(19) Europäisches Patentamt
European Patent Office
Office européen des brevets

(11) EP 2 426 135 A1

(12) **EUROPEAN PATENT APPLICATION**
published in accordance with Art. 153(4) EPC

(43) Date of publication:
**07.03.2012 Bulletin 2012/10**

(21) Application number: **10769499.4**

(22) Date of filing: **26.04.2010**

(51) Int Cl.:
***C07D 513/04*** (2006.01) ***A61K 31/415*** (2006.01)
***A61K 31/421*** (2006.01) ***A61K 31/437*** (2006.01)
***A61K 31/444*** (2006.01) ***A61K 31/4725*** (2006.01)
***A61K 31/5025*** (2006.01) ***A61K 31/506*** (2006.01)
***A61K 31/5377*** (2006.01) ***A61P 1/02*** (2006.01)
***A61P 1/04*** (2006.01) ***A61P 1/16*** (2006.01)
***A61P 1/18*** (2006.01) ***A61P 3/04*** (2006.01)
***A61P 3/10*** (2006.01) ***A61P 5/14*** (2006.01)
***A61P 7/06*** (2006.01) ***A61P 9/00*** (2006.01)
***A61P 9/04*** (2006.01) ***A61P 9/10*** (2006.01)

(86) International application number:
**PCT/JP2010/002998**

(87) International publication number:
**WO 2010/125799 (04.11.2010 Gazette 2010/44)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **27.04.2009 JP 2009108526**
**09.10.2009 JP 2009235769**
**26.02.2010 JP 2010043072**

(71) Applicant: **Shionogi & Co., Ltd.**
**Osaka-shi, Osaka 5410045 (JP)**

(72) Inventors:
- **TANIYAMA, Daisuke**
**Osaka-shi**
**Osaka 553-0002 (JP)**
- **MITSUOKA, Yasunori**
**Osaka-shi**
**Osaka 553-0002 (JP)**
- **HATA, Kayoko**
**Osaka-shi**
**Osaka 553-0002 (JP)**

(74) Representative: **UEXKÜLL & STOLBERG**
**Patentanwälte**
**Beselerstraße 4**
**22607 Hamburg (DE)**

(54) **UREA DERIVATIVE HAVING PI3K INHIBITORY ACTIVITY**

(57) Provided is a compound or a pharmaceutically acceptable salt thereof which inhibits the activity of PI3K to regulate many biological processes including the growth, differentiation, survival, proliferation, migration, metabolism, and the like of cells and is therefore useful for the prophylaxis/therapy of diseases including inflammatory diseases, arteriosclerosis, vascular/circulatory diseases, cancer/tumors, immune system diseases, cell proliferative diseases, infectious diseases, and the like. The above problem was solved by providing a urea derivative shown in the present specification, or a pharmaceutically acceptable salt thereof.

EP 2 426 135 A1

## Description

TECHNICAL FIELD

The present invention is related to: a compound that has phosphatidylinositol-3-kinase (hereinafter also referred to as "PI3K") inhibitory activity and is useful in the therapy/prophylaxis of a variety of phosphatidylinositol-3-kinase dependent diseases including cancers, inflammatory diseases, circulatory diseases, and the like; a salt thereof; or the like.

BACKGROUND ART

Phosphatidylinositol-3-kinase is an enzyme that catalyzes not only the production of a specific phospholipase, but also an intracellular mediator from phosphatidylinositol (hereinafter also referred to as "PI") of a membrane lipid. The 3'-OH group of phosphatidylinositol is phosphorylated, and thus, when phosphatidylinositol, phosphatidylinositol 4-phosphate, and phosphatidylinositol 4,5-bisphosphate are used as substrates, phosphatidylinositol 3-phosphate, phosphatidylinositol 3,4-bisphosphate, and phosphatidylinositol 3,4,5-triphosphate (PIP3) are produced respectively.

A phospholipid (PIP3) in which the hydroxyl group at the 3-position of the inositol ring is phosphorylated by this PI3K function as a second messenger that activates a serine/threonine kinase such as PDK1, Akt/PKB, and the like in a signal transduction route mediated by receptor stimulation. This second messenger is said to regulate many biological processes including growth, differentiation, survival, proliferation, migration and metabolism, and the like of cells.

PI3Ks are classified into three groups (*i.e.* Classes I to III) by primary structure, regulatory mechanism of activity, and specificity to a substrate. Among these, Class I is important in signaling.

Depending on the differences in the heterodimer, Class I is classified into IA (α, β, and δ), containing a subunit of 85 kDa, and IB (γ), containing a subunit of 101 kDa.

Class IA is associated with a variety of cell surface receptors such as hormones/growth factors and the like. For a signal transduction route, it is said to be a protein/kinase receptor type. Class IB is associated with a G protein receptor (GPCR), which is a receptor for chemokine and the like. Furthermore, it is said that when a specific tyrosine residue of a receptor is phosphorylated by stimulation of an activator such as a chemokine and the like, a regulatory subunit is bound to a catalytic subunit via the SH2 domain, and thereby the inhibitory activity of the regulatory subunit is reduced to exhibit enzyme activity.

PIP3 functions as a messenger in intracellular signaling. In the immediate downstream of PIP3, AKT (also known as protein kinase B (PKB)) and the like are known. It is said that in the downstream route thereof, a functional protein having the PH domain is activated and thereby a signal is transmitted.

PI3Kα and PI3Kβ are widely distributed in a variety of cells and are related to cell growth/glycometabolism. Based on these actions, inhibitors of PI3Kα and PI3Kβ are utilized as anticancer agents and the like. PI3Kδ and PI3Kγ exist mainly in blood and cells of the immune (lymphatic) system. PI3Kγ is also known to be widely distributed in inflammatory cells.

Regarding PI3Kγ, based on studies of knock-out mice and the like, it was found that a respiratory burst of neutrophils by a chemotactic factor and the migration of macrophage/neutrophil to an infection focus was blocked, functions of T cells/dendritic cells were thereby decreased, the degranulation of mast cells was thereby blocked, and anaphylaxis was thereby decreased. Accordingly, an inhibitor of PI3Kγ is considered useful as a therapeutic agent for these diseases. Furthermore, based on studies of arthritis, it is considered useful as an inhibitor of the inflammatory-cell infiltration in a part of a joint (Non-patent Documents 1 and 2). Furthermore, studies using a PI3Kγ inhibitor report the inhibition of mast cell activation (Non-patent Document 3), the inhibition of leukocyte activation/migration (Non-patent Documents 4 and 5), the inhibition of lymphocyte activation (Non-patent Document 6), and the like.

Based on these studies, a PI3Kγ inhibitor is believed to be useful in the therapy of the following diseases/disorders: thrombus; allergy/anaphylaxis (allergic diseases include, for example, asthma, atopic dermatitis, allergic rhinitis, and the like); inflammation such as pancreatitis (Non-patent Document 7), pneumonia, airway inflammation, chronic obstructive pulmonary disease (COPD) (Non-patent Documents 8 and 9), arthritis (e.g., articular rheumatism (Non-patent Documents 8 and 9), glomerulonephritis, and the like; systemic lupus erythematosus (SLE) (Non-patent Documents 8 and 9); autoimmune diseases; pulmonary disorder; circulatory diseases such as heart failure (systolic), cardiac ischemia (systolic), high blood pressure, and the like (Non-patent Document 10); wound healing; infectious diseases (Non-patent Document 11); cancer/tumors such as neoplasm (Patent Document 1); suppression of immune reaction in organ transplantation and autoimmune diseases (Patent Document 2); and the like.

Regarding PI3Kδ, based on studies of knock-out mice and the like, the B cell differentiation disorder of bone marrow is induced and in immunomodulation, the role thereof is expected.

PI3K is reported to be deeply involved in various stages of articular rheumatism, such as: T cell/B cell activation by presenting an antigen; inflammatory cell infiltration by neutrophil, macrophage, or the like; synovial cell proliferation;

mast cell activation; and the like (Non-Patent Document 12).

As examples of compounds that have PI3 kinase inhibitory activity, classically, wortmannin (Non-Patent Document 13), 2-(4-morpholinyl)-8-phenyl-4H-1-benzopyran-4-one (Patent Document 2), 17β-hydroxywortmannin and a derivative thereof (Patent Document 1), and the like are known.

Compounds that are disclosed in Patent Documents 3 to 16, Non-patent Documents 14 to 17, and the like are known to be urea derivatives useful as medicaments. Patent Document 17 discloses a urea derivative useful as an anti-inflammatory agent, but does not disclose PI3K inhibitory activity. Further, Patent Documents 18 to 23 disclose urea derivatives as imaging forming material, but do not disclose PI3K inhibitory activity. In addition, Patent Documents 24 to 27 disclose urea derivatives having PI3K inhibitory activity, but do not disclose compounds of the present invention.

PRIOR ART REFERENCES

[Patent Document]

[Patent Document 1] Japanese Laid-Open Publication No. 7-145051
[Patent Document 2] International Publication No. 95/29673 pamphlet
[Patent Document 3] International Publication No. 2001/098269 pamphlet
[Patent Document 4] International Publication No. 2001/098270 pamphlet
[Patent Document 5] International Publication No. 2000/035451 pamphlet
[Patent Document 6] International Publication No. 2000/035452 pamphlet
[Patent Document 7] International Publication No. 2000/035453 pamphlet
[Patent Document 8] International Publication No. 2000/035454 pamphlet
[Patent Document 9] International Publication No. 2000/035449 pamphlet
[Patent Document 10] International Publication No. 2002/100433 pamphlet
[Patent Document 11] International Publication No. 2002/051442 pamphlet
[Patent Document 12] International Publication No. 2001/010865 pamphlet
[Patent Document 13] International Publication No. 2003/072557 pamphlet
[Patent Document 14] International Publication No. 2005/113511 pamphlet
[Patent Document 15] International Publication No. 2007/115930 pamphlet
[Patent Document 16] International Publication No. 2007/115932 pamphlet
[Patent Document 17] International Publication No. 2008/039520 pamphlet
[Patent Document 18] Japanese Laid-Open Publication No. 2001-281820
[Patent Document 19] Japanese Laid-Open Publication No. 1994-003784
[Patent Document 20] Japanese Laid-Open Publication No. 1993-333503
[Patent Document 21] European Patent No. 578248 Specification
[Patent Document 22] European Patent No. 569979 Specification
[Patent Document 23] European Patent No. 572029 Specification
[Patent Document 24] International Publication No. 2007/115931 pamphlet
[Patent Document 25] United States Patent Application Publication No. 2007/0238730 Specification
[Patent Document 26] International Publication No. 2009/128520 pamphlet
[Patent Document 27] International Publication No. 2010/024258 pamphlet

[Non-patent document]

[Non-patent document 1] M. P. Wymann, et al., Biochemical Society Transactions 2003, 31, pp. 275-280
[Non-patent document 2] Rueckle T. et al., NATURE REVIEWS DRUG DISCOVERY 2006, 5 pp. 903-918
[Non-patent document 3] Laffargue M. et al., Immunity 2002 16: pp. 441-451
[Non-patent document 4] Hirsch E. et al., Science 2000 287: pp. 1049-1053
[Non-patent document 5] Li Z. et al., Science 2000 287; pp. 982-983
[Non-patent document 6] Sasaki T. et al., Science 2000 287; pp. 1040-1046
[Non-patent document 7] Lupia E. et al., Am J Pathol. 2004; 165, pp. 2003-2011
[Non-patent document 8] Barber DF et al., Nat Med 2005 11: pp. 933-935
[Non-patent document 9] Camps, Nat Med 2005 11: pp. 936-943
[Non-patent document 10] Campbell et al., Circ Res. 2005, 96, pp. 197-206

[Non-patent document 11] Yadav M. et al., J Immunol. 2006, 176, pp. 5494-503
[Non-patent document 12] Japanese Journal of Clinical Immunology, Vol. 30 2007, 5 pp.369-374
[Non-patent document 13] Ui MT et al., Trends Biochem. Sci., 1995, 20, pp. 303-307
[Non-patent document 14] Shao L. et al., Heteroatom Chemistry (2008) 19(1) pp. 2-6
[Non-patent document 15] Vovk, M. V. et al., Zhurnal Organichnoi ta Farmatsevtichnoi Khimii (2006), 4(4), pp. 63-66
[Non-patent document 16] Salvatore G. et al., Scientia Pharmaceutica (2000), 68(1), pp. 65-73
[Non-patent document 17] Romeo G. et al., Pharmazie (1999), 54(1), pp. 19-23

SUMMARY OF THE INVENTION

PROBLEMS TO BE SOLVED BY THE INVENTION

It is an object of the present invention to provide urea derivatives or pharmaceutically acceptable salts thereof which inhibit the activity of PI3K to regulate many biological processes including the growth, differentiation, survival, proliferation, migration, metabolism, and the like of cells, and are therefore useful for the prophylaxis/therapy of diseases including inflammatory diseases (allergic diseases (allergic dermatitis/allergic rhinitis, and the like), rheumatoid arthritis/ articular rheumatism, anaphylaxis, and the like), arteriosclerosis, vascular/circulatory diseases, cancer/tumors, immune system diseases, cell-proliferative diseases, infectious diseases, and the like.

MEANS FOR SOLVING PROBLEM

Accordingly, for example, the present invention provides the following items:
(1) A compound represented by formula (I):
[Formula 1]

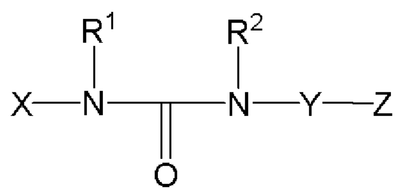

(I)

wherein
$R^1$ and $R^2$ are each independently hydrogen or substituted or unsubstituted alkyl;
X is a group represented by a formula selected from the following:
[Formula 2]

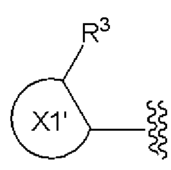

(X1)

or

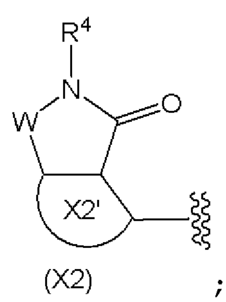

(X2) ;

X1' is a substituted or unsubstituted 6-membered aromatic hydrocarbon ring, a substituted or unsubstituted 5- or 6-membered aromatic heterocycle, a substituted or unsubstituted 5 to 7-membered non-aromatic hydrocarbon ring, or a substituted or unsubstituted 5 to 7-membered non-aromatic heterocycle; X2' is a substituted or unsubstituted monocyclic aromatic hydrocarbon ring, a substituted or unsubstituted monocyclic aromatic heterocycle, a substituted or unsubstituted monocyclic non-aromatic hydrocarbon ring, or a substituted or unsubstituted monocyclic non-aromatic

heterocycle;

$R^3$ is carboxy, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;

$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;

$R^4$ is hydrogen, cyano, substituted or unsubstituted alkyl, substituted or unsubstituted aryl, substituted or unsubstituted cycloalkyl, substituted or unsubstituted cycloalkenyl, substituted or unsubstituted heteroaryl, or substituted or unsubstituted heterocyclyl, and W is a group represented by the formula: $-(CR^5R^6)_a-$, or a group represented by the formula: $-(CR^7=CR^8)-$;

$R^5$ to $R^8$ are each independently hydrogen, halogen, cyano, hydroxy, carboxy, substituted or unsubstituted alkyl, substituted or unsubstituted alkoxy, substituted or unsubstituted acyl, substituted or unsubstituted carbamoyl, or substituted or unsubstituted amino;

a is 1 or 2;

Y is a group represented by a formula selected from the following:

[Formula 3]

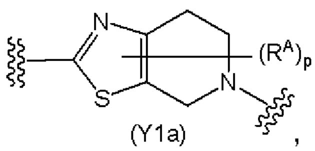
(Y1a) ,

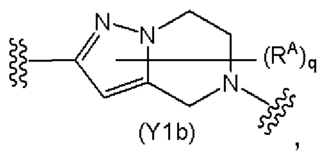
(Y1b) ,

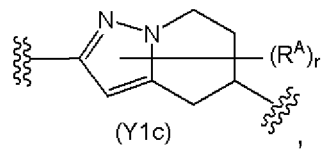
(Y1c) ,

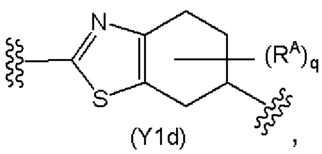
(Y1d) ,

[Formula 4]

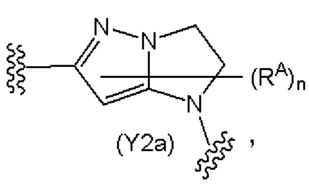
(Y2a) ,

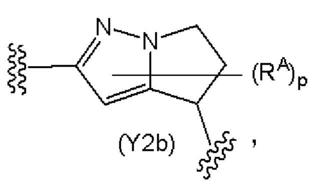
(Y2b) ,

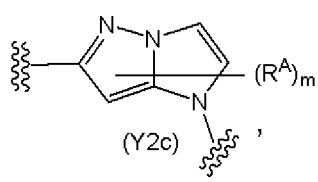
(Y2c) ,

[Formula 5]

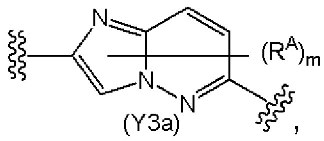
(Y3a),

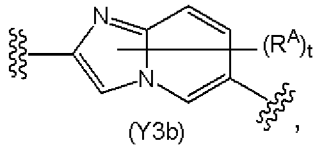
(Y3b),

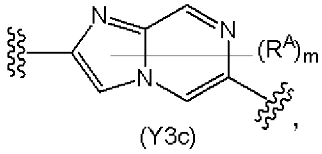
(Y3c),

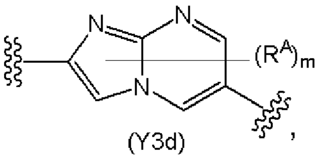
(Y3d),

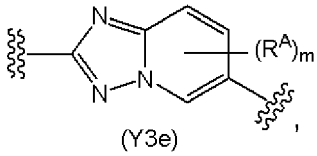
(Y3e),

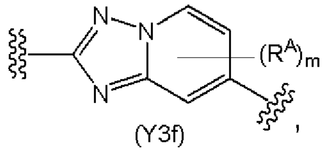
(Y3f),

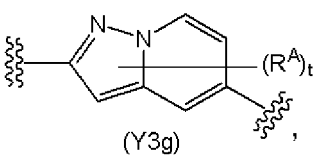
(Y3g),

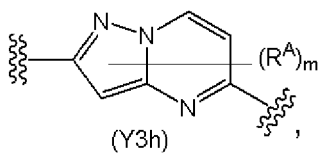
(Y3h),

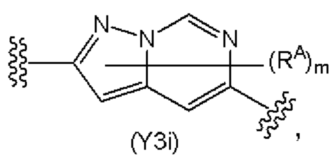
(Y3i),

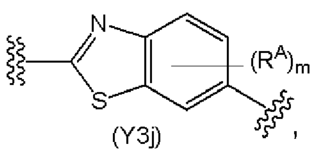
(Y3j),

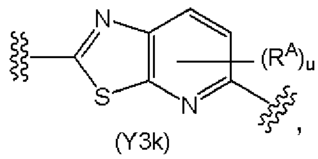
(Y3k),

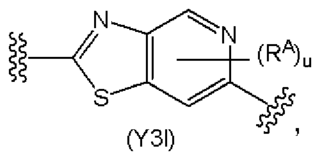
(Y3l),

[Formula 6]

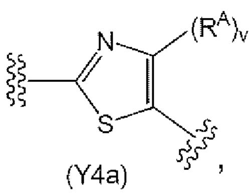
(Y4a),

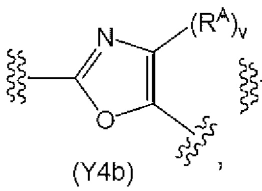
(Y4b),

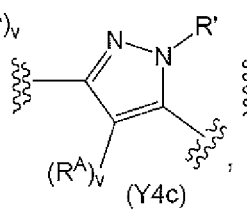
(Y4c),

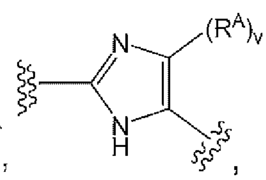
(Y4d),

[Formula 7]

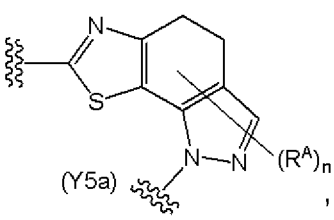
(Y5a),

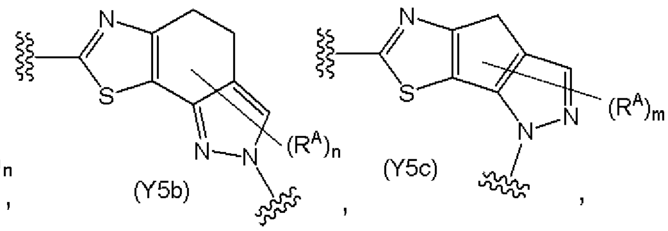
(Y5b), (Y5c),

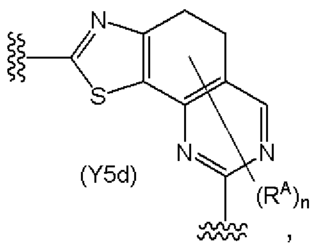
(Y5d),

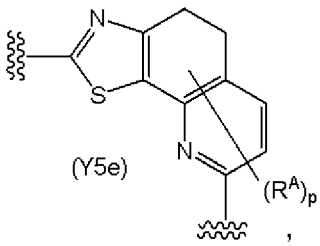
(Y5e),

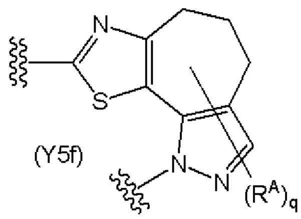
(Y5f),

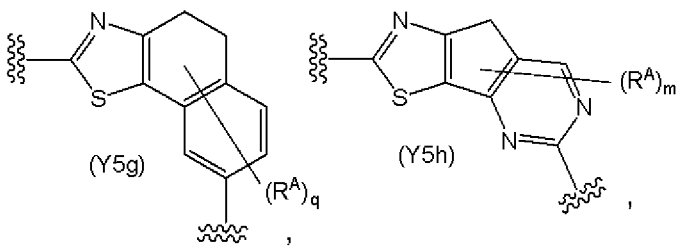
(Y5g), (Y5h),

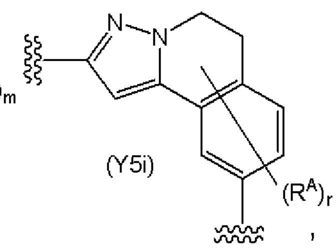
(Y5i),

[Formula 8]

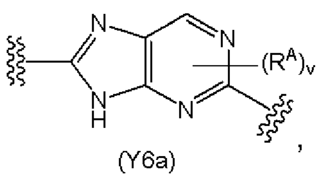
(Y6a)

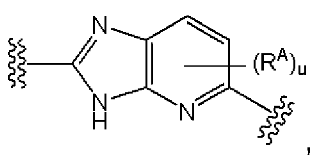
(Y6b)

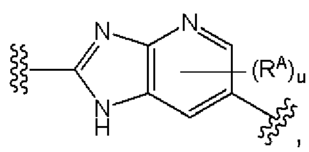
(Y6c)

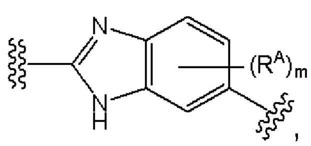
(Y6d)

[Formula 9]

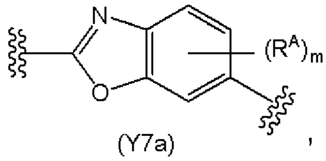
(Y7a) ,
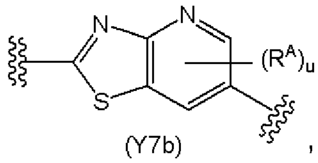
(Y7b) ,
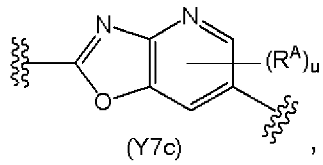
(Y7c) ,

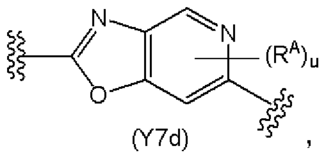
(Y7d) ,
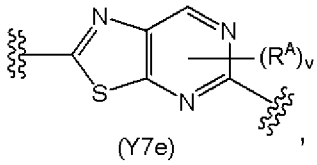
(Y7e) ,

[Formula 10]

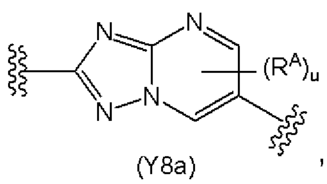
(Y8a) ,
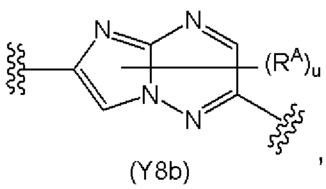
(Y8b) ,
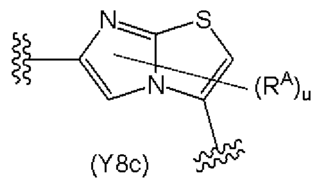
(Y8c)

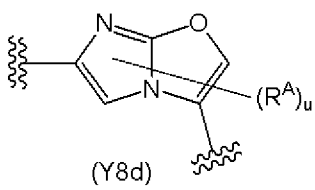
(Y8d) ,

[Formula 11]

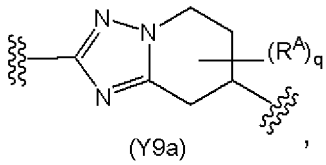
(Y9a) ,

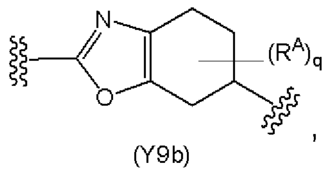
(Y9b) ,

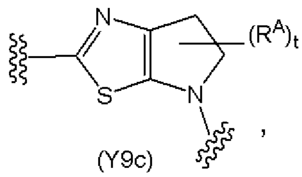
(Y9c) ,

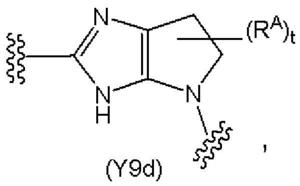
(Y9d) ,

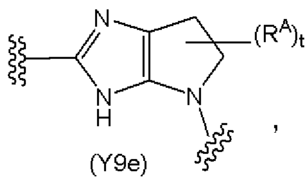
(Y9e) ,

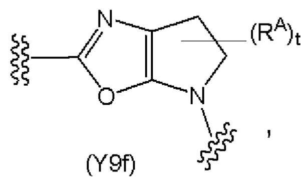
(Y9f) ,

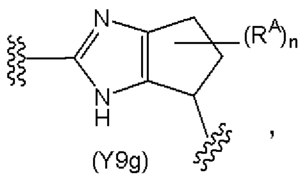
(Y9g) ,

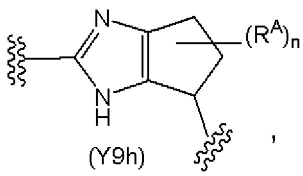
(Y9h) ,

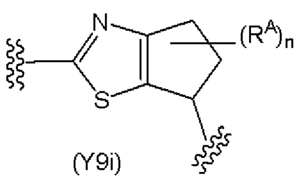
(Y9i)

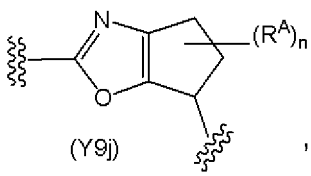
(Y9j) ,

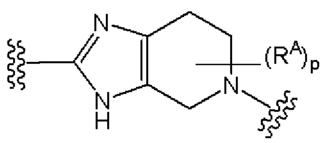
(Y9k) or

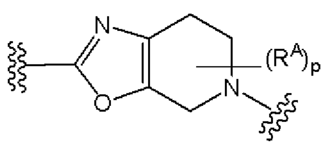
(Y9l) ;

$R^A$ is each independently halogen, cyano, carboxy, hydroxy, nitro, substituted or unsubstituted alkoxy, substituted or unsubstituted alkyl, substituted or unsubstituted alkenyl, substituted or unsubstituted alkynyl, substituted or unsubstituted amino, substituted or unsubstituted carbamoyl, substituted or unsubstituted acyl, substituted or unsubstituted aryl, substituted or unsubstituted cycloalkyl, substituted or unsubstituted cycloalkenyl, substituted or unsubstituted heteroaryl, substituted or unsubstituted heterocyclyl, a group represented by the formula: $-SO-R^{a'}$, a group represented by the formula: $-SO_2-R^{a'}$, or a group represented by the formula: $-SR^{a'}$;

$R^{a'}$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;

R' is hydrogen or substituted or unsubstituted alkyl;

m is an integer from 0 to 3;

n is an integer from 0 to 5;

p is an integer from 0 to 6;

q is an integer from 0 to 7;

r is an integer from 0 to 8;

t is an integer from 0 to 4;

u is an integer from 0 to 2;

v is 0 or 1; and

Z is substituted or unsubstituted cycloalkyl, substituted or unsubstituted cycloalkenyl, substituted or unsubstituted aryl, substituted or unsubstituted heteroaryl, or substituted or unsubstituted heterocyclyl,

with the proviso that:

when Y is a group represented by formula (Y4a) or a group represented by formula (Y4b), $R^A$ is not substituted or unsubstituted aryl;

when Y is a group represented by formula (Y4a), $R^3$ is not methyl or methoxy;

when Y is a group represented by formula (Y5a), $R^3$ is not carboxy, ethoxycarbonyl, phenyl, or alkyl substituted

with amino; and
when Y is a group represented by formula (Y5d), $R^3$ is not phenyl or alkyl substituted with amino, and wherein the following compound is excluded:

[Formula 12]

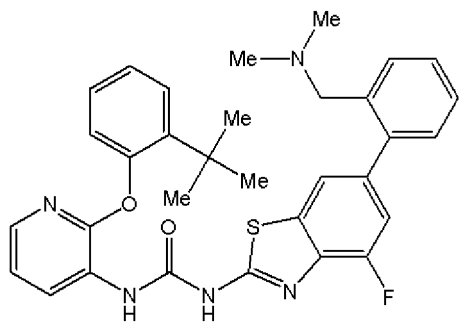
,

or a pharmaceutically acceptable salt thereof, or a solvate thereof.
(1A) A compound represented by formula (I):
[Formula 1A]

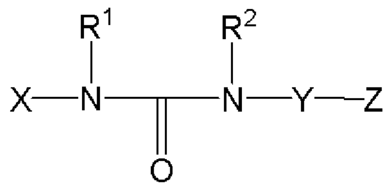
(I)

wherein
$R^1$ and $R^2$ are each independently hydrogen or substituted or unsubstituted alkyl;
X is a group represented by a formula selected from the following:
[Formula 2A]

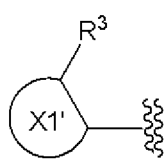
(X1)

or

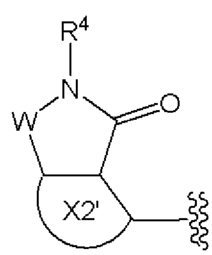
(X2)

;

X1' is a substituted or unsubstituted 6-membered aromatic hydrocarbon ring, a substituted or unsubstituted 5- or 6-membered aromatic heterocycle, a substituted or unsubstituted 3 to 7-membered non-aromatic hydrocarbon ring, or a substituted or unsubstituted 3 to 7-membered non-aromatic heterocycle; X2' is a substituted or unsubstituted monocyclic aromatic hydrocarbon ring, a substituted or unsubstituted monocyclic aromatic heterocycle, a substituted

or unsubstituted monocyclic non-aromatic hydrocarbon ring, or a substituted or unsubstituted monocyclic non-aromatic heterocycle;

$R^3$ is carboxy, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;

$R^a$ is hydrogen, substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;

$R^4$ is hydrogen, cyano, substituted or unsubstituted alkyl, substituted or unsubstituted aryl, substituted or unsubstituted cycloalkyl, substituted or unsubstituted cycloalkenyl, substituted or unsubstituted heteroaryl, or substituted or unsubstituted heterocyclyl, and W is a group represented by the formula: $-(CR^5R^6)_a-$ or a group represented by the formula: $-(CR^7=CR^8)-$;

$R^3$ to $R^8$ are each independently hydrogen, halogen, cyano, hydroxy, carboxy, substituted or unsubstituted alkyl, substituted or unsubstituted alkoxy, substituted or unsubstituted acyl, substituted or unsubstituted carbamoyl, or substituted or unsubstituted amino;

a is 1 or 2;

Y is a group represented by a formula selected from the following:

[Formula 3A]

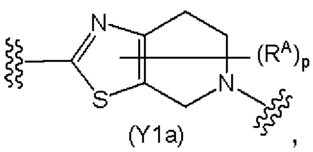
(Y1a) ,

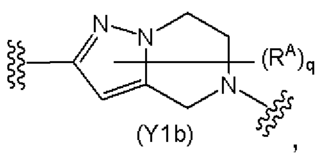
(Y1b) ,

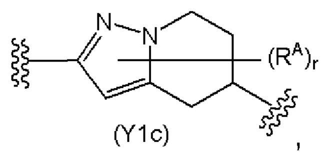
(Y1c) ,

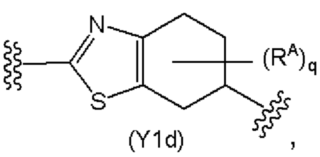
(Y1d) ,

[Formula 4A]

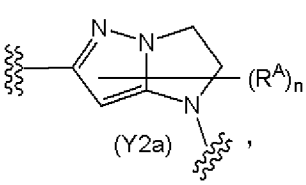
(Y2a) ,

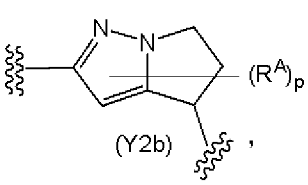
(Y2b) ,

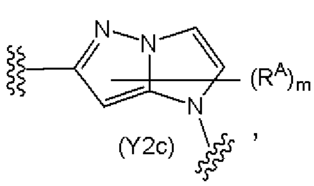
(Y2c) ,

[Formula 5A]

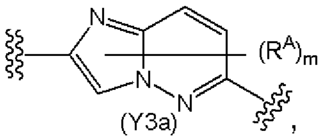
(Y3a),
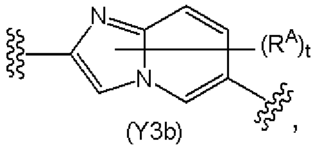
(Y3b),
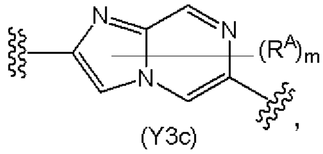
(Y3c),

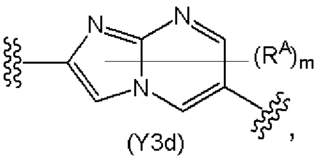
(Y3d),
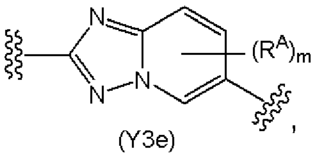
(Y3e),
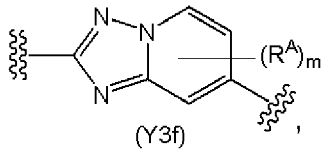
(Y3f),

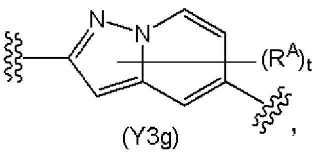
(Y3g),
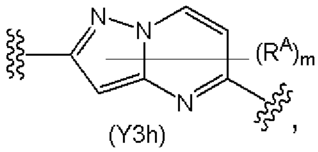
(Y3h),
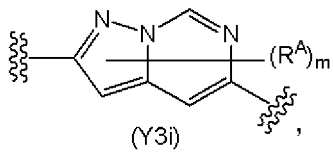
(Y3i),

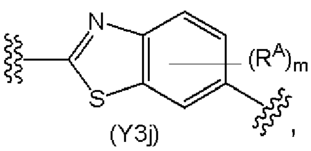
(Y3j),
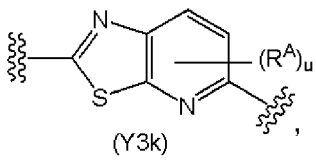
(Y3k),
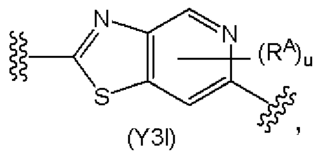
(Y3l),

[Formula 6A]

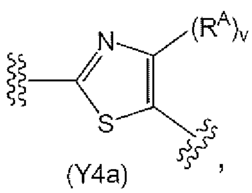
(Y4a),
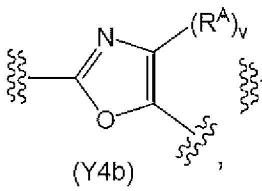
(Y4b),
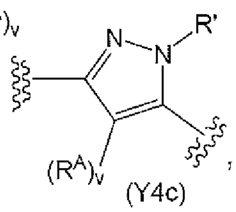
(Y4c),
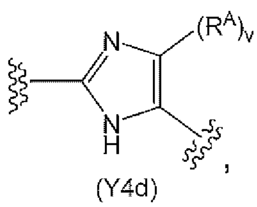
(Y4d),

[Formula 7A]

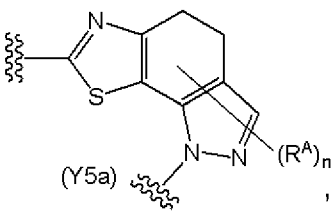
(Y5a) ,

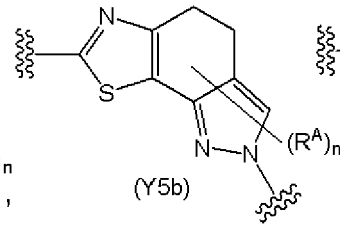
(Y5b) ,

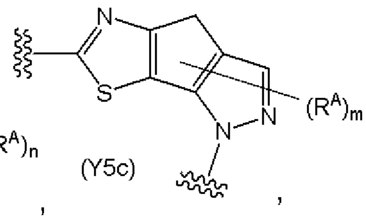
(Y5c) ,

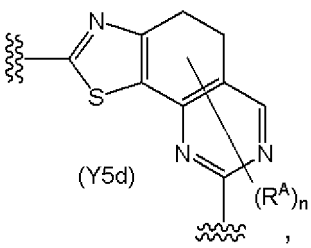
(Y5d) ,

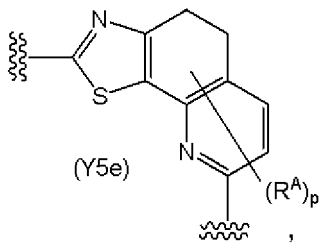
(Y5e) ,

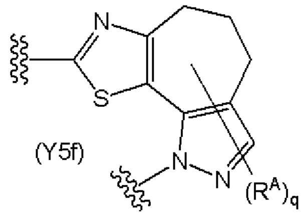
(Y5f) ,

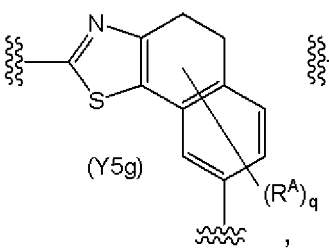
(Y5g) ,

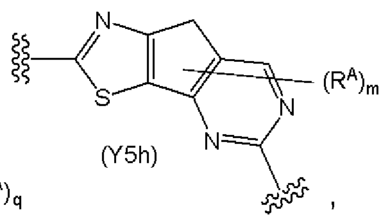
(Y5h) ,

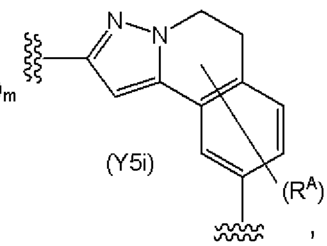
(Y5i) ,

[Formula 8A]

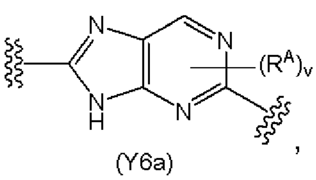
,
(Y6a)

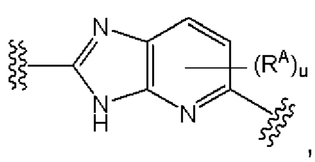
,
(Y6b)

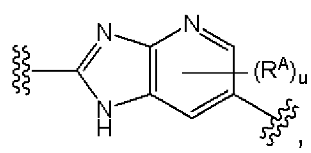
,
(Y6c)

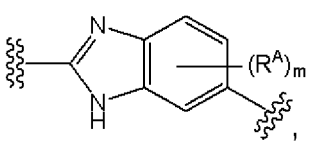
,
(Y6d)

[Formula 9A]

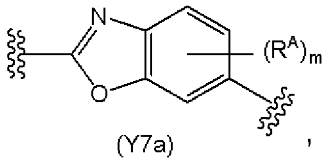
(Y7a) ,

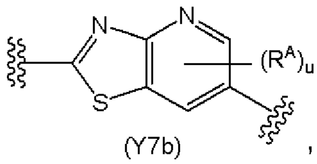
(Y7b) ,

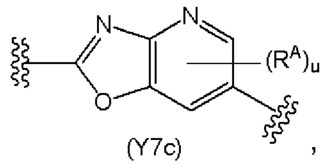
(Y7c) ,

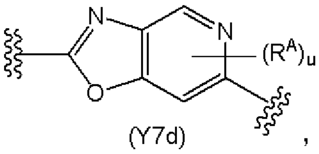
(Y7d) ,

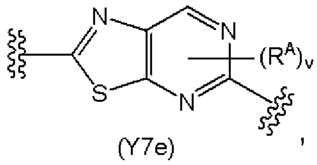
(Y7e) ,

[Formula 10A]

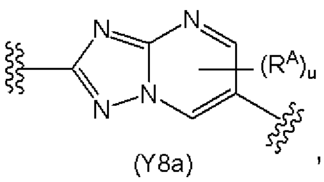
(Y8a) ,

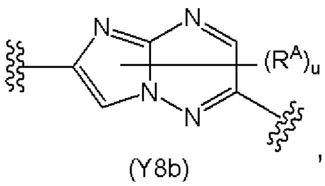
(Y8b) ,

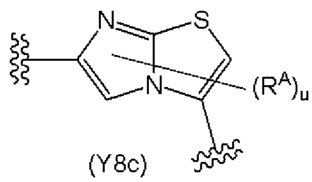
(Y8c)

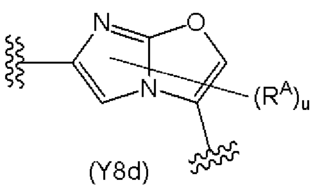
(Y8d) ,

[Formula 11A]

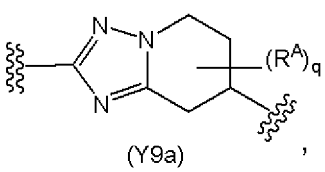

(Y9a) ,

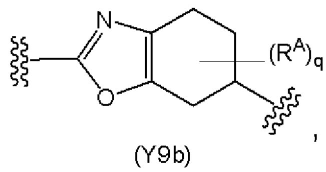

(Y9b) ,

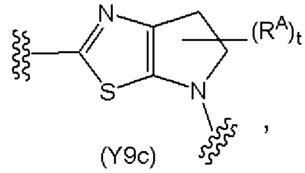

(Y9c) ,

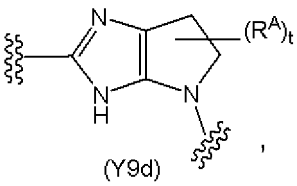

(Y9d) ,

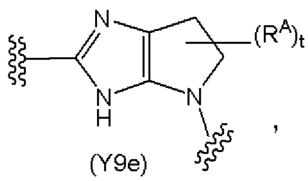

(Y9e) ,

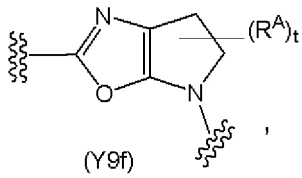

(Y9f) ,

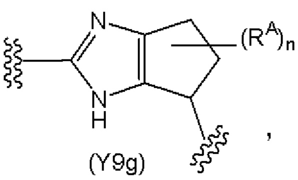

(Y9g) ,

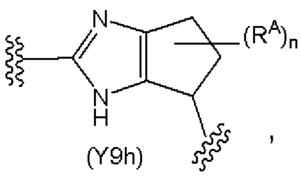

(Y9h) ,

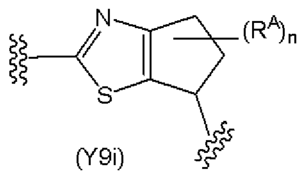

(Y9i)

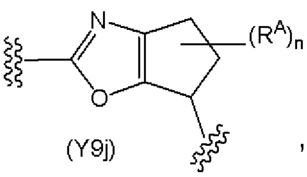

(Y9j) ,

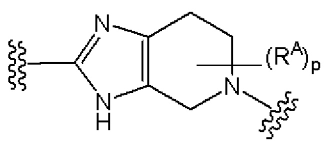

(Y9k) or

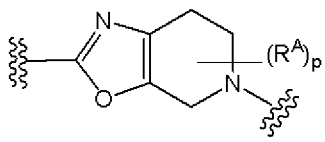

(Y9l) ;

$R^A$ is each independently halogen, cyano, carboxy, hydroxy, nitro, substituted or unsubstituted alkoxy, substituted or unsubstituted alkyl, substituted or unsubstituted alkenyl, substituted or unsubstituted alkynyl, substituted or unsubstituted amino, substituted or unsubstituted carbamoyl, substituted or unsubstituted acyl, substituted or unsubstituted aryl, substituted or unsubstituted cycloalkyl, substituted or unsubstituted cycloalkenyl, substituted or unsubstituted heteroaryl, substituted or unsubstituted heterocyclyl, a group represented by the formula:-SO-$R^{a'}$, a group represented by the formula: -$SO_2$-$R^{a'}$, or a group represented by the formula: -$SR^{a'}$;

$R^{a'}$ is hydrogen, substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;

R' is hydrogen or substituted or unsubstituted alkyl;

m is an integer from 0 to 3;

n is an integer from 0 to 5;

p is an integer from 0 to 6;

q is an integer from 0 to 7;

r is an integer from 0 to 8;

t is an integer from 0 to 4;

u is an integer from 0 to 2;

v is 0 or 1; and

Z is substituted or unsubstituted cycloalkyl, substituted or unsubstituted cycloalkenyl, substituted or unsubstituted aryl, substituted or unsubstituted heteroaryl, or substituted or unsubstituted heterocyclyl,

with the proviso that:

when Y is a group represented by formula (Y4a) or a group represented by formula (Y4b), $R^A$ is not substituted or unsubstituted aryl;

when Y is a group represented by formula (Y4a), $R^3$ is not methyl or methoxy;

when Y is a group represented by formula (Y5a), $R^3$ is not carboxy, ethoxycarbonyl, phenyl, or alkyl substituted with amino;

when Y is a group represented by formula (Y5c), $R^3$ is not phenyl, or alkyl substituted with substituted amino or imidazolidinone; and

when Y is a group represented by formula (Y5d), $R^3$ is not phenyl, or alkyl substituted with substituted amino, and wherein the following compound is excluded:

[Formula 12A]

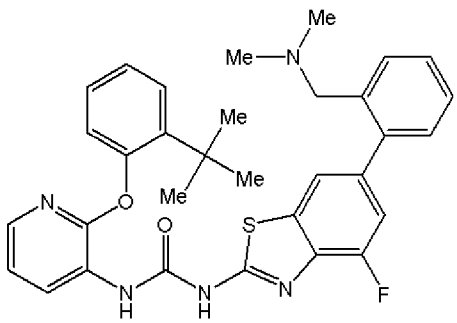

,

or a pharmaceutically acceptable salt thereof, or a solvate thereof.

(2) The compound according to item (1) or (1A), wherein X1' is a substituted or unsubstituted 6-membered aromatic hydrocarbon ring, a substituted or unsubstituted 5- or 6-membered aromatic heterocycle, a substituted or unsubstituted 5- or 6-membered non-aromatic hydrocarbon ring, or a substituted or unsubstituted 5- or 6-membered non-aromatic heterocycle, or a pharmaceutically acceptable salt thereof, or a solvate thereof.

(3) The compound according to item (1), (1A), or (2), wherein Y is a group represented by formula (Y1a), a group represented by the formula (Y1b), a group represented by formula (Y1c), or a group represented by formula (Y1d), or a pharmaceutically acceptable salt thereof, or a solvate thereof.

(4) The compound according to any of item (1), (1A), (2), and (3), wherein X is a group represented by formula (X1),

or a pharmaceutically acceptable salt thereof, or a solvate thereof.

(5) The compound according to any of item (1), (1A), (2) to (4), wherein $R^3$ is carboxy, substituted or unsubstituted heterocyclyl, substituted or unsubstituted acyl, substituted or unsubstituted alkoxycarbonyl, or substituted or unsubstituted carbamoyl,

or a pharmaceutically acceptable salt thereof, or a solvate thereof.

(5A) The compound according to any of item (1), (1A), (2) to (5), wherein $R^3$ is carboxy, substituted or unsubstituted heterocyclyl, substituted or unsubstituted acyl, substituted or unsubstituted alkyl, substituted or unsubstituted aryl, substituted or unsubstituted alkoxy, substituted or unsubstituted amino, substituted or unsubstituted alkoxycarbonyl, or substituted or unsubstituted carbamoyl,

or a pharmaceutically acceptable salt thereof, or a solvate thereof.

(6) The compound according to any of items (1), (1A), (2) to (5), and (5A), wherein X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring,

or a pharmaceutically acceptable salt thereof, or a solvate thereof.

(7) The compound according to any of items (1), (1A), (2) to (5), (5A), and (6), wherein Z is substituted or unsubstituted aryl, or substituted or unsubstituted heteroaryl,

or a pharmaceutically acceptable salt thereof, or a solvate thereof.

(8) The compound according to any of items (1), (1A), (2) to (5), (5A), (6), and (7), wherein Y is a group represented by formula (Y1a) and p is 0,

or a pharmaceutically acceptable salt thereof, or a solvate thereof.

(9) The compound according to any of items (1), (1A), (2) to (5), (5A), and (6) to (8), wherein $R^1$ and $R^2$ are both hydrogen,

or a pharmaceutically acceptable salt thereof, or a solvate thereof.

(9A) The compound according to any of items (1), (1A), (2) to (5), (5A), and (6) to (9), wherein Y is a group

represented by formula (Y3a),
or a pharmaceutically acceptable salt thereof, or a solvate thereof.

(9B) The compound according to any of items (1), (1A), (2) to (5), (5A), (6) to (9), and (9A), wherein Y is a group represented by formula (Y3a) and m is 0,
or a pharmaceutically acceptable salt thereof, or a solvate thereof.

(9C) The compound according to any of items (1), (1A), (2) to (5), (5A), (6) to (9), (9A), and (9B), wherein Y is a group represented by formula (Y1d),
or a pharmaceutically acceptable salt thereof, or a solvate thereof.

(9D) The compound according to any of items (1), (1A), (2) to (5), (5A), (6) to (9), (9A), (9B), and (9C), wherein Y is a group represented by formula (Y1d) and q is 0,
or a pharmaceutically acceptable salt thereof, or a solvate thereof.

(9E) The compound according to any of items (1), (1A), (2) to (5), (5A), (6) to (9), (9A), (9B), (9C), and (9D), wherein Y is a group represented by formula (Y2a),
or a pharmaceutically acceptable salt thereof, or a solvate thereof.

(9F) The compound according to any of items (1), (1A), (2) to (5), (5A), (6) to (9), (9A), (9B), (9C), (9D), and (9E), wherein Y is a group represented by formula (Y2a) and n is 0,
or a pharmaceutically acceptable salt thereof, or a solvate thereof.

(9G) The compound according to any of items (1), (1A), (2) to (5), (5A), (6) to (9), (9A), (9B), (9C), (9D), (9E), and (9F), wherein $R^3$ is substituted or unsubstituted carbamoyl, substituted or unsubstituted amino, or substituted or unsubstituted alkoxy,
or a pharmaceutically acceptable salt thereof, or a solvate thereof.

(10) A pharmaceutical composition containing the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.

(11) The pharmaceutical composition according to item (10), which is a phosphatidylinositol-3-kinase inhibitor.

(12) A phosphatidylinositol-3-kinase inhibitor containing the compound according to any of items (1), (1A), (2) to (5), (5A), (6) to (9), (9A), (9B), (9C), (9D), (9E), (9F), and (9G), or a pharmaceutically acceptable salt thereof, or a solvate thereof, as an active ingredient.

(13) The inhibitor according to item (12), which is specific to one or more types of α, β, γ, and δ of phosphatidylinositol-3-kinase inhibitors.

(14) The pharmaceutical composition according to items (10) and (11) for the treatment of phosphatidylinositol-3-kinase dependent diseases listed below:

encephalitis, myelitis and encephalomyelitis, meningitis, inflammatory polyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation

rejection, bone marrow transplantation rejection, graft-versus-host diseases, and the like; or burn, traumatic inflammation, and the like.

(15) A phosphatidylinositol-3-kinase inhibitor containing the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(16) A protein kinase B (AKT) inhibitor containing the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(17) An anticancer agent containing the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(18) An anti-inflammatory or a therapeutic agent for inflammatory diseases (such as pancreatitis, pneumonia, airway inflammation, COPD (such as pulmonary emphysema, chronic bronchitis, and the like), arthritis, glomerulonephritis, and the like) wherein the anti-inflammatory or the therapeutic agent contains the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(19) An antiallergic agent (asthma, atopic dermatitis, allergic rhinitis, and the like) containing the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(20) A therapeutic agent for immune system diseases wherein the therapeutic agent contains the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(21) An immunosuppressant containing the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(22) A therapeutic agent for autoimmune diseases wherein the therapeutic agent contains the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(23) An anti-circulatory-disease agent (such as antihypertensive agent and the like) wherein the agent contains the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(24) An anti-infective agent containing the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(25) A wound-healing agent containing the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(26) A method, a system, an apparatus, a kit, and the like for producing the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(27) A method, a system, an apparatus, a kit, and the like for preparing a pharmaceutical composition containing the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(28) A method, a system, an apparatus, a kit, and the like using the compound according to any of the preceding items (1), (1A), (2) to (5), (5A), (6) to (9), (9A), (9B), (9C), (9D), (9E), (9F), and (9G), or a pharmaceutically acceptable salt thereof, or a solvate thereof.
(29) The pharmaceutical composition according to item (10), which is a medicament for the treatment or prophylaxis of diseases, disorders, or conditions related to phosphatidylinositol-3-kinase.
(30) The pharmaceutical composition according to any of items (10), (11), and (29), which is a therapeutic agent and/or a prophylactic agent for inflammation.
(31) A method for the prophylaxis or therapy of inflammation, characterized by administering the compound according to any of items (1), (1A), (2) to (5), (5A), (6) to (9), (9A), (9B), (9C), (9D), (9E), (9F), and (9G), or a pharmaceutically acceptable salt thereof, or a solvate thereof.
(32) Use of the compound according to any of items (1), (1A), (2) to (5), (5A), (6) to (9), (9A), (9B), (9C), (9D), (9E), (9F), and (9G), or a pharmaceutically acceptable salt thereof, or a solvate thereof, for producing a therapeutic agent and/or a prophylactic agent for inflammation.
(33) The compound according to any of items (1), (1A), (2) to (5), (5A), (6) to (9), (9A), (9B), (9C), (9D), (9E), (9F), and (9G), or a pharmaceutically acceptable salt thereof, or a solvate thereof, for the therapy and/or prophylaxis of inflammation.
The present invention also provides, for example, the following items:
(1α) A compound represented by formula (I):

[Formula 1B]

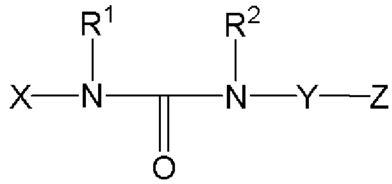

(I)

wherein
$R^1$ and $R^2$ are each independently hydrogen or substituted or unsubstituted alkyl;
X is a group represented by a formula selected from the following:
[Formula 2B]

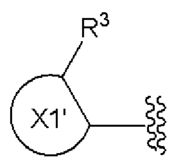

(X1)

or

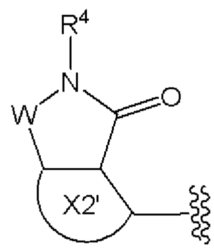

(X2) ;

X1' is a substituted or unsubstituted 6-membered aromatic hydrocarbon ring, a substituted or unsubstituted 5- or 6-membered aromatic heterocycle, a substituted or unsubstituted 3 to 7-membered non-aromatic hydrocarbon ring, or a substituted or unsubstituted 3 to 7-membered non-aromatic heterocycle; X2' is a substituted or unsubstituted monocyclic aromatic hydrocarbon ring, a substituted or unsubstituted monocyclic aromatic heterocycle, a substituted or unsubstituted monocyclic non-aromatic hydrocarbon ring, or a substituted or unsubstituted monocyclic non-aromatic heterocycle;
$R^3$ is carboxy, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-C(-R^a)=N-OH$, a group represented by the formula: $-SO-R^a$, a group represented by the formula: $-SO_2-R^a$, or a group represented by the formula: $-SR^a$;
$R^a$ is hydrogen, substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
$R^4$ is hydrogen, cyano, substituted or unsubstituted alkyl, substituted or unsubstituted aryl, substituted or unsubstituted cycloalkyl, substituted or unsubstituted cycloalkenyl, substituted or unsubstituted heteroaryl, or substituted or unsubstituted heterocyclyl, and W is a group represented by the formula: $-(CR^5R^6)_a-$ or a group represented by the formula: $-(CR^7=CR^8)-$;
$R^5$ to $R^8$ are each independently hydrogen, halogen, cyano, hydroxy, carboxy, substituted or unsubstituted alkyl, substituted or unsubstituted alkoxy, substituted or unsubstituted acyl, substituted or unsubstituted carbamoyl, or substituted or unsubstituted amino;
a is 1 or 2;
Y is a group represented by a formula selected from the following:
[Formula 3B]

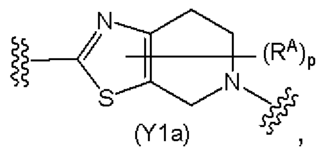
(Y1a),
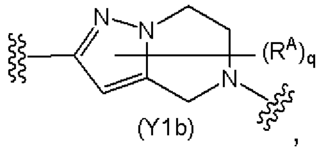
(Y1b),
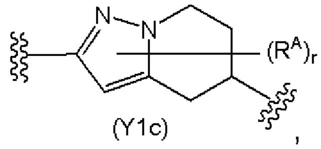
(Y1c),
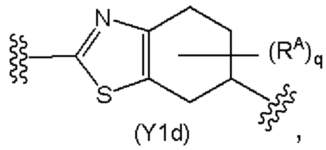
(Y1d),

[Formula 4B]

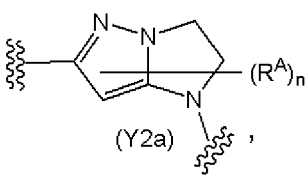
(Y2a),
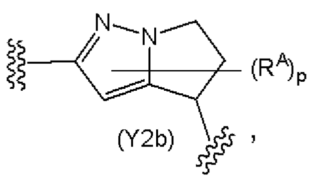
(Y2b),
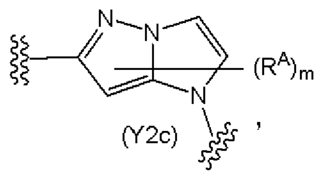
(Y2c),

[Formula 5B]

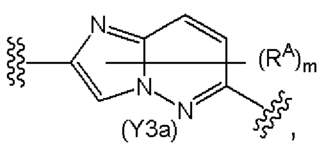
(Y3a),
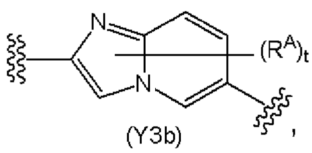
(Y3b),
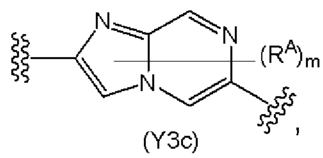
(Y3c),
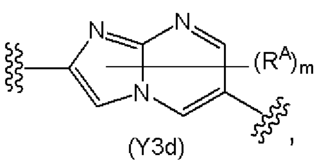
(Y3d),
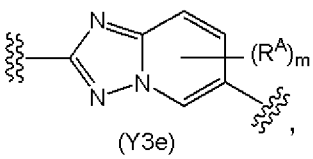
(Y3e),
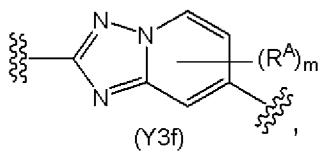
(Y3f),
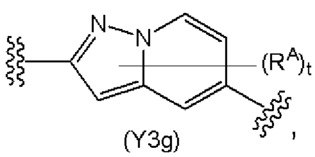
(Y3g),
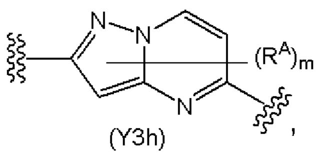
(Y3h),
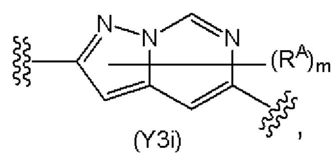
(Y3i),
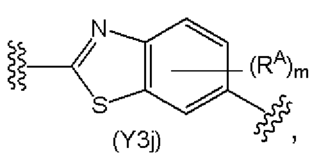
(Y3j),
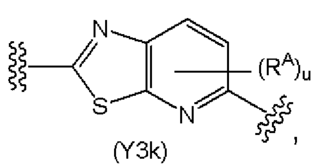
(Y3k),
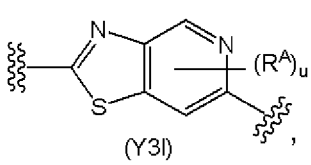
(Y3l),

[Formula 6B]

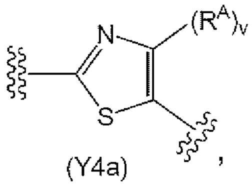
(Y4a),
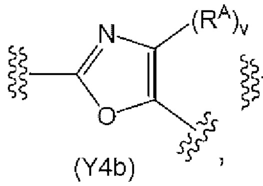
(Y4b),
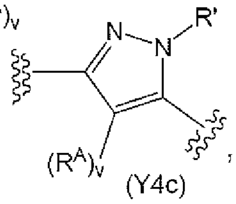
(Y4c),
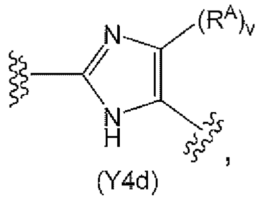
(Y4d),

[Formula 7B]

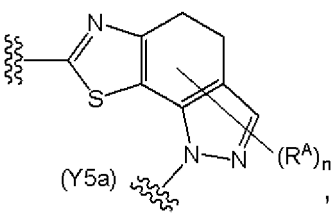
(Y5a),
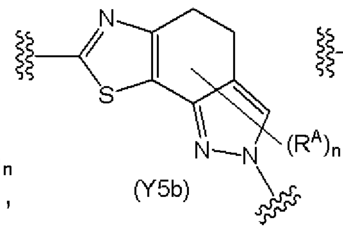
(Y5b),
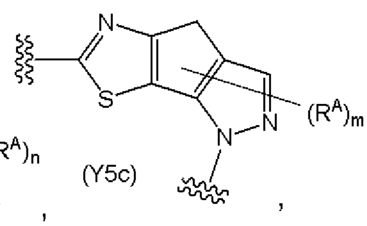
(Y5c),
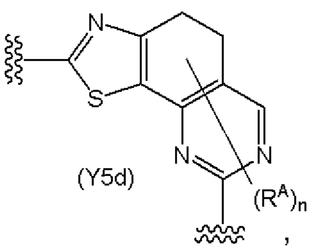
(Y5d),
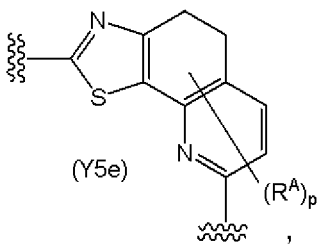
(Y5e),
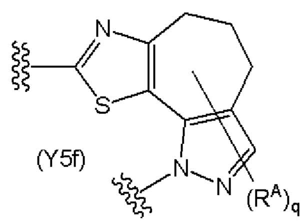
(Y5f),
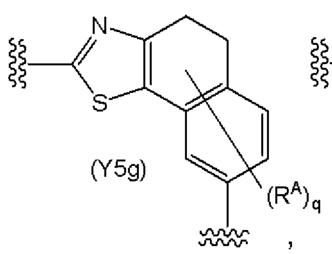
(Y5g),
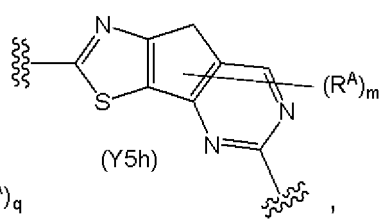
(Y5h),
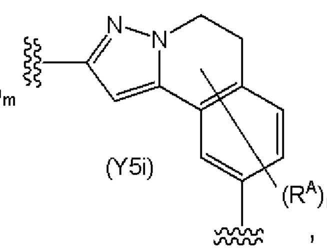
(Y5i),

[Formula 8B]

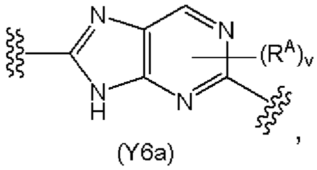
(Y6a) ,

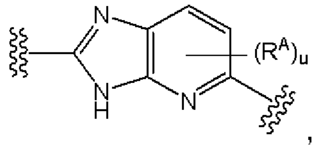
(Y6b) ,

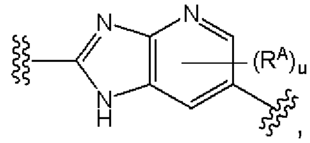
(Y6c) ,

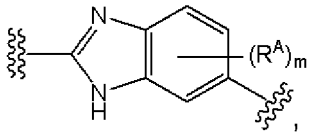
(Y6d) ,

[Formula 9B]

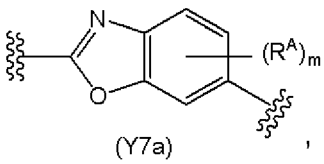
(Y7a) ,

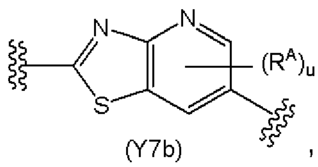
(Y7b) ,

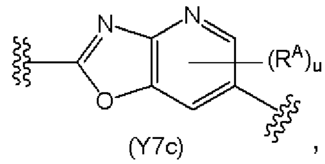
(Y7c) ,

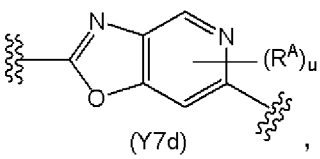
(Y7d) ,

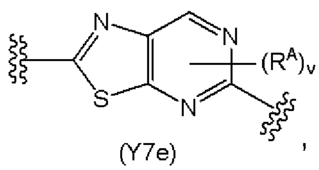
(Y7e) ,

[Formula 10B]

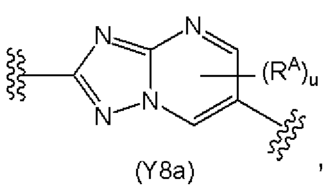
(Y8a) ,

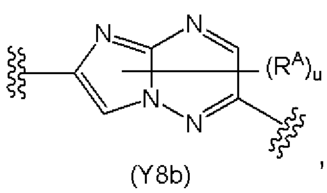
(Y8b) ,

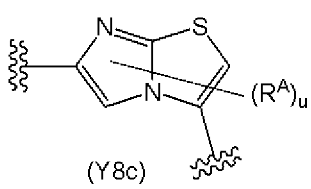
(Y8c)

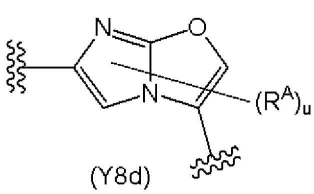
(Y8d) ,

[Formula 11B]

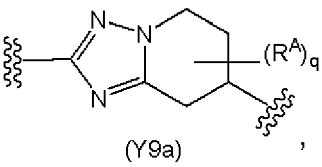
(Y9a) ,
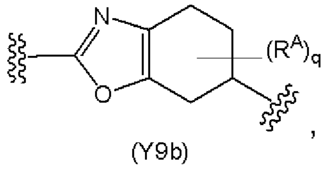
(Y9b) ,
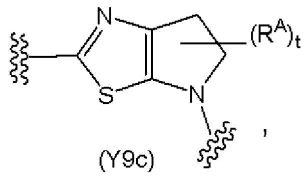
(Y9c) ,

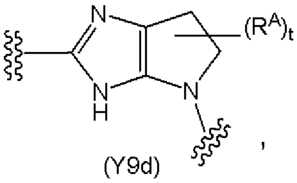
(Y9d) ,
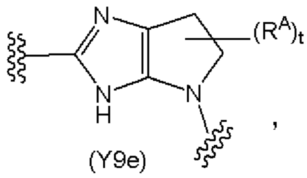
(Y9e) ,
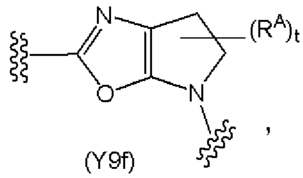
(Y9f) ,

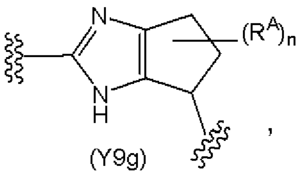
(Y9g) ,
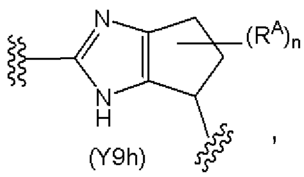
(Y9h) ,
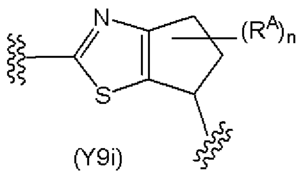
(Y9i)

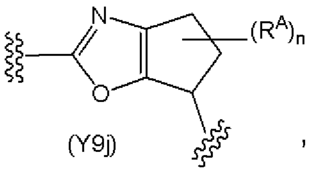
(Y9j) ,
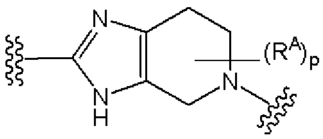
(Y9k)
or
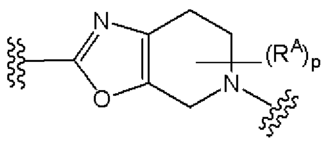
(Y9l)
;

$R^A$ is each independently halogen, cyano, carboxy, hydroxy, nitro, substituted or unsubstituted alkoxy, substituted or unsubstituted alkyl, substituted or unsubstituted alkenyl, substituted or unsubstituted alkynyl, substituted or unsubstituted amino, substituted or unsubstituted carbamoyl, substituted or unsubstituted acyl, substituted or unsubstituted aryl, substituted or unsubstituted cycloalkyl, substituted or unsubstituted cycloalkenyl, substituted or unsubstituted heteroaryl, substituted or unsubstituted heterocyclyl, a group represented by the formula: $-SO-R^{a'}$, a group represented by the formula: $-SO_2-R^{a'}$, or a group represented by the formula: $-SR^{a'}$;
$R^{a'}$ is hydrogen, substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
R' is hydrogen or substituted or unsubstituted alkyl;
m is an integer from 0 to 3;
n is an integer from 0 to 5;
p is an integer from 0 to 6;
q is an integer from 0 to 7;
r is an integer from 0 to 8;
t is an integer from 0 to 4;
u is an integer from 0 to 2;
v is 0 or 1;
Z is substituted or unsubstituted cycloalkyl, substituted or unsubstituted cycloalkenyl, substituted or unsubstituted aryl, substituted or unsubstituted heteroaryl, or substituted or unsubstituted heterocyclyl,
with the proviso that:

when Y is a group represented by formula (Y4a) or a group represented by formula (Y4b), $R^A$ is not substituted or unsubstituted aryl;
when Y is a group represented by formula (Y4a), $R^3$ is not methyl or methoxy;

when Y is a group represented by formula (Y5a), $R^3$ is not carboxy, ethoxycarbonyl, phenyl, or alkyl substituted with amino;
when Y is a group represented by formula (Y5c), $R^3$ is not phenyl, or alkyl substituted with substituted amino or imidazolidinone; and
when Y is a group represented by formula (Y5d), $R^3$ is not phenyl, or alkyl substituted with substituted amino, and wherein the following compound is excluded:

[Formula 12B]

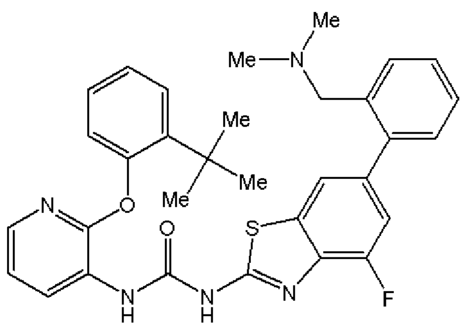

or a pharmaceutically acceptable salt thereof, or a solvate thereof.

(2α) The compound according to item (1α), wherein X1' is a substituted or unsubstituted 6-membered aromatic hydrocarbon ring, a substituted or unsubstituted 5- or 6-membered aromatic heterocycle, a substituted or unsubstituted 5- or 6-membered non-aromatic hydrocarbon ring, or a substituted or unsubstituted 5- or 6-membered non-aromatic heterocycle,
or a pharmaceutically acceptable salt thereof, or a solvate thereof.

(3α) The compound according to item (1α) or (2α), wherein X is a group represented by formula (X1),
or a pharmaceutically acceptable salt thereof, or a solvate thereof.

(4α) The compound according to any of items (1α) to (3α), wherein Y is a group represented by formula (Y1a), a group represented by formula (Y1b), a group represented by formula (Y1c), or a group represented by formula (Y1d),
or a pharmaceutically acceptable salt thereof, or a solvate thereof.

(5α) The compound according to any of items (1α) to (4α), wherein Y is a group represented by formula (Y1a) and p is 0,
or a pharmaceutically acceptable salt thereof, or a solvate thereof.

(6α) The compound according to any of items (1α) to (5α), wherein Y is a group represented by formula (Y1d) and q is 0 or 1,
or a pharmaceutically acceptable salt thereof, or a solvate thereof.

(7α) The compound according to any of items (1α) to (6α), wherein Y is a group represented by formula (Y2a), a group represented by formula (Y2b), or a group represented by formula (Y2c),
or a pharmaceutically acceptable salt thereof, or a solvate thereof.

(8α) The compound according to any of items (1α) to (7α), wherein Y is a group represented by formula (Y2a) and n is 0,
or a pharmaceutically acceptable salt thereof, or a solvate thereof.

(9α) The compound according to any of items (1α) to (8α), wherein Y is a group represented by formula (Y3a), a group represented by formula (Y3b), a group represented by formula (Y3c), a group represented by formula (Y3d), a group represented by formula (Y3e), a group represented by formula (Y3f), a group represented by formula (Y3g), a group represented by formula (Y3h), a group represented by formula (Y3i), a group represented by formula (Y3j), a group represented by formula (Y3k), or a group represented by formula (Y3l),
or a pharmaceutically acceptable salt thereof, or a solvate thereof.

(10α) The compound according to any of items (1α) to (9α), wherein Y is a group represented by formula (Y3a), a group represented by formula (Y3e), or a group represented by formula (Y3j) and m is 0,
or a pharmaceutically acceptable salt thereof, or a solvate thereof.

(11α) The compound according to any of items (1α) to (10α), wherein Y is a group represented by formula (Y3k) and u is 0,
or a pharmaceutically acceptable salt thereof, or a solvate thereof.

(12α) The compound according to any of items (1α) to (11α), wherein Y is a group represented by formula (Y4a), a group represented by formula (Y4b), a group represented by formula (Y4c), or a group represented by formula (Y4d),
or a pharmaceutically acceptable salt thereof, or a solvate thereof.
(13α) The compound according to any of items (1α) to (12α), wherein Y is a group represented by formula (Y4a) or a group represented by formula (Y4c), v is 0 or 1, and R' is hydrogen or substituted or unsubstituted alkyl,
or a pharmaceutically acceptable salt thereof, or a solvate thereof.
(14α) The compound according to any of items (1α) to (13α), wherein Y is a group represented by formula (Y5a), a group represented by formula (Y5b), a group represented by formula (Y5c), a group represented by formula (Y5d), a group represented by formula (Y5e), (Y5f), a group represented by formula (Y5g), a group represented by formula (Y5h), or a group represented by formula (Y5i),
or a pharmaceutically acceptable salt thereof, or a solvate thereof.
(15α) The compound according to any of items (1α) to (14α), wherein Y is a group represented by formula (Y5a) and n is 0 or 1,
or a pharmaceutically acceptable salt thereof, or a solvate thereof.
(16α) The compound according to any of items (1α) to (15α), wherein Y is a group represented by formula (Y6a), a group represented by formula (Y6b), a group represented by formula (Y6c), or a group represented by formula (Y6d),
or a pharmaceutically acceptable salt thereof, or a solvate thereof.
(17α) The compound according to any of items (1α) to (16α), wherein Y is a group represented by formula (Y7a), a group represented by formula (Y7b), a group represented by formula (Y7c), a group represented by formula (Y7d), or a group represented by formula (Y7e),
or a pharmaceutically acceptable salt thereof, or a solvate thereof.
(18α) The compound according to any of items (1α) to (17α), wherein Y is a group represented by formula (Y8a), a group represented by formula (Y8b), a group represented by formula (Y8c), or a group represented by formula (Y8d),
or a pharmaceutically acceptable salt thereof, or a solvate thereof.
(19α) The compound according to any of items (1α) to (18α), wherein Y is a group represented by formula (Y9a), a group represented by formula (Y9b), a group represented by formula (Y9c), a group represented by formula (Y9d), a group represented by formula (Y9e), a group represented by formula (Y9f), a group represented by formula (Y9g), a group represented by formula (Y9h), a group represented by formula (Y9i), a group represented by formula (Y9j), a group represented by formula (Y9k), or a group represented by formula (Y9l), or a pharmaceutically acceptable salt thereof, or a solvate thereof.
(20α) The compound according to any of items (1α) to (19α), wherein Z is substituted or unsubstituted aryl, substituted or unsubstituted heteroaryl, or substituted or unsubstituted heterocyclyl,
or a pharmaceutically acceptable salt thereof, or a solvate thereof.
(21α) The compound according to any of items (1α) to (20α), wherein $R^3$ is carboxy, substituted or unsubstituted alkyl, substituted or unsubstituted aryl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, substituted or unsubstituted alkylsulfonyl, substituted or unsubstituted sulfamoyl, or a group represented by the formula: $-C(-R^a)=N-OH$; and $R^a$ is hydrogen or substituted or unsubstituted alkyl,
or a pharmaceutically acceptable salt thereof, or a solvate thereof.
(22α) The compound according to any of item (21α), wherein $R^3$ is substituted or unsubstituted carbamoyl, substituted or unsubstituted amino, or substituted or unsubstituted alkoxy,
or a pharmaceutically acceptable salt thereof, or a solvate thereof.
(23α) The compound according to any of items (1α) to (22α), wherein $R^1$ and $R^2$ are both hydrogen,
or a pharmaceutically acceptable salt thereof, or a solvate thereof.
(24α) A pharmaceutical composition containing the compound according to any of items (1α) to (23α), or a pharmaceutically acceptable salt thereof, or a solvate thereof.
(25α) The pharmaceutical composition according to item (24α), which is a phosphatidylinositol-3-kinase inhibitor.
(26α) A phosphatidylinositol-3-kinase inhibitor containing the compound according to any of items (1α) to (23α), or a pharmaceutically acceptable salt thereof, or a solvate thereof, as an active ingredient.
(27α) The inhibitor according to item (26α), which is specific to one or more types of α, β, γ, and δ of phosphatidylinositol-3-kinase inhibitors.
(28α) The pharmaceutical composition according to items (24α) and (25α) for the treatment of phosphatidylinositol-3-kinase dependent diseases listed below:

encephalitis, myelitis and encephalomyelitis, meningitis, inflammatory polyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like; or burn, traumatic inflammation, and the like.

(29α) A phosphatidylinositol-3-kinase inhibitor containing the compound according to any of items (1α) to (23α), or a pharmaceutically acceptable salt thereof, or a solvate thereof.
(30α) A protein kinase B (AKT) inhibitor containing the compound according to any of items (1α) to (23α), or a pharmaceutically acceptable salt thereof, or a solvate thereof.
(31α) An anticancer agent containing the compound according to any of items (1α) to (23α), or a pharmaceutically acceptable salt thereof, or a solvate thereof.
(32α) An anti-inflammatory or a therapeutic agent for inflammatory diseases (such as pancreatitis, pneumonia, airway inflammation, COPD (such as pulmonary emphysema, chronic bronchitis, and the like), arthritis, glomerulonephritis, and the like) wherein the anti-inflammatory or the therapeutic agent contains the compound according to any of items (1α) to (23α), or a pharmaceutically acceptable salt thereof, or a solvate thereof.
(33α) An antiallergic agent (asthma, atopic dermatitis, allergic rhinitis, and the like) containing the compound according to any of items (1α) to (23α), or a pharmaceutically acceptable salt thereof, or a solvate thereof.
(34α) A therapeutic agent for immune system diseases wherein the therapeutic agent contains the compound according to any of items (1α) to (23α), or a pharmaceutically acceptable salt thereof, or a solvate thereof.
(35α) An immunosuppressant containing the compound according to any of items (1α) to (23α), or a pharmaceutically acceptable salt thereof, or a solvate thereof.
(36α) A therapeutic agent for autoimmune diseases wherein the therapeutic agent contains the compound according to any of items (1α) to (23α), or a pharmaceutically acceptable salt thereof, or a solvate thereof.
(37α) An anti-circulatory-disease agent (such as antihypertensive agent and the like) wherein the agent contains the compound according to any of items (1α) to (23α), or a pharmaceutically acceptable salt thereof, or a solvate thereof.
(38α) An anti-infective agent containing the compound according to any of items (1α) to (23α), or a pharmaceutically acceptable salt thereof, or a solvate thereof.
(39α) A wound-healing agent containing the compound according to any of items (1α) to (23α), or a pharmaceutically acceptable salt thereof, or a solvate thereof.
(40α) A method, a system, an apparatus, a kit, and the like for producing the compound according to any of items (1α) to (23α), or a pharmaceutically acceptable salt thereof, or a solvate thereof.
(41α) A method, a system, an apparatus, a kit, and the like for preparing a pharmaceutical composition containing the compound according to any of items (1α) to (23α), or a pharmaceutically acceptable salt thereof, or a solvate thereof.
(42α) A method, a system, an apparatus, a kit, and the like using the compound according to any of the preceding items (1α) to (23α), or a pharmaceutically acceptable salt thereof, or a solvate thereof.

(43α) The pharmaceutical composition according to item (24α), which is a medicament for the treatment or prophylaxis of diseases, disorders, or conditions related to phosphatidylinositol-3-kinase.

(44α) The pharmaceutical composition according to any of items (24α), (25α), and (43α), which is a therapeutic agent and/or a prophylactic agent for inflammation.

(45α) A method for the prophylaxis and/or therapy of inflammation, characterized by administering the compound according to any of items (1α) to (23α), or a pharmaceutically acceptable salt thereof, or a solvate thereof.

(46α) Use of the compound according to any of items (1α) to (23α), or a pharmaceutically acceptable salt thereof, or a solvate thereof, for producing a therapeutic agent and/or a prophylactic agent for inflammation.

(47α) The compound according to any of items (1α) to (23α), or a pharmaceutically acceptable salt thereof, or a solvate thereof, for the therapy and/or prophylaxis of inflammation.

The present invention also provides, for example, the following items:

(1β) A compound represented by formula (I):

[Formula 1C]

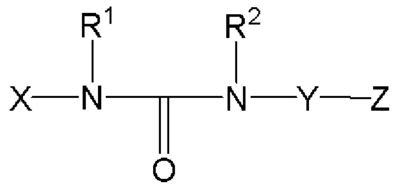

(I)

wherein

$R^1$ and $R^2$ are each independently hydrogen or substituted or unsubstituted alkyl;

X is a group represented by a formula selected from the following:

[Formula 2C]

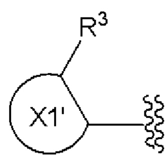

(X1)

or

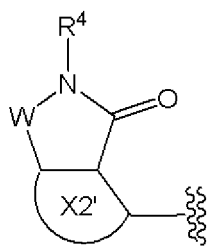

(X2) ;

X1' is a substituted or unsubstituted 6-membered aromatic hydrocarbon ring, a substituted or unsubstituted 5- or 6-membered aromatic heterocycle, a substituted or unsubstituted 3 to 7-membered non-aromatic hydrocarbon ring, or a substituted or unsubstituted 3 to 7-membered non-aromatic heterocycle; X2' is a substituted or unsubstituted monocyclic aromatic hydrocarbon ring, a substituted or unsubstituted monocyclic aromatic heterocycle, a substituted or unsubstituted monocyclic non-aromatic hydrocarbon ring, or a substituted or unsubstituted monocyclic non-aromatic heterocycle;

$R^3$ is carboxy, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-C(-R^a)=N-OH$, a group represented by the formula: $-SO-R^a$, a group represented by the formula: $-SO_2-R^a$, or a group represented by the formula: $-SR^a$;

$R^a$ is hydrogen, substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;

$R^4$ is hydrogen, cyano, substituted or unsubstituted alkyl, substituted or unsubstituted aryl, substituted or unsubstituted cycloalkyl, substituted or unsubstituted cycloalkenyl, substituted or unsubstituted heteroaryl, or substituted or unsubstituted heterocyclyl, and W is a group represented by the formula: $-(CR^5R^6)_a-$ or a group represented by the formula: $-(CR^7=CR^8)-$;

$R^5$ to $R^8$ are each independently hydrogen, halogen, cyano, hydroxy, carboxy, substituted or unsubstituted alkyl, substituted or unsubstituted alkoxy, substituted or unsubstituted acyl, substituted or unsubstituted carbamoyl, or substituted or unsubstituted amino;

a is 1 or 2;

Y is a group represented by a formula selected from the following:

[Formula 3C]

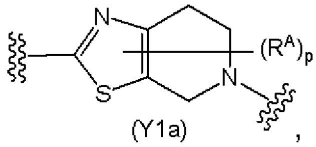
(Y1a) ,

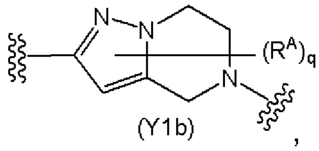
(Y1b) ,

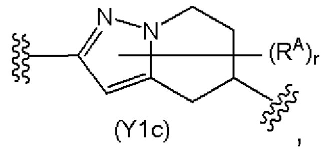
(Y1c) ,

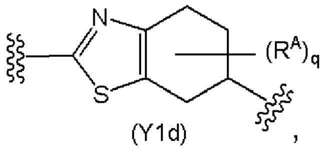
(Y1d) ,

[Formula 4C]

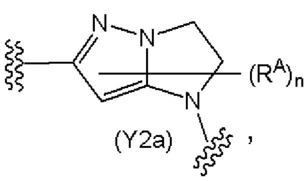
(Y2a) ,

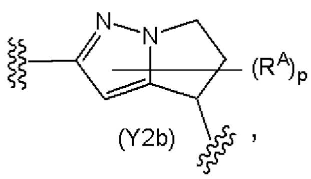
(Y2b) ,

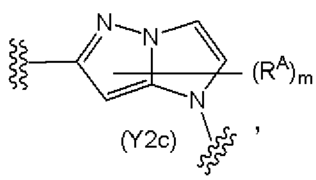
(Y2c) ,

[Formula 5C]

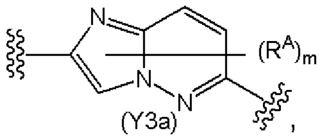
(Y3a),
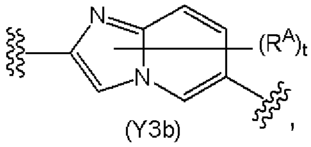
(Y3b),
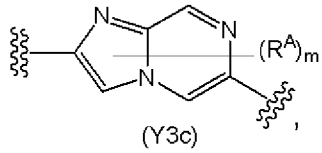
(Y3c),
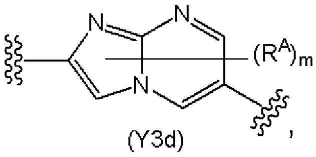
(Y3d),
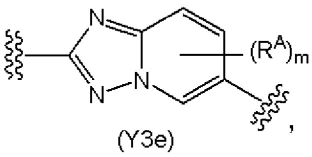
(Y3e),
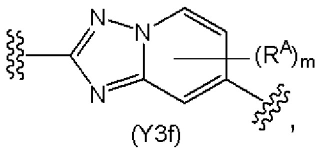
(Y3f),
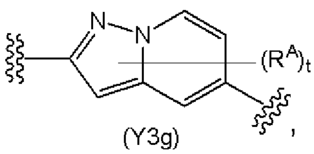
(Y3g),
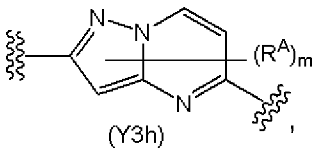
(Y3h),
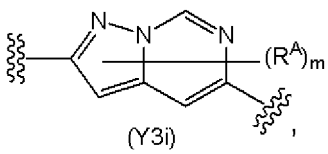
(Y3i),
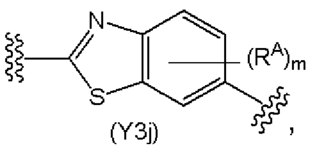
(Y3j),
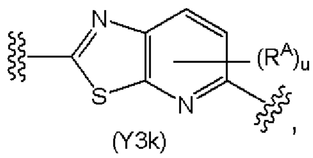
(Y3k),
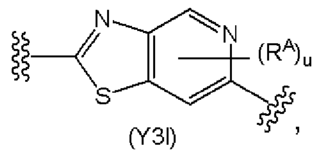
(Y3l),

[Formula 6C]

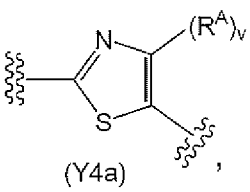
(Y4a),
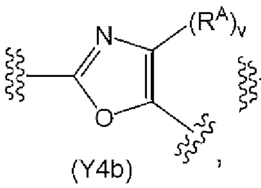
(Y4b),
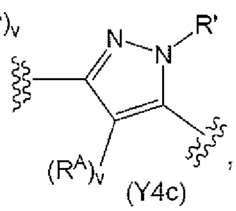
(Y4c),
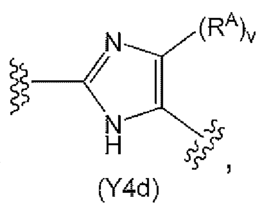
(Y4d),

[Formula 7C]

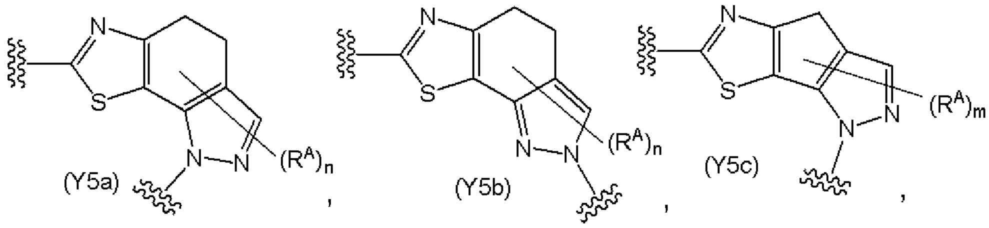

(Y5a), (Y5b), (Y5c),

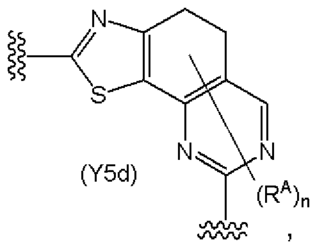

(Y5d),

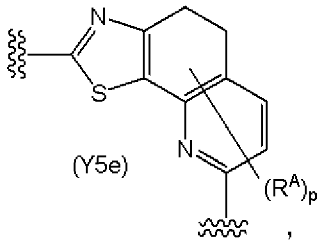

(Y5e),

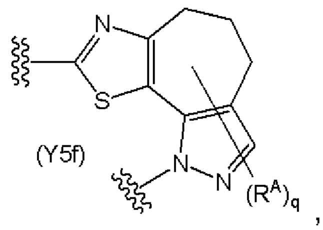

(Y5f),

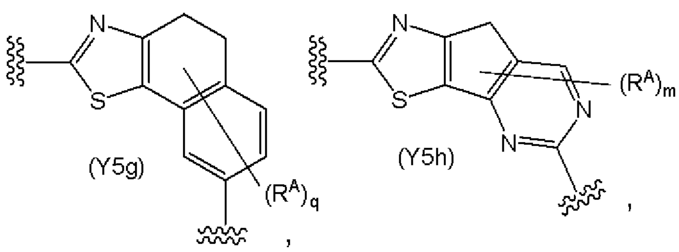

(Y5g), (Y5h),

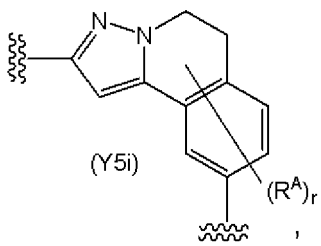

(Y5i),

[Formula 8C]

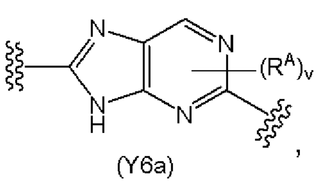

(Y6a),

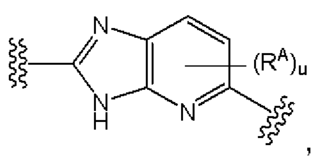

(Y6b),

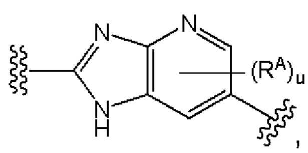

(Y6c),

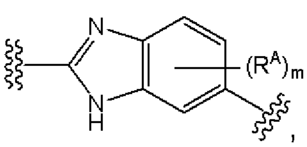

(Y6d)

[Formula 9C]

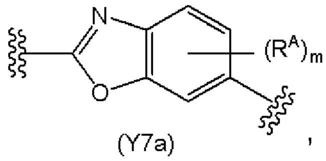
(Y7a) ,

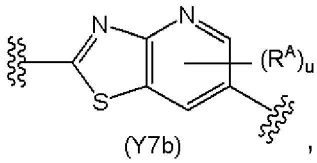
(Y7b) ,

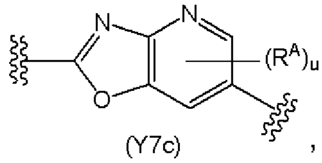
(Y7c) ,

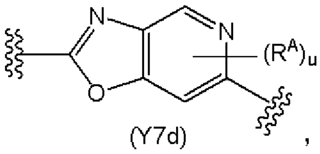
(Y7d) ,

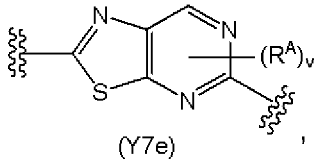
(Y7e) ,

[Formula 10C]

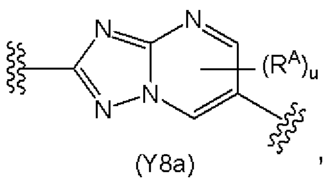
(Y8a) ,

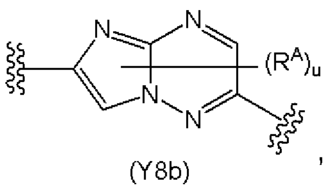
(Y8b) ,

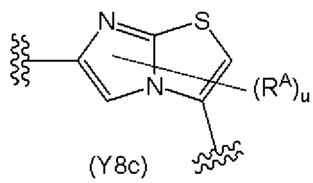
(Y8c)

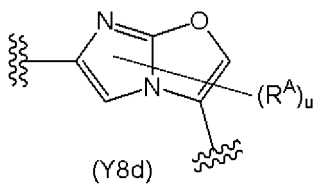
(Y8d) ,

[Formula 11C]

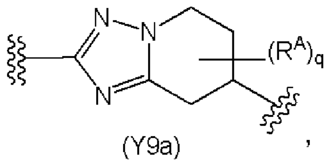

(Y9a) ,

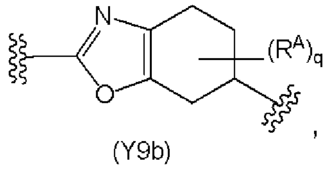

(Y9b) ,

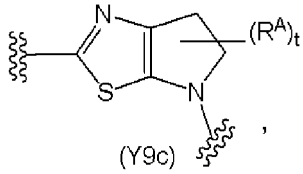

(Y9c) ,

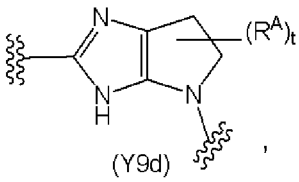

(Y9d) ,

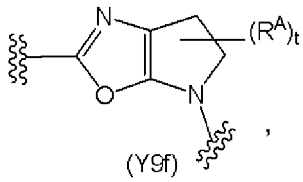

(Y9f) ,

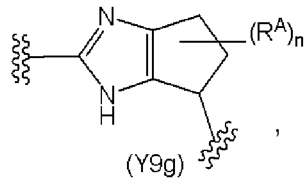

(Y9g) ,

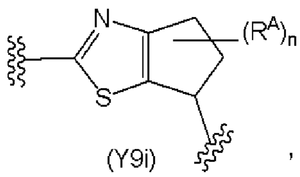

(Y9i) ,

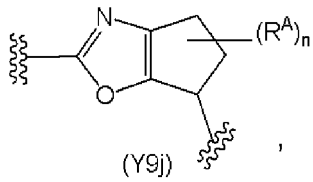

(Y9j) ,

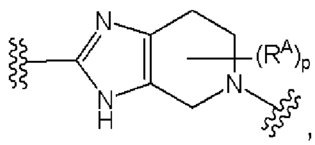

(Y9k) ,

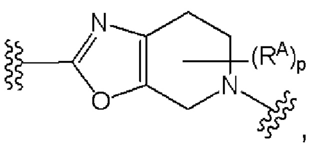

(Y9l) ,

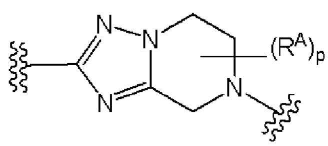

(Y9m) or

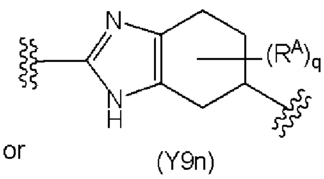

(Y9n) ;

$R^A$ is each independently halogen, cyano, carboxy, hydroxy, nitro, substituted or unsubstituted alkoxy, substituted or unsubstituted alkyl, substituted or unsubstituted alkenyl, substituted or unsubstituted alkynyl, substituted or unsubstituted amino, substituted or unsubstituted carbamoyl, substituted or unsubstituted acyl, substituted or unsubstituted aryl, substituted or unsubstituted cycloalkyl, substituted or unsubstituted cycloalkenyl, substituted or unsubstituted heteroaryl, substituted or unsubstituted heterocyclyl, a group represented by the formula: $-SO-R^{a'}$, a group represented by the formula: $-SO_2-R^{a'}$, or a group represented by the formula: $-SR^{a'}$;

$R^{a'}$ is hydrogen, substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;

R' is hydrogen or substituted or unsubstituted alkyl;

m is an integer from 0 to 3;

n is an integer from 0 to 5;

p is an integer from 0 to 6;

q is an integer from 0 to 7;

r is an integer from 0 to 8;

t is an integer from 0 to 4;

u is an integer from 0 to 2;

v is 0 or 1; and

Z is substituted or unsubstituted cycloalkyl, substituted or unsubstituted cycloalkenyl, substituted or unsubstituted aryl, substituted or unsubstituted heteroaryl, or substituted or unsubstituted heterocyclyl,

with the proviso that:

when Y is a group represented by formula (Y4a) or a group represented by formula (Y4b), $R^A$ is not substituted or unsubstituted aryl;

when Y is a group represented by formula (Y4a), $R^3$ is not methyl or methoxy;

when Y is a group represented by formula (Y5a), $R^3$ is not carboxy, ethoxycarbonyl, phenyl, or alkyl substituted with amino;

when Y is a group represented by formula (Y5c), $R^3$ is not phenyl, or alkyl substituted with substituted amino or imidazolidinone; and
when Y is a group represented by formula (Y5d), $R^3$ is not phenyl, or alkyl substituted with substituted amino, and wherein the following compound is excluded:

[Formula 12C]

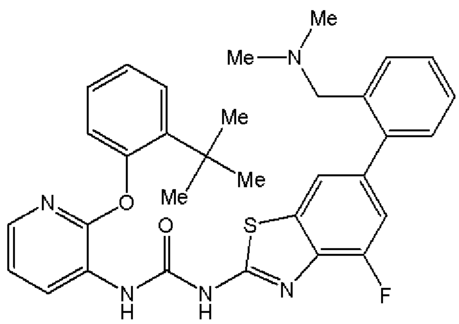

or a pharmaceutically acceptable salt thereof, or a solvate thereof.

(2β) The compound according to item (1β), wherein X1' is a substituted or unsubstituted 6-membered aromatic hydrocarbon ring, a substituted or unsubstituted 5- or 6-membered aromatic heterocycle, a substituted or unsubstituted 5- or 6-membered non-aromatic hydrocarbon ring, or a substituted or unsubstituted 5- or 6-membered non-aromatic heterocycle,
or a pharmaceutically acceptable salt thereof, or a solvate thereof.

(3β) The compound according to item (1β) or (2β), wherein X is a group represented by formula (X1),
or a pharmaceutically acceptable salt thereof, or a solvate thereof.

(4β) The compound according to any of items (1β) to (3β), wherein Y is a group represented by formula (Y1a), a group represented by formula (Y1b), a group represented by formula (Y1c), or a group represented by formula (Y1d),
or a pharmaceutically acceptable salt thereof, or a solvate thereof.

(5β) The compound according to any of items (1β) to (4β), wherein Y is a group represented by formula (Y1a) and p is 0 or 1,
or a pharmaceutically acceptable salt thereof, or a solvate thereof.

(6β) The compound according to any of items (1β) to (5β), wherein Y is a group represented by formula (Y1d) and q is 0 or 1,
or a pharmaceutically acceptable salt thereof, or a solvate thereof.

(7β) The compound according to any of items (1β) to (6β), wherein Y is a group represented by formula (Y2a), a group represented by formula (Y2b), or a group represented by formula (Y2c),
or a pharmaceutically acceptable salt thereof, or a solvate thereof.

(8β) The compound according to any of items (1β) to (7β), wherein Y is a group represented by formula (Y2a) and n is 0,
or a pharmaceutically acceptable salt thereof, or a solvate thereof.

(9β) The compound according to any of items (1β) to (8β), wherein Y is a group represented by formula (Y3a), a group represented by formula (Y3b), a group represented by formula (Y3c), a group represented by formula (Y3d), a group represented by formula (Y3e), a group represented by formula (Y3f), a group represented by formula (Y3g), a group represented by formula (Y3h), a group represented by formula (Y3i), a group represented by formula (Y3j), a group represented by formula (Y3k), or a group represented by formula (Y3l),
or a pharmaceutically acceptable salt thereof, or a solvate thereof.

(10β) The compound according to any of items (1β) to (9β), wherein Y is a group represented by formula (Y3a), (Y3e) or a group represented by formula (Y3j) and m is 0,
or a pharmaceutically acceptable salt thereof, or a solvate thereof.

(11β) The compound according to any of items (1β) to (10β), wherein Y is a group represented by formula (Y3k) and u is 0,
or a pharmaceutically acceptable salt thereof, or a solvate thereof.

(12β) The compound according to any of items (1β) to (11β), wherein Y is a group represented by formula (Y4a), a group represented by formula (Y4b), a group represented by formula (Y4c), or a group represented by formula

(Y4d),
or a pharmaceutically acceptable salt thereof, or a solvate thereof.

(13β) The compound according to any of items (1β) to (12β), wherein Y is a group represented by formula (Y4a) or a group represented by formula (Y4c), v is 0 or 1, and R' is hydrogen or substituted or unsubstituted alkyl,
or a pharmaceutically acceptable salt thereof, or a solvate thereof.

(14β) The compound according to any of items (1β) to (13β), wherein Y is a group represented by formula (Y5a), a group represented by formula (Y5b), a group represented by formula (Y5c), a group represented by formula (Y5d), a group represented by formula (Y5e), a group represented by formula (Y5f), a group represented by formula (Y5g), a group represented by formula (Y5h), or a group represented by formula (Y5i),
or a pharmaceutically acceptable salt thereof, or a solvate thereof.

(15β) The compound according to any of items (1β) to (14β), wherein Y is a group represented by formula (Y5a) and n is 0 or 1,
or a pharmaceutically acceptable salt thereof, or a solvate thereof.

(16β) The compound according to any of items (1β) to (15β), wherein Y is a group represented by formula (Y6a), a group represented by formula (Y6b), a group represented by formula (Y6c), or a group represented by formula (Y6d),
or a pharmaceutically acceptable salt thereof, or a solvate thereof.

(17β) The compound according to any of items (1β) to (16β), wherein Y is a group represented by formula (Y7a), a group represented by (Y7b), a group represented by (Y7c), a group represented by (Y7d), or a group represented by (Y7e),
or a pharmaceutically acceptable salt thereof, or a solvate thereof.

(18β) The compound according to any of items (1β) to (17β), wherein Y is a group represented by formula (Y8a), a group represented by formula (Y8b), a group represented by formula (Y8c), or a group represented by formula (Y8d),
or a pharmaceutically acceptable salt thereof, or a solvate thereof.

(19β) The compound according to any of items (1β) to (18β), wherein Y is a group represented by formula (Y9a), a group represented by formula (Y9b), a group represented by formula (Y9c), a group represented by formula (Y9d), a group represented by formula (Y9f), a group represented by formula (Y9g), a group represented by formula (Y9i), a group represented by formula (Y9j), a group represented by formula (Y9k), a group represented by formula (Y9l), a group represented by formula (Y9m), or a group represented by formula (Y9n),
or a pharmaceutically acceptable salt thereof, or a solvate thereof.

(20β) The compound according to any of items (1β) to (19β), wherein Z is substituted or unsubstituted aryl, substituted or unsubstituted heteroaryl, or substituted or unsubstituted heterocyclyl,
or a pharmaceutically acceptable salt thereof, or a solvate thereof.

(21β) The compound according to any of items (1β) to (20β), wherein $R^3$ is carboxy, substituted or unsubstituted alkyl, substituted or unsubstituted aryl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, substituted or unsubstituted alkylsulfonyl, substituted or unsubstituted sulfamoyl, or a group represented by the formula: $-C(-R^a)=N-OH$; $R^a$ is hydrogen or substituted or unsubstituted alkyl,
or a pharmaceutically acceptable salt thereof, or a solvate thereof.

(22β) The compound according to any of item (21β), wherein $R^3$ is substituted or unsubstituted carbamoyl, substituted or unsubstituted amino, or substituted or unsubstituted alkoxy,
or a pharmaceutically acceptable salt thereof, or a solvate thereof.

(23β) The compound according to any of items (1β) to (22β), wherein $R^1$ and $R^2$ are both hydrogen,
or a pharmaceutically acceptable salt thereof, or a solvate thereof.

(24β) A pharmaceutical composition containing the compound according to any of items (1β) to (23β), or a pharmaceutically acceptable salt thereof, or a solvate thereof.

(25β) The pharmaceutical composition according to item (24β), which is a phosphatidylinositol-3-kinase inhibitor.

(26β) A phosphatidylinositol-3-kinase inhibitor containing the compound according to any of items (1β) to (23β), or a pharmaceutically acceptable salt thereof, or a solvate thereof, as an active ingredient.

(27β) The inhibitor according to item (26β), which is specific to one or more types of α, β, γ, and δ of phosphatidylinositol-3-kinase inhibitors.

(28β) The pharmaceutical composition according to items (24β) and (25β) for the treatment of phosphatidylinositol-3-kinase dependent diseases listed below:

encephalitis, myelitis and encephalomyelitis, meningitis, inflammatory polyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like; or burn, traumatic inflammation, and the like.

(29β) A phosphatidylinositol-3-kinase inhibitor containing the compound according to any of items (1β) to (23β), or a pharmaceutically acceptable salt thereof, or a solvate thereof.
(30β) A protein kinase B (AKT) inhibitor containing the compound according to any of items (1β) to (23β), or a pharmaceutically acceptable salt thereof, or a solvate thereof.
(31β) An anticancer agent containing the compound according to any of items (1β) to (23β), or a pharmaceutically acceptable salt thereof, or a solvate thereof.
(32β) An anti-inflammatory or a therapeutic agent for inflammatory diseases (such as pancreatitis, pneumonia, airway inflammation, COPD (such as pulmonary emphysema, chronic bronchitis, and the like), arthritis, glomerulonephritis, and the like) wherein the anti-inflammatory or the therapeutic agent contains the compound according to any of items (1β) to (23β), or a pharmaceutically acceptable salt thereof, or a solvate thereof.
(33β) An antiallergic agent (asthma, atopic dermatitis, allergic rhinitis, and the like) containing the compound according to any of items (1β) to (23β), or a pharmaceutically acceptable salt thereof, or a solvate thereof.
(34β) A therapeutic agent for immune system diseases wherein the therapeutic agent contains the compound according to any of items (1β) to (23β), or a pharmaceutically acceptable salt thereof, or a solvate thereof.
(35β) An immunosuppressant containing the compound, or a pharmaceutically acceptable salt thereof, or a solvate thereof according to any of items (1β) to (23β).
(36β) A therapeutic agent for autoimmune diseases wherein the therapeutic agent contains the compound according to any of items (1β) to (23β), or a pharmaceutically acceptable salt thereof, or a solvate thereof.
(37β) An anti-circulatory-disease agent (such as antihypertensive agent and the like) wherein the agent contains the compound according to any of items (1β) to (23β), or a pharmaceutically acceptable salt thereof, or a solvate thereof.
(38β) An anti-infective agent containing the compound according to any of items (1β) to (23β), or a pharmaceutically acceptable salt thereof, or a solvate thereof.
(39β) A wound-healing agent containing the compound according to any of items (1β) to (23β), or a pharmaceutically acceptable salt thereof, or a solvate thereof.
(40β) A method, a system, an apparatus, a kit, and the like for producing the compound according to any of items (1β) to (23β), or a pharmaceutically acceptable salt thereof, or a solvate thereof.
(41β) A method, a system, an apparatus, a kit, and the like for preparing a pharmaceutical composition containing the compound according to any of items (1β) to (23β), or a pharmaceutically acceptable salt thereof, or a solvate thereof.
(42β) A method, a system, an apparatus, a kit, and the like using the compound according to any of the preceding items (1β) to (23β), or a pharmaceutically acceptable salt thereof, or a solvate thereof.
(43β) The pharmaceutical composition according to item (24β), which is a medicament for the treatment or

prophylaxis of diseases, disorders, or conditions related to phosphatidylinositol-3-kinase.

(44β) The pharmaceutical composition according to any of items (24β), (25β), and (43β), which is a therapeutic agent and/or a prophylactic agent for inflammation.

(45β) A method for the prophylaxis and/or therapy of inflammation, characterized by administering the compound according to any of items (1β) to (23β), or a pharmaceutically acceptable salt thereof, or a solvate thereof.

(46β) Use of the compound according to any of items (1β) to (23β), or a pharmaceutically acceptable salt thereof, or a solvate thereof, for producing a therapeutic agent and/or a prophylactic agent for inflammation.

(47β) The compound according to any of items (1β) to (23β), or a pharmaceutically acceptable salt thereof, or a solvate thereof, for the therapy and/or prophylaxis of inflammation.

Thus, these and other advantages of the present invention are apparent when the following detailed description is read.

EFFECT OF THE INVENTION

The present invention provides a medicament for the treatment of phosphatidylinositol-3-kinase dependent diseases; a compound used therefor; or a pharmaceutically acceptable salt thereof; or a prodrug thereof including a hydrate and the like thereof. The compound of the present invention exhibits excellent PI3-kinaseγ inhibitory activity as described in Examples below. Accordingly, the pharmaceutical composition of the present invention may be used for the prophylaxis and/or as a therapeutic agent for diseases such as encephalitis, myelitis and encephalomyelitis, meningitis, inflammatory polyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like, or used as a therapeutic agent for burn or traumatic inflammation.

The compound of the present invention is a compound having utility as a medicament. Here, utility as a medicament includes the following points: the compound has good metabolic stability; the induction of a drug-metabolizing enzyme is low; the inhibition of a drug-metabolizing enzyme which metabolizes another drug is low; the compound has high oral absorbency; the clearance is low; the half-life is sufficiently long to express the efficacy; or the like.

MODE FOR CARRYING OUT THE INVENTION

Hereinafter, the present invention is described with reference to embodiments. It should be understood that, throughout the present specification, the expression of a singular form includes the concept of its plural form unless specified otherwise. Accordingly, it should be understood that an article in singular form (for example, in the English language, "a," "an," "the," and the like) includes the concept of its plural form unless specified otherwise. Furthermore, it should be understood that the terms used herein are used in a meaning normally used in the art unless specified otherwise. Thus, unless defined otherwise, all technical and scientific terms used herein have the same meaning as those generally understood by those skilled in the art in the field to which the present invention pertains. If there is a

contradiction, the present specification (including definitions) precedes.

Each meaning of terms used herein is described below. In the present specification, each term is used in an unequivocal meaning. Both when used alone and in combination with another word, each term is used in the same meaning.

As used herein, the term "halogen" means fluorine, chlorine, bromine, and iodine. Examples thereof include fluorine, chlorine, and bromine.

As used herein, the term "alkyl" encompasses a linear or branched monovalent hydrocarbon group having 1 to 8 carbon atoms. Examples thereof include methyl, ethyl, *n*-propyl, isopropyl, *n*-butyl, isobutyl, sec-butyl, *tert*-butyl, *n*-pentyl, isopentyl, *neo*-pentyl, n-hexyl, isohexyl, *n*-heptyl, *n*-octyl, and the like. An example is C1-C6 alkyl. Another example is C1-C4 alkyl. When the carbon number is specifically designated, an "alkyl" having carbon in a range of the number is meant.

As used herein, the term "alkenyl" encompasses a linear or branched monovalent hydrocarbon group having 2 to 8 carbon atoms and one or more double bonds. Examples thereof include vinyl, allyl, 1-propenyl, 2-butenyl, 2-pentenyl, 2-hexenyl, 2-heptenyl, 2-octenyl, and the like. An example is C2-C6 alkenyl. Another example is C2-C4 alkenyl.

As used herein, the term "alkynyl" encompasses a linear or branched monovalent hydrocarbon group having 2 to 8 carbon atoms and one or more triple bonds. Examples thereof include ethynyl, 1-propynyl, 2-propynyl, 2-butynyl, 2-pentynyl, 2-hexynyl, 2-heptynyl, 2-octynyl, and the like. An example is C2-C6 alkynyl. Another example is C2-C4 alkynyl.

As used herein, the term "cycloalkyl" encompasses cycloalkyl having 3 to 8 carbon atoms. Examples thereof include cyclopropyl, cyclobutyl, cyclopentyl, cyclohexyl, cycloheptyl, and cyclooctyl. An example is C3-C6 cycloalkyl.

As used herein, the term "cycloalkenyl" encompasses cycloalkenyl having 3 to 8 carbon atoms. Examples thereof include cyclopropenyl, cyclobutenyl, cyclopentenyl, cyclohexenyl, cycloheptenyl, and, for example, C3-C6 cycloalkenyl.

As used herein, the term "alkoxy" includes methoxy, ethoxy, *n*-propoxy, isopropoxy, *n*-butoxy, isobutoxy, *sec*-butoxy, *tert*-butoxy, *n*-pentyloxy, isopentyloxy, 2-pentyloxy, 3-pentyloxy, *n*-hexyloxy, isohexyloxy, 2-hexyloxy, 3-hexyloxy, *n*-heptyloxy, *n*-octyloxy, and the like. An example is C1-C6 alkyloxy. Another example is C1-C5 alkyloxy. Another example is C1-C4 alkyloxy. When the carbon number is specifically designated, an "alkoxy" having carbon in a range thereof is meant.

As used herein, the term "alkylsulfonyl" includes methylsulfonyl, ethylsulfonyl, *n*-propylsulfonyl, isopropylsulfonyl, *n*-butylsulfonyl, isobutylsulfonyl, *sec*-butylsulfonyl, *tert*-butylsulfonyl, *n*-pentylsulfonyl, isopentylsulfonyl, 2-pentylsulfonyl, 3-pentylsulfonyl, *n*-hexylsulfonyl, isohexylsulfonyl, 2-hexylsulfonyl, 3-hexylsulfonyl, *n*-heptylsulfonyl, *n*-octylsulfonyl, and the like. An example is C1-C6 alkylsulfonyl. Another example is C1-C4 alkylsulfonyl.

As used herein, the term "alkoxycarbonyl" includes methoxycarbonyl, ethoxycarbonyl, *n*-propoxycarbonyl, isopropoxycarbonyl, *n*-butoxycarbonyl, *tert*-butoxycarbonyl, *n*-pentyloxycarbonyl, and the like. An example is C1-C4 alkyloxycarbonyl. Another example is C1-C2 alkyloxycarbonyl.

As used herein, the term "acyl" encompasses formyl, alkylcarbonyl, alkenylcarbonyl, cycloalkylcarbonyl, cycloalkenylcarbonyl, arylcarbonyl, heteroarylcarbonyl, heterocyclylcarbonyl. Examples thereof include acetyl, propionyl, butyloyl, benzoyl, and the like.

As used herein, the term "aryl" encompasses monovalent groups derived from a monocyclic or fused-cyclic aromatic hydrocarbon ring. Both in the cases where aryl is monocyclic and fused-cyclic, it may be bound at any possible position. Examples thereof include phenyl, 1-naphthyl, 2-naphthyl, anthryl, and the like. Examples include phenyl, 1-naphthyl, and 2-naphthyl. An example is phenyl.

As used herein, the term "aromatic hydrocarbon ring" encompasses aromatic 5- to 8-membered rings (preferably, 6-membered rings) containing only carbon atoms in the ring, or rings in which two or more of them are fused. Monocyclic aromatic hydrocarbon rings encompass those rings that are derived from a 5- to 8-membered (preferably, 6-membered) aromatic hydrocarbon ring and may have a bond on any of substitutable position on the ring. Fused aromatic hydrocarbon rings encompass those rings in which a 5- to 8-membered (preferably, 6-membered) monocyclic aromatic hydrocarbon ring is fused with one to four 5- to 8-membered (preferably, 6-membered) monocyclic aromatic hydrocarbon ring, and which may have a bond on any of substitutable position on the ring. Examples thereof include 6-membered aromatic hydrocarbon rings. Examples of aromatic hydrocarbon rings include a benzene ring, a naphthalene ring, an anthracene ring, and the like. Examples include a benzene ring, and a naphthalene ring.

As used herein, the term "non-aromatic hydrocarbon ring" encompasses non-aromatic 3- to 8-membered rings containing only carbon atoms in the ring, or rings in which two or more of them are fused. Monocyclic non-aromatic hydrocarbon rings encompass those rings that are derived from a 3- to 8-membered non-aromatic hydrocarbon ring and may have a bond on any of substitutable position on the ring. Fused non-aromatic hydrocarbon rings encompass those rings in which a 5-to 8-membereed monocyclic non-aromatic hydrocarbon ring is fused with one to four 5- to 8-membered monocyclic non-aromatic hydrocarbon ring and/or the aforementioned "aromatic hydrocarbon ring," and which may have a bond on any of substitutable position on the ring. When it is non-aromatic, it may be saturated or unsaturated. Examples thereof include 5- or 6-membered non-aromatic hydrocarbon rings. Examples of non-aromatic

hydrocarbon rings include a cyclopropane ring, a cyclobutane ring, a cyclopentane ring, a cyclohexane ring, a cycloheptane ring, a cyclooctane ring, a cyclopropene ring, a cyclobutene ring, a cyclopentene, cyclohexene ring, and a cycloheptene ring. Examples include a cyclopentane ring, a cyclohexane ring, a cyclopentene, a cyclohexene ring, and a tetrahydronaphthalene ring.

As used herein, the term "heteroaryl" encompasses monovalent groups derived from a 5- or 6-membered aromatic heterocyclic group containing one or more optionally-selected oxygen atoms, sulfur atoms, and/or nitrogen atoms in the ring. This may be fused with the aforementioned "aryl" and/or another heteroaryl at any possible position. Both in the cases that heteroaryl is monocyclic and fused-cyclic, it may be bound at any possible position. Examples thereof include pyrrolyl (e.g., 1-pyrrolyl, 2-pyrrolyl, 3-pyrrolyl), furyl (e.g., 2-furyl, 3-furyl), thienyl (e.g., 2-thienyl, 3-thienyl), imidazolyl (e.g., 2-imidazolyl, 4-imidazolyl), pyrazolyl (e.g., 1-pyrazolyl, 3-pyrazolyl, 4-pyrazolyl), isothiazolyl (e.g., 3-isothiazolyl), isoxazolyl (e.g., 3-isoxazolyl), oxazolyl (e.g., 2-oxazolyl, 4-oxazolyl, 5-oxazolyl), thiazolyl (e.g., 2-thiazolyl, 4-thiazolyl, 5-thiazolyl), pyridyl (e.g., 2-pyridyl, 3-pyridyl, 4-pyridyl), pyrazinyl (e.g., 2-pyrazinyl), pyrimidinyl (e.g., 2-pyrimidinyl, 4-pyrimidinyl), pyridazinyl (e.g., 3-pyridazinyl), tetrazolyl (e.g., 1H-tetrazolyl), oxadiazolyl (e.g., 1,3,4-oxadiazolyl), thiadiazolyl (e.g., 1,3,4-thiadiazolyl), indolizinyl (e.g., 2-indolizinyl, 6-indolizinyl), isoindolyl (e.g., 2-isoindolyl), indolyl (e.g., 1-indolyl, 2-indolyl, 3-indolyl), indazolyl (e.g., 3-indazolyl), purinyl (e.g., 8-purinyl), quinolizinyl (e.g., 2-quinolizinyl), isoquinolyl (e.g., 3-isoquinolyl), quinolyl (e.g., 2-quinolyl, 5-quinolyl), phthalazinyl (e.g., 1-phthalazinyl), naphthyridinyl (e.g., 2-naphthyridinyl), quinazolinyl (e.g., 2-quinazolinyl), cinnolinyl (e.g., 3-cinnolinyl), pteridinyl (e.g., 2-pteridinyl), carbazolyl (e.g., 2-carbazolyl, 4-carbazolyl), phenanthridinyl (e.g., 2-phenanthridinyl, 3-phenanthridinyl), acridinyl (e.g., 1-acridinyl, 2-acridinyl), dibenzofuranyl (e.g., 1-dibenzofuranyl, 2-dibenzofuranyl), benzimidazolyl (e.g., 2-benzimidazolyl), benzisoxazolyl (e.g., 3-benzisoxazolyl), benzoxazolyl (e.g., 2-benzoxazolyl), benzoxadiazolyl (e.g., 4-benzoxadiazolyl), benzisothiazolyl (e.g., 3-benzisothiazolyl), benzothiazolyl (e.g., 2-benzothiazolyl), benzofuryl (e.g., 3-benzofuryl), benzothienyl (e.g., 2-benzothienyl), dibenzothienyl (e.g., 2-dibenzothienyl), and the like.

As used herein, the term "heterocyclyl" encompasses monovalent groups derived from a 3- to 8-membered non-aromatic heterocycle containing one or more optionally-selected oxygen atoms, sulfur atoms, and/or nitrogen atoms in the ring. The aforementioned non-aromatic heterocycle may have a bond on any of substitutable position on the ring. In addition, such a non-aromatic heterocycle may further be bridged via a C1-C4 alkyl chain. Moreover, such a non-aromatic heterocycle may be fused with the foregoing "cycloalkyl" (including, for example, 3- to 6-membered rings), the foregoing "cycloalkenyl" (including, for example, 3- to 6-membered rings), the foregoing "aryl," the foregoing "heteroaryl," and/or other heterocyclyl. As long as the fused ring is non-aromatic as a ring, it may have an unsaturated bond in any ring. Examples thereof include pyrrolinyl (e.g., 1-pyrrolinyl, 2-pyrrolinyl, 3-pyrrolinyl), pyrrolidinyl (e.g., 1-pyrrolidinyl, 2-pyrrolidinyl, 3-pyrrolidinyl), pyrrolidinone, imidazolinyl (e.g., 1-imidazolinyl, 2-imidazolinyl, 4-imidazolinyl), imidazolidinyl (e.g., 1-imidazolidinyl, 2-imidazolidinyl, 4-imidazolidinyl), imidazolidinone, pyrazolinyl (e.g., 1-pyrazolinyl, 3-pyrazolinyl, 4-pyrazolinyl), pyrazolidinyl (e.g., 1-pyrazolidinyl, 3-pyrazolidinyl, 4-pyrazolidinyl), piperidinone, piperidino, piperidinyl (e.g., 2-piperidinyl, 3-piperidinyl, 4-piperidinyl), piperazinyl (e.g., 1-piperazinyl, 2-piperazinyl), morpholinyl (e.g., 2-morpholinyl, 3-morpholinyl), morpholino, tetrahydropyranyl(e.g., 2-tetrahydropyranyl, 3-tetrahydropyranyl, 4-tetrahydropyranyl), tetrahydrofuranyl (e.g., 2-tetrahydrofuranyl, 3-tetrahydrofuranyl), and the like.

As used herein, the term "aromatic heterocycle" encompass aromatic 5- to 8-membered rings containing one or more optionally-selected oxygen atoms, sulfur atoms, and/or nitrogen atoms in the ring, or rings in which two or more of them are fused. Monocyclic aromatic heterocycles encompass those rings derived from a 5- to 8-membered aromatic heterocycle that may contain one to four oxygen atoms, sulfur atoms, and/or nitrogen atoms in the ring. The monocyclic aromatic heterocycles may have a bond on any of substitutable position on the ring. The fused aromatic heterocycles encompass those rings in which a 5- to 8-membered monocyclic aromatic heterocycle that may contain one to four oxygen atoms, sulfur atoms, and/or nitrogen atoms in the ring are fused with a one to four 5- to 8-membered monocyclic aromatic hydrocarbon rings or other 5- to 8-membered monocyclic aromatic heterocycle. The fused aromatic heterocycles may have a bond on any of substitutable position on the ring. Examples thereof include 5- or 6-membered aromatic heterocycles. Examples of aromatic heterocycles include a pyrrole ring, a furan ring, a thiophene ring, an imidazole ring, a pyrazole ring, an isothiazole ring, an isoxazole ring, an oxazole ring, a thiazole ring, a pyridine ring, a pyrazine ring, a pyrimidine ring, a pyridazine ring, a tetrazole ring, an oxadiazole ring, a thiadiazole ring, an indolizine ring, an isoindole ring, an indole ring, an indazole ring, a purine ring, a quinolizine ring, an isoquinoline ring, a quinoline ring, a phthalazine ring, a naphthyridine ring, a quinazoline ring, a cinnoline ring, a pteridine ring, a carbazole ring, a phenanthridinyl ring, an acridine ring, a dibenzofurane ring, a benzimidazole ring, a benzisoxazole ring, a benzoxazole ring, a benzoxazole ring, a benzisothiazole ring, a benzothiazolyl ring, a benzofuran ring, a benzothiophene ring, a dibenzothiophene ring, and the like. Examples include a thiophene ring, a pyridine ring, a furan ring, a thiazole ring, an oxazole ring, and a pyrimidine ring.

As used herein, the term "non-aromatic heterocycle" encompasses non-aromatic 3- to 8-membered rings containing one or more optionally-selected oxygen atoms, sulfur atoms, and/or nitrogen atoms in the ring, or rings in which two or more of them are fused. In addition, such a non-aromatic heterocycle may be bridged via a C1-C4 alkyl chain. Moreover, such a non-aromatic heterocycle may be fused with the foregoing "aromatic hydrocarbon ring" (including, for example, 6-membered rings), the foregoing "non-aromatic hydrocarbon ring" (including, for example, 3- to 6-membered rings), the foregoing "aromatic heterocycle," and/or other "non-aromatic heterocycle." As long as the fused ring is a non-aromatic ring as a ring, it may have an unsaturated bond in any ring. Examples thereof include pyrroline ring, a pyrrolidine ring, a pyrrolidinone ring, an imidazoline ring, an imidazolidine ring, a pyrazoline ring, a pyrazolidine ring, a piperidinone ring, a piperidine ring, a piperazine ring, a morpholine ring, a tetrahydropyran ring, a tetrahydrofuran ring, a dihydropyran ring, a dihydrofuran ring, a benzodioxole ring, and the like. Examples include a dihydropyran ring, a tetrahydropyran ring, a dihydrofuran ring, and a tetrahydrofuran ring.

As used herein, the alkyl portion of "alkylcarbonyl" means the aforementioned "alkyl."

As used herein, the alkenyl portion of "alkenyloxy" and "alkenylcarbonyl" means the aforementioned "alkenyl."

As used herein, the cycloalkyl portion of "cycloalkylcarbonyl" and "cycloalkylsulfonyl" means the aforementioned "cycloalkyl."

As used herein, the cycloalkenyl portion of "cycloalkenylcarbonyl" means the aforementioned "cycloalkenyl."

As used herein, the aryl portion of "aryloxy," "arylcarbonyl," "aryloxycarbonyl," and "arylsulfonyl" means the aforementioned "aryl."

As used herein, the heteroaryl portion of "heteroaryloxy," "heteroarylcarbonyl," "heteroaryloxycarbonyl," and "heteroarylsulfonyl" means the aforementioned "heteroaryl."

As used herein, the heterocyclyl portion of "heterocyclyloxy," "heterocyclylcarbonyl," "heterocyclylsulfonyl," and "heterocyclyloxycarbonyl" means the aforementioned "heterocyclyl."

In the present specification, substituents of the "substituted or unsubstituted 6-membered aromatic hydrocarbon ring," "substituted or unsubstituted 5- or 6-membered aromatic heterocycle," "substituted or unsubstituted 3 to 7-membered non-aromatic hydrocarbon ring," "substituted or unsubstituted 3 to 7-membered non-aromatic heterocycle," "substituted or unsubstituted 5- or 6-membered non-aromatic hydrocarbon ring," "substituted or unsubstituted 5- or 6-membered non-aromatic heterocycle," "substituted or unsubstituted monocyclic aromatic hydrocarbon ring," "substituted or unsubstituted monocyclic aromatic heterocycle," "substituted or unsubstituted monocyclic non-aromatic hydrocarbon ring," "substituted or unsubstituted monocyclic non-aromatic heterocycle," "substituted or unsubstituted alkyl," "substituted or unsubstituted alkenyl," "substituted or unsubstituted alkynyl," "substituted or unsubstituted aryl," "substituted or unsubstituted cycloalkyl," "substituted or unsubstituted cycloalkenyl," "substituted or unsubstituted heteroaryl," "substituted or unsubstituted heterocyclyl," "substituted or unsubstituted acyl," "substituted or unsubstituted alkoxycarbonyl," "substituted or unsubstituted carbamoyl," "substituted or unsubstituted alkoxy," and "substituted or unsubstituted alkylsulfonyl" are selected from the group consisting of, for example, hydroxy, carboxy, halogen, halogenated alkyl (e.g., $CF_3$, $CH_2CF_3$, $CH_2CCl_3$) , nitro, nitroso, cyano, alkyl (e.g., methyl, ethyl, isopropyl, *tert*-butyl), alkenyl (e.g., vinyl), alkynyl (e.g., ethynyl), cycloalkyl (e.g., cyclopropyl, adamanthyl), cycloalkylalkyl (e.g., cyclohexylmethyl, adamanthylmethyl), cycloalkenyl (e.g., cyclopropenyl), aryl (e.g., phenyl, naphthyl), arylalkyl (e.g., benzyl, phenethyl), heteroaryl (e.g., pyridyl, furyl), heteroarylalkyl (e.g., pyridylmethyl), heterocyclyl (e.g., piperidyl), heterocyclylalkyl (e.g., morpholylmethyl), alkoxy (e.g., methoxy, ethoxy, propoxy, butoxy), halogenated alkyloxy (e.g., $OCF_3$), alkenyloxy (e.g., vinyloxy, allyloxy), aryloxy (e.g., phenyloxy), alkoxycarbonyl (e.g., methoxycarbonyl, ethoxycarbonyl, *tert*-butoxycarbonyl), aryl-alkyloxy (e.g., benzyloxy), unsubstituted amino, substituted amino [e.g., alkylamino (e.g., methylamino, ethylamino, dimethylamino), acylamino (e.g., acetylamino, benzoylamino), arylalkylamino (e.g., benzylamino, tritylamino), hydroxyamino], alkylaminoalkyl (e.g., di-ethylaminomethyl), alkylaminocarbonyl (e.g., N-methylaminocarbonyl, N-ethylaminocarbonyl, N-n-propylamino-carbonyl, N-isopropylaminocarbonyl), alkylcarbonyl (e.g., formyl, acetyl, propionyl), alkylsulfonyl (e.g., methylsulfonyl, ethylsulfonyl), heterocyclylsulfonyl (e.g., piperidinylsulfonyl), sulfamoyl, oxo, and the like. Substitution may occur with 1 to 4 of such substituents.

As used herein, the term "substituted or unsubstituted amino" encompasses amino that may be substituted at one or two positions with the "alkyl," the "cycloalkyl," the "aryl," the "heteroaryl," the "heterocyclyl," the "acyl," the "alkoxycarbonyl," the "alkylsulfonyl," the "arylsulfonyl," the "heteroarylsulfonyl," and/or the "heterocyclylsulfonyl." The substituents may further be substituted with a substituent described below. Examples of the substituents include alkyl, alkenyl, aryl, heteroaryl, alkylcarbonyl, arylcarbonyl, heteroarylcarbonyl, heterocyclylcarbonyl, alkoxycarbonyl, aryloxycarbonyl, heteroaryloxycarbonyl, heterocyclyloxycarbonyl, sulfamoyl, alkylsulfonyl, carbamoyl, cycloalkylsulfonyl, arylsulfonyl, heteroarylsulfonyl, heterocyclylsulfonyl, hydroxy, sulfonyl, sulfinyl, amino, halogen, cyano, alkoxy, carboxy, and the like. Examples of the "substituted or unsubstituted amino" include amino, methylamino, dimethylamino, ethylamino, diethylamino, ethylmethylamino, cyclohexylamino, benzylamino, acetylamino, benzoylamino, methoxycarbonylamino, methylsulfonylamino, tetrahydropyranylamino, tetrahydrofuranylamino, morpholinoamino, morpholinylamino, piperidinylamino, piperazinylamino, and the like. Examples include amino, methylamino, dimethylamino, ethylmethylamino, diethylamino, acetylamino, methylsulfonylamino, tetrahydropyranylamino, tetrahydrofuranylamino, morpholinoamino, piperidinylamino, and the like.

As used herein, the term "substituted or unsubstituted carbamoyl" encompasses substituted or unsubstituted aminocarbonyl in which the substituted or unsubstituted amino portion is the "substituted or unsubstituted amino." Examples of the "substituted or unsubstituted carbamoyl" include carbamoyl, N-methylcarbamoyl, N,N-dimethylcarbamoyl, N-ethyl-N-methylcarbamoyl, N,N-diethylcarbamoyl, N-n-propylaminocarbamoyl, N-isopropylcarbamoyl, N-morpholinocarbamoyl, N-tetrahydrofuranylcarbamoyl, N-piperidylcarbamoyl, N-tetrahydropyranylcarbamoyl, N-benzylcarbamoyl, N-acetylcarbamoyl, N-methylsulfonylcarbamoyl, N-(2,2,2-trifluoroethyl)carbamoyl, N-(2-hydroxy-1-methylethyl)-carbamoyl, and the like. Examples include carbamoyl, N-methylcarbamoyl, N,N-dimethylcarbamoyl, N-n-propylaminocarbamoyl, N-isopropylcarbamoyl, N-morpholinocarbamoyl, N-tetrahydrofuranylcarbamoyl, N-piperidylcarbamoyl, N-tetrahydropyranylcarbamoyl, N-methylsulfonylcarbamoyl, N-(2,2,2-trifluoroethyl)carbamoyl, N-(2-hydroxy-1-methylethyl)carbamoyl, and the like.

As used herein, the term "substituted or unsubstituted sulfamoyl" encompasses substituted or unsubstituted aminosulfonyl in which the substituted or unsubstituted amino portion is the "substituted or unsubstituted amino." Examples of the "substituted or unsubstituted sulfamoyl" include sulfamoyl, N-methylsulfamoyl, N,N-dimethylsulfamoyl, N-ethyl-N-methylsulfamoyl, N,N-diethylsulfamoyl, N-*n*-propylaminosulfamoyl, N-isopropylsulfamoyl, N-morpholinosulfamoyl, N-tetra-hydrofuranylsulfamoyl, N-piperidylsulfamoyl, N-tetra-hydropyranylsulfamoyl, N-benzylsulfamoyl, N-acetylsulfamoyl, N-methylsulfonylsulfamoyl, and the like. Examples include sulfamoyl, N-methylsulfamoyl, N,N-dimethylsulfamoyl, N-*n*-propylaminosulfamoyl, N-isopropylsulfamoyl, N-morpholinosulfamoyl, N-tetrahydrofuranyl-sulfamoyl, N-piperidylsulfamoyl, N-tetrahydropyranyl-sulfamoyl, N-methylsulfonylsulfamoyl, and the like.

Pharmaceutically acceptable salts of compounds of the present invention include the following salts.

Examples of basic salts thereof include: alkali metal salts such as sodium salts, potassium salts, and the like; alkaline-earth metal salts such as calcium salts, magnesium salts, and the like; ammonium salts; aliphatic amine salts such as trimethylamine salts, triethylamine salts, dicyclohexylamine salts, ethanolamine salts, diethanolamine salts, triethanolamine salts, procaine salts, meglumine salts, diethanolamine salts, ethylenediamine salts, and the like; aralkylamine salts such as N,N-dibenzylethylenediamine salts, benethamine salts, and the like; aromatic heterocyclic amine salts such as pyridine salts, picoline salts, quinoline salts, isoquinoline salts, and the like; quaternary ammonium salts such as tetramethylammonium salts, tetraethylammonium salts, benzyltrimethylammonium salts, benzyltriethylammonium salts, benzyltributylammonium salts, methyltrioctylammonium salts, tetrabutylammonium salts, and the like; basic amino acid salts such as arginine salts, lysine salts; and the like.

Examples of acidic salts include: inorganic acid salts such as hydrochloride, sulfate, nitrate, phosphate, carbonate, bicarbonate, perchlorate, and the like; organic acid salts such as acetate, propionate, lactate, maleate, fumarate, tartrate, malate, citrate, ascorbate, and the like; sulfonate such as methanesulfonate, isethionate, benzenesulfonate, p-toluenesulfonate; acidic amino acids salts such as aspartate, glutamate, and the like.

As a pharmaceutically acceptable prodrug of the present invention, any form known in the art can be adopted. A prodrug refers to a compound that, taking advantage of a metabolic machinery *in vivo,* does not exhibit a pharmaceutical effect or merely exhibits very low activity in its original form, but is modified so as to, when metabolized *in vivo*, thereby exhibit or increase pharmacological activity for the first time. Examples of prodrugs can include not only salts, solvates, and the like, but also esters, amides, and the like.

The term "solvate" means a solvate of a compound of the present invention, or a pharmaceutically acceptable salt thereof. Examples thereof include solvates formed with alcohol (e.g., ethanol), hydrates, and the like. Examples of hydrates can include monohydrate, dihydrate, and the like.

Moreover, one or more hydrogen, carbon, or other atoms in the compound of formula (I) may be replaced with isotopes of hydrogen, carbon, or other atoms respectively. The compound of formula (I) encompasses all of isotopically labeled compounds of the compound of formula (I). Such "isotopically labeling," "an isotopically labeled compound," and the like of the compound of formula (I) are each encompassed by the present invention, and are useful for studies on metabolized drug pharmacokinetics and studies on binding assay, and/or a diagnostic tool. Furthermore, they are also useful as medicaments.

Examples of isotopes that may be incorporated in the compound of formula (I) include hydrogen, carbon, nitrogen, oxygen, phosphorus, sulfur, fluorine, and chlorine, such as $^{2}H$ (D), $^{3}H$, $^{13}C$, $^{14}C$, $^{15}N$, $^{18}O$, $^{17}O$, $^{31}P$, $^{32}P$, $^{35}S$, $^{18}F$, and $^{36}Cl$ respectively. An isotopically labeled compound of the present invention can be prepared using a well-known method in the relevant technical field. For example, a deuterium ($^{2}H$ (D))-labeled compound of formula (I) can be synthesized by using a deuterated reagent, deuterium oxide, or deuterium. For example, a tritium-labeled compound of formula (I) can be prepared by introducing a tritium to a certain compound of formula (I), for example, through a catalytic dehalogenation reaction using a tritium. This method may comprise reacting with an appropriately-halogenated precursor of the compound of formula (I) with tritium gas in the presence of an appropriate catalyst, such as Pd/C, and in the presence or absent of a base. For another appropriate method of preparing a tritium-labeled compound, the document: Isotopes in the Physical and Biomedical Sciences, Vol. 1, Labeled Compounds (Part A), Chapter 6 (1987) can be referred to. A $^{14}C$-labeled compound can be prepared by using a raw material having $^{14}C$.

(Preferred Embodiments)

Preferred embodiments of the present invention are illustrated in (A-0) to (F-2') below. Each symbol is defined

the same as above. Compounds shown by combining the contents described in (A-0) with the contents described in (A-1) to (F-2') are encompassed.

(A-0)

Each substituent is defined as in the foregoing item (1), (1A), (1α), or (1β) unless specified otherwise.
In general formula (I):
[Formula 13]

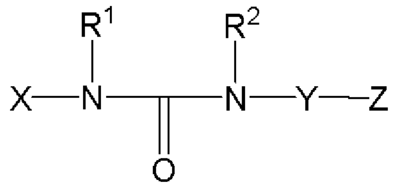

(I)

,

X includes groups represented by the following formulas:
[Formula 14]

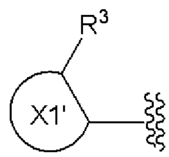

(X1)

or

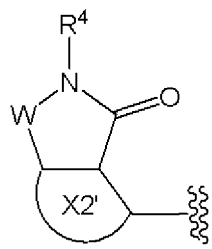

(X2)

.

Examples of X include groups represented by formula (X1).
In formula (X1), X1' includes substituted or unsubstituted aromatic hydrocarbon rings, a substituted or unsubstituted aromatic heterocycle, substituted or unsubstituted non-aromatic hydrocarbon rings, or substituted or unsubstituted non-aromatic heterocycles.
Examples of X1' include substituted or unsubstituted 6-membered aromatic hydrocarbon rings, substituted or unsubstituted 5- or 6-membered aromatic heterocycles, substituted or unsubstituted 3 to 7-membered non-aromatic hydrocarbon rings, or substituted or unsubstituted 3 to 7-membered non-aromatic heterocycles.
Examples of X1' include substituted or unsubstituted 6-membered aromatic hydrocarbon rings, substituted or unsubstituted 5- or 6-membered aromatic heterocycles, substituted or unsubstituted 3 to 7-membered non-aromatic hydrocarbon rings.
Examples of X1 include 6-membered aromatic hydrocarbon rings (benzene), 5- or 6-membered aromatic heterocycles (thiophene, pyridine, furan, thiazole, oxazole, pyrimidine, pyrazole), 5- or 6-membered non-aromatic hydrocarbon rings (cyclopentene, cyclohexane, cyclopentane).
In formula (X1), it is meant that a ring-constituting atom bound to $R^3$ (which ring-constituting atom is a carbon or nitrogen atom) and a ring-constituting atom bound to a nitrogen atom in formula (I) (which ring-constituting atom is a carbon or nitrogen atom) are adjacent to each other in (X1') ring. The same also applies in (X2).
Furthermore, X includes groups represented by formula (X2).
In formula (X2), X2' includes substituted or unsubstituted monocyclic aromatic hydrocarbon rings, substituted or unsubstituted monocyclic aromatic heterocycles, substituted or unsubstituted monocyclic non-aromatic hydrocarbon rings, or substituted or unsubstituted monocyclic non-aromatic heterocycles.
Examples of X2' include substituted or unsubstituted monocyclic aromatic hydrocarbon rings, or substituted or unsubstituted monocyclic aromatic heterocycles.
$R^1$ and $R^2$ include, each independently, hydrogen or substituted or unsubstituted alkyl.
Examples of $R^1$ and $R^2$ are both hydrogen.

$R^3$ includes carboxy, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, groups represented by the formula: -SO-$R^a$, groups represented by the formula: -SO-$R^a$, or groups represented by the formula: -S$R^a$

Examples of $R^3$ include carboxy, substituted or unsubstituted alkyl, substituted or unsubstituted aryl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, or substituted or unsubstituted amino.

Examples of $R^3$ include carboxy, substituted or unsubstituted alkyl, substituted or unsubstituted aryl, substituted or unsubstituted heterocyclyl, substituted or unsubstituted acyl, substituted or unsubstituted alkoxycarbonyl, substituted or unsubstituted carbamoyl, substituted or unsubstituted alkoxy, or substituted or unsubstituted amino.

Examples of $R^3$ include carboxy, substituted or unsubstituted alkyl (wherein a substituent includes alkyl, amino, or hydroxy), unsubstituted aryl, substituted heterocyclyl (wherein a substituent includes alkyl), substituted acyl (wherein a substituent includes heterocyclyl, alkyl, or aryl), unsubstituted alkoxycarbonyl, substituted carbamoyl (wherein a substituent includes alkyl or cycloalkyl), substituted or unsubstituted alkoxy (wherein a substituent includes halogen, aryl, amino, or carboxy), or substituted amino (wherein a substituent includes acyl).

In the above formula, $R^a$ includes substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl.

Examples of $R^a$ include substituted or unsubstituted alkyl, or substituted or unsubstituted amino.

$R^4$ includes hydrogen, cyano, substituted or unsubstituted alkyl, substituted or unsubstituted aryl, substituted or unsubstituted cycloalkyl, substituted or unsubstituted cycloalkenyl, substituted or unsubstituted heteroaryl, or substituted or unsubstituted heterocyclyl.

Examples of $R^4$ include hydrogen, or substituted or unsubstituted alkyl.

W includes groups represented by the formula: $-(CR^5R^6)_a-$, or groups represented by the formula: $-(CR^7=CR^8)-$.

$R^5$ to $R^8$ in the above formula include, each independently, hydrogen, halogen, cyano, hydroxy, carboxy, substituted or unsubstituted alkyl, substituted or unsubstituted alkoxy, substituted or unsubstituted acyl, substituted or unsubstituted carbamoyl, or substituted or unsubstituted amino.

In addition, a in the above formula includes 1 or 2.

Y includes groups represented by the following formulas:

[Formula 15]

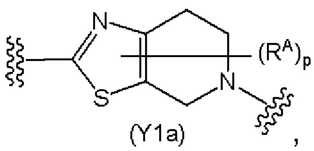
(Y1a) ,

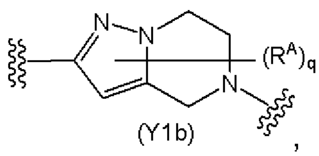
(Y1b) ,

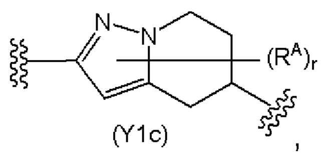
(Y1c) ,

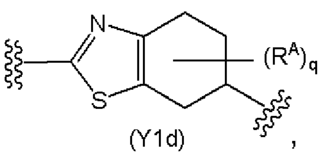
(Y1d) ,

[Formula 16]

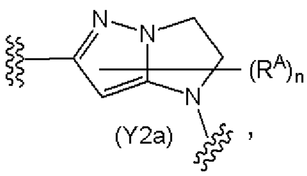
(Y2a),
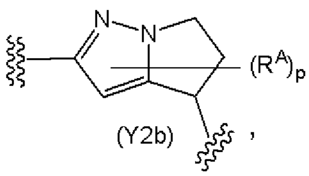
(Y2b),
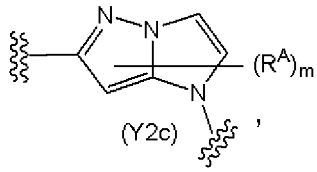
(Y2c),

1

[Formula 17]

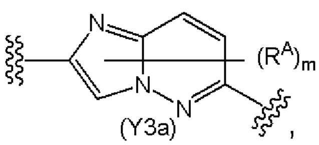
(Y3a),
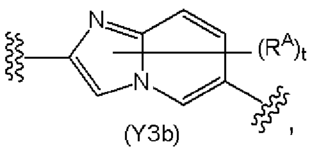
(Y3b),
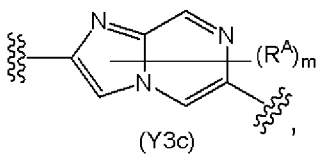
(Y3c),
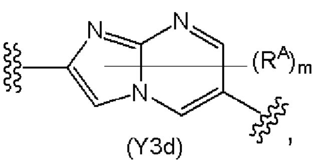
(Y3d),
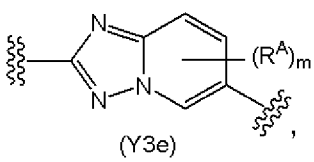
(Y3e),
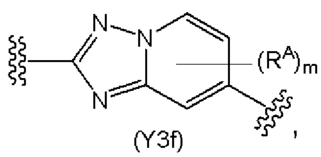
(Y3f),
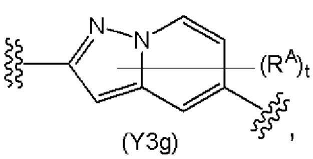
(Y3g),
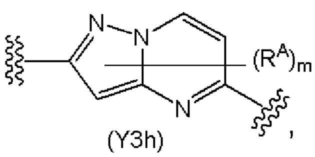
(Y3h),
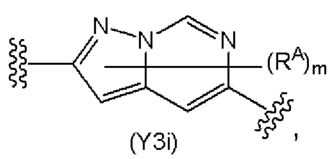
(Y3i),
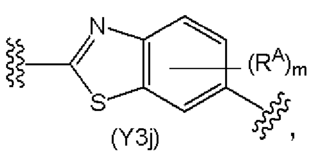
(Y3j),
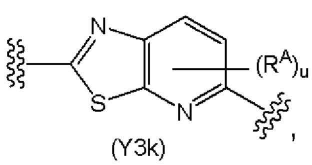
(Y3k),
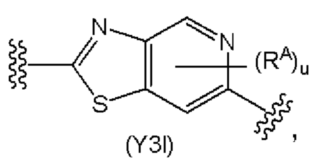
(Y3l),

[Formula 18]

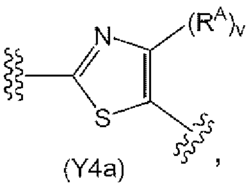
(Y4a),
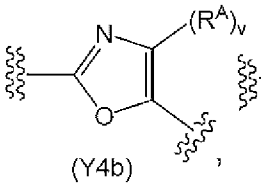
(Y4b),
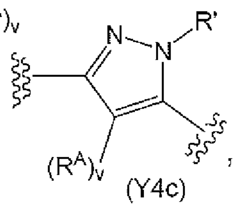
(Y4c),
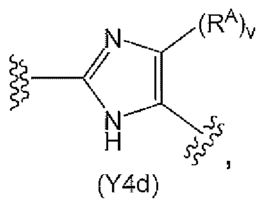
(Y4d),

[Formula 19]

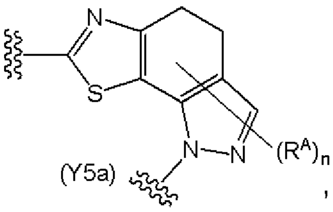
(Y5a),

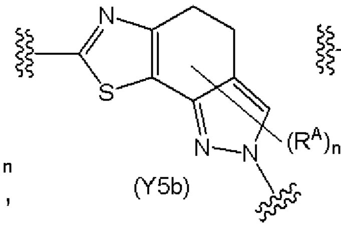
(Y5b),

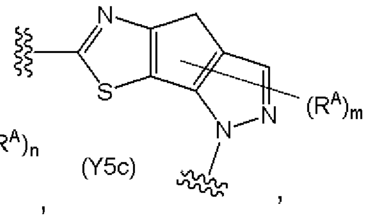
(Y5c),

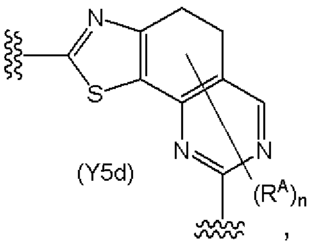
(Y5d),

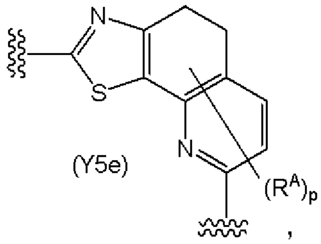
(Y5e),

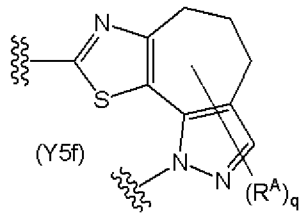
(Y5f),

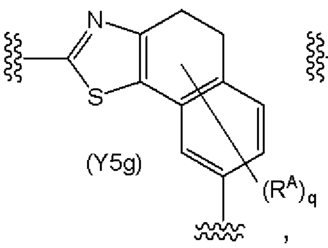
(Y5g),

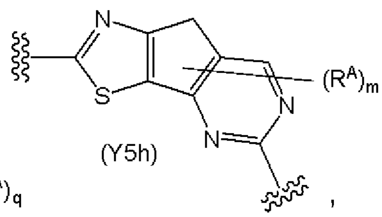
(Y5h),

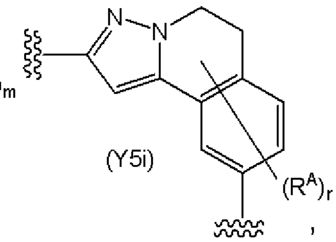
(Y5i),

[Formula 20]

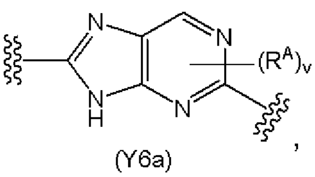
(Y6a),

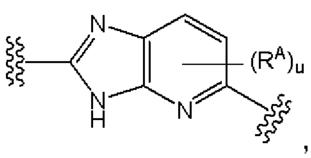
(Y6b),

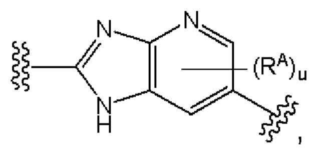
(Y6c),

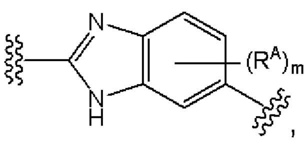
(Y6d)

[Formula 21]

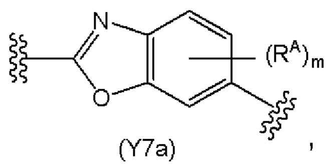
(Y7a) ,

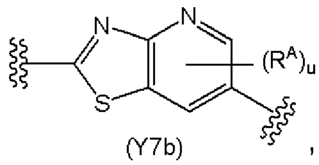
(Y7b) ,

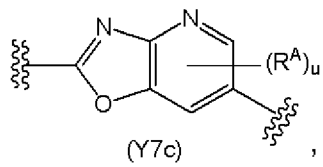
(Y7c) ,

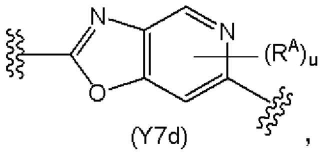
(Y7d) ,

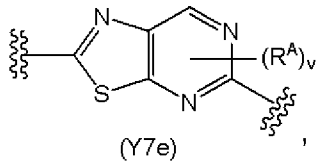
(Y7e) ,

[Formula 22]

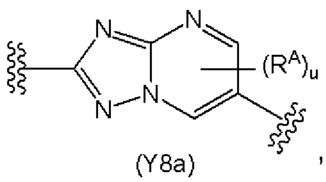
(Y8a) ,

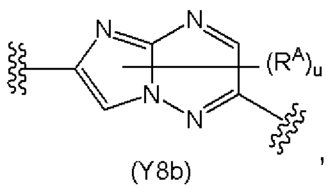
(Y8b) ,

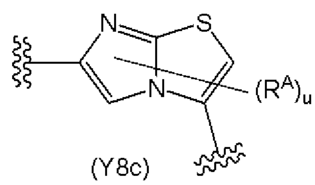
(Y8c)

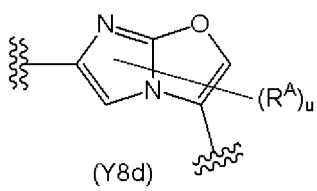
(Y8d) ,

[Formula 23]

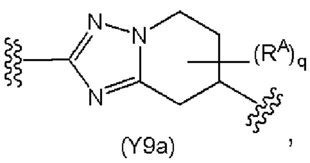
(Y9a) ,

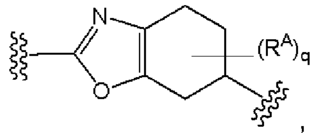
(Y9b) ,

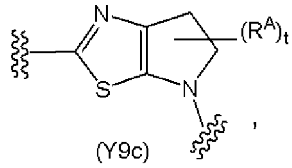
(Y9c) ,

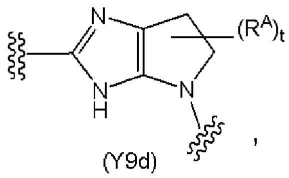
(Y9d) ,

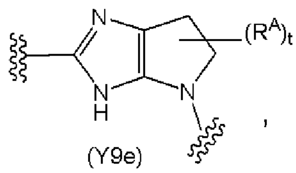
(Y9e) ,

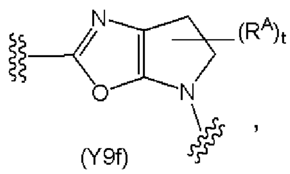
(Y9f) ,

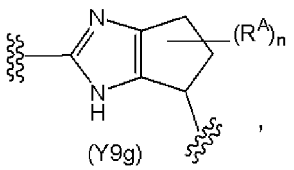
(Y9g) ,

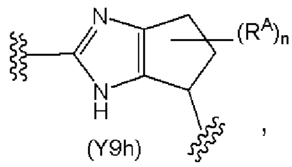
(Y9h) ,

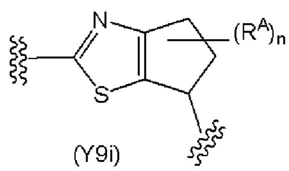
(Y9i)

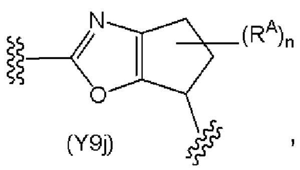
(Y9j) ,

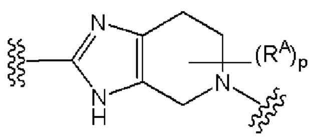
(Y9k) or

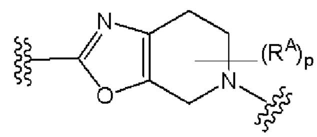
(Y9l) .

In the present specification, when a bond from substituent $R^A$ is described across two ring (for example, (Y1a), (Y1b), and the like), it indicates that substitution with $R^A$ may occur on either ring. Further, in the present specification, when substitution with a bond from substituent $R^A$ occurs on only one ring (for example, (Y1d), (Y3j), and the like), it indicates that substitution with $R^A$ may occur on the ring. The above-described respective groups defined as "Y" have bonds on the left and right sides of each ring, respectively. The left bond is to bind to a "N" atom of general formula (I), and the right bond is to bind to substituent "Z" of general formula (I).

Examples of Y include groups represented by formula (Y1a), groups represented by formula (Y1b), groups represented by formula (Y1c), or groups represented by formula (Y1d).

Examples of Y include groups represented by formula (Y1a), groups represented by formula (Y3a), groups represented by formula (Y3b), groups represented by formula (Y3j), groups represented by formula (Y3k), groups represented by formula (Y4a), groups represented by formula (Y5a), or groups represented by formula (Y6d).

Examples of Y include groups represented by formula (Y1a).

Examples of Y include groups represented by formula (Y1d).

Examples of Y include groups represented by formula (Y2a).

Examples of Y include groups represented by formula (Y3a).

$R^A$ in the above formulas is, each independently, halogen, cyano, carboxy, hydroxy, nitro, substituted or unsubstituted alkoxy, substituted or unsubstituted alkyl, substituted or unsubstituted alkenyl, substituted or unsubstituted alkynyl, substituted or unsubstituted amino, substituted or unsubstituted carbamoyl, substituted or unsubstituted acyl, substituted or unsubstituted aryl, substituted or unsubstituted cycloalkyl, substituted or unsubstituted cycloalkenyl, substituted or unsubstituted heteroaryl, substituted or unsubstituted heterocyclyl, groups represented by the formula: $-SO-R^{a'}$, groups represented by the formula: $-SO_2-R^{a'}$, or groups represented by the formula: $-SR^{a'}$;

$R^{a'}$ includes substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl.

$R^A$ in the above formulas can be bound to any substitutable position on the ring.

R' in the above formulas includes hydrogen or substituted or unsubstituted alkyl.

In the above formulas,

m includes integers from 0 to 3,
n includes integers from 0 to 5,
p includes integers from 0 to 6,
q includes integers from 0 to 7,
r includes integers from 0 to 8,
t includes integers from 0 to 4,
u includes integers from 0 to 2, and
v includes 0 or 1.

In the above formula, for example, p is 0.

Z includes substituted or unsubstituted cycloalkyl, substituted or unsubstituted cycloalkenyl, substituted or unsubstituted aryl, substituted or unsubstituted heteroaryl, or substituted or unsubstituted heterocyclyl.

Examples of Z include substituted or unsubstituted aryl, or substituted or unsubstituted heteroaryl.

Examples of Z include substituted or unsubstituted phenyl, substituted or unsubstituted pyrimidyl, substituted or unsubstituted thiazolyl, substituted or unsubstituted oxazolyl, substituted or unsubstituted pyridyl, or substituted or unsubstituted triazyl.

However, when Y is a group represented by formula (Y4a) or a group represented by formula (Y4b), $R^A$ is not substituted or unsubstituted aryl;
when Y is a group represented by formula (Y4a), $R^3$ is not methyl or methoxy;
when Y is a group represented by formula (Y5a), $R^3$ is not carboxy, ethoxycarbonyl, phenyl, or alkyl substituted with amino;
when Y is a group represented by formula (Y5c), $R^3$ is not phenyl, or alkyl substituted with substituted amino or imidazolidinone; and
when Y is a group represented by formula (Y5d), $R^3$ is not phenyl or alkyl substituted with amino.

In addition, the present invention is not the compound shown below:

[Formula 24]

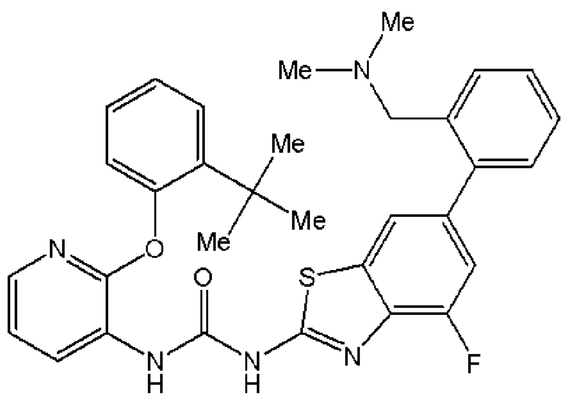

(A-1)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y1a) or (Y1d);
p is 0;
q is 0; and
Z is substituted or unsubstituted aryl, or substituted or unsubstituted heteroaryl (each substituent is defined the same as the foregoing item (1), (1A), (1$\alpha$), or (1$\beta$) unless specified otherwise).

(A-1')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y1a) or (Y1d);
p is 0;
q is 0; and
Z is substituted or unsubstituted heteroaryl (each substituent is defined the same as the foregoing item (1), (1A), (1α), or (1β) unless specified otherwise).

(A-2)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y1a);
p is 0; and
Z is substituted or unsubstituted aryl, or substituted or unsubstituted heteroaryl (each substituent is defined the same as the foregoing item (1), (1A), (1α), or (1β) unless specified otherwise).

(A-2')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y1a);
p is 0; and
Z is substituted or unsubstituted heteroaryl (each substituent is defined the same as the foregoing item (1), (1A), (1α), or (1β) unless specified otherwise).

(A-3)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y1b) or (Y1c);
q is 0;

r is 0; and
Z is substituted or unsubstituted aryl, or substituted or unsubstituted heteroaryl (each substituent is defined the same as the foregoing item (1), (1A), (1α), or (1β) unless specified otherwise).

(A-3')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y1b) or (Y1c);
q is 0;
r is 0; and
Z is substituted or unsubstituted heteroaryl (each substituent is defined the same as the foregoing item (1), (1A), (1α), or (1β) unless specified otherwise).

(A-4)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y1b);
q is 0; and
Z is substituted or unsubstituted aryl, or substituted or unsubstituted heteroaryl (each substituent is defined the same as the foregoing item (1), (1A), (1α), or (1β) unless specified otherwise).

(A-4')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y1b);
q is 0; and
Z is substituted or unsubstituted heteroaryl (each substituent is defined the same as the foregoing item (1), (1A), (1α), or (1β) unless specified otherwise).

(A-5)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y1a) or (Y1d);
p is 0;
q is 0; and
Z is substituted or unsubstituted aryl, or substituted or unsubstituted heteroaryl (each substituent is defined the same as the foregoing item (1), (1A), (1α), or (1β) unless specified otherwise).

(A-5')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y1a) or (Y1d);
p is 0;
q is 0; and
Z is substituted or unsubstituted heteroaryl (each substituent is defined the same as the foregoing item (1), (1A), (1α), or (1β) unless specified otherwise).

(A-6)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y1a);
p is 0; and
Z is substituted or unsubstituted aryl, or substituted or unsubstituted heteroaryl (each substituent is defined the same as the foregoing item (1), (1A), (1α), or (1β) unless specified otherwise).

(A-6')

In general formula (I),
$R^1$ and $R^2$ are both hydrogen;

X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y1a);
p is 0; and
Z is substituted or unsubstituted heteroaryl (each substituent is defined the same as the foregoing item (1), (1A), (1α), or (1β) unless specified otherwise).

(A-7)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y1d);
p is 0; and
Z is substituted or unsubstituted aryl, or substituted or unsubstituted heteroaryl (each substituent is defined the same as the foregoing item (1), (1A), (1α), or (1β) unless specified otherwise).

(A-7')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y1d);
p is 0; and
Z is substituted or unsubstituted heteroaryl (each substituent is defined the same as the foregoing item (1), (1A), (1α), or (1β) unless specified otherwise).

(A-8)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or

a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y1d);
p is 0; and
Z is substituted or unsubstituted aryl, or substituted or unsubstituted heteroaryl (each substituent is defined the same as the foregoing item (1), (1A), (1α), or (1β) unless specified otherwise).

(A-8')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y1d);
p is 0; and
Z is substituted or unsubstituted heteroaryl (each substituent is defined the same as the foregoing item (1), (1A), (1α), or (1β) unless specified otherwise).

(B-1)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y2a) or (Y2c);
m is 0;
n is 0; and
Z is substituted or unsubstituted aryl, or substituted or unsubstituted heteroaryl (each substituent is defined the same as the foregoing item (1), (1A), (1α), or (1β) unless specified otherwise).

(B-1')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y2a) or (Y2c);
m is 0;
n is 0; and
Z is substituted or unsubstituted heteroaryl (each substituent is defined the same as the foregoing item (1), (1A), (1α),

or (1β) unless specified otherwise).

(B-2)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y2a) or (Y2c);
m is 0;
n is 0; and
Z is substituted or unsubstituted aryl, or substituted or unsubstituted heteroaryl (each substituent is defined the same as the foregoing item (1), (1A), (1α), or (1β) unless specified otherwise).

(B-2')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y2a) or (Y2c);
m is 0;
n is 0; and
Z is substituted or unsubstituted heteroaryl (each substituent is defined the same as the foregoing item (1), (1A), (1α), or (1β) unless specified otherwise).

(B-3)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y2a);
m is 0;
n is 0; and
Z is substituted or unsubstituted aryl, or substituted or unsubstituted heteroaryl (each substituent is defined the same

as the foregoing item (1), (1A), (1α), or (1β) unless specified otherwise).

(B-3')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y2a);
m is 0;
n is 0; and
Z is substituted or unsubstituted heteroaryl (each substituent is defined the same as the foregoing item (1), (1A), (1α), or (1β) unless specified otherwise).

(B-4)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y2a);
m is 0;
n is 0; and
Z is substituted or unsubstituted aryl, or substituted or unsubstituted heteroaryl (each substituent is defined the same as the foregoing item (1), (1A), (1α), or (1β) unless specified otherwise).

(B-4')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y2a);
m is 0;
n is 0; and
Z is substituted or unsubstituted heteroaryl (each substituent is defined the same as the foregoing item (1), (1A), (1α),

or (1β) unless specified otherwise).

(C-1)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y3a);
m is 0; and
Z is substituted or unsubstituted aryl, or substituted or unsubstituted heteroaryl (each substituent is defined the same as the foregoing item (1), (1A), (1α), or (1β) unless specified otherwise).

(C-1')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y3a);
m is 0; and
Z is substituted or unsubstituted heteroaryl (each substituent is defined the same as the foregoing item (1), (1A), (1α), or (1β) unless specified otherwise).

(C-2)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y3a);
m is 0; and
Z is substituted or unsubstituted aryl, or substituted or unsubstituted heteroaryl (each substituent is defined the same as the foregoing item (1), (1A), (1α), or (1β) unless specified otherwise).

(C-2')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y3a);
m is 0; and
Z is substituted or unsubstituted heteroaryl (each substituent is defined the same as the foregoing item (1), (1A), (1α), or (1β) unless specified otherwise).

(C-3)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y3b), (Y3c), (Y3d), (Y3e), (Y3f), or (Y3j);
t is 0;
m is 0; and
Z is substituted or unsubstituted aryl, or substituted or unsubstituted heteroaryl (each substituent is defined the same as the foregoing item (1), (1A), (1α), or (1β) unless specified otherwise).

(C-3')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y3b), (Y3c), (Y3d), (Y3e), (Y3f), or (Y3j);
t is 0;
m is 0; and
Z is substituted or unsubstituted heteroaryl (each substituent is defined the same as the foregoing item (1), (1A), (1α), or (1β) unless specified otherwise).

(C-4)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or

a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y3b), (Y3c), (Y3d), (Y3e), (Y3f), or (Y3j);
t is 0;
m is 0; and
Z is substituted or unsubstituted aryl, or substituted or unsubstituted heteroaryl (each substituent is defined the same as the foregoing item (1), (1A), (1α), or (1β) unless specified otherwise).

(C-4')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y3b), (Y3c), (Y3d), (Y3e), (Y3f), or (Y3j);
t is 0;
m is 0; and
Z is substituted or unsubstituted heteroaryl (each substituent is defined the same as the foregoing item (1), (1A), (1α), or (1β) unless specified otherwise).

(C-5)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y3a), (Y3b), (Y3c), (Y3d), (Y3e), (Y3f), or (Y3j);
t is 0;
m is 0; and
Z is substituted or unsubstituted aryl, or substituted or unsubstituted heteroaryl (each substituent is defined the same as the foregoing item (1), (1A), (1α), or (1β) unless specified otherwise).

(C-5')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y3a), (Y3b), (Y3c), (Y3d), (Y3e), (Y3f), or (Y3j);
t is 0;

m is 0; and
Z is substituted or unsubstituted heteroaryl (each substituent is defined the same as the foregoing item (1), (1A), (1α), or (1β) unless specified otherwise).

(C-6)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y3a), (Y3b), (Y3c), (Y3d), (Y3e), (Y3f), or (Y3j);
t is 0;
m is 0; and
Z is substituted or unsubstituted aryl, or substituted or unsubstituted heteroaryl (each substituent is defined the same as the foregoing item (1), (1A), (1α), or (1β) unless specified otherwise).

(C-6')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y3a), (Y3b), (Y3c), (Y3d), (Y3e), (Y3f), or (Y3j);
t is 0;
m is 0; and
Z is substituted or unsubstituted heteroaryl (each substituent is defined the same as the foregoing item (1), (1A), (1α), or (1β) unless specified otherwise).

(D-1)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted heterocyclyl, substituted or unsubstituted alkoxycarbonyl, or substituted or unsubstituted carbamoyl;
Y is a group represented by formula (Y4a), (Y4b), (Y4c), or (Y4d);
v is 0 or 1;
$R^A$ is halogen, cyano, carboxy, hydroxy, nitro, substituted or unsubstituted alkoxy, or substituted or unsubstituted alkyl;

R' is hydrogen or substituted or unsubstituted alkyl; and
Z is substituted or unsubstituted aryl, or substituted or unsubstituted heteroaryl (each substituent is defined the same as the foregoing item (1), (1A), (1α), or (1β) unless specified otherwise).

(D-1')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted heterocyclyl, substituted or unsubstituted alkoxycarbonyl, or substituted or unsubstituted carbamoyl;
Y is a group represented by formula (Y4a), (Y4b), (Y4c), or (Y4d);
v is 0 or 1;
$R^A$ is halogen, cyano, carboxy, hydroxy, nitro, substituted or unsubstituted alkoxy, or substituted or unsubstituted alkyl;
R' is hydrogen or substituted or unsubstituted alkyl; and
Z is substituted or unsubstituted heteroaryl (each substituent is defined the same as the foregoing item (1), (1A), (1α), or (1β) unless specified otherwise).

(D-2)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y4a), (Y4b), (Y4c), or (Y4d);
v is 0 or 1;
$R^A$ is halogen, cyano, carboxy, hydroxy, nitro, substituted or unsubstituted alkoxy, or substituted or unsubstituted alkyl;
R' is hydrogen or substituted or unsubstituted alkyl; and
Z is substituted or unsubstituted aryl, or substituted or unsubstituted heteroaryl (each substituent is defined the same as the foregoing item (1), (1A), (1α), or (1β) unless specified otherwise).

(D-2')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y4a), (Y4b), (Y4c), or (Y4d);
v is 0 or 1;
$R^A$ is halogen, cyano, carboxy, hydroxy, nitro, substituted or unsubstituted alkoxy, or substituted or unsubstituted alkyl;

R' is hydrogen or substituted or unsubstituted alkyl; and
Z is substituted or unsubstituted heteroaryl (each substituent is defined the same as the foregoing item (1), (1A), (1α), or (1β) unless specified otherwise).

(E-1)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkoxy, substituted or unsubstituted heterocyclyl, or substituted or unsubstituted carbamoyl;
Y is a group represented by formula (Y5a), (Y5b), (Y5c), (Y5d), (Y5e), (Y5g), (Y5h), or (Y5i);
$R^A$ is substituted or unsubstituted aryl, substituted or unsubstituted cycloalkyl, substituted or unsubstituted cycloalkenyl, substituted or unsubstituted heteroaryl, or substituted or unsubstituted heterocyclyl;
n is 0 or 1;
m is 0 or 1;
q is 0 or 1;
r is 0 or 1; and
Z is substituted or unsubstituted aryl, or substituted or unsubstituted heteroaryl (each substituent is defined the same as the foregoing item (1), (1A), (1α), or (1β) unless specified otherwise).

(E-1')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkoxy, substituted or unsubstituted heterocyclyl, or substituted or unsubstituted carbamoyl;
Y is a group represented by formula (Y5a), (Y5b), (Y5c), (Y5d), (Y5e), (Y5g), (Y5h), or (Y5i);
$R^A$ is substituted or unsubstituted aryl, substituted or unsubstituted cycloalkyl, substituted or unsubstituted cycloalkenyl, substituted or unsubstituted heteroaryl, or substituted or unsubstituted heterocyclyl;
n is 0 or 1;
m is 0 or 1;
q is 0 or 1;
r is 0 or 1; and
Z is substituted or unsubstituted heteroaryl (each substituent is defined the same as the foregoing item (1), (1A), (1α), or (1β) unless specified otherwise).

(E-2)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkenyl, substituted or unsubstituted alkynyl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y5a), (Y5b), (Y5c), (Y5d), (Y5e), (Y5g), (Y5h), or (Y5i);
$R^A$ is substituted or unsubstituted aryl, substituted or unsubstituted cycloalkyl, substituted or unsubstituted cycloalkenyl,

substituted or unsubstituted heteroaryl, or substituted or unsubstituted heterocyclyl;
n is 0 or 1;
m is 0 or 1;
q is 0 or 1;
r is 0 or 1; and
Z is substituted or unsubstituted aryl, or substituted or unsubstituted heteroaryl (each substituent is defined the same as the foregoing item (1), (1A), (1α), or (1β) unless specified otherwise).

(E-2')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkenyl, substituted or unsubstituted alkynyl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y5a), (Y5b), (Y5c), (Y5d), (Y5e), (Y5g), (Y5h), or (Y5i);
$R^A$ is substituted or unsubstituted aryl, substituted or unsubstituted cycloalkyl, substituted or unsubstituted cycloalkenyl, substituted or unsubstituted heteroaryl, or substituted or unsubstituted heterocyclyl;
n is 0 or 1;
m is 0 or 1;
q is 0 or 1;
r is 0 or 1; and
Z is substituted or unsubstituted heteroaryl (each substituent is defined the same as the foregoing item (1), (1A), (1α), or (1β) unless specified otherwise).

(F-1)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;
Y is a group represented by formula (Y7a), (Y7c), or (Y7d);
m is 0;
u is 0; and
Z is substituted or unsubstituted aryl, or substituted or unsubstituted heteroaryl (each substituent is defined the same as the foregoing item (1), (1A), (1α), or (1β) unless specified otherwise).

(F-1')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted acyl, substituted or unsubstituted amino, substituted or unsubstituted alkyl, substituted or unsubstituted alkoxy, substituted or unsubstituted heterocyclyl, substituted or unsubstituted alkoxycarbonyl, or substituted or unsubstituted carbamoyl;

Y is a group represented by formula (Y7a), (Y7c), or (Y7d);
m is 0;
u is 0; and
Z is substituted or unsubstituted heteroaryl (each substituent is defined the same as the foregoing item (1), (1A), (1α), or (1β) unless specified otherwise).

(F-2)

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y7a), (Y7c), or (Y7d);
m is 0;
u is 0; and
Z is substituted or unsubstituted aryl, or substituted or unsubstituted heteroaryl (each substituent is defined the same as the foregoing item (1), (1A), (1α), or (1β) unless specified otherwise).

(F-2')

In general formula (I),
$R^1$ and $R^2$ are both hydrogen;
X is a group represented by formula (X1);
X1' is a substituted or unsubstituted 6-membered aromatic hydrocarbon ring, a substituted or unsubstituted 5- or 6-membered aromatic heterocycle, or a substituted or unsubstituted 5- or 6-membered non-aromatic hydrocarbon ring;
$R^3$ is substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-SO-R^a$, a group represented by the formula: $-SO_2-R^a$, or a group represented by the formula: $-SR^a$;
$R^a$ is substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
Y is a group represented by formula (Y7a), (Y7c), or (Y7d);
m is 0;
u is 0; and
Z is substituted or unsubstituted heteroaryl (each substituent is defined the same as the foregoing item (1), (1A), (1α), or (1β) unless specified otherwise).
Preferred embodiments of the present invention are further illustrated in (G-1) below. Each symbol is defined the same as above.

(G-1)

Each substituent is defined the same as the foregoing item (1), (1A), (1α), or (1β) unless specified otherwise.
In general formula (I):
[Formula 25]

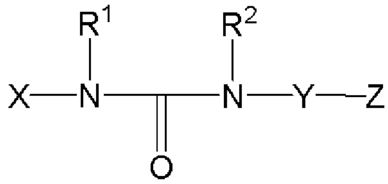

(I)

,

X includes groups represented by the following formulas:

[Formula 26]

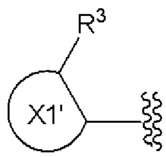

(X1)

or

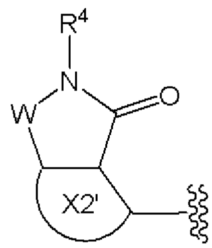

(X2)

.

Examples of X include groups represented by formula (X1).

In formula (X1), X1' includes substituted or unsubstituted aromatic hydrocarbon rings, substituted or unsubstituted aromatic heterocycles, substituted or unsubstituted non-aromatic hydrocarbon rings, or substituted or unsubstituted non-aromatic heterocycles.

Examples of X1' include substituted or unsubstituted 6-membered aromatic hydrocarbon rings, substituted or unsubstituted 5- or 6-membered aromatic heterocycles, substituted or unsubstituted 3- to 7-membered non-aromatic hydrocarbon rings, or substituted or unsubstituted 3- to 7-membered non-aromatic heterocycles.

Examples of X1' include substituted or unsubstituted 6-membered aromatic hydrocarbon rings, substituted or unsubstituted 5- or 6-membered aromatic heterocycle, substituted or unsubstituted 5- or 6-membered non-aromatic hydrocarbon rings, or substituted or unsubstituted 5- or 6-membered non-aromatic heterocycles.

Examples of X1' include substituted or unsubstituted 6-membered aromatic hydrocarbon rings (benzene), substituted or unsubstituted 5- or 6-membered aromatic heterocycles (thiophene, pyridine, furan, thiazole, oxazole, pyrimidine, and pyrazole), substituted or unsubstituted 5- or 6-membered non-aromatic hydrocarbon rings (cyclopentene, cyclohexane, and cyclopentane), or substituted or unsubstituted 5-or 6-membered non-aromatic heterocycles (dihydropyran, tetrahydropyran, dihydrofuran, tetrahydrofuran, and pyrrolidine).

Here, substituents of the substituted 6-membered aromatic hydrocarbon ring, substituted 5- or 6-membered aromatic heterocycle, substituted 3- to 7-membered non-aromatic hydrocarbon ring, or substituted 3- to 7-membered non-aromatic heterocycle as X1' include halogen, C1-C3 alkyl, halogenated C1-C3 alkyl, or C1-C3 alkoxy.

In formula (X1), it is meant that a ring-constituting atom bound to $R^3$ (which ring-constituting atom is a carbon or nitrogen atom) and a ring-constituting atom bound to a nitrogen atom in formula (I) (which ring-constituting atom is a carbon or nitrogen atom) are adjacent to each other in (X1') ring. The same applies in (X2).

Furthermore, X includes groups represented by formula (X2).

In formula (X2), X2' includes substituted or unsubstituted monocyclic aromatic hydrocarbon rings, substituted or unsubstituted monocyclic aromatic heterocycles, substituted or unsubstituted monocyclic non-aromatic hydrocarbon rings, or substituted or unsubstituted monocyclic non-aromatic heterocycles.

Examples of X2' include substituted or unsubstituted monocyclic aromatic hydrocarbon rings, or substituted or unsubstituted monocyclic aromatic heterocycles.

For example, X2' is a substituted or unsubstituted monocyclic aromatic hydrocarbon ring (benzene).

$R^1$ and $R^2$ include, each independently, hydrogen or substituted or unsubstituted alkyl.

Examples of $R^1$ and $R^2$ include, each independently, hydrogen or substituted or unsubstituted C1-C6 alkyl.

For example, $R^1$ and $R^2$ are both hydrogen.

$R^3$ is carboxy, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-C(-R^a)=N-OH$, a group represented by the formula: $-SO-R^a$, a group represented by the formula: $-SO_2-R^a$, or a group represented by the formula: $-SR^a$; $R^a$ includes hydrogen, substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl.

Examples of $R^3$ include carboxy, substituted or unsubstituted alkyl, substituted or unsubstituted aryl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, substituted or unsubstituted alkylsulfonyl, substituted or unsubstituted sulfamoyl, or groups represented by the formula: $-C(-R^a)=N-OH$.

Examples of $R^3$ include carboxy, substituted or unsubstituted C1-C6 alkyl, substituted or unsubstituted aryl (phenyl), substituted or unsubstituted heteroaryl, substituted or unsubstituted heterocyclyl (tetrahydropyranyl, tetrahydrofuranyl, pyrrolidinyl, or piperazinyl), substituted or unsubstituted acyl (C1-C6 alkylcarbonyl, arylcarbonyl (phenylcarbonyl), heterocyclylcarbonyl (piperidinocarbonyl, or morpholinocarbonyl)), substituted or unsubstituted C1-C6 alkoxycarbonyl, substituted or unsubstituted carbamoyl, substituted or unsubstituted C1-C6 alkoxy, substituted or unsubstituted amino, substituted or unsubstituted C1-C6 alkylsulfonyl, substituted or unsubstituted sulfamoyl, or a group represented by the formula: $-C(-CH_3)=N-OH$.

Examples of $R^3$ include substituted or unsubstituted carbamoyl, substituted or unsubstituted amino, or substituted or unsubstituted C1-C6 alkoxy.

Examples of $R^3$ include unsubstituted carbamoyl, or carbamoyl substituted at one or more positions with a substituent(s) selected from substituted or unsubstituted C1-C6 alkyl (wherein substituents thereof include halogen, hydroxy, cyano, and the like), substituted or unsubstituted C1-C6 alkenyl (wherein substituents thereof include halogen, hydroxy, cyano, and the like), substituted or unsubstituted C1-C6 alkynyl (wherein substituents thereof include halogen, hydroxy, cyano, and the like), substituted or unsubstituted C3-C6 cycloalkyl (wherein substituents thereof include halogen, hydroxy, cyano, and the like), and substituted or unsubstituted heterocyclyl (tetrahydrofuranyl, tetrahydropyranyl, morpholino, morpholinyl, piperazinyl, pyrrolidinyl, or piperidinyl).

Examples of $R^3$ include unsubstituted amino, or amino substituted at one or more positions with a substituent (s) selected from substituted or unsubstituted C1-C6 alkyl (wherein substituents thereof include halogen, hydroxy, cyano, and the like), substituted or unsubstituted alkenyl (wherein substituents thereof include halogen, hydroxy, cyano, and the like), substituted or unsubstituted alkynyl (wherein substituents thereof include halogen, hydroxy, cyano, and the like), substituted or unsubstituted C3-C6 cycloalkyl (wherein substituents thereof include halogen, hydroxy, cyano, and the like), and substituted or unsubstituted heterocyclyl (for example, tetrahydrofuranyl, tetrahydropyranyl, morpholino, morpholinyl, piperazinyl, pyrrolidinyl, and piperidinyl).

Examples of $R^3$ include unsubstituted C1-C6 alkoxy, or C1-C6 alkoxy substituted at one or more positions with a substituent(s) selected from halogen, hydroxy, cyano, substituted or unsubstituted C3-C6 cycloalkyl (wherein substituents thereof include halogen, hydroxy, cyano, and the like), or substituted or unsubstituted heterocyclyl(tetrahydrofuranyl, tetrahydropyranyl, morpholino, morpholinyl, piperazinyl, pyrrolidinyl, or piperidinyl).

Examples of $R^a$ include hydrogen, substituted or unsubstituted C1-C6 alkyl, or substituted or unsubstituted amino.

$R^4$ includes hydrogen, cyano, substituted or unsubstituted alkyl, substituted or unsubstituted aryl, substituted or unsubstituted cycloalkyl, substituted or unsubstituted cycloalkenyl, substituted or unsubstituted heteroaryl, or substituted or unsubstituted heterocyclyl.

Examples of $R^4$ include hydrogen, or substituted or unsubstituted C1-C6 alkyl.

W includes groups represented by the formula: $-(CR^5R^6)_a-$ or groups represented by the formula: $-(CR^7=CR^8)-$.

For example, W includes groups represented by the formula: $-(CR^5R^6)_a-$.

$R^5$ to $R^8$ in the above formulas include, each independently, hydrogen, halogen, cyano, hydroxy, carboxy, substituted or unsubstituted alkyl, substituted or unsubstituted alkoxy, substituted or unsubstituted acyl, substituted or unsubstituted carbamoyl, or substituted or unsubstituted amino.

Examples of $R^5$ to $R^8$ include hydrogen.

Further, a in the above formulas includes 1 or 2.

Y includes groups represented by the following formulas:

[Formula 27]

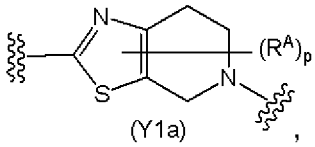
(Y1a),
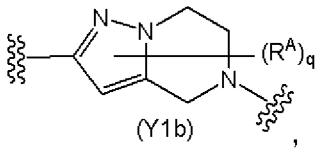
(Y1b),
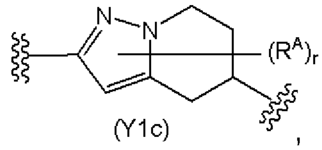
(Y1c),
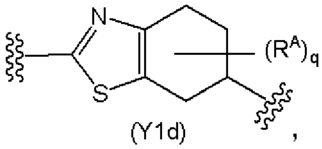
(Y1d),

[Formula 28]

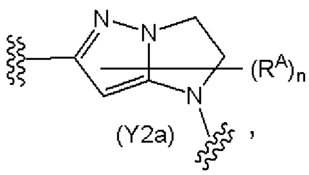
(Y2a),
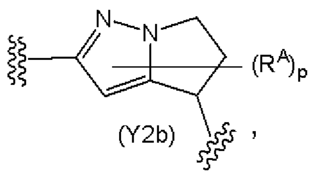
(Y2b),
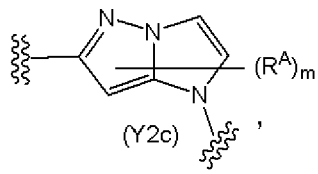
(Y2c),

[Formula 29]

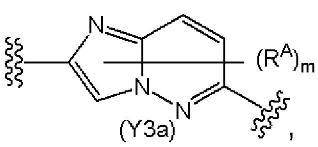
(Y3a),
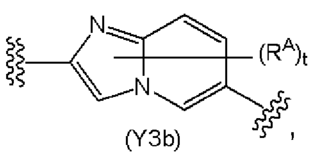
(Y3b),
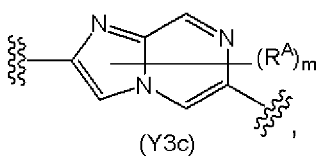
(Y3c),
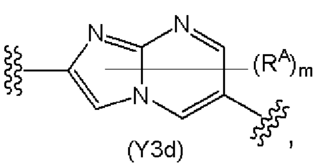
(Y3d),
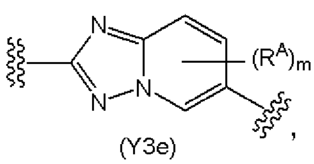
(Y3e),
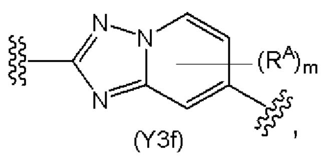
(Y3f),
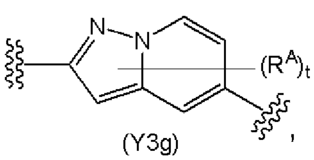
(Y3g),
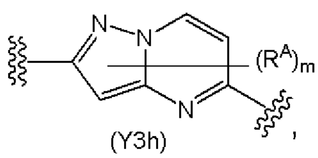
(Y3h),
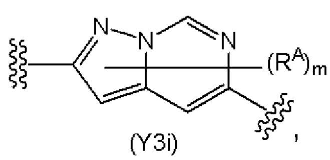
(Y3i),
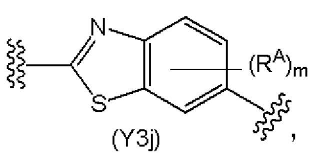
(Y3j),
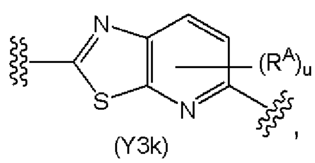
(Y3k),
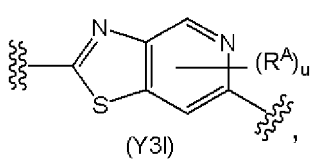
(Y3l),

[Formula 30]

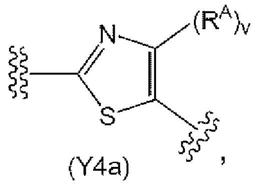
(Y4a),
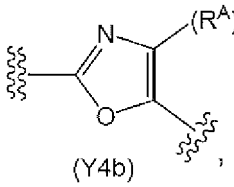
(Y4b),
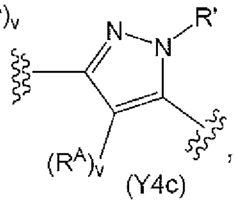
(Y4c),
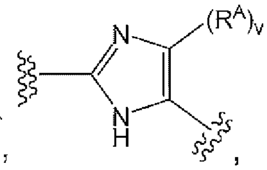
(Y4d),

[Formula 31]

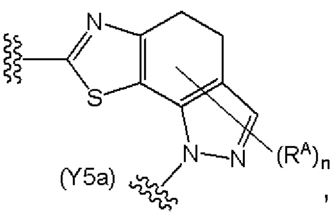
(Y5a),
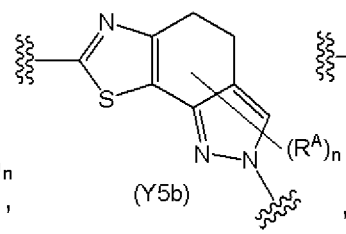
(Y5b),
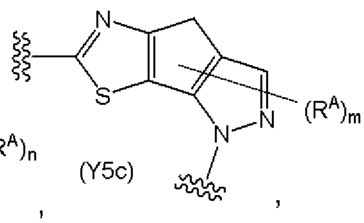
(Y5c),
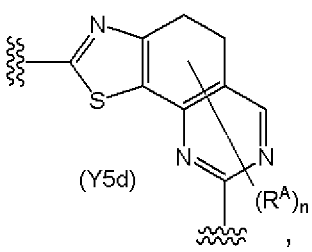
(Y5d),
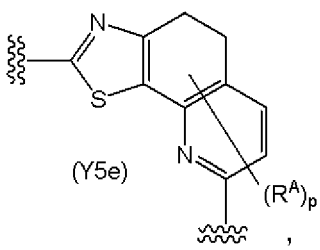
(Y5e),
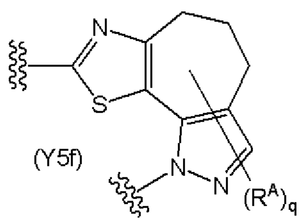
(Y5f),
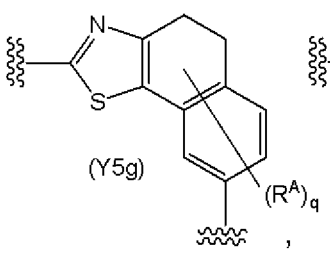
(Y5g),
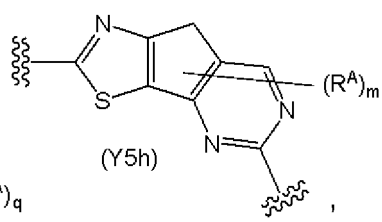
(Y5h),
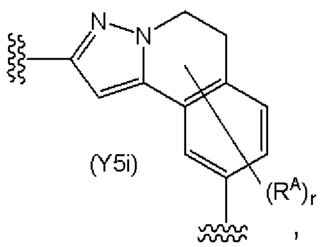
(Y5i),

[Formula 32]

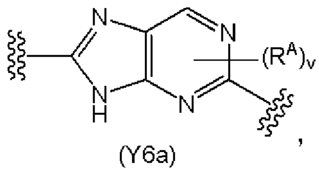
(Y6a) ,

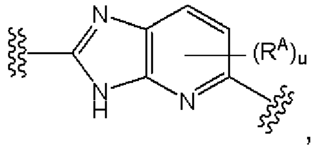
(Y6b) ,

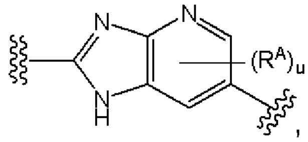
(Y6c) ,

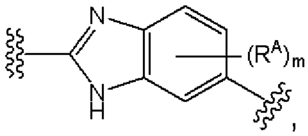
(Y6d) ,

[Formula 33]

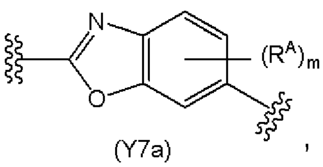
(Y7a) ,

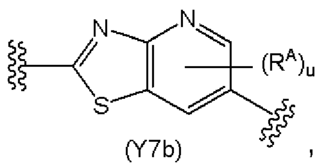
(Y7b) ,

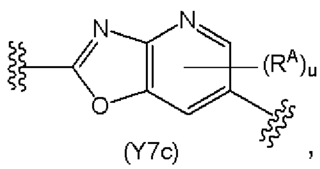
(Y7c) ,

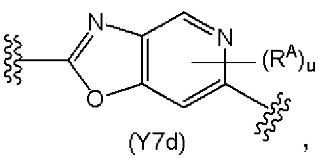
(Y7d) ,

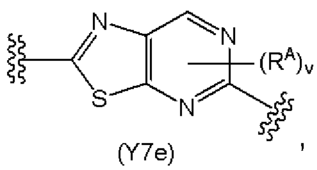
(Y7e) ,

[Formula 34]

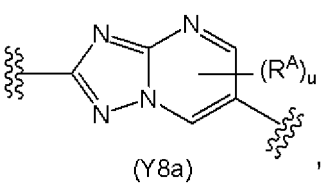
(Y8a) ,

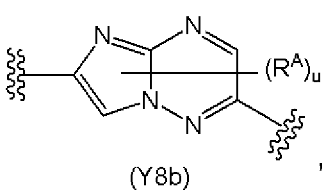
(Y8b) ,

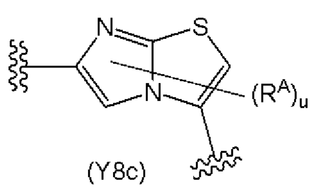
(Y8c)

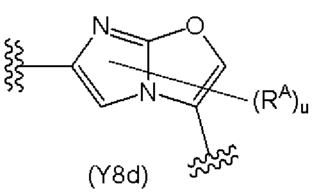
(Y8d) ,

[Formula 35]

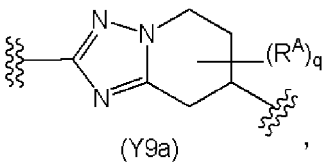
(Y9a) ,
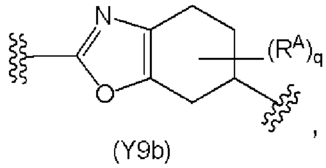
(Y9b) ,
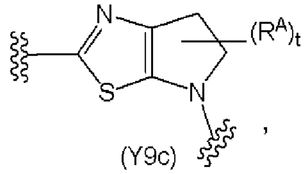
(Y9c) ,
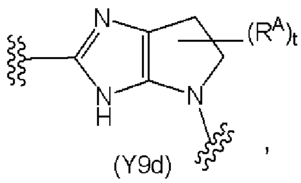
(Y9d) ,
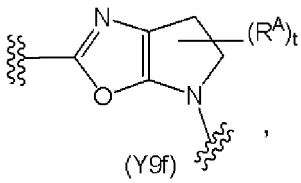
(Y9f) ,
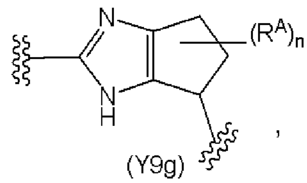
(Y9g) ,
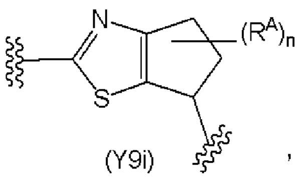
(Y9i) ,
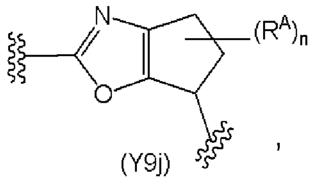
(Y9j) ,
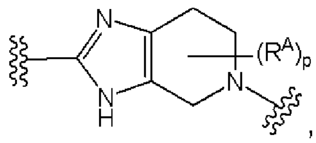
(Y9k) ,
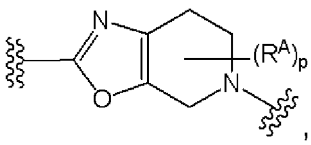
(Y9l) ,
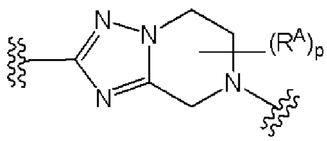
(Y9m) or
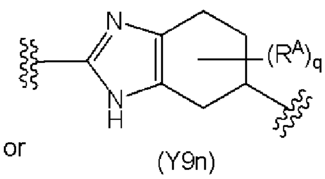
(Y9n) .

In the present specification, when a bond from substituent $R^A$ is described across two ring (for example, (Y1a), (Y1b), and the like), it indicates that substitution with $R^A$ may occur on either ring. Furthermore, in the present specification, when substitution with a bond from substituent $R^A$ occurs on only one ring (for example, (Y1d), (Y3j), and the like), it indicates that substitution with $R^A$ may occurs on the ring. The above-described respective groups defined as "Y" have bonds on the left and right sides of each ring, respectively. The left bond is to bind to a "N" atom of general formula (I), and the right bond is to bind to substituent "Z" of general formula (I).

Examples of Y include groups represented by formula (Y1a), groups represented by formula (Y1b), groups represented by formula (Y1c), or groups represented by formula (Y1d).

Examples of Y include groups represented by formula (Y1a).

Examples of Y include groups represented by formula (Y1d).

Examples of Y include groups represented by formula (Y2a), groups represented by formula (Y2b), or groups represented by formula (Y2c).

Examples of Y include groups represented by formula (Y2a).

Examples of Y include groups represented by formula (Y3a), groups represented by formula (Y3b), groups represented by formula (Y3c), groups represented by formula (Y3d), groups represented by formula (Y3e), groups represented by formula (Y3f), groups represented by formula (Y3g), groups represented by formula (Y3h), groups represented by formula (Y3i), groups represented by formula (Y3j), groups represented by formula (Y3k), or groups represented by formula (Y31).

Examples of Y include groups represented by formula (Y3a), groups represented by formula (Y3b), groups represented by formula (Y3e), groups represented by formula (Y3f), groups represented by formula (Y3g), groups represented by formula (Y3h), groups represented by formula (Y3j), or groups represented by formula (Y3k).

Examples of Y include groups represented by formula (Y3a), groups represented by formula (Y3e), groups represented by formula (Y3j), or groups represented by formula (Y3k).

Examples of Y include groups represented by formula (Y3a).

Examples of Y include groups represented by formula (Y4a), groups represented by formula (Y4b), groups represented by formula (Y4c), or groups represented by formula (Y4d).

Examples of Y include groups represented by formula (Y4a) or groups represented by formula (Y4c).

Examples of Y include groups represented by formula (Y5a), groups represented by formula (Y5b), groups represented by formula (Y5c), groups represented by formula (Y5d), groups represented by formula (Y5e), groups represented by formula (Y5f), groups represented by formula (Y5g), groups represented by formula (Y5h), or groups represented by formula (Y5i).

Examples of Y include groups represented by formula (Y5a).

Examples of Y include groups represented by formula (Y5c).

Examples of Y include groups represented by formula (Y6a), groups represented by formula (Y6b), groups represented by formula (Y6c), or groups represented by formula (Y6d).

Examples of Y include groups represented by formula (Y6b) or groups represented by formula (Y6d).

Examples of Y include groups represented by formula (Y7a), groups represented by formula (Y7b), groups represented by formula (Y7c), groups represented by formula (Y7d), or groups represented by formula (Y7e).

Examples of Y include groups represented by formula (Y7a), groups represented by formula (Y7c), or groups represented by formula (Y7d).

Examples of Y include groups represented by formula (Y7b) or groups represented by formula (Y7e).

Examples of Y include groups represented by formula (Y8a), groups represented by formula (Y8b), groups represented by formula (Y8c), or groups represented by formula (Y8d).

Examples of Y include groups represented by formula (Y9a), groups represented by formula (Y9b), groups represented by formula (Y9c), groups represented by formula (Y9d), groups represented by formula (Y9f), groups represented by formula (Y9g), groups represented by formula (Y9i), groups represented by formula (Y9j), groups represented by formula (Y9k), groups represented by formula (Y91), groups represented by formula (Y9m), or groups represented by formula (Y9n).

Examples of Y include groups represented by formula (Y9k) or groups represented by formula (Y9m).

$R^A$ in the above formulas is, each independently, halogen, cyano, carboxy, hydroxy, nitro, substituted or unsubstituted alkoxy, substituted or unsubstituted alkyl, substituted or unsubstituted alkenyl, substituted or unsubstituted alkynyl, substituted or unsubstituted amino, substituted or unsubstituted carbamoyl, substituted or unsubstituted acyl, substituted or unsubstituted aryl, substituted or unsubstituted cycloalkyl, substituted or unsubstituted cycloalkenyl, substituted or unsubstituted heteroaryl, substituted or unsubstituted heterocyclyl, a group represented by the formula: $-SO-R^{a'}$, a group represented by the formula: $-SO_2-R^{a'}$, or a group represented by the formula: $-SR^{a'}$;

$R^{a'}$ includes substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl.

$R^A$ in the above formulas can be bound to any substitutable position on the ring.

Examples of $R^A$ include each independently halogen, or substituted or unsubstituted C1-C3 alkyl.

Examples of $R^A$ include, each independently, substituted or unsubstituted aryl or substituted or unsubstituted heteroaryl.

R' in the above formulas includes hydrogen or substituted or unsubstituted alkyl.

Examples of R' include hydrogen or substituted or unsubstituted C1-C6 alkyl.

In the above formulas,

m includes integers from 0 to 3,
n includes integers from 0 to 5,
p includes integers from 0 to 6,
q includes integers from 0 to 7,
r includes integers from 0 to 8,
t includes integers from 0 to 4,
u includes integers from 0 to 2,
v includes 0 or 1.

For example, m includes 0.

For example, n includes 0 or 1.

For example, p includes 0 or 1.

For example, q includes 0.

For example, u includes 0.

For example, v includes 0 or 1.

Z includes substituted or unsubstituted cycloalkyl, substituted or unsubstituted cycloalkenyl, substituted or unsubstituted aryl, substituted or unsubstituted heteroaryl, or substituted or unsubstituted heterocyclyl.

Examples of Z include substituted or unsubstituted aryl, substituted or unsubstituted heteroaryl, or substituted or unsubstituted heterocyclyl.

Examples of Z include substituted or unsubstituted phenyl, substituted or unsubstituted pyrimidyl, substituted

or unsubstituted pyrazinyl, substituted or unsubstituted pyridazinyl, substituted or unsubstituted thiazolyl, substituted or unsubstituted oxazolyl, substituted or unsubstituted pyridyl, substituted or unsubstituted pyrazolyl, substituted or unsubstituted triazyl, substituted or unsubstituted piperidinyl, or substituted or unsubstituted benzopiperidinyl.

However, when Y is a group represented by formula (Y4a) or a group represented by formula (Y4b), $R^A$ is not substituted or unsubstituted aryl;

when Y is a group represented by formula (Y4a), $R^3$ is not substituted or unsubstituted alkyl, or substituted or unsubstituted alkoxy;

when Y is a group represented by formula (Y5a), $R^3$ is not substituted or unsubstituted alkyl, carboxy, substituted or unsubstituted alkoxycarbonyl, or substituted or unsubstituted aryl;

when Y is a group represented by formula (Y5c) or a group represented by formula (Y5d), $R^3$ is not substituted or unsubstituted alkyl, or substituted or unsubstituted aryl.

In addition, the present invention is not the compound shown below:

[Formula 36]

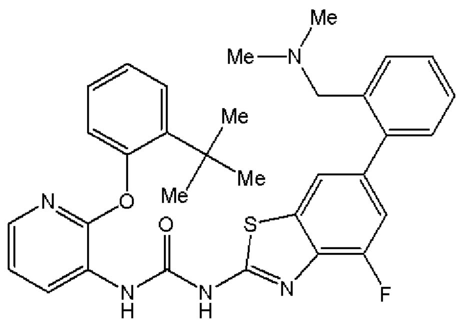

Compounds of general formula (I), or pharmaceutically acceptable salts thereof, or solvates thereof encompass compounds shown on the basis of all possible combinations of all alternatives of respective substituents exemplified in (G-1) above, or pharmaceutically acceptable salts, or solvates thereof.

(Production Method)

General production methods of the compound of the present invention are illustrated hereinbelow. Furthermore, with regard to extraction, purification, and the like, treatments as in usual experiments of organic chemistry may be carried out.

Synthesis of the compound of the present invention can be carried out by reference to a known method in the relevant field.

As a raw material compound, the following is available: commercially available compounds; those described in Patent Documents 3 to 17 and 24 to 27 and Non-patent Documents 14 to 17; those described in the present specification; those described in other documents cited herein; and other known compounds.

Regarding some of the compound of the present invention, a tautomer, regioisomer, or optical isomer thereof may exist. The present invention encompasses all possible isomers including these, and mixtures thereof.

When it is desired to obtain a salt of the compound of the present invention, in the case that the compound of the present invention is obtained in salt form, it may be purified as it is. Furthermore, in the case that it is obtained in free form, it may be dissolved or suspended in an appropriate organic solvent and then an acid or base may be added thereto to form a salt thereof using a general method.

Furthermore, the compound of the present invention and a pharmaceutically acceptable salt thereof may exist in form of adduct with water or any kind of solvent (hydrate or solvate). These adducts are also encompassed by the present invention.

Derivatives thereof are converted in the body and consequently activated, which are named "prodrug" herein. It is understood that examples of prodrugs include not only the aforementioned salts and solvates, but also esters (e.g., alkyl ester and the like), amides, and the like.

Various examples of compounds of the present invention are listed in Examples. By reference to these, those skilled in the art can produce and use compounds that are not illustrated in the present invention.

The present invention is also related to a system, an apparatus, and a kit for producing the compound of the present invention. It is understood that, as elements of such a system, an apparatus, and a kit, matters known in the

relevant field are available, and those skilled in the art can appropriately design them.

(General Synthesis Method)

Representative general synthesis methods of the compound of the present invention described in the Examples are shown in General Synthesis Methods 1 to 18. Compounds described in the Examples are synthesized mainly according to these methods, but methods are not specifically limited thereto. Reaction solvents, bases, palladium catalysts, and phosphine ligands, which are available in producing the compounds, are listed below. Of them, the preferred ones are described in General Synthesis Methods 1 to 18, but those are not specifically limited thereto.

(1) Reaction solvents: N,N-dimethylformamide (DMF), N-methyl-2-pyrrolidone (NMP), N,N-dimethylacetamide (DMA), dimethylsulfoxide, aromatic hydrocarbons (e.g., toluene, benzene, xylene, and the like), saturated hydrocarbons (e.g., cyclohexane, hexane, and the like), halogenated hydrocarbons (e.g., dichloromethane, chloroform, 1,2-dichloroethane, and the like), ethers (e.g., tetrahydrofuran, diethyl ether, dioxane, 1,2-dimethoxyethane, and the like), esters (e.g., methyl acetate, ethyl acetate, and the like), ketones (e.g., acetone, methylethylketone, and the like), nitriles (e.g., acetonitrile, and the like), alcohols (e.g., methanol, ethanol, *t*-butanol, and the like), water and mixed solvents thereof, and the like.
(2) Bases: metal hydrides (e.g., sodium hydride and the like), metal hydroxides (e.g., sodium hydroxide, potassium hydroxide, lithium hydroxide, barium hydroxide, and the like), metal carbonate salts (e.g., sodium carbonate, potassium carbonate, calcium carbonate, cesium carbonate, and the like), metal alkoxides (e.g., sodium methoxide, sodium ethoxide, potassium t-butoxide, and the like), sodium hydrogen carbonate, metallic sodium, organic amines (e.g., triethylamine, diisopropylethylamine, 1,8-diazabicyclo[5.4.0]undec-7-ene (DBU), pyridine, 4-dimethylaminopyridine, 2,6-lutidine, and the like), alkyl lithium(*n*-butyl lithium (n-BuLi), sec-butyl lithium (sec-BuLi), *tert*-butyl lithium (*tert*-BuLi)), and the like.
(3) Palladium catalysts used in Pd coupling: $Pd(PPh_3)_4$, $PdCl_2(dppf)$, $PdCl_2(PPh_3)_2$, $Pd(OAc)_2$, $Pd(dba)_2$, $Pd_2(dba)_3$, $PdCl_2$, and the like.
(4) Phosphine ligands: $PPh_3$, BINAP, Xantphos, S-Phos, X-Phos, DPPF, $P(t\text{-}Bu)_3$, tris(o-tolyl)phosphine, and the like.

(General Synthesis Method 1) Synthesis method of Compound I-A:
[Formula 37]

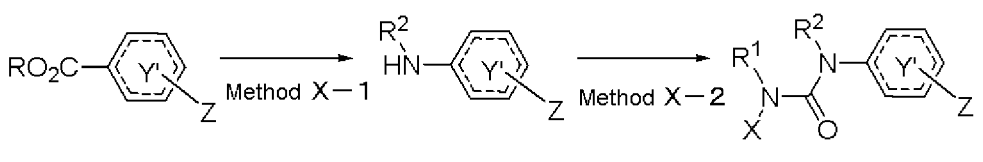


A1 B1 Compound I−A ,

wherein each symbol is defined the same as above and R represents C1-C6 alkyl, and wherein the formula:
[Formula 38]

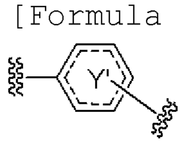

represents Y in formula (I).
This is a method for synthesizing Compound I-A using Methods X-1 and X-2. Hereinafter, Method X-1 and Method X-2 are described in detail.
(1-1) Method X-1:
[Formula 39]

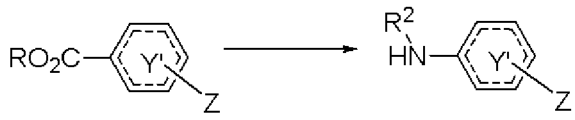

A1 B1

Step 1:

[Formula 40]

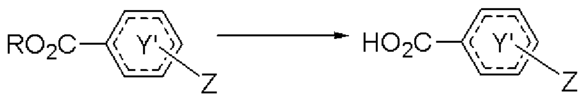

A1 A2 ,

wherein each symbol is defined the same as above. As Compound represented by formula (A1), a known compound may be used, or a known compound derived from a known compound according to a conventional method may be used.

The above step is of hydrolyzing a compound represented by formula (A1) in the presence of base to synthesize a compound represented by formula (A2).

Bases include the bases described in (2) above. A base is preferably a metal hydroxide (e.g., sodium hydroxide, potassium hydroxide, lithium hydroxide, barium hydroxide, and the like).

Reaction solvents include the solvents described in (1) above.

Preferably, a reaction may be carried out in an aqueous solution and an alcohol solution of a metal hydroxide. A reaction temperature and a reaction time are not specifically limited, but a reaction may be carried out at temperatures from -20˚C to a solvent-refluxing temperature for 0.5 to 36 hours.

Step 2:

[Formula 41]

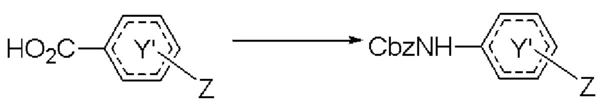

A2 A3 ,

wherein each symbol is defined the same as above and Cbz represents a benzyloxycarbonyl group.

The above step is of reacting a compound represented by formula (A2) with an azidation reagent or azide compound followed by pyrolysis to cause a Curtius rearrangement, and further treatment with an alcohol which forms a protecting group to synthesize a compound represented by formula (A3).

Sodium azide, hydrogen azide, diphenylphosphoryl azide, and the like can be used as azidation reagents or azide compounds. A solvent described in (1) above can be used as a solvent. For a preferable example, a reaction in a benzylalcohol solution produces an amine protected with benzyloxycarbonyl. With regard to reaction temperatures, the reaction with an azidation reagent or azide compound is usually carried out at a low temperature (e.g., 0˚C and the like), and the pyrolysis is usually carried out under a heating condition (e.g., 100˚C and the like). A reaction time is not specifically limited, but the reaction may be carried out for 0.5 to 12 hours.

Step 3:

[Formula 42]

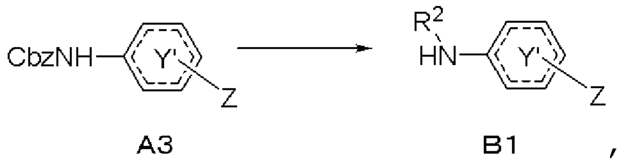

wherein each symbol is defined the same as above and Cbz represents a benzyloxycarbonyl group.
The above step is of deprotecting a compound represented by formula (A3) from a benzyloxycarbonyl group to synthesize a compound represented by formula (B1) A deprotection method is carried out in accordance with a method described in "Protective Groups in Organic Synthesis," by Theodora W. Greene (John Wiley & Sons, Inc., New York, second ed., 1991). When $R^2$ is substituted or unsubstituted alkyl, reductive alkylation can introduce $R^2$. Furthermore, after $R^2$ is introduced to a compound represented by formula (A3), deprotection from Cbz may be carried out.
(1-2) method X-2:
[Formula 43]

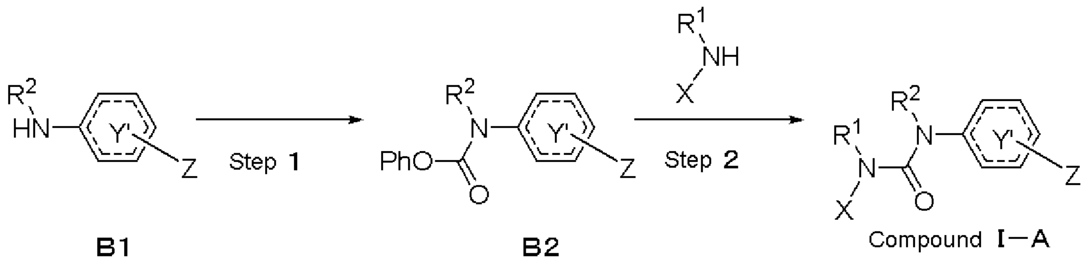


wherein each symbol is defined the same as above and Ph is a phenyl group.
The above step is of reacting a compound represented by formula (B1) with phenyl chloroformate to form a compound represented by formula (B2), and then reacting a compound represented by the following formula:
[Formula 44]

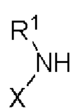

to synthesize Compound I-A.
In the reaction of Step 1, a solvent described in (1) above and a base described in (2) above can be used. Preferably, the reaction may be carried out using ethers (e.g., tetrahydrofuran, diethyl ether, dioxane, 1,2-dimethoxyethane, and the like) as solvent, and an organic amine (e.g., triethylamine and the like) as a base. A reaction temperature and a reaction time are not specifically limited, but the reaction may be carried out at -20 to 50˚C for 0.5 to 12 hours. *p*-Nitrophenyl chloroformate also can be used instead of phenyl chloroformate.
In the reaction of Step 2, a solvent described in (1) above can be used. Preferably, the reaction may be carried out using dimethylsulfoxide. A reaction temperature and a reaction time are not specifically limited, but the reaction may be carried out at -20 to 50˚C for 0.5 to 12 hours.
Furthermore, the following methods, X-3 and X-4, are illustrated as other methods for synthesizing Compound I-A.
(General Synthesis Method 2) Synthesis method of Compounds I-A and I-A':
[Formula 45]

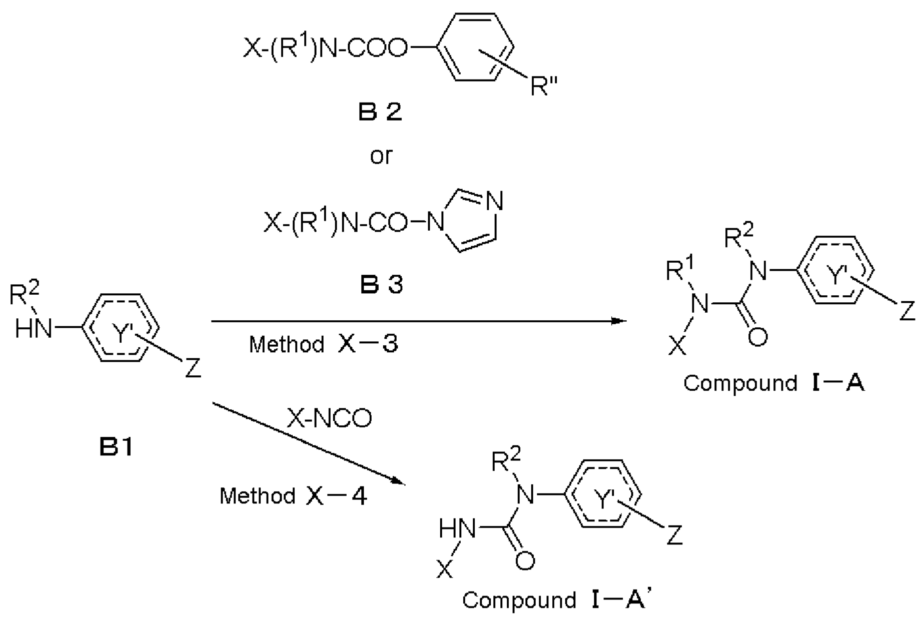

B 2
or
B 3
B1
Compound I−A
Compound I−A'

,

wherein each symbol is defined the same as above, and a known compound may be used for formula (B1), or a compound derived from a known compound by a conventional method may be used, and wherein the formula:
[Formula 46]

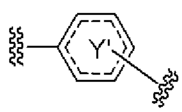

represents Y in formula (I) and R" represents hydrogen or a nitro group.

(2-1) Method X-3:

This method is a method of reacting a compound represented by formula (B1) with an active carbamate represented by formula (B2), formula (B3), or the like in the presence or absence of a base to synthesize Compound I-A.
A reaction solvent described in (1) above can be used as a reaction solvent. DMF, NMP, DMA, THF, dioxane, or DMSO is preferred among others, but the reaction solvent is not specifically limited as long as it does not react under the present condition.
A base described in (2) above can be used as a base. Preferably, examples of the bases include metal hydrides (e.g., sodium hydride and the like), metal hydroxides (e.g., sodium hydroxide, potassium hydroxide, lithium hydroxide, barium hydroxide, and the like), metal carbonate salts (e.g., sodium carbonate, calcium carbonate, cesium carbonate, and the like), organic amines, and the like. A base is not always used, but can be used as required.
A reaction temperature and a reaction time are not specifically limited, but the temperature can be from a temperature under an icebath-cooling condition to the boiling point of a solvent. Usually, the reaction is carried out at room temperature. When the progress of the reaction is slow, there are some cases where heating can accelerate the reaction.

(2-2) Method X-4:

This method is a method of reacting a compound represented by formula (B1) with a compound of the formula: X-NCO in the presence or absence of a base to synthesize Compound I-A'.
A reaction solvent described in (1) above can be used as a reaction solvent. DMF, NMP, DMA, THF, DMSO,

or dioxane is preferred among others, but the reaction solvent is not specifically limited as long as it does not react under the present condition.

A based described in (2) above can be used as a base. Preferably, examples of the base include metal hydrides (e.g., sodium hydride and the like), organic amines, metal hydroxides (e.g., sodium hydroxide, potassium hydroxide, lithium hydroxide, barium hydroxide, and the like), metal carbonate salts (e.g., sodium carbonate, calcium carbonate, cesium carbonate, and the like), and the like. A reaction temperature and a reaction time are not specifically limited, but the temperature can be from a temperature under an icebath-cooling condition to the boiling point of a solvent. Usually, the reaction is carried out at room temperature. When the progress of the reaction is slow, there are some cases where heating can accelerate the reaction.

Furthermore, Compound I-A" can be synthesized using the following method X-5.

(General Synthesis Method 3) Synthesis method of Compound I-A" (Method X-5):

[Formula 47]

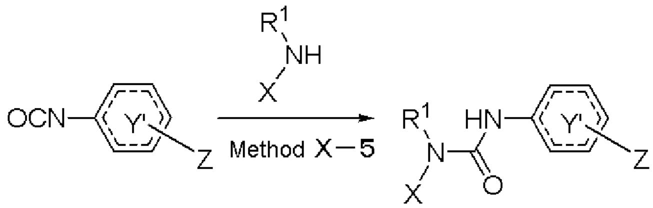


C1

Compound I-A" ,

wherein each symbol is defined the same as above, and a known compound may be used for formula (C1), or a compound derived from a known compound by a conventional method may be used.
This method is a method of reacting a compound represented by formula (C1) with a compound of the formula: X-($R^1$)NH in the presence of a base to synthesize Compound I-A". The reaction may be carried out under the same reaction condition as that in Method X-4 of General Synthesis Method (2-2).

(General Synthesis Method 4) Synthesis Method of Compound I-B (Method Z-1)

[Formula 48]

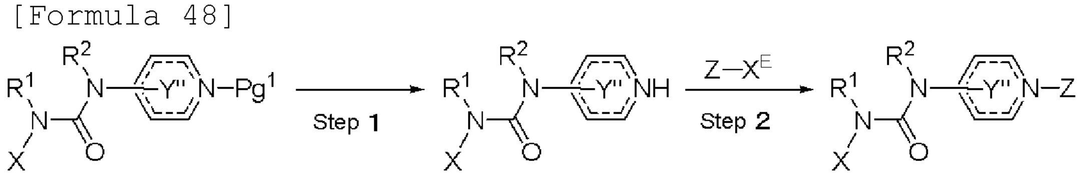


E1

E2

Compound I-B

wherein each symbol is defined the same as above; $X^E$ includes halogen (e.g., Cl, Br, I, and the like) or a leaving group such as -OMs, -OTs, -OTf, -ONs, or the like, wherein "Ms" represents a methanesulfonyl group, "Ts" represents a *p*-toluenesulfonyl group, "Tf" represents a trifluoromethanesulfonyl group, and "Ns" represents an o-nitrobenzenesulfonyl group; and $Pg^1$ represents an amino protecting group (e.g., *t*-butoxycarbonyl (Boc) group, benzyl group, acetyl group, benzoyl group, benzyloxycarbonyl group, and the like), and wherein the formula:

[Formula 49]

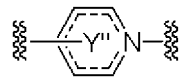

represents Y in formula (I); and known compounds may be used for a compound represented by formula (E1) and a compound represented by the formula: Z-$X^E$, or compounds derived from a known compound by a conventional method may be used.

The above steps are the steps for synthesizing Compound I-B of the present invention by, in Step 1, deprotecting a compound represented by formula (E1) from a $Pg^1$ group, and further, in Step 2, reacting a compound represented by formula (E2) with the above-described Z-$X^E$ in the presence of a base. A base described in (2) above can be used as a base.

Step 1 is conducted using a method described in "Protective Groups in Organic Synthesis," by Theodora W. Greene (John Wiley & Sons, Inc., New York, second ed., 1991) as a deprotection method.

In Step 2, Compound I-B of the present invention can be synthesized by reacting a compound represented by formula (E2) with the above-described Z-$X^E$ in the presence or absence of a base, a palladium catalyst, and a phosphine ligand.

Compound I-B of the present invention can be synthesized by, for example, carrying out a substitution reaction of a compound represented by formula (E2) with a compound represented by the formula: Z-$X^E$ using N,N-dimethylformamide or dimethylsulfoxide as solvent and a metal carbonate salt (e.g., sodium carbonate, potassium carbonate, calcium carbonate, cesium carbonate, and the like) as a base. The reaction may be carried out for 0.5 to 24 hours at temperatures from -20˚C to a temperature at which a solvent used is refluxed.

(General Synthesis Method 5) Synthesis method of a boronic acid derivative (Method Z-2):

[Formula 50]

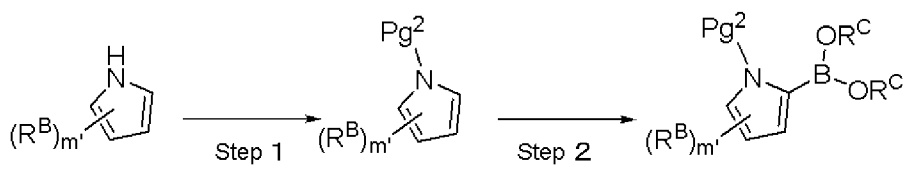


F1 F2 F3 ,

wherein each symbol is defined the same as above; $R^B$ represents hydrogen or substituted or unsubstituted alkyl; $Pg^2$ represents an amino protecting group (e.g., *t*-butoxycarbonyl (Boc) group, benzyl group, acetyl group, benzoyl group, benzyloxycarbonyl group, and the like), and wherein m' is an integer from 0 to 3; $R^c$ can include hydrogen or alkyl; substituents can include any suitable substituents; two occurrence of $R^c$ may be taken together to form a ring; and a known compound may be used for formula (F1), or a compound derive from a known compound by a conventional method may be used.

The above method is for synthesizing a boronic acid derivative of formula (F3), which is used in the present invention, from a compound represented by formula (F1) through Steps 1 and 2 of Method Z-2 described below. The detail is described below.

Step 1: The protection of an amino group

[Formula 51]

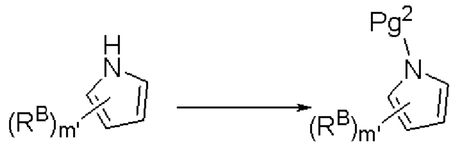

F1 F2

wherein each symbol is defined the same as above; a known compound may be used for formula (F1), or a compound derived from a known compound by a conventional method may be used; and $Pg^2$ represents an amino protecting group.

The above step is of introducing a protecting group ($Pg^2$ in the formula) to the nitrogen of a compound represented by formula (F1) Carbamate functional groups such as *t*-butoxycarbonyl (Boc) group, benzyloxycarbonyl (Cbz) group, and the like, and methoxymethyl group (MOM) group, 2-(trimethylsilyl)ethoxymethyl (SEM) group, 2-tetrahydropyranyl (THP) group, and the like can be used as protecting groups, but are not specifically limited thereto. The foregoing protecting groups can be introduced in accordance with a known method. However, for example, in the case of *t*-butoxycarbonyl group, a reaction may be carried out using di-*t*-butyl dicarbonate in the presence of an organic base such as triethylamine or the like or an inorganic base such as sodium hydroxide, sodium hydrogen carbonate, sodium carbonate, or the like and using DMF, NMP, DMA, dichloromethane or a mixed solvent thereof with water as solvent. There are some cases where catalytic addition of N,N-4-dimethylaminopyridine (DMAP) accelerates the progress of the reaction. It is preferable that reaction temperatures are from about 0˚C to room temperature, but it may be selected depending on the progress of the reaction.

Step 2: Synthesis of a boronic acid derivative:

[Formula 52]

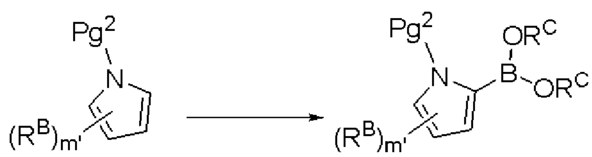

F2 F3

wherein each symbol is defined the same as above; $Pg^2$ represents an amino protecting group; $R^c$ can include hydrogen or alkyl; substituents include any suitable substituent; two occurrence of $R^c$ may be taken together to form a ring; and a known compound may be used for formula (F2), or a compound derived from a known compound by a conventional method may be used.

The above step is for producing a boronic acid derivative of formula (F3) by reacting a compound of formula (F2) with a boric acid compound in the presence of a base. For example, with regard to conversion to a boronic ester, a compound represented by formula (F2) is lithiated with alkyl lithium such as *n*-butyl lithium or the like before a reaction with a boric ester, and consequently can be converted to a compound represented by formula (F3). Reaction solvents include THF, dioxane, and the like, and are not specifically limited as long as they are not reacted with alkyl lithium. The temperature of the lithiation reaction is preferably from a low temperature of about -78˚C to about 0˚C, and may be warmed to room temperature after the boric ester is added. As a boric ester, a methyl ester or isopropyl ester is preferred. After the reaction, addition of water, an aqueous diluted hydrochloric acid solution, or the like can convert it to an organic boronic acid.

Furthermore, a compound represented by formula (F3) can be obtained by reacting a compound represented by formula (F2) in the presence of a palladium catalyst described (3) above, a base, a reaction solvent, and bis(pinacolato) diboran at temperatures from room temperature to about the boiling point of the solvent.

In a similar way, a boronic acid derivative of pyrimidine, a boronic acid derivative of thiazole, a boronic acid derivative of oxazole, a boronic acid derivative of pyridine, a boronic acid derivative of triazine, and the like can be synthesized. Commercially available boronic acid reagents also can be used.

Using Method Z-3 in addition to Method Z-1 described above, a Z moiety of the present invention can be synthesized.

(General Synthesis Method 6) Synthesis method of Compound I-C using a boronic acid derivative (Method Z-3)

[Formula 53]

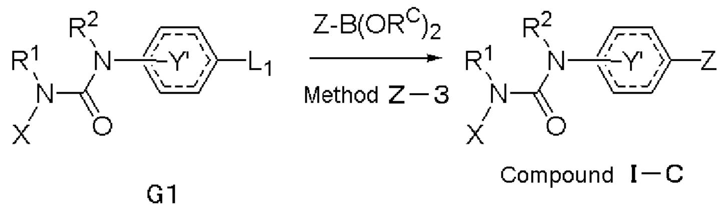


wherein each symbol is defined the same as above; a known compound may be used for formula (G1), or a compound derived from a known compound by a conventional method may be used; $L_1$ represents halogen or a leaving group, wherein chlorine, iodine, and bromine are preferred as halogen, and a OTf group (a trifluoromethanesulfonate ester) is preferred as a leaving group; and wherein the formula:

[Formula 54]

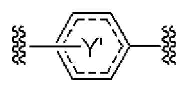

represents Y of formula (I).

The above step is of carrying out Suzuki coupling reaction using a compound represented by formula (G1) and a boronic acid derivative and thereby introducing group Z to synthesize Compound I-C.

In this step, it is preferable to carry out the Suzuki coupling reaction in the presence of a palladium catalyst described in (3), optionally a phosphine ligand described in (4), and a base described in (2). With regard to an organic boronic acid derivative, a commercially available compound is used as if it can be obtained. An organic boronic acid derivative also can be synthesized according to Method Z-2.

A reaction solvent described in (1) can be used as a reaction solvent. Dioxane, DMF, 1,2-dimethoxyethane (DME), lower alcohol, toluene, and mixed solutions thereof are preferred, but the reaction solvents are not specifically limited thereto as long as solvents do not react under the present condition. The reaction temperature is not specifically limited, but the reaction can be carried out at temperatures from room temperature to 200˚C. When the reactivity is low, it is possible to adjust a temperature by warming appropriately. Solids or solutions of $Na_2CO_3$, $K_3PO_4$, $K_2CO_3$, NaOH, $Cs_2CO_3$ and the like are preferred as bases.

(General Synthesis Method 7) Methods Y-1 and Y-2: When Y is represented by the following:

[Formula 55]

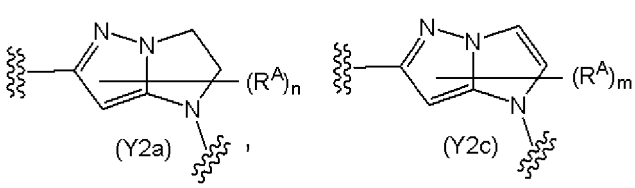

[Formula 56]

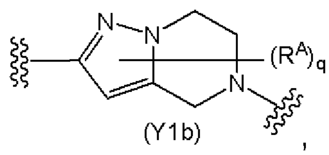

(Y1b)

that is, when Y is represented by:

[Formula 57]

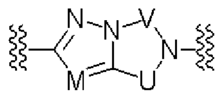

wherein V represents -($CR^GR^H$)m"- or -$CR^I$=$CR^J$-; U represents a single bond or -($CR^KR^L$)n"-; M represents -C=; $R^G$ to $R^L$ are hydrogen, substituted or unsubstituted alkyl, substituted or unsubstituted alkoxy, and the like; m is 2; and n is 1,

then a compound represented by formula (N1) is synthesized using Method Y-1 or Method Y-2.

[Formula 58]

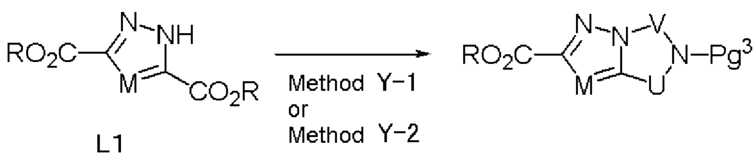


L1

N1

wherein each symbol is defined the same as above, and $Pg^3$ represents an amino protecting group (e.g., *t*-butoxycarbonyl group, benzyl group, acetyl group, benzoyl group, benzyloxycarbonyl group and the like).

The detailed steps of Method Y-1 and Method Y-2 are described below.

(7-1) Synthesis method (Method Y-1) of a compound represented by formula (N1) when U is a single bond, that is, when Y is represented by the following:

[Formula 59]

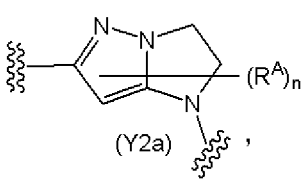

(Y2a) ,

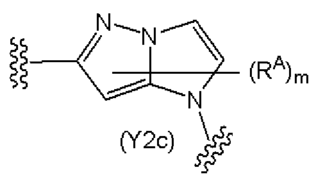

(Y2c)

Step 1

[Formula 60]

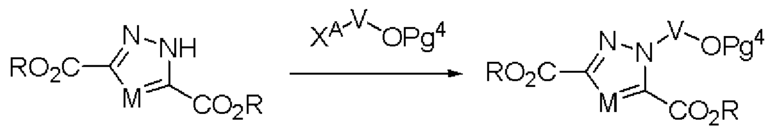

L1 L2

wherein each symbol is defined the same as above; R includes C1-C6 alkyl; $X^A$ includes halogen (e.g., Cl, Br, I, and the like) or a leaving group such as -OMs, -OTs, -OTf, -ONs, and the like; and wherein "Ms" represents methanesulfonyl group, "Ts" represents p-toluenesulfonyl group, "Tf" represents trifluoromethanesulfonyl group, "Ns" represents *o*-nitrobenzenesulfonyl group; $Pg^4$ represents a hydroxy protecting group (e.g., benzyl group, p-methoxybenzyl group, acetyl group, and the like); and a known compound may be used as a compound represented by formula (L1) or the formula:

[Formula 61]

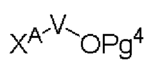

or a compound derived from a known compound by a conventional method may be used.
A compound represented by formula (L1) can be reacted with a compound represented by the formula:

[Formula 62]

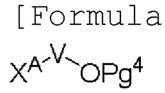

in the presence of a base to synthesize a compound represented by formula (L2).

Reaction solvents include solvents described in (1) above. Bases include bases described in (2) above. Preferably, the reaction may be carried out using ethers (e.g., tetrahydrofuran, diethyl ether, dioxane, and the like), N,N-dimethylformamide, acetonitrile as a reaction solvent, and a metal carbonate salt (e.g., sodium carbonate, potassium carbonate, calcium carbonate, cesium carbonate, and the like) as a base. A reaction temperature and a reaction time are not specifically limited, but the reaction may be carried out for 0.5 to 12 hours at temperatures from -20°C to a temperature at which a solvent used is refluxed.

Step 2

[Formula 63]

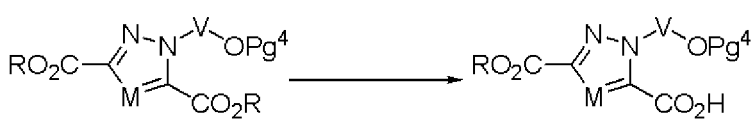

L2 L3

wherein each symbol is defined the same as above.
A compound represented by formula (L2) can be hydrolyzed in the presence of a base to synthesize a compound represented by formula (L3).

A base described in (2) above can be used as a base. Preferably, the base is a metal hydroxide (e.g., sodium

hydroxide, potassium hydroxide, lithium hydroxide, barium hydroxide, and the like). A solvent described in (1) above can be used as a reaction solvent. Preferably, the reaction may be carried out in an aqueous solution and an alcohol solution of a metal hydroxide. A reaction temperature and a reaction time are not specifically limited, but the reaction may be carried out for 0.5 to 36 hours at temperatures from -20 to 50˚C.

Step 3

[Formula 64]

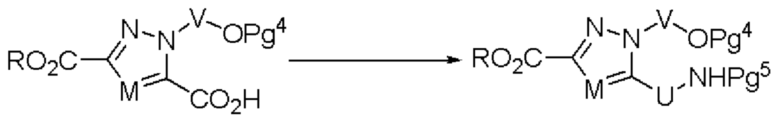

L3 L4

wherein each symbol is defined the same as above and $Pg^5$ represents an amino protecting group (e.g., *t*-butoxycarbonyl group, benzyl group, acetyl group, benzoyl group, benzyloxycarbonyl group, and the like).
A compound represented by formula (L3) can be reacted with an azidation reagent or azide compound followed by pyrolysis to cause a Curtius rearrangement, and further treatment with an alcohol which forms a protecting group to synthesize a compound represented by formula (L4).

Sodium azide, hydrogen azide, diphenylphosphoryl azide, and the like can be used as azidation reagents or azide compounds. A solvent described (1) above can be used as a solvent. For a preferable example, a reaction in a *t*-butylalcohol solution produces an amine protected with a *t*-butoxycarbonyl group. With regard to reaction temperatures, the reaction with an azidation reagent or azide compound is usually carried out at a low temperature (e.g., 0˚C and the like), and the pyrolysis is usually carried out under a heating condition (e.g., 100˚C and the like). A reaction time is not specifically limited, but the reaction may be carried out for 0.5 to 12 hours.

Step 4

[Formula 65]

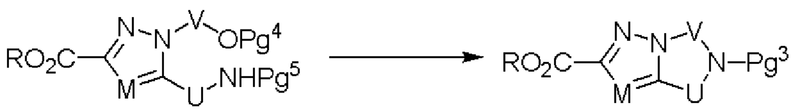

L4 N1

wherein each symbol is defined the same as above.
A compound represented by formula (N1), that is, a compound having a formula described below:

[Formula 66]

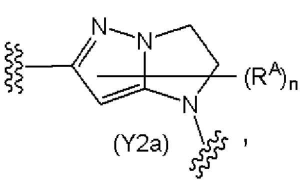

(Y2a),

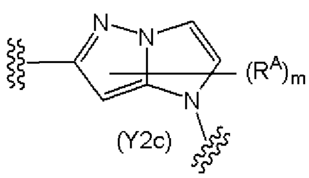

(Y2c)

can be synthesized by deprotecting a compound represented by formula (L4) from the $Pg^4$ group and then intramolecularly cyclizing the resulting alcohol.

A deprotection method is carried out in accordance with a method described in "Protective Groups in Organic Synthesis," by Theodora W. Greene (John Wiley & Sons, Inc., New York, second ed., 1991). For example, when a

protecting group is a benzyl or *p*-methoxybenzyl group, a catalytic reduction reaction may be carried out in the presence of hydrogen.

A method of an intramolecular cyclization may be carried out under a condition of Mitsunobu reaction. Phosphine reagents include phosphine ligands described in (4) above. For a preferable example, $PPh_3$, $P(n\text{-}Bu)_3$, or the like may be used. DEAD, DIAD, ADDP, and the like can be used as azodicarboxylate esters and amides. A reaction temperature and a reaction time are not specifically limited, but the reaction may be carried out at temperatures from -20 to 50˚C for 0.5 to 12 hours. With regard to a method of an intramolecular cyclization, an OH group may be converted to halogen (e.g., Cl, Br, I, and the like) or a leaving group (which represents, for example, OTf, OMs, or the like) described in Method Z-1 of the foregoing (General Synthesis Method 4) to carry out an intramolecular cyclization in the presence of a base. In the conversion of an OH group to a halogen, after the reaction with methanesulfonyl chloride to form a OMs group, the OMs group is substituted with $Cl^-$ that generates in the reaction system and consequently the chlorination can be attained. In addition, a reaction with carbon tetrabromide, bromine, NCS, or NBS in the presence of $PPh_3$ may be carried out.

A solvent described in (1) above can be used as a solvent. Preferably, the reaction may be carried out using N,N-dimethylformamide, halogenated hydrocarbons (e.g., dichloromethane, chloroform, 1,2-dichloroethane, and the like), or ethers (e.g., tetrahydrofuran, diethyl ether, dioxane, 1,2-dimethoxyethane, and the like). A reaction temperature and a reaction time are not specifically limited, but the reaction may be carried out at temperatures from -20 to 50˚C for 0.5 to 12 hours. A base described in (2) above can be used as a base in an intramolecular cyclization reaction. Preferably, the reaction may be carried out using a metal hydride (e.g., sodium hydride and the like), a metal alkoxide (e.g., sodium methoxide, sodium ethoxide, potassium t-butoxide, and the like).

(7-2) Synthesis method (Method Y-2) of a compound represented by formula (N1) when U is $-(CR^KR^L)n''-$, that is, when Y is represented by the following:

[Formula 67]

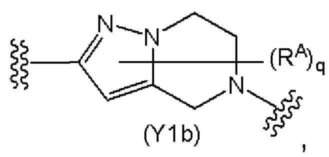

(Y1b) ,

Step 1

[Formula 68]

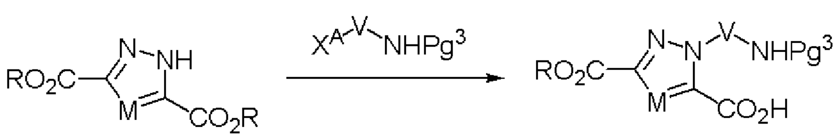

L1 M2

wherein each symbol is defined the same as above, and $X^A$ represents halogen (e.g., Cl, Br, I, and the like) or a leaving group (e.g., OTf, OMs, and the like) described in Method Z-1 of the foregoing (General Synthesis Method 4); $Pg^3$ represents an amino protecting group (e.g., *t*-butoxycarbonyl group, acetyl group, benzoyl group, benzyl group, benzyloxycarbonyl group, and the like); and known compounds can be used as compounds represented by formula (L1) or the formula:

[Formula 69]

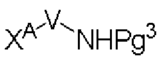

or a compound derived from a known compound by a conventional method may be used.

By reacting a compound represented by formula (L1) with a compound represented by the formula:

[Formula 70]

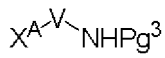

in the presence of a base using the same method as Step 1 of Method Y-1 described above and further reacting the resulting compound using the same method as Step 2 of Method Y-1, a compound represented by formula (M2) can be synthesized. The above step can be carried out under the same reaction condition as Step1 and Step 2 of Method Y-1 described above.

With regard to reaction condition of Step 1, preferably, the reaction may be carried out by using N,N-dimethylformamide (DMF), nitriles (e.g., acetonitrile and the like), or the like as solvent at temperatures from -20 to 50˚C for 0.5 to 12 hours. With regard to Step 2, preferably, the reaction may be carried out using alcohols (e.g., methanol, ethanol, t-butanol, and the like), water, halogenated hydrocarbons (e.g., dichloromethane, chloroform, 1,2-dichloroethanme, and the like), and the like as solvent at temperatures from -20 to 50˚C for 0.5 to 24 hours.

Step 2

[Formula 71]

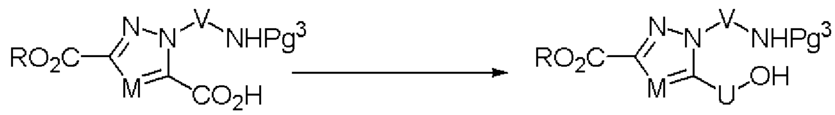

M2 M3

wherein each symbol is defined the same as above.

An alcohol represented by formula (M3) can be synthesized by reacting a compound represented by formula (M2) with N,N'-carbonyldiimidazole to convert the carboxyl group of a compound represented by formula (M2) to -C(=O)Im (wherein Im is imidazole), followed by reduction.

For example, sodium tetrahydroborate, lithium tetrahydroborate, or the like is used as a reducing agent. A solvent described in (1) above or the like can be used as a solvent. Preferably, ethers (e.g., tetrahydrofuran, diethyl ether, dioxane, 1,2-dimethoxyethane, and the like) can be used. A reaction temperature and a reaction time are not specifically limited, but the reaction may be carried out at temperatures from -20 to 50˚C for 0.5 to 12 hours.

Step 3

[Formula 72]

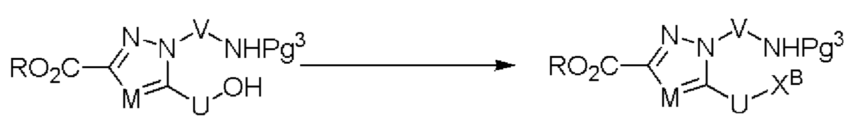

M3 M4

wherein each symbol is defined the same as above, and $X^B$ represents halogen (e.g., Cl, Br, I, and the like) or a leaving group (e.g., OTf, OMs, and the like) described in Method Z-1 of the foregoing (General Synthesis Method 4).

A compound represented by formula (M4) can be synthesized by halogenating a compound represented by formula (M3).

For example, it is possible to react the OH group of a compound represented by formula (M3) with methanesulfonyl chloride to convert it to OMs group, followed by chlorination by substitution with $Cl^-$ that generates in the reaction system.

With regard to a reaction of halogenating an alcohol, an alcohol may be reacted in the presence of $PPh_3$ with carbon tetrabromide, bromine, NCS, or NBS. A solvent described in (1) above can be used as a solvent. The reaction

may be carried out using, preferably, halogenated hydrocarbons (e.g., dichloromethane, chloroform, 1,2-dichloroethane, and the like), or ethers (e.g., tetrahydrofuran, diethyl ether, dioxane, 1,2-dimethoxyethane, and the like). A reaction temperature and a reaction time are not specifically limited, but the reaction may be carried out at temperatures from -20 to 50°C for 0.5 to 12 hours.

Step 4

[Formula 73]

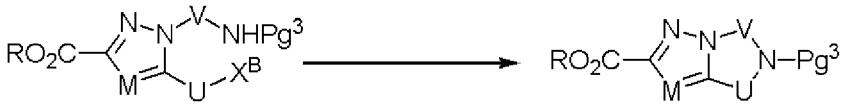

M4 N1

wherein each symbol is defined the same as above, and $X^B$ represents halogen (e.g., Cl, Br, I, and the like) or a leaving group (e.g., OTf, OMs, and the like) described in Method Z-1 of the foregoing (General Synthesis Method 4). By intramolecularly cyclizing a compound represented by formula (M4) in the presence of a base, a compound of formula (N1), that is, a compound having a group represented by the following:

[Formula 74]

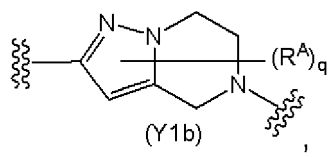

(Y1b) ,

can be synthesized.

A base described in (2) above can be used as a base. Preferably, the reaction may be carried out using a metal hydride (e.g., sodium hydride and the like), metal alkoxide (e.g., sodium methoxide, sodium ethoxide, potassium t-butoxide, and the like). A solvent described in (1) above can be used as a solvent. Preferably, the reaction may be carried out using N,N-dimethylformamide, halogenated hydrocarbons (e.g., dichloromethane, chloroform, 1,2-dichloroethane, and the like), or ethers (e.g., tetrahydrofuran, diethyl ether, dioxane, 1,2-dimethoxyethane, and the like). A reaction temperature and a reaction time are not specifically limited, but the reaction may be carried out at temperatures from -20 to 50°C for 0.5 to 12 hours.

Moreover, by combining the foregoing Methods X-1, X-2, Y-1, Y-2, and Z-1 as shown in the schemes below, Compound I-D of the invention can be synthesized.

[Formula 75]

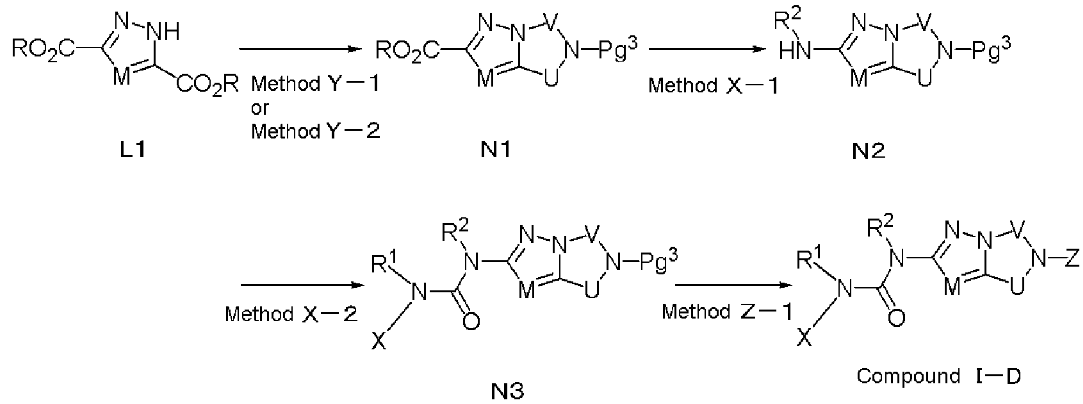


L1 N1 N2 N3 Compound I-D

wherein each symbol is defined the same as above, and a known compound may be used as a compound represented by formula (L1), or a compound derived from a known compound by a conventional method may be used.

(General Synthesis Method 8) When Y is represented by the following:

[Formula 76]

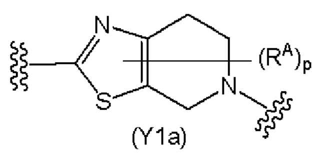

(Y1a)

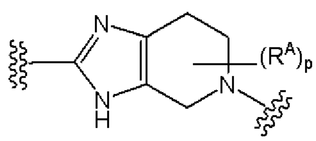

(Y9k)

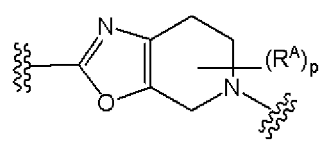

(Y9l)

that is, when Y is represented by the following:

[Formula 77]

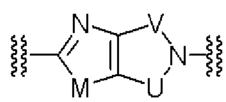

M=S, O, $NR^M$

wherein each symbol is defined the same as above; U is $-CH_2-$; and M represents S, O, and $NR^M$ wherein $R^M$ represents hydrogen, substituted or unsubstituted alkyl, substituted or unsubstituted alkoxy, or the like, then Compound I-E can be synthesized using Methods Y-3 and Z-1 described below.

[Formula 78]

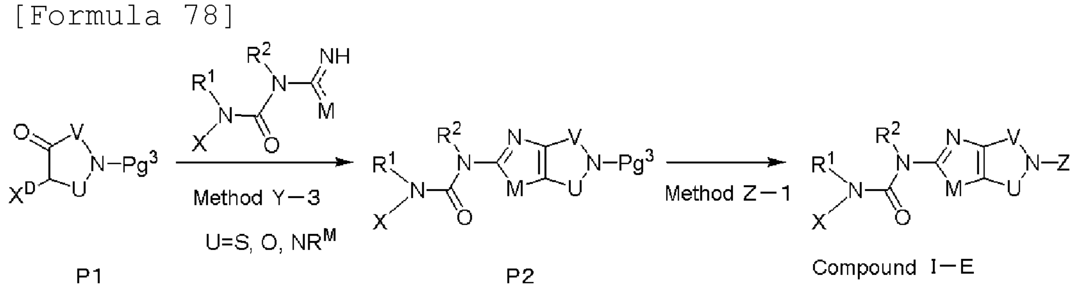


P1 P2 Compound I-E

wherein each symbol is defined the same as above; $X^D$ represents halogen (e.g., Cl, Br, I, and the like); $Pg^3$ represents an amino protecting group (e.g., t-butoxycarbonyl (Boc) group, benzyl group, benzyloxycarbonyl group, and the like); and a known compound may be used as a compound represented by formula (P1) or a compound represented by the formula:

[Formula 79]

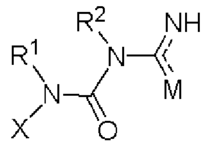

or a compound derived from a known compound by a conventional method may be used.
By reacting a compound represented by formula (P1) with a compound represented by the following:

[Formula 80]

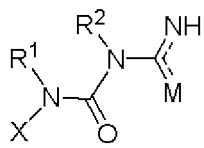

a compound of formula (P2) can be synthesized. Moreover, by reacting a compound of formula (P2) in the same manner as Method Z-1 described above, Compound I-E, that is, a compound having a group represented by the following:

[Formula 81]

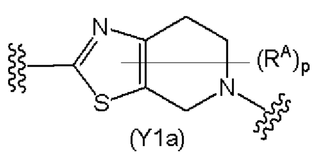

(Y1a)

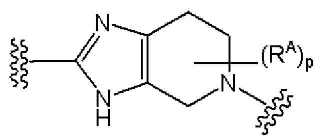

(Y9k)

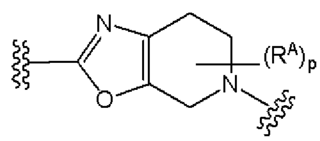

(Y9l)

can be synthesized.

(General Synthesis Method 9) When Y is represented by the following:

[Formula 81A]

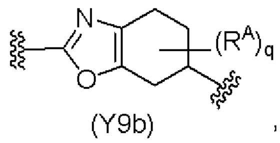
(Y9b) ,
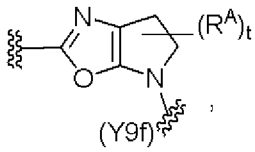
(Y9f) ,
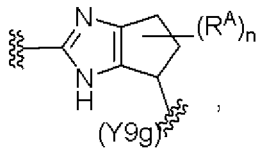
(Y9g) ,
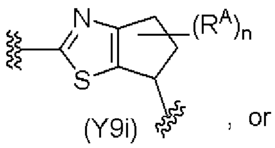
(Y9i) , or
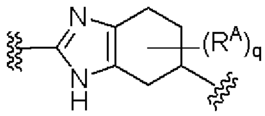
(Y9n)

that is, when Y is represented by the following:

[Formula 81B]

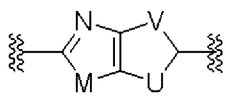
(M=S, O, $NR^M$)

wherein each symbol is defined the same as above; U is $-CH_2-$; and M represents S, O, and $NR^M$ wherein $R^M$ represents hydrogen, substituted or unsubstituted alkyl, substituted or unsubstituted alkoxy, or the like, then Compound 1-F can be synthesized by Method Y-4 and X-2, X-3, or X-4.

[Formula 81C]

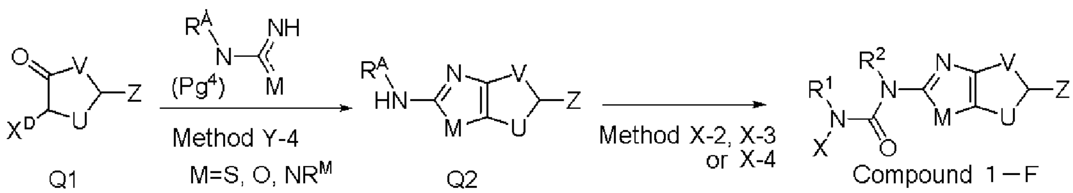


wherein each symbol is defined the same as above; $X^D$ represents halogen (e.g., Cl, Br, I, and the like); and $Pg^4$ represents an amino protecting group (e.g., t-butoxycarbonyl (Boc) group, benzyl group, benzyloxycarbonyl group, and the like).

(General Synthesis Method 10) When Y is a group represented by the following:

[Formula 82]

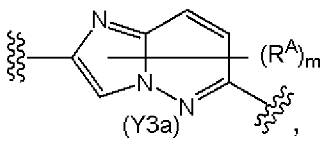
(Y3a),
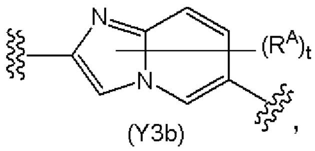
(Y3b),
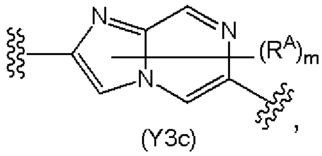
(Y3c),
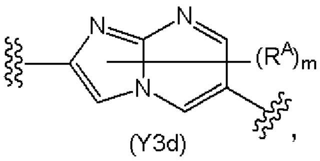
(Y3d),
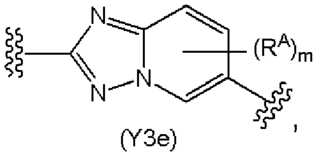
(Y3e),
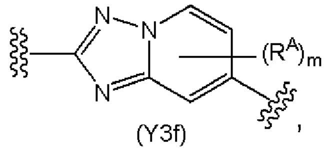
(Y3f),
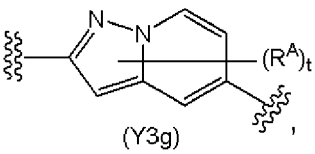
(Y3g),
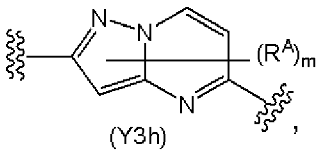
(Y3h),
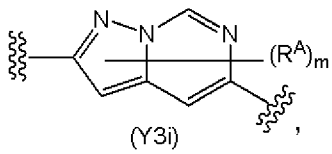
(Y3i),
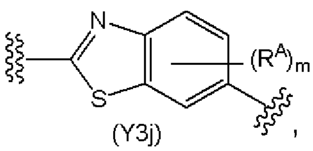
(Y3j),
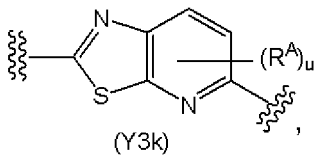
(Y3k),
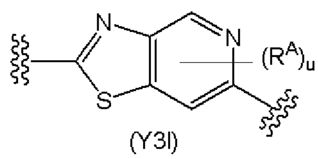
(Y3l)

an amine that is an intermediate of a group represented by (Y3a) to (Y31) shown above can be synthesized by reference to methods described in International Publication No. 2007/095588, International Publication No. 2009/010530, or the like. A urea derivative can be synthesized from the amine by reference to General Synthesis Methods 1, 2, 3, and 4 described above.

(General Synthesis Method 11) When Y is a group represented by the following:

[Formula 83]

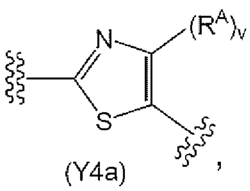
(Y4a),
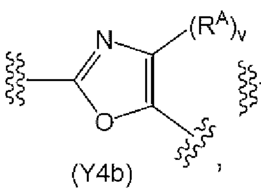
(Y4b),
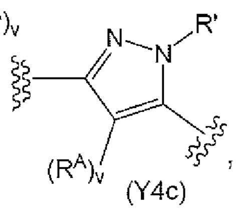
(Y4c),
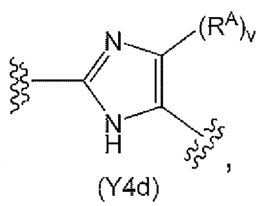
(Y4d),

an amine that is an intermediate of a group represented by (Y4a) to (Y4d) shown above can be synthesized by reference to methods described in Patent Document 13, International Publication No. 2007/129052, or the like. A urea derivative can be synthesized from the amine by reference to General Synthesis Methods 1, 2, 3, and 4 described above.

(General Synthesis Method 12) When Y is a group represented by the following:

[Formula 84]

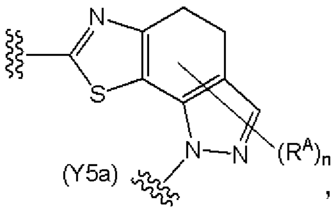
(Y5a) ,
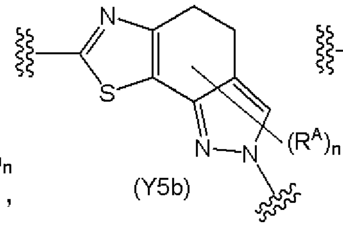
(Y5b) ,
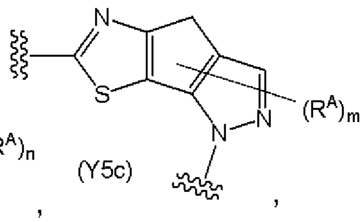
(Y5c) ,
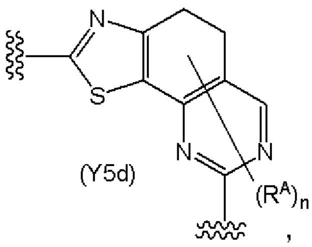
(Y5d) ,
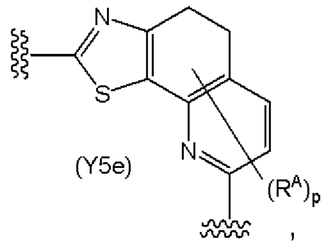
(Y5e) ,
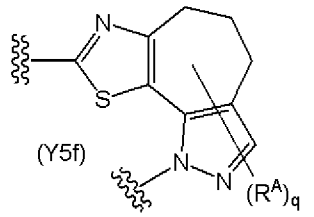
(Y5f) ,
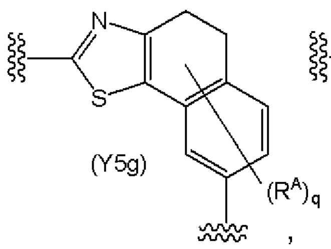
(Y5g) ,
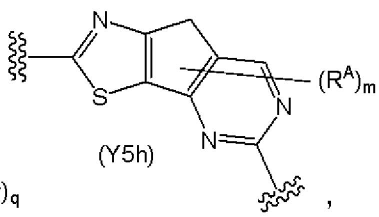
(Y5h) ,
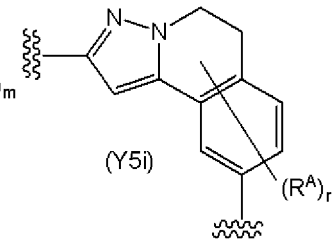
(Y5i)

an amine that is an intermediate of a group represented by (Y5a) to (Y5h) shown above can be synthesized by reference to methods described in Patent Document 15 or 16, or the like. In addition, an amine that is an intermediate of a group represented by (Y5i) shown above can be synthesized by reference to a method described in Journal of the Chemical Society, Perkin Transactions 1: Organic and Bio-Organic Chemistry (1972-1999), (1), 9-12; 1986. A urea derivative can be synthesized from the amine by reference to General Synthesis Methods 1, 2, 3, and 4 described above.

(General Synthesis Method 13) When Y is a group represented by the following:

[Formula 85]

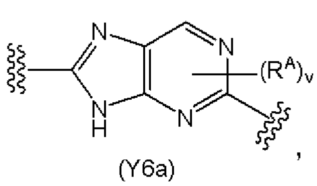
(Y6a) ,
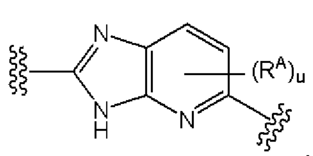
(Y6b) ,
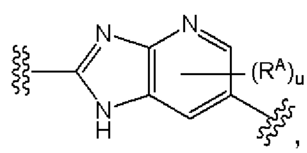
(Y6c) ,
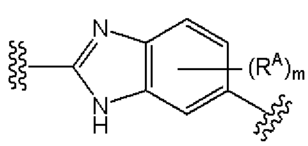
(Y6d)

an amine that is an intermediate of a group represented by (Y6a) to (Y6d) shown above can be synthesized by reference to methods described in International Publication No. 2008/116129, International Publication No. 2008/118468, International Publication No. 2007/135398, or the like. In addition, a urea derivative can be synthesized from the amine by reference to General Synthesis Methods 1, 2, 3, and 4 described above.

(General Synthesis Method 14) When Y is a group represented by the following:

[Formula 86]

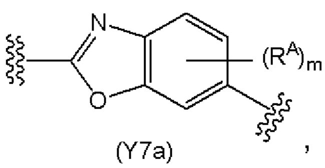
(Y7a) ,
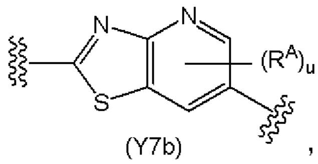
(Y7b) ,
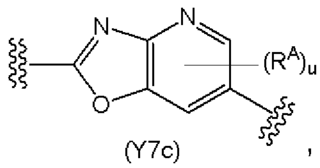
(Y7c) ,

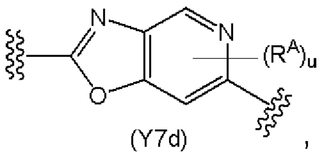
(Y7d) ,
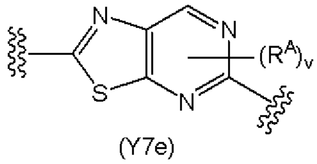
(Y7e)

an amine that is an intermediate of a group represented by (Y7a) to (Y7e) shown above can be synthesized by reference to methods described in International Publication No. 2009/017822, International Publication No. 2008/152390, or the like. A urea derivative can be synthesized from the amine by reference to General Synthesis Methods 1, 2, 3, and 4 described above.

(General Synthesis Method 15) When Y is a group represented by the following:

[Formula 87]

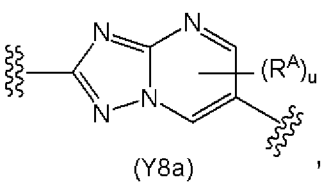
(Y8a) ,
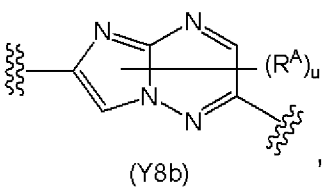
(Y8b) ,
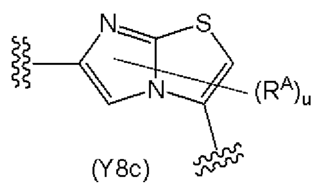
(Y8c)

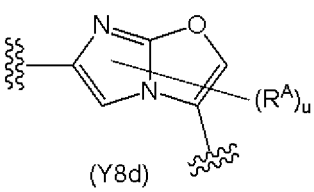
(Y8d)

an amine that is an intermediate of a group represented by (Y8a) to (Y8d) shown above can be synthesized by reference to methods described in International Publication No. 2009/021990, International Publication No. 2009/021992, or the like. A urea derivative can be synthesized from the amine by reference to General Synthesis Methods 1, 2, 3, and 4 described above.

(General Synthesis Method 16) When Y is a group represented by the following:

[Formula 88]

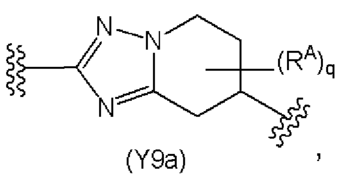

(Y9a) ,

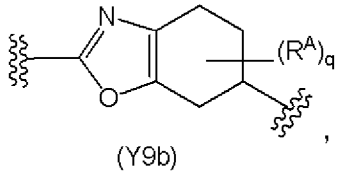

(Y9b) ,

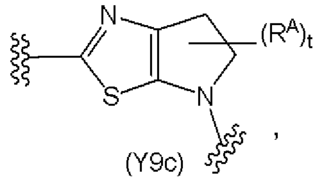

(Y9c) ,

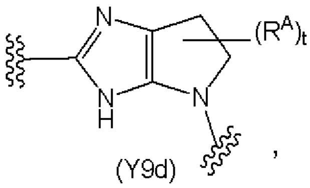

(Y9d) ,

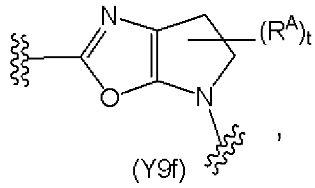

(Y9f) ,

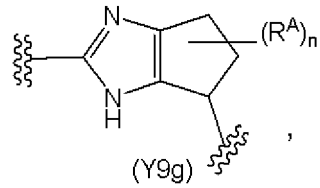

(Y9g) ,

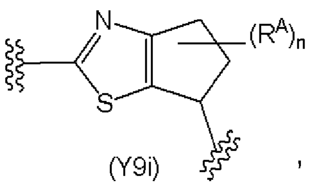

(Y9i) ,

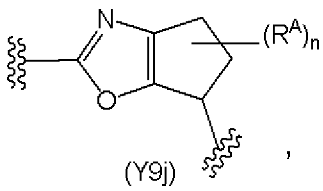

(Y9j) ,

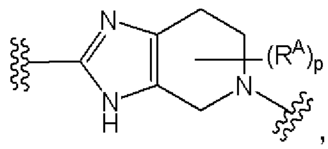

(Y9k) ,

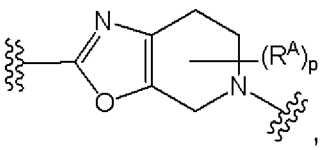

(Y9l) ,

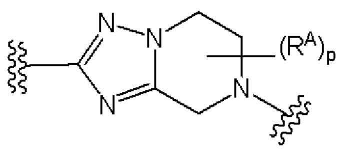

(Y9m) or

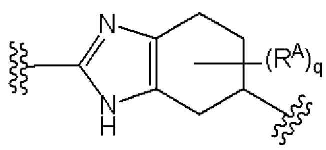

(Y9n)

an amine that is an intermediate of a group represented by (Y9a) to (Y9n) shown above can be synthesized by reference to General Synthesis Methods in the present specification. A urea derivative can be synthesized from the amine by reference to General Synthesis Methods 1, 2, 3, and 4 described above.

(General Synthesis Method 17) When Y is a group represented by the following:

[Formula 89]

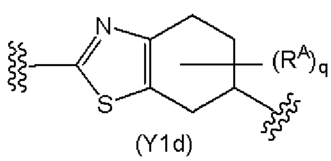

(Y1d)

Y can be synthesized by reference to Bioorganic & Medicinal Chemistry Letters, 15(22), 4910-4914; 2005. For example, Y can be synthesized in accordance with the following scheme.

[Formula 90]

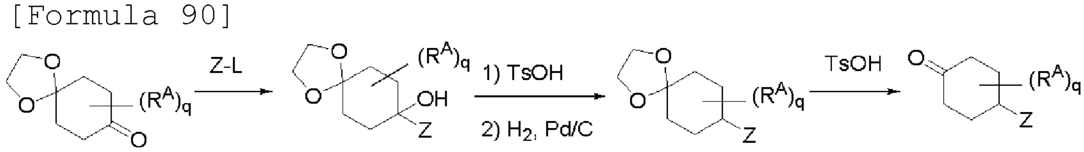


H1 H2 H3 H4

wherein each symbol is defined the same as above; a known compound may be used as a compound represented by formula (H1), or a compound derived from a known compound by a conventional method may be used; and L represents lithium halide or magnesium halide.

Moreover, in accordance with Journal of Medicinal Chemistry, 30(3), 494-8; 1987, a compound represented by formula (H5) can be synthesized from a compound represented by formula (H4).

[Formula 91]

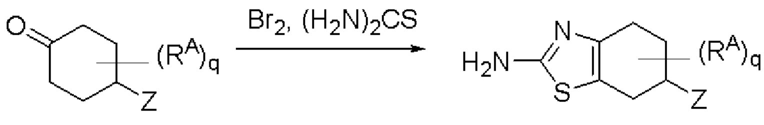

H4 H5

wherein each symbol is defined the same as above.

As another method, in accordance with Journal of Medicinal Chemistry, 51(10), 3005-3019; 2008, a compound represented by formula (H5) can be synthesized.

[Formula 92]

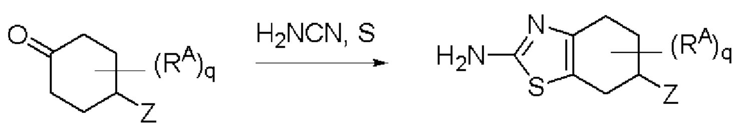

H4 H5

wherein each symbol is defined the same as above.

A urea derivative can be synthesized from a compound represented by formula (H5) in accordance with General Synthesis Methods 1, 2, 3, and 4.

(General Synthesis Method 18) When Y is a group represented by the following:

[Formula 93]

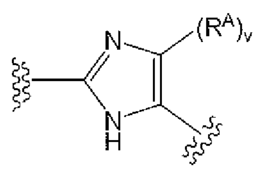

(Y4d)

Y can be synthesized by reference to Journal of Combinatorial Chemistry, 10(1), 118-122; 2008. For example, Y can be synthesized in accordance with the following scheme.

[Formula 94]

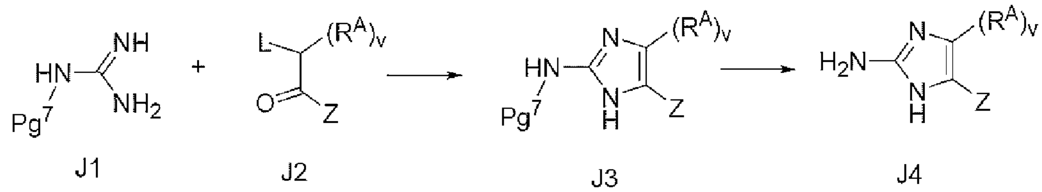


wherein each symbol is defined the same as above; a known compound may be used as a compound represented by formula (J1), or a compound derived from a known compound by a conventional method may be used; $Pg^7$ represents an amino protecting group (e.g., acetyl group and the like); L represents halogen (e.g., Br, I, and the like) or a leaving group (e.g., OTf group (trifluoromethanesulfonate ester) and the like).

A urea derivative can be synthesized from a compound represented by formula (J4) in accordance with General Synthesis Methods 1, 2, 3, and 4 described above.

(General Synthesis Method 19) When Y is a group represented by the following:

[Formula 95]

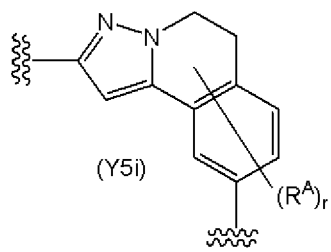


Y can be synthesized by reference to Journal of the Chemical Society, Perkin Transactions 1, (1), 9-12; 1986. For example, Y can be synthesized in accordance with the following scheme.

[Formula 96]

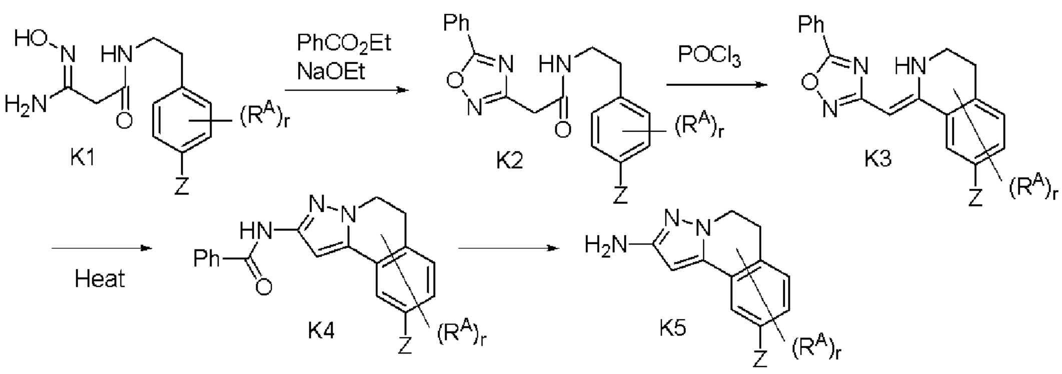


wherein each symbol is defined the same as above, and a known compound may be used as a compound represented by formula (K1), or a compound derived from a known compound by a conventional method may be used. After a compound represented by formula (K4) is synthesized in accordance with the foregoing reference, a compound represented by formula (K5) can be synthesized by the deprotection of the benzoyl group. A urea derivative can be synthesized from a compound represented by formula (K5) in accordance with General Synthesis Methods 1, 2, 3, and 4 described above.

In the above general synthesis methods, reaction steps are not limited to the above ones, and the compound

of the present invention can be synthesized after changing the order of reactions. For example, Compound I-D of the present invention can be synthesized by reacting the compound represented by formula (N1) using Method Z-1 of General Synthesis Method 4, Method X-1 of General Synthesis Method 1, and Method X-2 thereof in that order.

The compound of the present invention can be protected with a protecting group(s). For example, it can be produced by protecting an appropriate substituent using a method known in the relevant field among, typically, halogen (I, Br, Cl, F, and the like), lower (which, here, typically refers to C1-C6, but is not limited thereto) alkoxy, lower alkylthio, lower alkylsulfonyloxy, arylsulfonyloxy, and the like). Examples of such protecting groups can include protecting groups, such as ethoxycarbonyl, *t*-butoxycarbonyl, acetyl, benzyl, and the like, which are described in "Protective Groups in Organic Synthesis," written by T. W. Green (John Wiley & Sons Inc., second ed., 1991), or the like. Methods for the introduction and removal of a protecting group are methods commonly used in synthetic organic chemistry (see, for example, methods described in "Protective Groups in Organic Synthesis," written by T. W. Greene (John Wiley & Sons Inc., second ed., 1991), or the like) or can be obtained in accordance therewith. Furthermore, a functional group included in each substituent can be converted by a known method (for example, those described in Comprehensive Organic Transformations, written by R. C. Larock (1989), and the like) in addition to the above production methods. Some of the compounds of the present invention can be used as a synthetic intermediate, leading to a new derivative. Intermediates and target compounds produced in each of the above production methods can be isolated and purified by a purification method commonly used in synthetic organic chemistry, for example, subjecting them to neutralization, filtration, extraction, washing, drying, concentration, recrystallization, any kind of chromatography, or the like. Furthermore, intermediates can be subjected to a next reaction without further purification.

(Medicament)

The compound of the present invention or a pharmaceutically acceptable salt can be administered alone as it is, but it is usually preferable to provide it as a variety of pharmaceutical formulations. Furthermore, those pharmaceutical formulations are used for an animal and a human.

With regard to an administration route, it is preferable to use the most effective route on therapy. It can be peroral administration, or parenteral administration, for example, intrarectal; intraoral; subcutaneous; intramuscular; intravenous; and the like.

Dosage forms include capsule, tablet, granule, powder, syrup, emulsion, suppository, injection, and the like. A liquid preparation, such as emulsion and syrup, which is suitable for oral administration, can be produced using: water; sugars such as sucrose, sorbite, fructose, and the like; glycols such as polyethylene glycol, propylene glycol, and the like; oils such as sesame oil, olive oil, soybean oil, and the like; antiseptics such as *p*-hydroxybenzoate esters, and the like; and flavors such as strawberry flavor, peppermint, and the like. Furthermore, a capsule, a tablet, a powder, a granule, and the like can be produced using: an excipient such as lactose, glucose, sucrose, mannite, and the like; a disintegrator such as starch, sodium alginate and the like; a lubricant such as magnesium stearate, talc, and the like; a binder such as polyvinyl alcohol, hydroxypropylcellulose, gelatin, and the like; surfactant such as fatty ester and the like; and a plasticizer such as glycerin and the like.

A formulation suitable for parenteral administration preferably consists of a sterilized-water-based formulation containing an active compound and being isotonic to blood of a recipient. For example, in the case of injection, a solution for an injection is prepared using: a carrier consisting of a salt solution, a glucose solution, or a mixture of salt water and a glucose solution; and the like.

A topical formulation is prepared by dissolving or suspending an active compound in one or more kinds of media, such as mineral oil, petroleum, polyalcohol, and the like, or other bases used for a topical pharmaceutical formulation. A formulation for enteral administration is prepared using a general carrier such as cacao butter, hydrogenated fat, hydrogenated fatty carboxylic acid, and the like, and then provided as a suppository.

In the present invention, to a parenteral agent can be added one or more kinds of auxiliary ingredients selected from glycols, oils, flavors, antiseptics (including antioxidants), excipients, disintegrators, lubricants, binders, surfactants, plasticizer, and the like exemplified in an oral agent.

An effective dose and the frequency of administration of a compound of the present invention or a pharmaceutically acceptable salt thereof are different according to administration form, the age of a patient, weight, characteristics or the severity, and the like of a condition to be treated. Generally, a dose is 0.01 to 1000 mg/person per day, preferably 5 to 500 mg/person per day, and a frequency of administration is preferably once per day or divided administration.

All compounds of the present invention are immediately applicable to therapeutic use as kinase inhibitors for controlling kinase dependent diseases in mammals, particularly, kinase inhibitors related to phosphatidylinositol-3-kinase.

Compounds of the present invention are preferably such compounds as having an $IC_{50}$ value in a range of 0.1 nmol/L to 10 μmol/L. A certain compound of the present invention wherein the compound is capable of selectively inhibiting one (e.g., α, β, γ, or δ) or more of four types of Class I phosphatidylinositol-3-kinase can be selected. For

example, by utilizing a compound selectively inhibiting only γ type, merely diseases related to inflammation, such as a lymphocyte and the like can be treated. In the case that a compound is α-type selective, the utility as a selective anticancer agent can be found.

Phosphatidylinositol-3-kinase dependent diseases include inflammatory diseases (allergic diseases (allergic dermatitis/allergic rhinitis, and the like), articular rheumatism, anaphylaxis, and the like), arteriosclerosis, vascular/circulatory diseases, cancer/tumors (hyperproliferative malfunction), immune system diseases, cell-proliferative diseases, infectious diseases, and the like initiated/maintained by unusual phosphatidylinositol-3-kinase enzyme activity. Examples thereof include psoriasis, pulmonary fibrosis, glomerulonephritis, cancers, atherosclerosis, and antiangiogenesis (e.g., tumor growth, diabetic retinopathy). Specifically, for example, the pharmaceutical composition of the present invention may be used for the prophylaxis and/or as a therapeutic agent for diseases such as encephalitis, myelitis and encephalomyelitis, meningitis, inflammatory polyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like, or used as a therapeutic agent for burn or traumatic inflammation.

The present invention is also related to a system, an apparatus, and a kit for producing a pharmaceutical composition of the present invention. It is understood that, as elements of such a system, an apparatus, and a kit, matters publicly known in the relevant field are available, and those skilled in the art can appropriately design them.

The present invention is also related to a system, an apparatus, and a kit using a compound of the present invention, a pharmaceutically acceptable salt thereof, or a prodrug thereof (such as a hydrate thereof and the like). It is understood that, as elements of such a system, an apparatus, and a kit, matters publicly known in the relevant field are available, and those skilled in the art can appropriately design them.

Wortmannin, which is a classical PI3K inhibitor, has low inhibition selectivity, high toxicity, and the like, and consequently is highly cytotoxic. Thus, by using a usual test to measure cytotoxicity, a PI3K inhibitor (or another class of a kinase inhibitor) that intends to cause an unpreferable side effect due to lack of the selectivity can be identified.

Compounds of the present invention have utility as a medicament. Here, utility as a medicament includes the following points: the compound has good metabolic stability; the induction of a drug-metabolizing enzyme is low; the inhibition of a drug-metabolizing enzyme which metabolizes another drug is low; the compound has high oral absorbency; the clearance is low; the half-life is sufficiently long to express the efficacy; or the like.

Reference including scientific literature, patents, patent applications, and the like cited herein is incorporated herein by reference in its entirety at the same level as the case where each reference is specifically described.

Hereinafter, Examples describe the constitution of the present invention in more detail, but the present invention is not limited thereto. Regarding reagents and the like used below, those commercially available were used unless specified otherwise.

EXAMPLES

Hereinafter, the present invention is described in more detail with Examples. However, the technical scope of the present invention is not limited by the Examples and the like.

With regard to instruments used, measurement conditions, and the like, those described below were adopted.

In the Examples and the specification, abbreviations described below are used.

[Formula 97]

| Abbreviation | Name |
|---|---|
| HATU | O-(7-Azabenzotriazol-1-yl)-1,1,3,3-tetramethyluronium hexafluorophosphate |
| HOBt | 1-Hydroxybenzotriazole |
| HOAt | 1-Hydroxy-7-azabenzotriazole |
| HOSu | 1-Hydroxysuccinimide |
| WSCD | Water-soluble carbodiimide |
| NMP | *N*-Methylpyrrolidone |
| DMSO | Dimethylsulfoxide |
| THF | Tetrahydrofuran |
| DMF | N,N-Dimethylformamide |
| DMA | *N,N*-Dimethylacetamide |
| BINAP | 2,2'-Bis(diphenylphosphino)-1,1'-binaphthyl |
| S-Phos | 2-Dicyclohexylphosphino-2',6'-dimethoxybiphenyl |
| X-Phos | 2-Dicyclohexylphosphino-2',4',6'-triisopropylbiphenyl |
| DPPF | 1,1'-Bis(diphenylphosphino)ferrocene |
| Xantphos | 9,9-Dimethyl-4,5-bis(diphenylphosphino)xanthene |
| $P(t\text{-}Bu)_3$ | Tri(*t*-butyl)phosphine |
| $P(n\text{-}Bu)_3$ | Tri(*n*-butyl)phosphine |
| $PPh_3$ | Triphenylphosphine |
| $Pd(PPh_3)_4$ | Tetrakistriphenylphosphine palladium |
| $Pd(OAc)_2$ | Palladium acetate |
| $Pd_2(dba)_3$ | Tris(dibenzylideneacetone)bispalladium |
| $Pd(dba)_2$ | Bis(dibenzylideneacetone)palladium |
| $PdCl_2(dppf)$ | [1,1'-Bis(diphenylphosphino)ferrocene]-dichloropalladium(II)-dichloromethane complex |
| $PdCl_2(PPh_3)_2$ | Bis(triphenylphosphine)palladium chloride |
| $PdCl_2$ | Palladium chloride |
| DEAD | Diethyl azodicarboxylate |
| DIAD | Diisopropyl azodicarboxylate |
| ADDP | 1,1'-(Azodicarbonyl)dipiperidine |
| MBI | Mechanism-Based Inhibition |
| FAT | Fluctuation Ames Test |

[Formula 98]

| | |
|---|---|
| MCS | ***N*-Chlorosuccinimide** |
| NBS | ***N*-Bromosuccinimide** |
| Ph | **Phenyl** |
| Me | **Methyl** |
| Boo | ***t*-Butoxycarbonyl** |
| Cbz | **Benzyloxycarbonyl** |

With regard to LC/MS, measurements were performed under conditions described below.

(Method A)

Column: Waters Phenomenex Luna C18 (2) (5 μm, 50 x 4.6 mm)
Flow rate: 3 mL/min
UV detection wavelength: 254 nm
Mobile phase: [A] was aqueous 0.1% formic-acid-containing solution, and [B] was 0.1% formic-acid-containing solution in acetonitrile.

Mobile phase used a mixture of 90% of solvent [A] and 10% of solvent [B], in a 0 to 3 min time period the gradient of solvent [B] increased from 10% to 100%. After 3 min, a solution of 100% of [B] was used as the mobile phase.

(Method B)

Column: Waters Xbridge C18 (5 μm 50 x 4.6 mm)
Flow rate: 2 mL/min
UV detection wavelength: 254 nm
Mobile phase: [A] was aqueous 10 mmol/L ammonium-carbonate-containing solution, and [B] was acetonitrile.

Mobile phase used a mixture of 90% of solvent [A] and 10% of solvent [B], in a 0 to 3 min time period the gradient of solvent [B] increased from 10% to 100%. After 3 min, a solution of 100% of [B] was used as the mobile phase.

(Method C)

Column: Shimadzu Shim-pack XR-ODS (2.2 μm, 50 x 3.0 mm)
Flow rate: 1.6 mL/min
UV detection wavelength: 254 nm
Mobile phase: [A] was aqueous 0.1% formic-acid-containing solution, and [B] was 0.1% formic-acid-containing solution in acetonitrile.

Mobile phase used a mixture of 90% of solvent [A] and 10% of solvent [B], in a 0 to 3 min time period the gradient of solvent [B] increased from 10% to 100%. After 3 min, a solution of 100% of [B] was used as the mobile phase.

(Method D)

Column: Waters Xbrigde C18 (5 μm, 4.6 x 50 mm)
Flow rate: 3 mL/min
UV detection wavelength: 254 nm
Mobile phase: [A] was aqueous 0.1% formic-acid-containing solution, and [B] was 0.1% formic-acid-containing solution in acetonitrile.

Mobile phase used a mixture of 90% of solvent [A] and 10% of solvent [B], in a 0 to 3 min time period the gradient of solvent [B] increased from 10% to 100%. After 3 min, a solution of 100% of [B] was used as the mobile phase.

(Method F)

Column: Gemini-NX (5 μm, 4.6 x 50 mm) (Phenomenex)

Flow rate: 3 mL/min
UV detection wavelength: 254 nm
Mobile phase: [A] was aqueous 0.1% formic-acid-containing solution, and [B] was 0.1% formic-acid-containing solution in methanol.

Mobile phase used a mixture of 95% of solvent [A] and 5% of solvent [B], in a 0 to 3.5 min time period the gradient of solvent [B] increased from 5% to 100%. After 3.5 min, a solution of 100% of [B] was used as the mobile phase.

(Synthesis examples)

(Reference example 1)

[Formula 99]

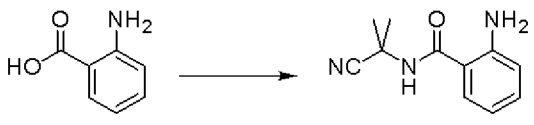

1 2

To a solution of Compound 1 (150 mg, 1.09 mmol) in dimethylformamide (3.0 mL), 2-amino-2-methylpropanenitrile (148 mg, 2.19 mmol), 0-(7-azabenzotriazol-1-yl)-N,N,N',N'-tetramethyluronium hexafluorophosphate (499 mg, 1.31 mmol) and triethylamine (364 μL, 2.63 mmol) were added. The reaction mixture was stirred under flow of nitrogen at room temperature for 4 and a half hours. Aqueous saturated sodium hydrogen carbonate solution was then added to the reaction mixture, followed by extraction with ethyl acetate. The extract was washed sequentially with water and saturated brine, and then dried over anhydrous magnesium sulfate. After concentrating *in vacuo*, purification by silica gel column chromatography (*n*-hexane:ethyl acetate = 4:1 2:1) yielded Compound 2 (139 mg).
LC-MS (Method A): 1.00 min, $[M+H]^+$ = 204
$^1$H-NMR (DMSO-$d_6$) δ: 8.40 (1H, s), 7.51 (1H, d, J = 8.1 Hz), 7.18 (1H, t, J = 8. 1 Hz), 6.72 (1H, d, J = 8. 1 Hz), 6.52 (1H, t, J = 8.1 Hz), 6.41 (2H, s), 1.67 (6H, s).

(Reference example 2)

[Formula 100]

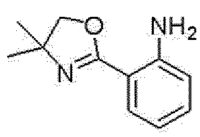

3

Compound 3 was synthesized in accordance with the reference (Journal of the Chemical Society, Perkin Transaction 1, 2000, 999-1001).

(Reference example 3)

[Formula 101]

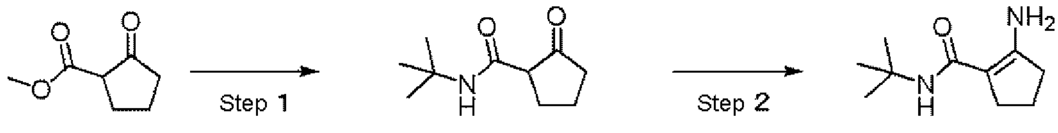


4 5 6

Step 1

To a solution of Compound 4 (500 mg, 3.52 mmol) in toluene (5.0 mL), *t*-butylamine (739 μL, 7.03 mmol) and 4-dimethylaminopyridine (129 mg, 1.06 mmol) were added. The reaction mixture was then stirred under flow of nitrogen at 100°C for 20 hours. The reaction mixture was concentrated *in vacuo,* and then purified by silica gel column chromatography (*n*-hexane:ethyl acetate = 4:1 → 2:1) to yield Compound 5 (417 mg).
$^1$H-NMR ($CDCl_3$) δ: 6.56 (1H, brs), 2.90 (1H, t, J= 9.1 Hz), 2.36-2.27 (4H, m), 2.08-2.00 (1H, m), 1.87-1.76 (1H, m), 1.35 (9H, s).

Step 2

To a solution of Compound 5 (87.9 mg, 0.480 mmol) in methanol (1.0 mL), ammonium acetate (185 mg, 2.40 mmol) was added. The reaction mixture was then stirred under flow of nitrogen at room temperature for 20 hours. After the reaction mixture was concentrated *in vacuo,* the residue was dissolved in methylene chloride (10 mL), washed sequentially with water and saturated brine, and then dried over anhydrous sodium sulfate. Concentrating *in vacuo* yielded mixture 6 (84.4 mg). $^1$H-NMR ($CDCl_3$) δ: 5.72 (2H, brs), 4.85 (1H, brs), 2.45 (2H, t, J = 7.5 Hz), 2.39 (2H, t, J = 6.8 Hz), 1.87-1.80 (2H, m), 1.38 (9H, s).

(Reference example 4)

[Formula 102]

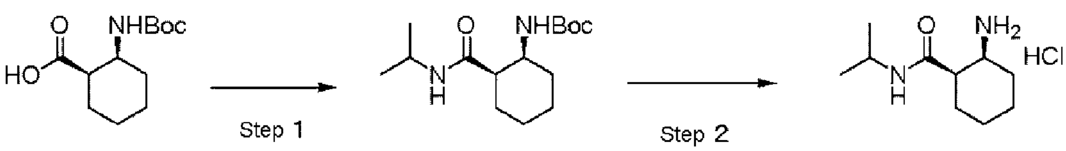


(rac)-7 (rac)-8 (rac)-9

Step 1

To a solution of Compound 7 (150 mg, 0.617 mmol) in dimethylformamide (2.0 mL), isopropylamine (79 μL, 0.925 mmol), O-(7-azabenzotriazol-1-yl)-N,N,N',N'-tetramethyluronium hexafluorophosphate (281 mg, 0.740 mmol) and triethylamine (205 μL, 1.48 mmol) were added. The reaction mixture was then stirred under flow of nitrogen at room temperature overnight. Water was then added to the reaction mixture. The precipitated solid was collected and then washed with water to yield Compound 8 (166 mg).
$^1$H-NMR (DMSO-$d_6$) δ: 7.51 (1H, d, J = 7.8 Hz), 6.25 (1H, s), 3.83-3.75 (2H, m), 2.37 (1H, brs), 1.78-1.72 (2H, m), 1.47-1.23 (15H, m), 1.01 (6H, t, J = 5.7 Hz).

Step 2

To a solution of Compound 8 (60.0 mg, 0.211 mmol) in methylene chloride (1.5 mL), hydrochloric acid (4 mol/L, a dioxane solution) (264 μL, 1.06 mmol) was added. The reaction mixture was then stirred at room temperature for 2 hours. Evaporating the solvent yielded mixture 9 (50.3 mg).
$^1$H-NMR (DMSO-$d_6$) δ: 8.14 (1H, d, J = 6.6 Hz), 8.06 (2H, s), 3.88-3.85 (1H, m), 2.62 (1H, brs), 1.92-1.84 (2H, m),

1.67-1.24 (6H, m), 1.07-1.05 (6H, m).

(Reference example 5)

[Formula 103]

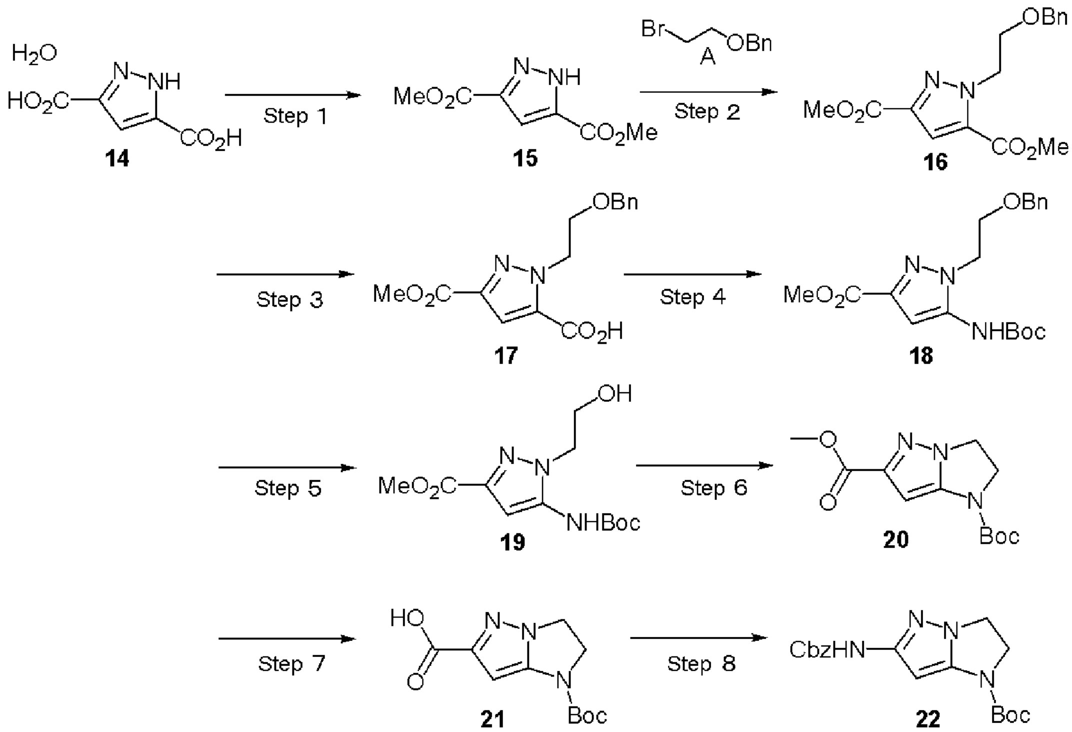


Step 1

To a solution of Compound 14 (60 g, 345 mmol) in methanol (600 mL), under flow of nitrogen, thionyl chloride (75 mL, 1034 mmol) was added at 0˚C. The solution was then heated at reflux for 4 hours. The reaction mixture was concentrated *in vacuo* to yield crude product 15 (64.3 g).
LC/MS (Method A): 0.93 min, $[M+H]^+$ = 185.

Step 2

To an acetonitrile (250 mL) solution of crude product 15 (24.5 g) obtained from Step 1, benzyl-2-bromoethyl ether A (27.3 mL, 173 mmol) and potassium carbonate (27.5 g, 199 mmol) were added. The solution was then heated at reflux under flow of nitrogen for 4 hours and a half. The reaction mixture was filtered and then the filtrate was concentrated *in vacuo.* The residue was dissolved in ethyl acetate, washed sequentially water and saturated brine, then dried over anhydrous magnesium sulfate and concentrated *in vacuo* to yield crude product 16 (54.6 g).
LC/MS (Method A): 1.97 min, $[M+H]^+$ = 319.

Step 3

To a methanol (250 mL) solution of crude product 16 (40.8 g) obtained from Step 2, potassium hydroxide (2.0 mol/L, a methanol solution) (64.1 mL, 128 mmol) was added. The reaction mixture was stirred under flow of nitrogen at room temperature overnight, and then concentrated *in vacuo.* The residue was dissolved in water, acidified, and then extracted with ethyl acetate. The extract was dried over anhydrous magnesium sulfate, and then concentrated *in vacuo* to yield crude product 17 (50.8 g).
LC/MS (Method A): 1.54 min, $[M+H]^+$ = 305.

Step 4

To a *t*-butylalcohol (110 mL) solution of crude product 17 (16.1 g) obtained from Step 3, under flow of nitrogen, diphenylphosphoryl azide (13.7 mL, 63.4 mmol) and triethylamine (9.53 mL, 68.7 mmol) were added. The solution was then stirred at 100˚C for 4 hours. Water was then added to the reaction mixture, followed by extraction with ethyl acetate. The extract was washed sequentially with water and saturated brine, and then dried over anhydrous magnesium sulfate. After concentrating *in vacuo*, purification by silica gel column chromatography (*n*-hexane:ethyl acetate = 2:1 → 1:1) yielded Compound 18 (13.1 g, 66%).
LC/MS (Method A): 2.13 min, $[M+H]^+$ = 376
$^1$H-NMR ($CDCl_3$) δ: 7.91 (1H, s), 7.35-7.25 (5H, m), 6.82 (1H, s), 4.51 (2H, s), 4.37 (2H, t, J = 4.3 Hz), 3.90 (3H, s), 3.85 (2H, t, J = 4.3 Hz), 1.44 (9H, s).

Step 5

To a solution of Compound 18 (13.1 g, 34.9 mmol) in methanol (150 mL), 20% palladium hydroxide-carbon (50% of water content, 1.3 g) was added. The solution was then stirred under flow of hydrogen at room temperature for 6 hours. The reaction mixture was filtered, and then the filtrate was concentrated *in vacuo* to yield Compound 19 (10.3 g, 100%).
LC/MS (Method A): 1.29 min, $[M+H]^+$ = 286
$^1$H-NMR ($CDCl_3$) δ: 7.87 (1H, s), 6.75 (1H, s), 4.28 (2H, s), 4.02 (2H, s), 3.89 (3H, s), 3.02 (1H, brs), 1.50 (9H, s).

Step 6

To a solution of Compound 19 (5.52 g, 19.4 mmol) in tetrahydrofuran (250 mL), under flow of nitrogen, tri-*n*-butylphosphine (5.73 mL, 23.2 mmol) and 1,1'-(azodicarbonyl)dipiperidine (5.86 g, 23.2 mmol) were added at 0˚C. The reaction mixture was stirred at room temperature for 2 hours and a half. The reaction mixture was filtrated, and then the filtrate was concentrated *in vacuo.* Water was then added to the residue, followed by extraction with ethyl acetate. The extract was washed sequentially with water and saturated brine, and then dried over anhydrous magnesium sulfate. After concentrating *in vacuo,* purification by silica gel column chromatography (n-hexane:ethyl acetate = 1:1 → 1:2) yielded Compound 20 (4.59 g, 89%).
LC/MS (Method A): 1.54 min, $[M+H]^+$ = 268
$^1$H-NMR ($CDCl_3$) δ: 6.17 (1H, s), 4.39 (4H, s), 3.91 (3H, s), 1.57 (9H, s).

Step 7

To a solution of Compound 20 (3.51 g, 13.1 mmol) in tetrahydrofuran:methanol (1:1, 50 mL), aqueous 1 mol/L lithium hydroxide solution (26.3 mL, 26.3 mmol) was added. The reaction mixture was stirred at room temperature for 1 hour and a half, and then concentrated *in vacuo.* The residue was dissolved in water, acidified, and then extracted with ethyl acetate. The extract was dried over anhydrous magnesium sulfate, and then concentrated *in vacuo* to yield Compound 21 (2.50 g, 75%).
LC/MS (Method A): 1.26 min, $[M+H]^+$ = 254
$^1$H-NMR (DMSO-$d_6$) δ: 12.64 (1H, brs), 5.95 (1H, s), 4.34 (4H, s), 1.50 (9H, s).

Step 8

To a suspension of Compound 21 (2.50 g, 9.87 mmol) in toluene (50 mL), under flow of nitrogen, benzylalcohol (1.23 mL, 11.9 mmol), diphenylphosphoryl azide (2.55 mL, 11.9 mmol), and triethylamine (1.78 mL, 12.8 mmol) were added. The reaction mixture was then stirred at 100˚C for 5 hours and a half. Water was then added to the reaction mixture, followed by extraction with ethyl acetate. The extract was concentrated *in vacuo,* and then the residue was washed with ethyl acetate:methanol (1:1) to yield Compound 22 (2.19 g, 62%).
LC/MS (Method A): 1.98 min, $[M+H]^+$ = 359
$^1$H-NMR (DMSO-$d_6$) δ: 9.99 (1H, brs), 7.40-7.33 (5H, m), 5.83 (1H, brs), 5.12 (2H, s), 4.21 (2H, t, J = 7.2 Hz), 4.12 (2H, t, J = 7.2 Hz), 1.48 (9H, s).

(Example 1-1)

[Formula 104]

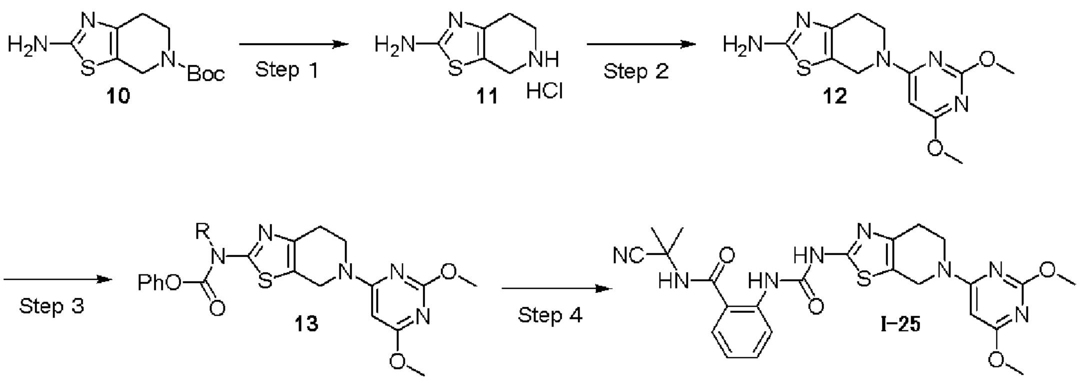


R = H or COOPh

Step 1

Compound 10 was synthesized in accordance with the reference (Heterocycles, 2004, 63(7), 1555-1561). To a solution of Compound 10 (5.00 g, 19.5 mmol) in methylene chloride (75 mL), hydrochloric acid (4 mol/L, a dioxane solution) (24.5 mL, 98.0 mmol) was added. The reaction mixture was then stirred at room temperature for 1 hour and a half. The precipitated solid was collected to yield mixture 11 (4.16 g).
LC-MS (Method A): 0.18 min, $[M+H]^+$ = 156
$^1$H-NMR (DMSO-$d_6$) δ: 9.86 (2H, s), 9.23 (2H, brs), 4.05 (2H, s), 3.34 (2H, s), 2.78 (2H, s).

Step 2

To a dimethylacetamide (20 mL) suspension of mixture 11 (1.99 g) obtained from Step 1, 4-chloro-2,6-dimethoxypyrimidine (1.53 g, 8.72 mmol) and potassium carbonate (4.82 g, 34.9 mmol) were added. The solution was then stirred under flow of nitrogen at 100˚C for 5 hours. Aqueous saturated sodium hydrogen carbonate solution was then added to the reaction mixture, followed by extraction with ethyl acetate. The extract was dried over anhydrous magnesium sulfate, concentrated *in vacuo,* and then purified by amino silica gel column chromatography (chloroform) to yield Compound 12 (1.87 g).
LC-MS (Method A): 0.75 min, $[M+H]^+$ = 294
$^1$H-NMR ($CDCl_3$) δ: 5.57 (1H, s), 4.91 (2H, brs), 4.59 (2H, s), 3.93-3.90 (8H, m), 2.72 (2H, s).

Step 3

To a suspension of Compound 12 (104 mg, 0.354 mmol) in methylene chloride (2.0 mL), under icebath-cooling, pyridine (86 μL, 1.06 mmol) and phenyl chloroformate (116 μL, 0.919 mmol) were added. The solution was then stirred under flow of nitrogen at room temperature for 1 hour. Aqueous saturated sodium hydrogen carbonate solution was then added to the reaction mixture, followed by extraction with ethyl acetate. The extract was washed sequentially with water and saturated brine, and then dried over anhydrous magnesium sulfate to yield mixture 13 (203 mg).
LC-MS (Method A): 1.81 min, $[M+H]^+$ = 414
LC-MS (Method A): 2.24 min, $[M+H]^+$ = 534.

Step 4

To a solution of Compound 2 (55.3 mg, 0.272 mmol) and triethylamine (94 μL, 0.680 mmol) in dimethylsulfoxide (1.5 mL), mixture 13 (93.5 mg) obtained from Step 3 (described above) was added. The solution was then stirred under flow of nitrogen at room temperature overnight. Aqueous saturated sodium hydrogen carbonate solution was then added to the reaction mixture, followed by extraction with ethyl acetate. The extract was washed sequentially with water and saturated brine, and then dried over anhydrous magnesium sulfate. After concentrating *in vacuo*, purification by silica gel column chromatography (*n*-hexane:ethyl acetate = 2:1 → 2:3) yielded Compound I-25 (13.8 mg).
LC-MS (Method A): 1.74 min, $[M+H]^+$ = 523
$^1$H-NMR (DMSO-$d_6$) δ: 11.70 (1H, s), 9.98 (1H, brs), 8.97 (1H, s), 8.10 (1H, d, J = 7.4 Hz), 7.68 (1H, d, J = 7.4 Hz), 7.52 (1H, t, J = 7.4 Hz), 7.15 (1H, t, J = 7.4 Hz), 5.89 (1H, s), 4.69 (2H, brs), 3.94 (2H, brs), 3.82 (3H, s), 3.81 (3H, s), 2.69

(2H, brs), 1.71 (6H, s).

(Example 1-2)

[Formula 105]

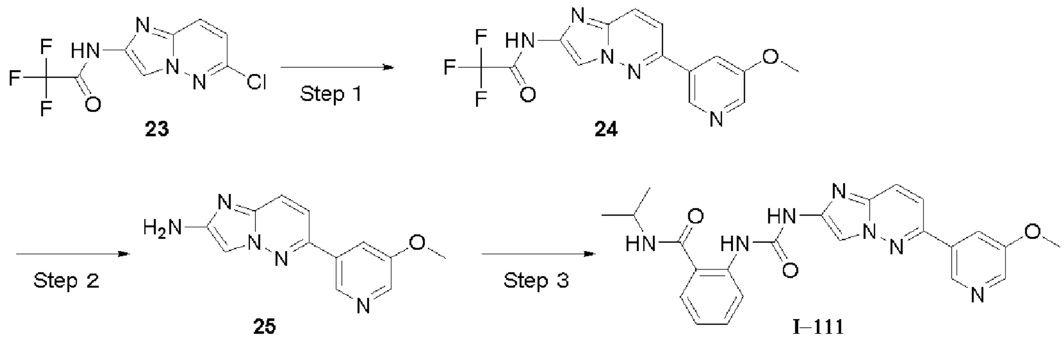


23 24 25 I-111

Step 1

Compound 23 was synthesized using a method described in the Patent Document (International Publication No. 2007/095588 pamphlet). A suspension of Compound 23 (1.50 g, 5.67 mmol), 3-methoxy-5-(4,4,5,5-tetramethyl-1,3,2-dioxyboran-2-yl)pyridine (2.10 g, 8.93 mmol), palladium acetate (90 mg, 0.40 mmol), triphenylphosphine (320 mg, 1.22 mmol), potassium phosphate (3.80 g, 17.90 mmol), and dioxane (12 mL) was allowed to react under irradiation of microwave at 100˚C for 1 hour. The mixture was then partitioned between water and chloroform, and extracted. The chloroform layer was dried over sodium sulfate, and then the solvent was evaporated off. The resulting residue was purified by column chromatography (chloroform/methanol) to yield Compound 24 (172 mg) as a liver brown solid.
$^1$H NMR (DMSO-$d_6$) $\delta$ = 3.95 (3H, s, $OCH_3$), 7.97 (1H, d, J = 9.5Hz), 7.99 (1H, m), 8.23 (1H, d, J = 9.5Hz), 8.44 (1H, d, J = 2.9Hz), 8.46 (1H, s), 8.86 (1H, d, J = 1.7Hz), and 12.68 (1H, br, NH).

Step 2

To a solution of Compound 24 (170 mg, 0.504 mmol) in dioxane (20 mL), aqueous 1 mol/L sodium hydroxide solution (5 mL) was added. After the solution was stirred at room temperature for 2 hours, 2 mol/L hydrochloric acid (2 mL) was added thereto, and then partitioned between water and chloroform, and extracted. The chloroform layer was dried over sodium sulfate, and then the solvent was evaporated off to yield target compound 25 (125 mg).
$^1$H NMR (DMSO-$d_6$) $\delta$ = 3.93 (3H, s, $OCH_3$), 5.60 (2H, br, $NH_2$), 7.40(1H, s), 7.61 (1H, d, 9.2Hz), 7.74 (1H, d, J = 9.2Hz), 7.87 (1H, dd, $J_1$ = 2.8 Hz, $J_2$ = 1.8Hz), 8.36 (1H, d, J = 2.9Hz), and 8.76 (1H, d, J = 1.8Hz).

Step 3

To a suspension of Compound 25 (85 mg, 0.353 mmol) in dichloromethane (8.5 mL), carbonyldiimidazole (85 mg) was added. The suspension was then stirred at 40˚C for 3 hours. The precipitate in the resulting reaction suspension was filtered, washed with dichloromethane, and then subjected to a next reaction. To the precipitate, THF (15 mL), 2-amino-N-isopropylbenzamide (80 mg, 0.45 mmol), and diisopropylethylamine (80 $\mu$L) were then added. The mixture was then stirred at 50˚C for 8 hours. The precipitate was removed by filtration, and then the solvent in the filtrate was evaporated off to give the residue. Water was added to the residue to solidify it. The solid was filtered and then washed with methanol to yield target compound I-111 (20 mg) as a white solid.
$^1$H NMR (DMSO-$d_6$) $\delta$ = 1.19 (6H, d, J = 6.5 Hz, $CHCH_3$), 3.95 (3H, s, $OCH_3$), 4.13 (1H, q, 6.9 Hz, $CHCH_3$), 7.07 (1H, t, J = 7.5Hz), 7.45 (1H, m), 7.60 (1H, m), 7.85 (1H, d, J = 9.4Hz), 7.96 (1H, t, J = 2.4Hz), 8.08 (1H, d, J = 9.4Hz), 8.18 (1H, d, J = 8.4Hz), 8.21 (1H, s), 8.42 (1H, d, J = 2.9Hz), 8.44 (1H, m), 8.84 (1H, d, J = 1.7Hz), 10.14 (1H, s), and 10.59 (1H, s).
LC-MS (Method D) 1.51 min, $[M+H]^+$ = 446.

(Example 1-3)

[Formula 106]

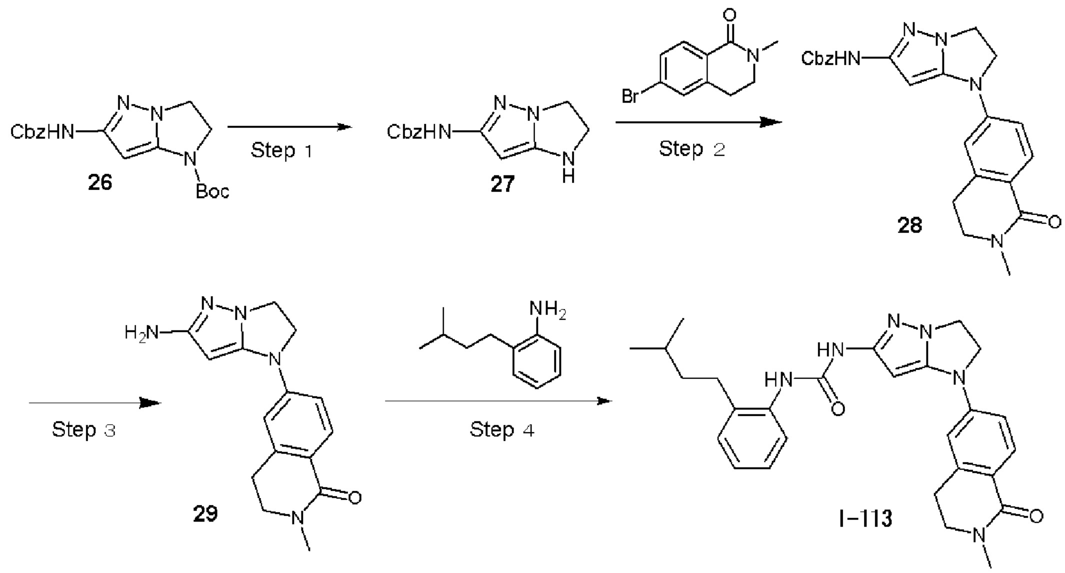


Step 1

Compound 26 was synthesized in accordance with a method described in Reference example 5. To a solution of Compound 26 (35.0 g, 98 mmol) in 1,4-dioxane (250 mL), hydrochloric acid (4 mol/L, a dioxane solution) (122 mL, 488 mmol) was added at room temperature. The reaction mixture was then stirred at 40˚C for 30 minutes and subsequently at 60˚C for 3 hours and a half. After cooling to room temperature, the reaction mixture was concentrated *in vacuo.* Dichloromethane (250 mL) and triethylamine (40.6 mL, 293 mmol) were added to the residue, and then the resulting mixture was stirred at room temperature for 16 hours and a half. The reaction mixture was then concentrated *in vacuo.* The residue was suspended in methanol (100 mL) and chloroform (100 mL), and collected by filtration. The residue was washed with methanol, and then dried in *vacuo* to yield Compound 27 (22.9 g, 89 mmol).
LC-MS (Method C): 1.35 min, $[M+H]^+$ = 259
$^1$H-NMR: (DMSO-$d_6$) δ: 9.82 (1H, s), 7.42-7.28 (5H, m), 5.70 (1H, s), 5.37 (1H, s), 5.09 (2H, s), 3.90-3.81 (2H, m), 3.74-3.66 (2H, m).

Step 2

Compound 27 was synthesized from Step 1, and 5-bromo-2-2-methyl-2H-1,2,3,4-tetrahydroisoquinolin-1-one was synthesized using a method described in United States Patent Application Publication No. 2006/0063799. To a solution of Compound 27 (10 g, 38.7 mmol) and 5-bromo-2-2-methyl-2H-1,2,3,4-tetrahydroisoquinolin-1-one (10.23 g, 42.6 mmol) in 1,2-dimethoxyethane (150 mL), tris(dibenzylideneacetone)dipalladium (1.77 g, 1.93 mmol), dicyclohexyl (2',4',6'-triisopropylbiphenyl-2-yl)phosphine (3.69 g, 7.74 mmol), and potassium phosphate (11.51 g, 54.2 mmol) were added. The mixture was then heated at reflux under flow of nitrogen for 2 hours. After cooling to room temperature, tris (dibenzylideneacetone)dipalladium (1.77 g, 1.93 mmol) and dicyclohexyl(2',4',6'-triisopropylbiphenyl-2-yl)phosphine (3.69 g, 7.74 mmol) were added thereto. The mixture was refluxed under flow of nitrogen for 1 hour. After cooling to room temperature, the reaction mixture was filtered. The residue was washed sequentially with 1,2-dimethoxyethane and water, air-dried, and then dried *in vacuo* at 70˚C to yield crude product 28 (14.4 g).
LC-MS (Method C): 1.82 min, $[M+H]^+$ = 418
$^1$H-NMR (DMSO-$d_6$) δ: 10.04 (1H, s), 7.88-7.80 (1H, m), 7.45-7.30 (5H, m), 6.98-6.92 (1H, m), 6.85-6.80 (1H, m), 6.11 (1H, s), 5.14 (2H, s), 4.44-4.32 (2H, m), 4.28-4.19 (2H, m), 3.56-3.47 (2H, m), 3.03-2.96 (5H, m).

Step 3

To a methanol solution (260 mL) of mixture 28 (13.3 g) obtained from Step 2, formic acid (130 mL, 3.38 mol) and palladium carbon (10 wt%, wet) (4.0 g, 37.6 mmol) were added. The reaction mixture was then refluxed under flow of nitrogen for 2 hours, and then cooled to room temperature and filtered. The residue was washed with methanol, and then the filtrate was concentrated *in vacuo.* Hydrochloric acid (2 mol/L, a methanol solution) (130 mL, 260 mmol) was

added to the mixture, and then the mixture was stirred at room temperature for 30 minutes. After standing overnight at room temperature, the reaction mixture was concentrated *in vacuo,* and then ethyl acetate was added to the residue, and concentrated *in vacuo.* This operation was repeated twice. Thereafter a crude product was washed with ethyl acetate. This crude product was suspended in diethyl ether, and then water (120 mL), sodium hydrogen carbonate (6 g), and water (60 mL) were added thereto. The reaction mixture was stirred for 30 minutes at room temperature, filtered, washed with water, and then air-dried. The crude product was dissolved in mixed solvent of chloroformmethanol, and then filtered. The filtrate was then concentrated in *vacuo.* The residue was washed with mixed solvent of hexane-ethyl acetate, and then dried *in vacuo* to yield Compound 29 (7.86 g, 78% (2 steps)). LC-MS (Method D): 0.62 min, $[M+H]^+$ = 284
$^1$H-NMR: (DMSO-$d_6$) δ: 7.81 (1H, d, J = 8.08Hz), 6.90 (1H, d, J = 8.08Hz), 6.78 (1H, s), 5.28 (1H, s), 4.73 (2H, s), 4.30-4.23 (2H, m), 4.04-3.96 (2H, m), 3.02-2.93 (5H, m) .

Step 4

To an icebath-cooled dichloromethane suspension (1.5 mL) of Compound 29 (30 mg, 0.106 mmol) obtained from Step 3, under flow of nitrogen, pyridine (13 μL, 0.159 mmol) and 4-nitrophenyl chloroformate (27.7 mg, 0.138 mmol) were added. The solution was then stirred at room temperature for 2 and a half hours. The reaction mixture was icebath-cooled, and then 2-isopentylaniline (51.9 mg, 0.318 mmol) and triethylamine (73 μL, 0.529 mmol) were added thereto. The reaction mixture was then stirred at room temperature for 24 hours. Under icebath-cooling, aqueous saturated sodium carbonate and water were then added to the reaction mixture. Chloroform was added to the mixed solution, and then the precipitate was filtered. The residue was washed sequentially with aqueous saturated sodium bicarbonate, water, and ethyl acetate, and then dried *in vacuo.* Ethyl acetate and methanol were added to the crude product, and then the mixture was warmed up. After cooling to room temperature, the mixture was filtered. The residue was washed sequentially with warm ethyl acetate and warm methanol, and then filtrate was concentrated *in vacuo.* Ethyl acetate, methanol, and diisopropyl ether were added sequentially to the concentrated residue, followed by filtration. The residue was washed with mixed solution of diisopropyl ether and ethyl acetate, and then dried *in vacuo* to yield Compound I-113 (2.2 mg, 4.4%).
LC-MS (Method C): 2.38 min, $[M+H]^+$ = 473.

(Example 1-4)

[Formula 107]

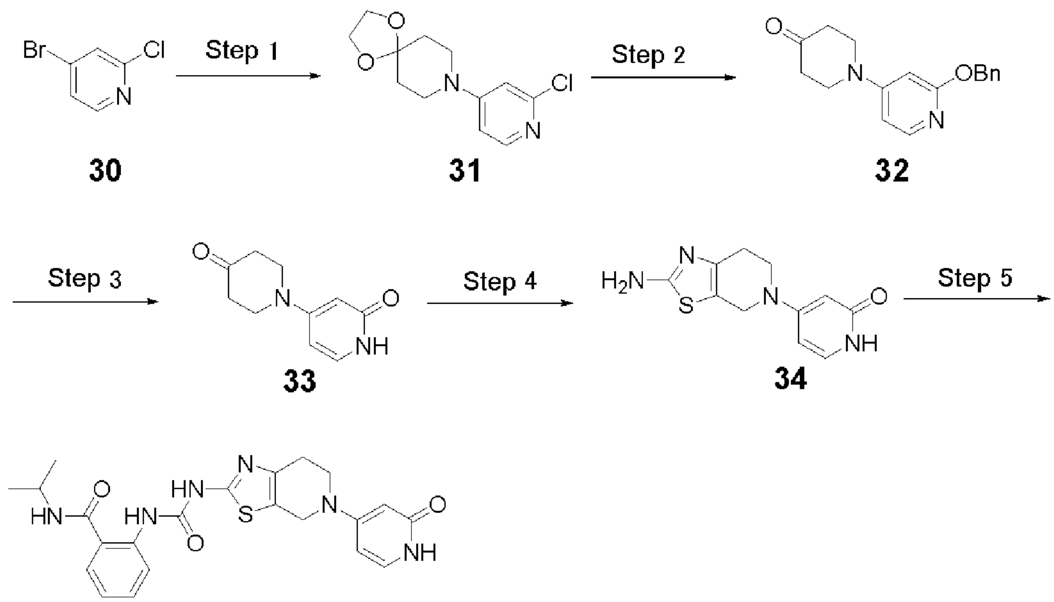

30 31 32
33 34
I-178

Step 1

Compound 30 (6.72 g), which was a commercial product, was dissolved in DMF (25 ml), and then 1,4-dioxa-8-azaspiro[4,5]decane (7.50 g) and potassium carbonate (7.24 g) were added thereto. The solution was then stirred at 90˚C for 6 hours. The reaction mixture was cooled to room temperature, and then water was added thereto, followed by extraction with ethyl acetate. The extract was washed with water, dried over anhydrous magnesium sulfate, and then concentrated *in vacuo.* The resulting residue was purified by silica gel column chromatography (*n*-hexane:ethyl acetate = 1:0 → 0:1) to yield Compound 31 (6.44 g).
$^1$H-NMR (DMSO-$d_6$) δ: 7.92 (1H, d, J = 6.08 Hz), 6.88-6.86 (2H, m), 3.92 (4H, s), 3.49-3.47 (4H, m), 1.65-1.62 (4H, m) .

Step 2

Under flow of nitrogen, N,N'-dimethylimidazolidin-2-one (60 ml) was added to benzyl alcohol (3.06 g), and then, under icebath-cooling, 60% sodium hydride was added thereto. The reaction mixture was warmed to room temperature, and then stirred for 15 minutes. Compound 31 (3.00 g) was added to the reaction mixture, and then stirred further 10 minutes. The reaction mixture was then warmed to 90˚C, and then stirred for 7 hours. The reaction mixture was cooled to room temperature, and then water was added thereto, followed by extraction with ethyl acetate. The extract was washed with water, dried over anhydrous magnesium sulfate, and then concentrated *in vacuo.* THF (50 ml) and aqueous 2 mol/L hydrochloric acid solution (17.6 ml) were added to the resulting residue, and then the mixture was stirred at 60˚C for 2 hours. The reaction mixture was cooled to room temperature, and then aqueous saturated sodium hydrogen carbonate solution was added thereto, followed by extraction with ethyl acetate. The extract was dried over anhydrous magnesium sulfate, and then concentrated *in vacuo.* The resulting residue was purified by silica gel column chromatography (n-hexane:ethyl acetate = 1:0 → 0:1) to yield Compound 32 (2.57 g).
$^1$H-NMR (DMSO-$d_6$) δ: 7.84 (1H, d, J = 6.08 Hz), 7.43-7.28 (5H, m), 6.63 (1H, dd, J = 6.08, 2.03 Hz), 6.27 (1H, d, J = 2.03 Hz), 5.30 (2H, s), 3.70 (4H, t, J = 6.08 Hz), 2.40 (4H, t, J = 6.08 Hz).

Step 3

Compound 32 (2.57 g) was dissolved in methanol (25 ml) and ethyl acetate (25 ml), and then wet 10% Pd carbon powder (515 mg) was added thereto. The solution was stirred under hydrogen atmosphere for 2 hours and 45 minutes at room temperature. Dichloromethane was added to the reaction mixture, followed by filtration through Celite. The filtrate was then concentrated *in vacuo.* Ethyl acetate was added to the resulting residue, and then the solid was collected by filtration to yield Compound 33 (1.73 g).
$^1$H-NMR (DMSO-$d_6$) δ: 10.57 (1H, brs), 7.10 (1H, dd, J = 27.12, 7.35 Hz), 6.03 (1H, dq, J = 22.56, 3.46 Hz), 5.42 (1H, dd, J = 24.84, 2.53 Hz), 3.63 (2H, t, J = 6.08 Hz), 3.24-3.16 (2H, m), 2.42 (2H, t, J = 6.08 Hz), 1.63-1.60 (2H, m).

Step 4

To Compound 33 (1.73 g), cyanamide (0.758 g), and sulfur (0.578 g), under flow of nitrogen, pyridine (17 ml) was added. The solution was warmed to 100˚C, and then stirred for 4 hours. The reaction mixture was then concentrated *in vacuo.* Methanol was added to the resulting residue, and then the solid was collected by filtration to yield Compound 34 (2.01 g).
$^1$H-NMR (DMSO-$d_6$) δ: 10.62 (1H, brs), 7.11 (1H, d, J = 7.60 Hz), 6.81 (2H, s), 6.08 (1H, dd, J = 7.60, 2.53 Hz), 5.45 (1H, d, J = 2.53 Hz), 4.25 (2H, s), 3.61 (2H, t, J = 5.83 Hz).

Step 5

To Compound 34 (100 mg) and carbonyldiimidazole (CDI) (98 mg), under flow of nitrogen, DMF (1 ml) was added. The solution was warmed to 50˚C, and then stirred for 1 hour and a half. The reaction mixture was then cooled to room temperature. The insoluble was collected by filtration, and then washed with dichloromethane to yield an ocherous solid (120 mg).
To a portion of the obtained ocherous solid (50 mg), commercially available 2-amino-N-isopropylbenzamide (28.6 mg) and DMF (0.5 ml) were added. The solution was stirred at 50˚C for 1 hour and a half, and then 2-amino-N-isopropylbenzamide (10.4 mg) and DMF (0.5 ml) were added thereto, subsequently stirring further about 5 hours. The reaction mixture was cooled to room temperature, and then ethyl acetate and water were added to precipitate solid. The solid was collected by filtration, and then washed sequentially with methanol and dichloromethane to yield Compound I-178 (48.4 mg).
LCMS (Method C): 1.38 min, $[M+H]^+$ = 453.05

$^1$H-NMR (DMSO-$d_6$) δ: 11.62 (1H, brs), 10.62 (1H, brs), 10.31 (1H, s), 8.45 (1H, d, J = 8.11 Hz), 8.14 (1H, d, J = 8.62 Hz), 7.62 (1H, dd, J = 7.60, 1.52 Hz), 7.47-7.43 (1H, m), 7.14-7.07 (2H, m), 6.12 (1H, dd, J = 7.60, 2.53 Hz), 5.50 (1H, d, J = 2.53 Hz), 4.41 (2H, s), 4.15-4.07 (1H, m), 3.67 (2H, t, J = 5.58 Hz), 2.69-2.66 (2H, m), 1.18 (6H, d, J = 6.59 Hz).

(Example 1-5)

[Formula 108]

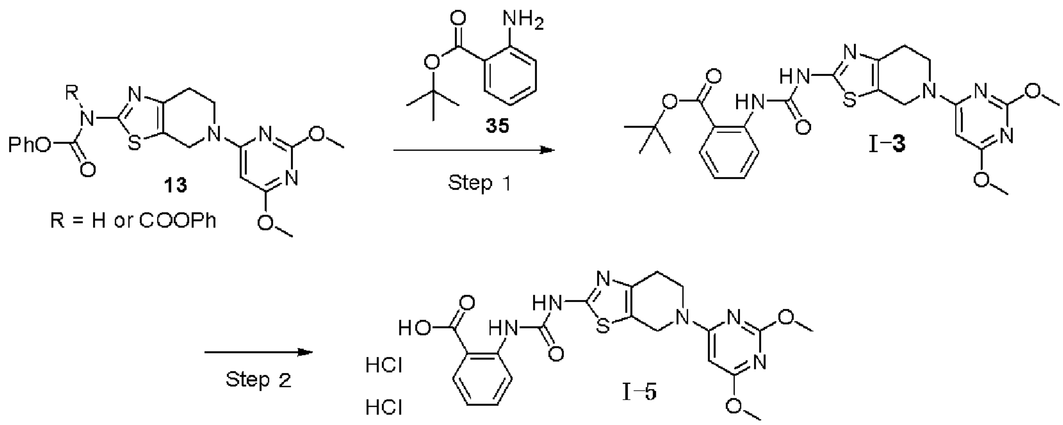


Step 1

Mixture 13 was synthesized in accordance with Example 1-1. To a solution of Compound 35 (52.6 mg, 0.272 mmol) and triethylamine (94 μL, 0.680 mmol) in dimethylsulfoxide (0.3 mL), a solution of mixture 13 (56.2 mg) in dimethylsulfoxide (1.0 mL) was added. The solution was stirred under flow of nitrogen at room temperature for 4 hours. Water was then added to the reaction mixture, followed by extraction with ethyl acetate. The extract was washed sequentially with water and saturated brine, and then dried over anhydrous magnesium sulfate. After concentrating *in vacuo*, purification by silica gel column chromatography (*n*-hexane:ethyl acetate = 2:1 → 1:1) yielded Compound I-3 (35.1 mg) .
LC/MS (Method A): 2.43 min, $[M+H]^+$ = 513
$^1$H-NMR (DMSO-$d_6$) δ: 11.77 (1H, s), 10.25 (1H, s), 8.25 (1H, d, J = 8.8 Hz), 7.90 (1H, d, J = 8.6 Hz), 7.59-7.55 (1H, m), 7.14-7.11 (1H, m), 5.89 (1H, s), 4.69 (2H, s), 3.94 (2H, s), 3.82 (3H, s), 3.81 (3H, s), 2.69 (2H, s), 1.58 (9H, s).

Step 2

To a solution of Compound I-3 (31.2 mg, 0.061 mmol) in methylene chloride (1.0 mL), hydrochloric acid (4 mol/L, a dioxane solution) (0.91 mL, 3.65 mmol) was added. The reaction mixture was then stirred at room temperature overnight. The solvent was evaporated off *in vacuo* to yield Compound I-5 (32.1 mg).
LC/MS (Method A): 1.83 min, $[M+H]^+$ = 457
$^1$H-NMR (DMSO-$d_6$) δ: 10.59 (1H, s), 8.33 (1H, d, J = 8.1 Hz), 7.96 (1H, d, J = 7. 8 Hz), 7.60-7.56 (1H, m), 7.13-7.10 (1H, m), 5.99 (1H, s), 4.76 (2H, s), 4.00 (2H, s), 3.87 (6H, s), 2.72 (2H, s).

(Example 1-6)

[Formula 109]

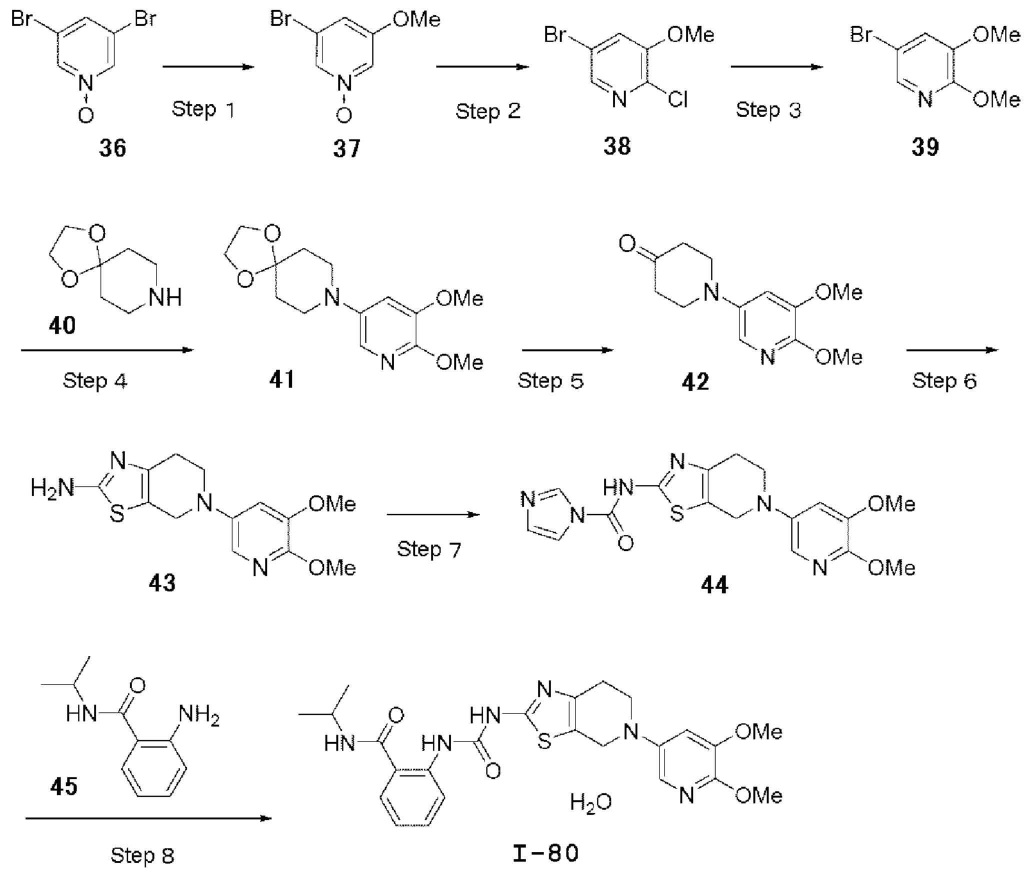


Step 1

Compound 36 (16.44 g), which was a commercial product, was suspended in DME (79 mL), and then a solution of potassium hydroxide (4.56 g) in methanol (79 mL) was added thereto. The solution was then stirred under reflux for 5 hours. The reaction mixture was cooled to room temperature, and then dichloromethane was added thereto. The insoluble was then filtered. After the filtrate was concentrated, methanol and dichloromethane were added thereto, and then the insoluble was filtered again. The resulting filtrate was concentrated to yield Compound 37 (12.84 g).
$^1$H-NMR ($CDCl_3$) δ: 8.02 (1H, m), 7.90 (1H, m), 7.03 (1H, m), 3.85 (3H, s).

Step 2

To Compound 37 (8.87 g), under flow of nitrogen, chloroform (166 mL) was added. Under icebath-cooling, phosphorus oxychloride was then added thereto. The reaction mixture was stirred under reflux for 7 hours. The reaction mixture was cooled, and then aqueous sodium hydrogen carbonate solution and aqueous potassium carbonate solution were added thereto, followed by extraction with dichloromethane. The extract was washed with water, dried over anhydrous magnesium sulfate, and then concentrated *in vacuo.* The resulting residue was purified by silica gel column chromatography (hexane:ethyl acetate = 1:0 → 9:1) to yield Compound 38 (7.07 g).
$^1$H-NMR ($CDCl_3$) δ: 8.06 (1H, d, J = 1.85 Hz), 7.33 (1H, d, J = 1.85 Hz), 3.93 (3H, s).

Step 3

To Compound 38 (11.30 g), under flow of nitrogen, methanol (25 mL) and 28% sodium methoxide/methanol solution (41.75 g) were added. The solution was then stirred under reflux for 7 hours. The reaction mixture was cooled and then concentrated. Water was then added thereto, followed by extraction with ethyl acetate. The extract was washed with saturated brine, then dried over anhydrous magnesium sulfate and concentrated in *vacuo.* The resulting residue

was purified by silica gel column chromatography (hexane:ethyl acetate = 1:0 → 9:1) to yield Compound 39 (10.48 g).
$^1$H-NMR ($CDCl_3$) δ: 7.78 (1H, d, J = 2.01 Hz), 7.14 (1H, d, J = 2.01 Hz), 3.99 (3H, s), 3.87 (3H, s).

Step 4

To a solution of Compound 39 (6.87 g) in toluene (69 mL), under flow of nitrogen, commercially available Compound 40 (2.57 g), palladium dibenzylacetone complex (577 mg), Ru phos (1176 mg), and sodium butoxide (3.63 g) were added. The solution was warmed to 100°C and then stirred for 2 hours. The reaction mixture was cooled, and then the insoluble was filtered. The filtrate was concentrated, and then the resulting residue was purified by silica gel column chromatography (hexane:ethyl acetate = 7:3 → 4:6) to yield Compound 41 (7.55 g).
$^1$H-NMR ($CDCl_3$) δ: 7.37 (1H, d, J = 2.03 Hz), 6.83 (1H, d, J = 2.03 Hz), 4.00 (4H, s), 3.97 (3H, s), 3.86 (3H, s), 3.20 (4H, m), 1.88 (4H, m).

Step 5

To a solution of Compound 41 (9.76 g) in THF (105 mL), 2 mol/L hydrochloric acid (52 mL) was added. The solution was warmed to 40°C and then stirred for 3 hours. The reaction mixture was cooled, and then neutralized with sodium hydrogen carbonate. The reaction mixture was concentrated, and then extracted with chloroform. The extract was dried over anhydrous magnesium sulfate, and then concentrated *in vacuo.* The resulting residue was purified by silica gel column chromatography (hexane:ethyl acetate = 6:4 → 4:6) to yield Compound 42 (6.66 g).
$^1$H-NMR ($CDCl_3$) δ: 7.42 (1H, d, J = 2.44 Hz), 6.85 (1H, d, J = 2.44 Hz), 3.99 (3H, s), 3.89 (3H, s), 3.46 (4H, m), 2.60 (4H, m).

Step 6

To a solution of Compound 42 (8.88 g) in pyridine (17 mL), cyanamide (3.16 g) and sulfur (0.578 g) were added. Under flow of nitrogen, the solution was warmed to 100°C, and then stirred for 2 to 4 hours. The reaction mixture was then concentrated *in vacuo.* Chloroform was added to the resulting residue, which was then collected by filtration to yield Compound 43 (6.26 g).

Step 7

To Compound 43 (8.86 g), under flow of nitrogen, chloroform (100 mL) and CDI (12.20 g) were added. The solution was warmed to 50°C, and then stirred for 2 to 3 hours. The reaction mixture was then cooled to room temperature. The insoluble was collected by filtration, and then washed with chloroform to yield Compound 44 (11.16 g).

Step 8

To Compound 44 (10.75 g), THF (335 mL), commercially available Compound 45 (5.95 g) and diisopropylethylamine (5.8 mL) were added. The mixture was stirred at 50°C for 2 hours. The reaction mixture was cooled to room temperature, and then aqueous saturated ammonium chloride solution was added thereto, followed by extraction with ethyl acetate. The extract was washed sequentially with water and saturated brine, then dried over anhydrous magnesium sulfate and concentrated *in vacuo.* The solid precipitated from the concentrated solution was collected by filtration, and then dissolved in methanol and tetrahydrofuran. Activated carbon was added to the solution, and the suspension was then stirred. The suspension was filtered, and then the filtrate was concentrated. The precipitated solid was then filtrated and collected. The obtained solid was suspended in water, and then filtered. The obtained solid was, without being dried, dissolved in methanol and tetrahydrofuran, and then activated carbon was added thereto. The mixture was stirred and then filtered. The filtrate was then concentrated. The solid precipitated from the concentrated solution was collected by filtration to yield Compound 46 (6.79 g) as a yellow solid.
$^1$H-NMR (DMSO-$d_6$) δ: 11.61 (1H, s), 10.32 (1H, s), 8.45 (1H, d, J = 7.60 Hz), 8.16 (1H, d, J = 8.11Hz), 7.63 (1H, dd, J = 7.86, 1.27 Hz), 7.48-7.43 (1H, m), 7.36 (1H, d, J = 2.53 Hz), 7.14 (1H, d, J = 2.53 Hz), 7.12-7.08 (1H, m), 4.29 (2H, s),4.12 (1H, td, J = 13.69, 6.93 Hz), 3.79 (6H, d, J = 3.55 Hz), 3.54 (2H, t, J = 5.58 Hz), 2.70 (2H, s), 1.16 (6H, dd, J = 18.00, 6.84 Hz).

(Example 1-7)

[Formula 110]

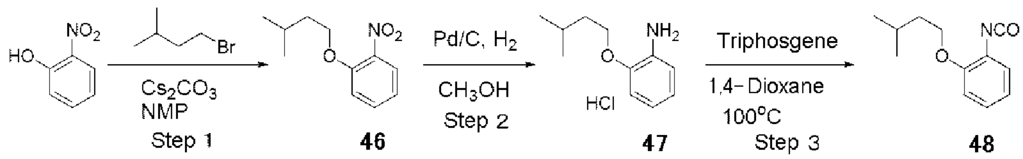


Step 1

A solution of o-nitrophenol (55.1 g, 0.396 mol) in NMP (400 mL), 1-bromo-3-methylbutane (71.8 g, 0.52 mol) and potassium carbonate (82 g, 0.594 mol) were added. The solution was stirred at 80˚C overnight. The reaction mixture was cooled, and then poured into iced water (2L), followed by extraction with ethyl acetate. The ethyl acetate layer was washed sequentially with water and brine, and then dried over sodium sulfate. After the solvent was evaporated off *in vacuo,* the resulting residue (97.88 g) was subject to the next step as it was.

Step 2

To a solution of 46 (97.88 g, 0.468 mol) in methanol (400 mL), 10% palladium carbon (9.79 g) was added. The solution was then stirred under hydrogen atmosphere at 40˚C overnight. The reaction mixture was then filtered through a kieselguhr pad. Hydrochloric acid was added to the filtrate, which was then concentrated to yield 47 as a hydrochloride salt (60 g, y. 62.5%).

Step 3

To a solution of 47 (30 g, 0.168 mol) in dioxane (200 mL), triphosgene (19.84 g, 0.0067 mol) was added. The solution was then refluxed for 3 hours. The solvent was evaporated off, and then partitioned between methyl tert-butyl ether (MTBE) and water. The organic layer was washed sequentially with water and brine, and then dried over sodium sulfate. The solvent was evaporated off to yield target compound 48 as a brown oil (25 g, y. 72.5%).
In the following scheme, $Ac_2O$ denotes acetic anhydride, DMAP denotes N,N-4-dimethylaminopyridine, DCM reflux denotes dichloromethane reflux, $Pd(PPh_3)_2Cl_2$ denotes bis(triphenylphosphine)palladium chloride, and aq. HCl denotes hydrochloric acid.

[Formula 111]

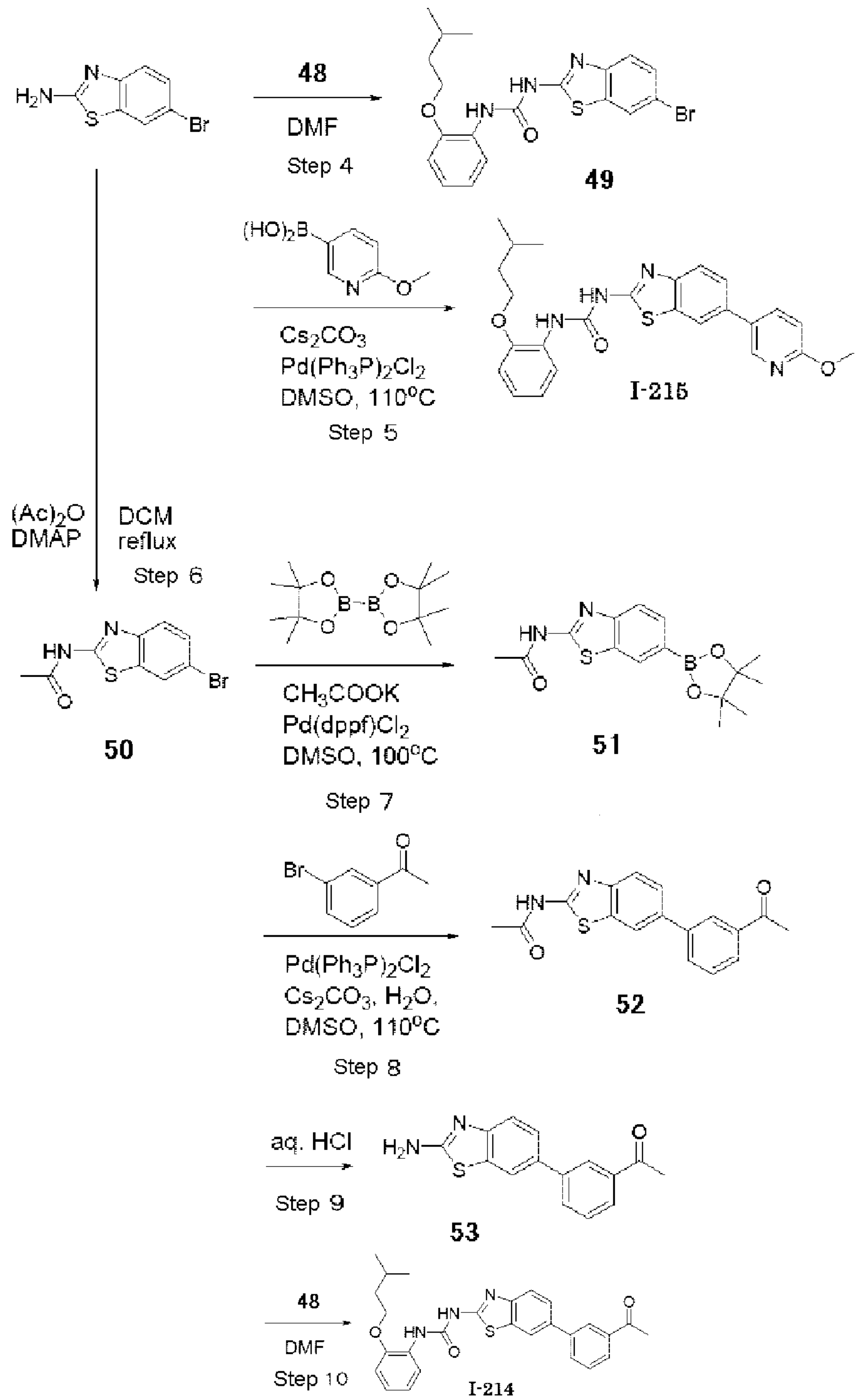


Step 4

To a solution of 2-amino-6-bromobenzothiazole (160 mg, 0.7 mmol) in DMF (7 mL), 48 (200 mg, 0.9 mmol) was added. The solution was then stirred at room temperature overnight. Ethyl acetate and water were then added to the reaction mixture. The organic layer was washed sequentially with water and brine, and then dried over sodium sulfate. The solvent was evaporated off to yield 49 (280 mg, y. 70%).

Step 5

To a solution of 49 (80 mg, 0.18 mmol) in DMSO (7 mL), cesium carbonate (117 mg), 6-methoxypyridinyl-3-boronic acid (34 mg), and $Pd(PPh_3)_2Cl_2$ (26 mg) were added. The solution was then stirred under nitrogen atmosphere at 110˚C overnight. The reaction mixture was cooled, and then partitioned between ethyl acetate and water. The organic layer was washed sequentially with water and brine, and then dried over sodium sulfate. The solvent was then evaporated off. The resulting residue was purified by preparative HPLC to yield I-215 (9 mg, y. 10.5%) as a pale brown solid.
$^1$H-NMR (DMSO-$d_6$): 11.65 (s, 1H), 8.54 (s, 1H), 8.53 (d, 1H), 8.25 (s, 1H), 8.05 (m, 2H), 7.75 (m, 2H), 7.10 (m, 2H), 6.91 (m, 2H), 4.13 (t, 2H), 3.90 (s, 3H), 1.86 (m, 1H), 1.75 (m, 2H), 0.95 (d, 6H).
LC/MS (Method D): 2.63 min, $[M+H]^+$ = 463.14.

Step 6

To a solution of 2-amino-6-bromobenzothiazole (10 g, 0.044 mol) in dichloromethane (150 mL), DMAP (7 g) and acetic anhydride (18 g) were added. The solution was then refluxed overnight. The reaction mixture was concentrated, and then water was added thereto. The precipitate was collected by filtration, then washed with water and dried to yield target compound 50 (10.2 g, y. 85%).

Step 7

To a solution of 50 (5 g, 0.019 mol) in DMSO (100 mL), 4,4,4',4',5,5,5',5'-octamethyl-2,2'-bi(1,3,2-dioxaborolane) (9.7 g), potassium acetate (7.5 g), and $Pd(dppf)Cl_2$ (1.5 g) were added. The solution was stirred at 100˚C overnight. The reaction mixture was cooled, and then partitioned between ethyl acetate and water. The organic layer was washed sequentially with water and brine, and then dried over sodium sulfate. The solvent was evaporated off to yield target compound 51 as a white solid (4.2 g, y. 70%).
$^1$H-NMR (DMSO-$D_6$): 8.32 (s, 1H), 7.88 (d, 1H, J = 8.1), 7.76 (d, 1H, J = 8.1), 2.27 (s, 3H), 1.37 (s, 12H).

Step 8

To a solution of 51 (200 mg, 6 mmol) in DMSO and water, 1-(3-bromophenyl)ethanone (200 mg), cesium carbonate (400 mg), and $Pd(PPh_3)_2Cl_2$ (30 mg) were added. The solution was then stirred under nitrogen atmosphere at 110˚C overnight. The reaction mixture was cooled, and then partitioned between ethyl acetate and water. The organic layer was washed sequentially with water and brine, and then dried over sodium sulfate. The solvent was evaporated off to yield target compound 52 (170 mg, y. 89%).

Step 9

To 52 (1.3 g, 4.2 mmol), 3 mol/L hydrochloric acid (10 mL) was added. The mixture was refluxed for 3 hours, and then the solvent was evaporated off. The resulting solid was collected by filtration, and then washed with aqueous sodium hydrogen carbonate solution to yield target compound 53 (0.92 g, y. 83.6%).

Step 10

To a solution of 53 (150 mg, 0.56 mmol) in DMF (10 mL), 48 (170 mg) was added. The solution was then stirred at room temperature overnight. The reaction mixture was then partitioned between ethyl acetate and water. The organic layer was washed sequentially with water and brine, and then dried over sodium sulfate. The solvent was then evaporated off. The resulting residue was purified by silica column chromatography to yield target compound I-214 as a white solid (130 mg, y. 70%).
$^1$H-NMR (DMSO-$d_6$): 11.68 (s, 1H), 8.36 (s, 1H), 8.26 (s, 1H), 8.14 (d, 1H, J = 7.8), 8.01 (dd, 2H), 7.78 (s, 2H), 7.66 (t, 1H), 7.10-6.91 (m, 3H), 4.13 (t, 2H), 2.68 (s, 3H), 1.99 (m, .1H), 1.87 (m, 2H), 0.97 (d, 6H, J = 6.3). LC/MS (Method D): 2.64 min, $[M+H]^+$ = 474.15.

(Example 1-8)

In the following scheme, SCN-$CO_2$Et denotes ethoxycarbonyl isothiocyanate, DCM denotes dichloromethane, DIPEA denotes diisopropylethylamine, EtOH denotes ethanol, MeOH denotes methanol, DME denotes 1,2-dimethoxyethane, and Ary-$B(OH)_2$ denotes 3-acetylphenylboronic acid.
[Formula 112]

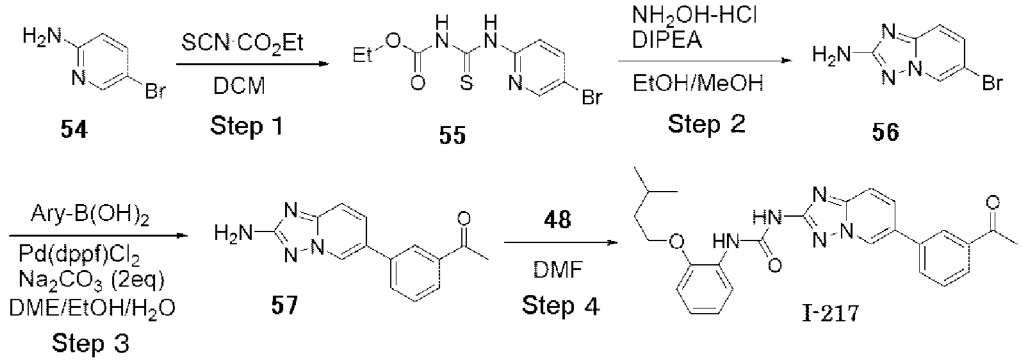


Step 1

To a solution of 2-amino-5-bromopyridine (54: 1.73 g, 10 mmol) in dichloromethane (20 mL), under cooling to 0 to 5˚C, ethoxycarbonyl isothiocyanate (1.29 g) was added dropwise. After the solution was stirred at room temperature overnight, the solvent was evaporated off. The resulting residue was collected by filtration, and then washed with petroleum ether to yield target compound 55 (2.8 g, y. 93%).

Step 2

To a solution of hydroxylamine hydrochloride (345 mg) in ethanol-methanol (1:1) (10 mL), diisopropylethylamine (0.5 mL) was added. The solution was then stirred at room temperature for 1 hour. To the solution, 55 (303 mg, 1 mmol) was added, followed by reflux. After the starting material was consumed, the reaction mixture was cooled. The precipitate was collected by filtration, and then washed sequentially with water, ethanol-methanol (1:1), and diethyl ether to yield target compound 56 (100 mg, y. 51%).
$^1$H-NMR (300 Mz) (DMSO): 6.13 (s, 2H), 7.02 (d, J = 9 Hz, 1H), 7.54 (d, J = 9 Hz, 1H), 8.92 (s, 1H).

Step 3

To a solution of 56 (620 mg, 3 mmol) in DME/$H_2O$/ethanol (7:3:2) (24 mL), 3-acetylphenylboronic acid (550 mg), Pd(dppf)$Cl_2$ (60 mg), and sodium carbonate (480 mg) were added. The solution was then refluxed under nitrogen atmosphere overnight. After the reaction mixture was concentrated, the residue was purified by silica gel column chromatography (ethyl acetate/petroleum ether 1:1) to yield target compound 57 (580 mg, y. 82%) as a white solid.

Step 4

To a solution of 57 (252 mg, 1 mmol) in DMF (10 mL), o-isopentyloxyphenyl isocyanate (48, 400 mg) was added. The solution was heated to 50˚C and stirred overnight. Water, methanol, and ethanol (10 mL each) were then added to the reaction mixture. The precipitate was collected by filtration, and then washed with ethyl acetate to yield target compound I-217 (40 mg, y. 12%) as a white solid.
LC/MS (Method D): 2.36 min, $[M+H]^+$ = 458.17.
$^1$H-NMR (300 Mz) (DMSO): 0.91 (d, J = 3 Hz, 6H), 1.86 (s, 3H), 2.70 (s, 3H), 4.14 (t, J = 6 Hz, 2H), 6.93 (s, 1H), 7.00 (s, 1H), 7.08 (s, 1H), 7.73 (t, J = 9 Hz, 2H), 7.98 to 8.35 (m, 5H), 9.31 (s, 1H), 10.50 (s, 1H), 10.80 (s, 1H).

(Example 1-9)

In the following scheme, KSCN denotes potassium thiocyanate, AcOH denotes acetic acid, $Boc_2O$ denotes dit-butyl dicarbonate, DMAP denotes N,N-4-dimethylaminopyridine, EtOH denotes ethanol, and $Et_3N$ denotes triethylamine.

[Formula 113]

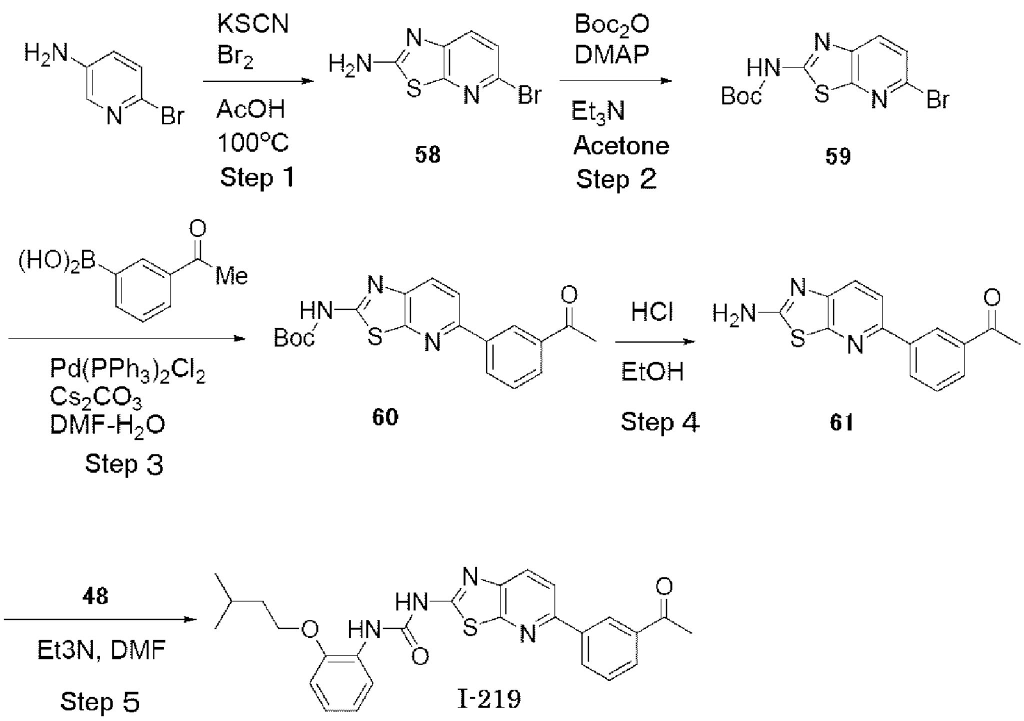


Step 1

To a solution of 2-bromo-5-aminopyridine (3 g, 17.3 mmol) and potassium thiocyanate (4.2 g) in acetic acid (20 mL), bromine (3.3 g) was added dropwise under icebath-cooling. The solution was then stirred for 1 hour and subsequently at 100˚C overnight. After the reaction mixture was cooled, water was added thereto. The precipitate was collected by filtration, then subjected to a next reaction without further purification.

Step 2

To a solution of 58 (3.98 g, 17.3 mmol), $Boc_2O$ (4.91 g), and triethylamine (2.5 g) in acetone (50 mL), DMAP (84 mg) was added. The solution was heated to 50˚C and stirred overnight, and then concentrated. The residue was purified by column chromatography to yield target compound 59 (2.81 g, y. 10.5%).

Step 3

To a solution of 59 (1 g, 4.34 mmol) in DMF (30 mL) and water (8 mL), 3-acetylphenylboronic acid (0.6 g), cesium carbonate (2.96 g), and $Pd(PPh_3)_2Cl_2$ (217 mg) were added. The solution was then heated and stirred under nitrogen atmosphere at 116˚C overnight. The reaction mixture was cooled, partitioned between ethyl acetate and water. The ethyl acetate layer was washed with brine, and then dried over sodium sulfate. The solvent was evaporated off, and then the resulting residue 60 (1 g, y. 91%) was subjected to a next reaction without further purification.

Step 4

To a solution of 60 (1.0 g, 3 mmol) in ethanol (100 mL), 12 mol/L hydrochloric acid (20 mL) was added. The solution was heated to 90˚C and stirred for 4 hours, and then concentrated. The resulting residue 61 (400 mg, y. 73%) was subjected to a next reaction without further purification.

Step 5

To a solution of 61 (722 mg, 2.68 mmol) in DMF (30 mL), triethylamine (542 mg) and 48 (1.1 g) were added.

The reaction mixture heated to 40˚C and stirred, then partitioned between ethyl acetate and water. The ethyl acetate layer was washed sequentially with diluted hydrochloric acid and brine, and then concentrated. The resulting residue was purified by thin layer chromatography to yield Compound I-219 as a pale brown solid (60 mg, y. 4.7%).
LC/MS (Method D): 2.58 min, $[M+H]^+$ = 465.14.
$^1$H-NMR (300 Mz) (DMSO-$d_6$): 11.850 (s, 1H), 8.701 (s, 1H), 8.653 (s, 1H), 8.403 (d, 1H, J = 8.1 HZ), 8.166 (m, 3H), 8.037 (d, 1H, J = 7.8Hz), 7.693 (t, 1H), 7.110-6.925 (m, 3H), 4.135 (t, 2H), 2.690 (s, 3H), 1.892-1.826 (m, 1H), 1.775-1.706 (m, 2H), 0.920 (d, 6H).

(Example 1-10)

In the following scheme, AcOH denotes acetic acid, BuLi denotes *n*-butyl lithium, BrCN denotes bromine cyanide, EtOH denotes ethanol, Ar-$B(OH)_2$ denotes 3-acetylphenylboronic acid, and $Et_3N$ denotes triethylamine.
[Formula 114]

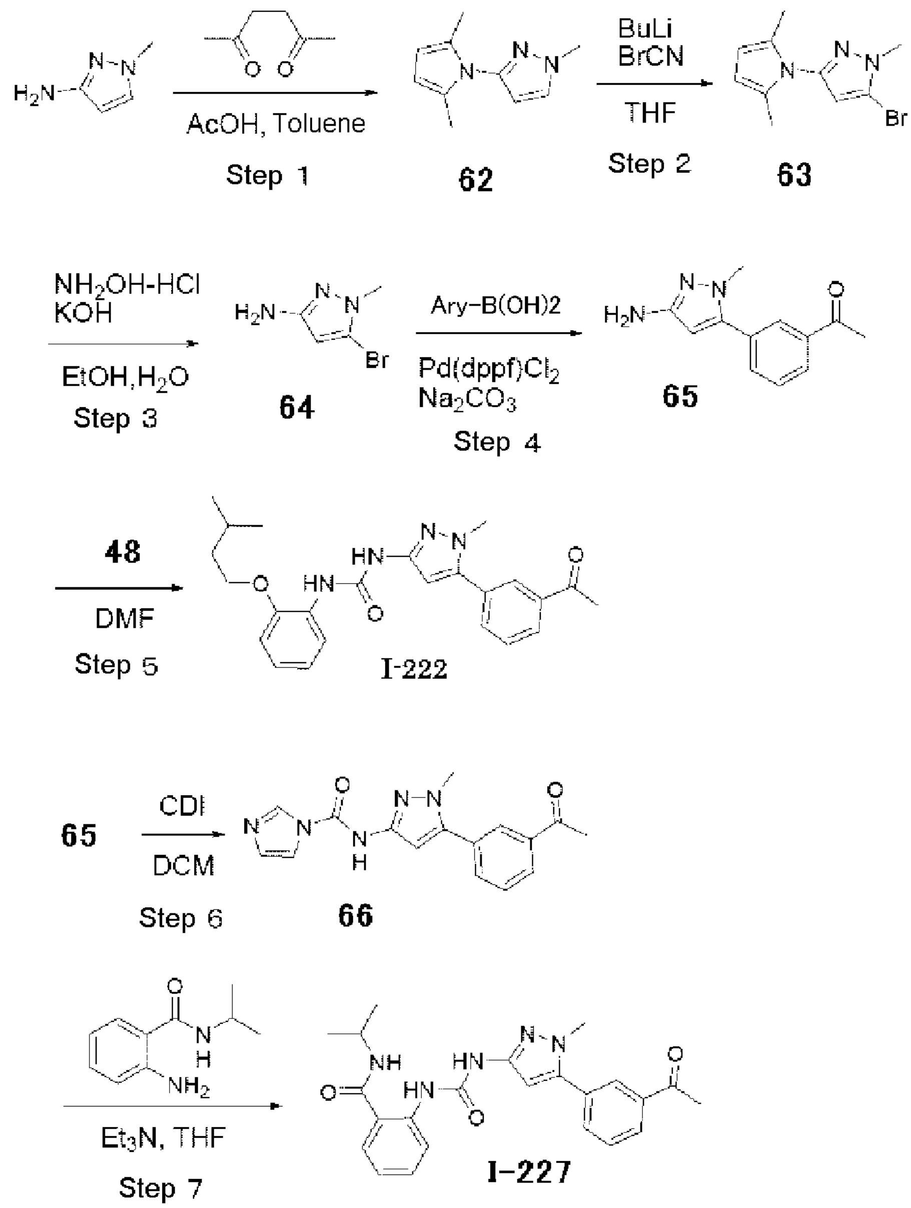


Step 1

To a solution of 1-methyl-3-aminopyrazole (50 g, 0.52 mol) in toluene (2 L), hexane-2,5-dione (58.5 g) and acetic acid (12.5 mL) were added. The solution was then refluxed for 3 hours. After the reaction mixture was cooled, water (0.6 L) was added thereto. The mixture was neutralized with sodium carbonate, and then separated. The organic layer was then concentrated. The residue was purified by silica gel chromatography (ethyl acetate/petroleum ether 1:10) to yield 62 as a brown solid (57 g, y. 63%).

$^1$H-NMR (500 Mz) ($CDCl_3$): 2.10 (s, 6H), 3.92 (s, 3H), 5.84 (s, 1H), 6.14 (d, J = 3.5 Hz, 1H), 7.38 (d, J = 3.5 Hz, 1H).

Step 2

A solution of 62 (3.5 g, 20 mmol) in THF (100 mL) was cooled to -78°C, and then 1.6 mol/L *n*-butyl lithium solution in THF (20 mL) was added dropwise thereto. The solution was stirred at the same temperature for 1.5 hours,

and then a solution of bromine cyanide (6.2 g) in THF (10 mL) was added thereto, subsequently warming to room temperature. The reaction solvent was concentrated, and then the residue was partitioned between ether and water. The ether layer was concentrated, and then the residue was purified by silica gel chromatography (ethyl acetate/petroleum ether 1:10) to yield 63 as a brown solid (2.8 g, y. 80%).
$^1$H-NMR (300 Mz) (DMSO): 2.02 (s, 6H), 3.85 (s, 3H), 5.75 (s, 2H), 6.70 (s, 1H).

Step 3

To a solution of hydroxylamine hydrochloride (3.06 g) in ethanol (20 mL), a solution of potassium hydroxide (1.26 g) in water-ethanol (1:1, 40 mL), and 63 (2.3 g, 9 mmol) were added. The solution was then refluxed overnight. The reaction mixture was then concentrated. The resulting residue was purified by silica gel column chromatography (ethyl acetate/petroleum ether 1:5) to yield 64 as a white solid (1.45 g, y. 75%).
LC/MS (Method D): 0.58 min, $[M+H]^+$ = 175.99, 178.01.
$^1$H-NMR (300 Mz) (DMSO): 3.44 (d, J = 93.5 Hz, 3H), 4.77 (s, 2H), 5.53 (s, 1H).

Step 4

To a solution of 64 (400 mg, 2.3 mmol) in DME/$H_2O$/ethanol (7:3:2) (24 mL), 3-acetylphenylboronic acid (546 mg), Pd(dppf)$Cl_2$ (50 mg), and sodium carbonate (490 mg) were added. The solution was then refluxed under nitrogen atmosphere overnight. The reaction mixture was concentrated, and then purified by silica gel column chromatography (ethyl acetate/petroleum ether 1:1) to yield 65 as a white solid (530 mg, y. 92%).
$^1$H NMR (300 Mz): 7.97 (m, 2H), 7.709 (m, 1H), 7.61 (m, 1H), 5.64 (s, 1H), 4.63 (s, 2H), 3.60 (s, 3H), 2.63 (s, 3H).

Step 5

To a solution of 65 (510 mg, 2.35 mmol) in THF (20 mL), 48 (943 mg) was added. The solution was heated to 40˚C and stirred overnight, and then water, methanol, and ethanol (10 mL each) were added. The precipitate was collected by filtration, then washed with ethyl acetate to yield target compound I-222 as a white solid (280 mg, y. 28%).
LC/MS (Method D): 2.30 min, $[M+H]^+$ = 421.22.
$^1$H-NMR (300 Mz) (DMSO): 0.95 (d, J = 11 Hz, 6H), 1.70-1.87 (m, 3H), 2.65 (s, 3H), 3.78 (s, 3H), 4.04-4.10 (m, 2H), 6.46 (s, 1H), 6.84-6.96 (m, 2H), 7.02 (d, J = 13 Hz, 1H), 7.67 (t, J = 13 Hz, 1H), 7.83 (d, J = 12.5 Hz, 1H), 8.03 (t, J = 11 Hz, 2H), 8.16 (d, J = 11 Hz, 1H), 9.74 (d, J = 7 Hz, 1H).

Step 6

To a solution of 65 (215 mg, 1 mmol) in dichloromethane (4 mL), CDI (324 mg) was added. The reaction mixture was then refluxed for 2 hours, and then concentrated. The residue was washed with dichloromethane to yield target compound 66 as a white solid (110 mg, y. 45%).

Step 7

To a solution of 66 (100 mg, 0.38 mmol) in THF (4 mL), anthranilic acid isopropyl amide (102 mg) and triethylamine (0.3 mL) were added. After reflux, the reaction mixture was concentrated. The resulting residue was purified by preparative HPLC to yield I-227 as a white solid (27 mg, y. 33%).
$^1$H-NMR (300 Mz) (DMSO): 1.25 (t, J = 13.5 Hz, 6H), 3.64 (s, 3H), 3.83 (s, 3H), 4.12-4.15 (m, 1H), 6.14 (d, J = 13 Hz, 1H), 6.52 (s, 1H), 7.01 (t, J = 12 Hz, 1H), 7.41 (t, J = 15 Hz, 2H), 7.54-7.47 (m, 2H), 7.92-8.04 (m, 3H), 8.33 (d, J = 13.5 Hz, 1H).
LC/MS (Method D): 1.64 min, $[M+H]^+$ = 420.17.

(Example 1-11)

[Formula 115]

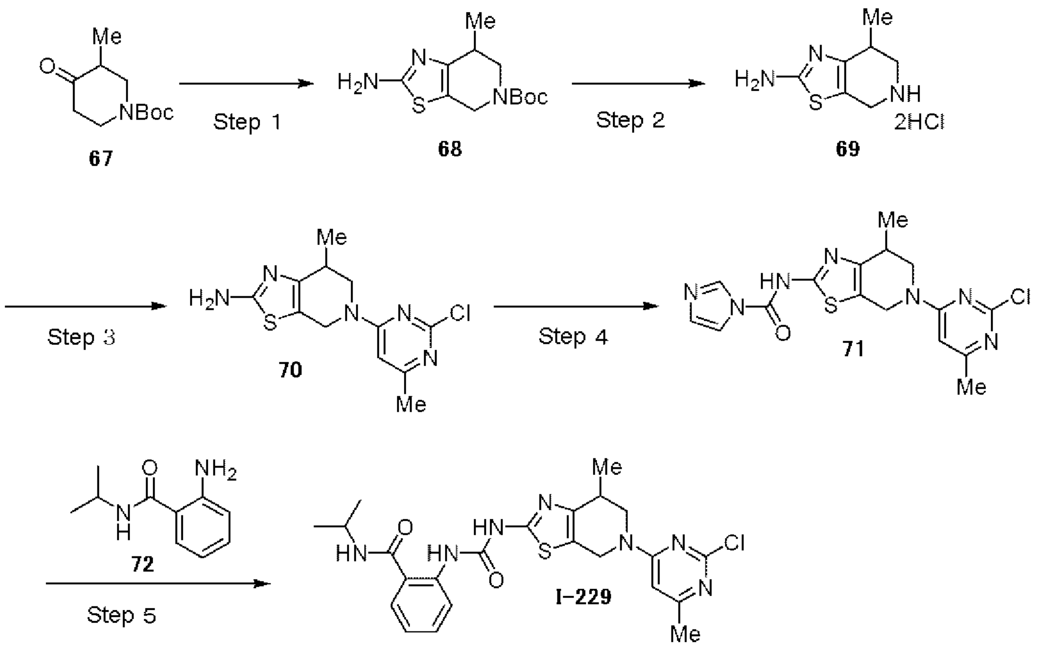


67 68 69 70 71 72 I-229

Step 1

To a solution of tert-butyl-3-methyl-4-oxopiperidine-1-carboxylate (Compound 67: 200 mg, 0.938 mmol) in pyridine (1 mL), sulfur (60.1 mg, 1.876 mmol) and cyanamide (79 mg, 1.876 mmol) were added. The solution was then stirred at 100˚C for 3 hours. The reaction mixture was poured into iced water, and then extracted with ethyl acetate. The organic layer was washed with water, and then dried over anhydrous sodium sulfate. The solvent was concentrated *in vacuo,* and then purification by silica gel column chromatography (chloroform:methanol = 100:1 → 100:2) yielded Compound 68 as a pale yellow foam (79 mg, 31.3%).
LC/MS (Method C): 1.06 min, $[M+H]^+$ = 267
$^1$H-NMR ($CDCl_3$) δ: 1.21 (3H, d, J = 6.9 Hz), 1.48 (9H, s), 2.83 (1H, m), 3.24-3.84 (2H, m), 4.28-4.60 (2H, m), 4.83 (2H, s).

Step 2

To a solution of Compound 68 (79 mg, 0.293 mmol) in methylene chloride (1.0 mL), hydrochloric acid (4 mol/l solution in dioxane) (0.367 mL, 1.466 mmol) was added. The solution was stirred at room temperature for 2 hours. The solvent was evaporated off *in vacuo* to yield Compound 69 as a pale brown solid (71 mg, 100%). Compound 69 was subjected to the next step without further purification.

Step 3

To a solution of Compound 69 (71 mg, 0.293 mmol) in N-methylpyrrolidone (1 mL), 2,4-dichloro-6-methylpyrimidine (47.8 mg, 0.293 mmol) and diisopropylethylamine (0.256 mL, 1.465 mmol) were added. The solution was then stirred at 90˚C for 1 hour and 30 minutes. The reaction mixture was poured into iced water, and then extracted with ethyl acetate. The organic layer was washed with water, and then dried over anhydrous sodium sulfate. The solvent was concentrated *in vacuo* to yield Compound 70 as a brown oil (108 mg, 100%).
LC/MS (Method C): 0.92 min, $[M+H]^+$ = 296.

Step 4

To a solution of Compound 70 (87 mg, 0.293 mmol) in methylene chloride (3 mL), 1,1'-carbonyldiimidazole (61.8 mg, 0.831 mmol). The solution was then stirred at 40˚C for 3 hours. The precipitate was collected by filtration to

yield Compound 71 as a pale brown solid (40 mg, 35%). Compound 71 was subjected to the next step without further purification.

Step 5

To a solution of Compound 71 (60 mg, 0.154 mmol) in tetrahydrofuran (3 mL), commercially available Compound 72 (28 mg, 0.157 mmol) and diisopropylethylamine (0.032 mL, 0.185 mmol) were added. The solution was then stirred at 50˚C for 3 hours and 30 minutes. The reaction mixture was poured into iced water, and then extracted with ethyl acetate. The organic layer was washed with water, and then dried over anhydrous sodium sulfate. The solvent was then concentrated *in vacuo.* Elution through silica gel column chromatography (chloroform:methanol = 100:1), and subsequent recrystallization from ethyl acetate/n-hexane (1:3) yielded Compound I-229 as a pale yellow solid (6.1 mg, 7.9%).
LC/MS (Method C): 1.06 min, $[M+H]^+$ = 501
$^1$H-NMR ($CDCl_3$) δ: 1.20-1.35 (9H, m), 2.37 (3H, s), 3.04 (1H, m), 3.51 (1H, m), 4.10 (1H, m), 4.22 (1H, m), 4.60-4.70 (2H, m), 6.05 (1H, d, J = 7.80 Hz), 6.34 (1H, s), 7.06 (1H, m), 7.42 (1H, m), 7.47 (1H, m), 8.40 (1H, d, J = 7.80 Hz), 11.12 (1H, s).

(Example 1-12)

[Formula 116]

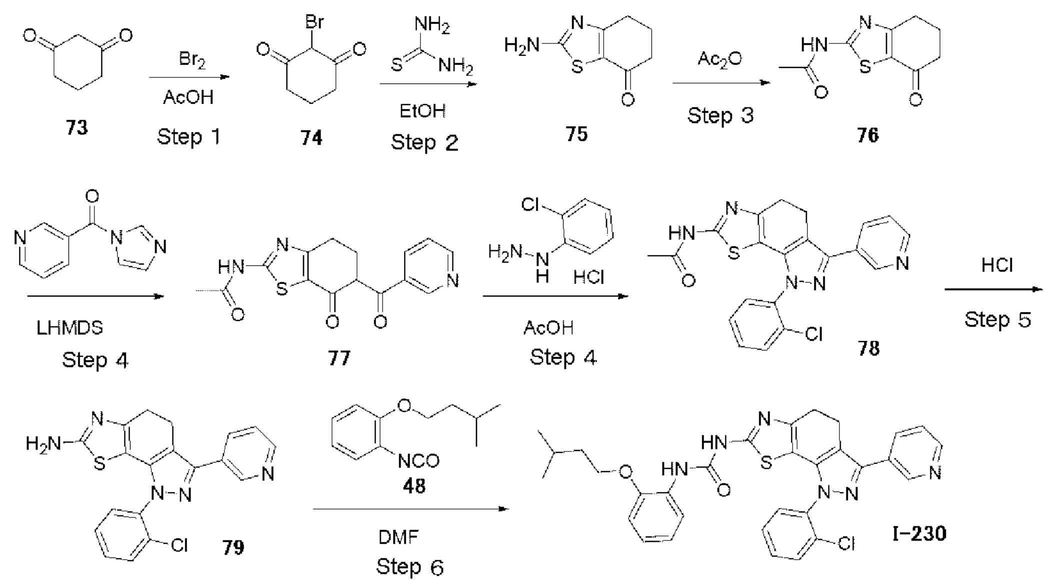


Step 1

To a solution of 1,3-cyclohexanedione 73 (11.2 g, 100 mmol) in acetic acid (120 mL), a solution of bromine (16 g) in acetic acid (30 mL) was added dropwise under icebath-cooling. After stirring at room temperature for 3 hours, the reaction mixture was filtered, and then washed with petroleum ether to yield 74 as a white solid (19.2 g, y. 98%).

Step 2

To a suspension of thiourea (3.8 g) in ethanol (100 mL), 74 (9.5 g, 50 mmol) was added. The suspension was then stirred under reflux overnight. The reaction mixture was cooled, filtered, and then washed sequentially with water and ethanol to yield 75 as a yellow solid (3.1 g, y. 42%).
$^1$H-NMR (300 Mz) (DMSO): 1.99-2.05 (m, 2H), 2.39 (t, J = 5.5 Hz, 2H), 2.70 (t, J = 6 Hz, 2H), 8.55-8.66 (m, 2H).

Step 3

Acetic anhydride (5 mL) was added to 75 (500 mg, 3.5 mmol). After stirring under reflux for 5 hours, the reaction

mixture was cooled and then filtered to yield 76 as a brown solid (360 mg, y. 60%).
$^1$H-NMR (300 Mz) (DMSO): 2.05-2.09 (m, 2H), 2.49 (m, 5H), 2.85 (t, J = 6.5 Hz, 2H), 12.56 (s, 1H).

Step 4

To a solution of nicotinic acid (250 mg) in THF (5 mL), carbonyldiimidazole (468 mg) was added. The solution was then stirred at room temperature for 3 hours to obtain a solution of nicotinic acid imidazolide in THF.
To a solution of 76 (210 mg, 1 mmol) in THF/DMF (1:1) (4 mL), 1 mol/L LHMDS/THF solution (2 mL) was added dropwise at -50˚C. The reaction mixture was stirred for 30 minutes, and then a solution of nicotinic acid imidazolide in THF, which had been previously prepared, was added. The solution was then stirred at -20˚C for 1 hour. Diluted hydrochloric acid, ethyl acetate, and water were then added to the reaction mixture, followed by extraction with ethyl acetate. The organic layer was concentrated, and then the resulting brown solid residue (77) was subjected to a next reaction without further purification.
To a solution of 77 in acetic acid (3 mL), 2-chlorophenylhydrazine hydrochloride (176 mg) was added. The reaction mixture was stirred at 60˚C for 3 hours, and then concentrated. The resulting residue was purified by preparative HPLC to yield 78 as a yellow solid (80 mg, y. 13% for 2 steps).
$^1$H-NMR (300 Mz) ($CDCl_3$): 2.26 (s, 3H), 3.14-3.27 (m, 4H), 7.51-7.67 (m, 5H), 8.49 (d, J = 8 Hz, 1H), 8.70(d, J = 5 Hz, 1H), 9.15 (s, 1H).

Step 5

Concentrated hydrochloric acid (10 mL) was added to 78 (1.6 g), and then the mixture was stirred at 80˚C for a period of time. After cooling to room temperature, the reaction mixture was adjusted with sodium hydroxide to pH = 10-11. The precipitate was collected by filtration, then purified by preparative HPLC to yield 79 as yellow solid (230 mg, y. 16%).

Step 6

To a solution of 79 (200 mg, 0.53 mmol) in DMF (4 mL), 48 (350 mg) was added. The solution was stirred at 50˚C overnight, and then water was added thereto. The mixture was filtered, and then washed sequentially with methanol, ethanol, and ethyl acetate. Purification by preparative HPLC and recrystallization (acetonitrile/water) yielded I-230 as a white solid (24 mg, y. 7.7%).
$^1$H-NMR (300 Mz) (DMSO): 0.95 (d, 6H), 1.66-1.85 (m, 3H), 3.03 (t, J = 8 Hz,2H), 3.22 (s, 2H), 4.06 (t, J = 6.5 Hz, 2H), 6.85-7.06 (m, 3H), 7.49-7.53 (m, 1H), 7.61 (t, J = 7 Hz, 1H), 7.72 (t, J = 7.5 Hz, 1H), 7.82 (d, J = 7.5 Hz, 1H), 7.97 (d, J = 13 Hz, 1H), 8.12 (d, J = 8 Hz, 1H), 8.38 (s, 1H), 8.59 (d, J = 5 Hz, 1H), 8.95 (s, 1H), 11.52 (s, 1H).

(Results)

The following tables show values of the physical properties (retention time, mass spectrum, and measurement condition in LC/MS) for Compound Nos. I-1 to I-150 and I-152 to I-229. It is noted that * in the tables indicates that a carbon atom having it attached is asymmetric carbon.
[Table 1-1]

| Compound No. | Structural Formula | LC/MS RetentionTime (min) | [M+H]+ | LC Method |
|---|---|---|---|---|
| I-1 | 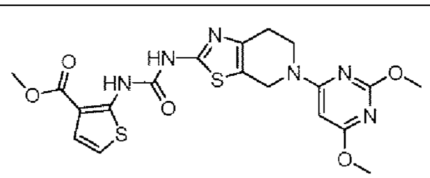 | 1.95 | 477 | A |

(continued)

| Compound No. | Structural Formula | LC/MS RetentionTime (min) | [M+H]+ | LC Method |
|---|---|---|---|---|
| I-2 | 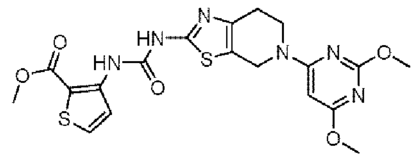 | 1.94 | 477 | A |
| I-3 | 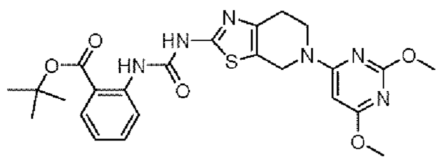 | 2.41 | 513.1 | A |
| I-4 | 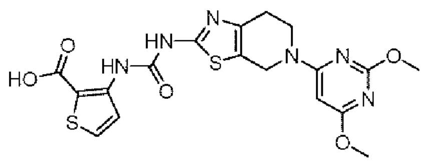 | 1.8 | 463 | A |
| I-5 | 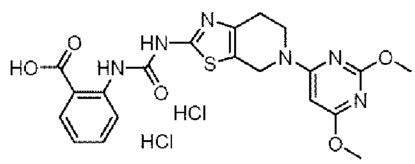 | 1.83 | 457 | A |
| I-6 | 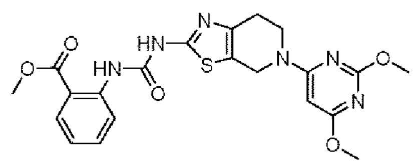 | 1.99 | 471 | A |

[Table 1-2]

| Compound No. | Structural Formula | LC/MS Retention Time (min) | [M+H]+ | LC Method |
|---|---|---|---|---|
| I-7 | 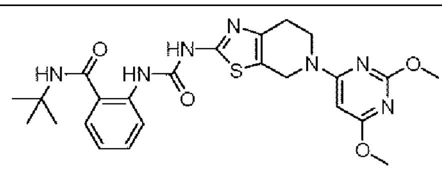 | 1.97 | 512.1 | A |
| I-8 | 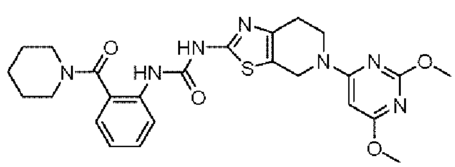 | 1.82 | 524.1 | A |

(continued)

| Compound No. | Structural Formula | LC/MS Retention Time (min) | [M+H]+ | LC Method |
|---|---|---|---|---|
| I-9 | 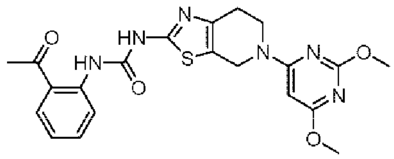 | 1.83 | 455 | A |
| I-10 | 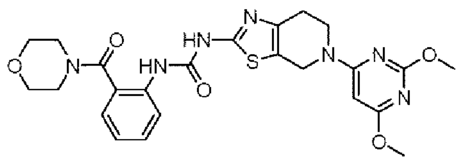 | 1.52 | 526 | A |
| I-11 | 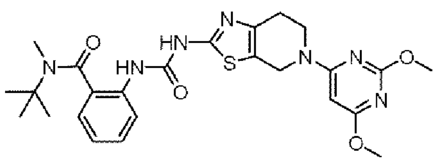 | 1.94 | 526 | A |
| I-12 | 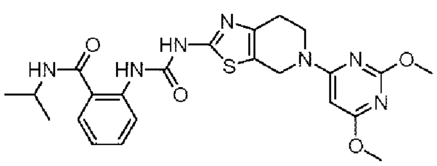 | 1.81 | 498.1 | A |

[Table 1-3]

| Compound No. | Structural Formula | LC/MS Retention Time (min) | [M+H]+ | LC Method |
|---|---|---|---|---|
| I-13 | 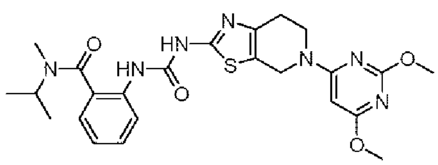 | 1.77 | 512.1 | A |
| I-14 | 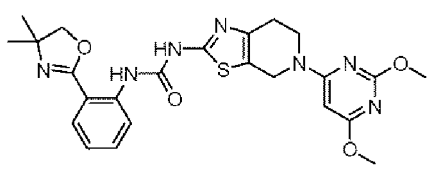 | 2.26 | 510.1 | A |
| I-15 | 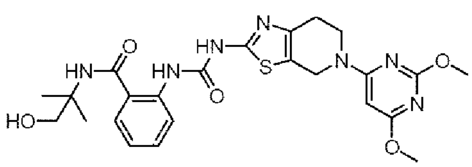 | 1.62 | 528.1 | A |

(continued)

| Compound No. | Structural Formula | LC/MS Retention Time (min) | [M+H]+ | LC Method |
|---|---|---|---|---|
| I-16 | 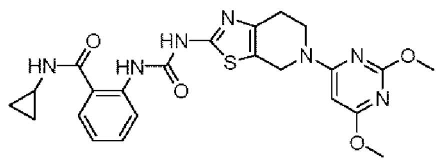 | 1.71 | 496 | A |
| I-17 | 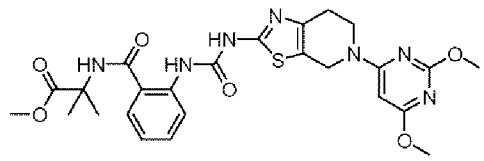 | 1.83 | 556.1 | A |
| I-18 | 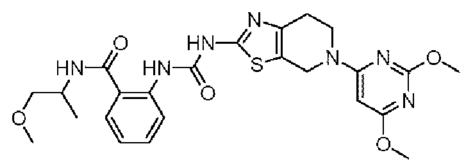 | 1.72 | 528.1 | A |

[Table 1-4]

| Compound No. | Structural Formula | LC/MS Retention Time (min) | [M+H]+ | LC Method |
|---|---|---|---|---|
| I-19 | 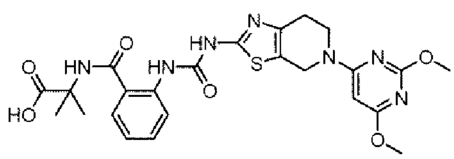 | 1.63 | 542.1 | A |
| I-20 | 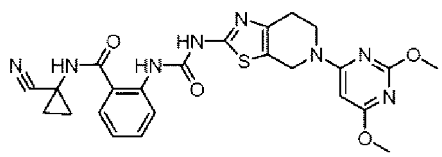 | 1.68 | 521.1 | A |
| I-21 | 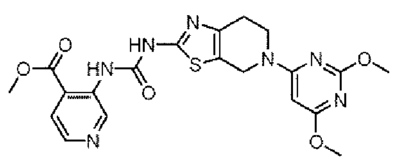 | 1.49 | 472 | A |
| I-22 | 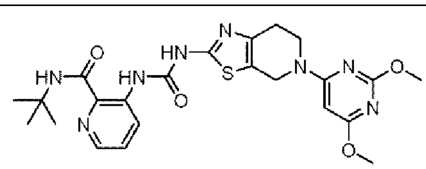 | 2.29 | 513.1 | A |

(continued)

| Compound No. | Structural Formula | LC/MS Retention Time (min) | [M+H]+ | LC Method |
|---|---|---|---|---|
| I-23 | 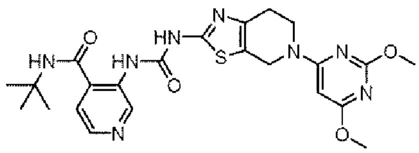 | 1.5 | 513.3 | A |
| I-24 | 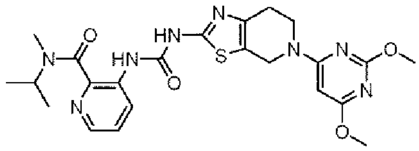 | 1.58 | 513.2 | A |

[Table 1-5]

| Compound No. | Structural Formula | LC/MS Retention Time (min) | [M+H]+ | LC Method |
|---|---|---|---|---|
| I-25 | 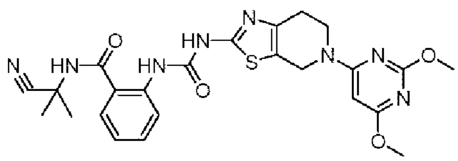 | 1.74 | 523.2 | A |
| I-26 | 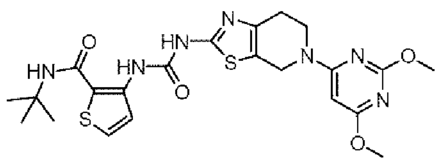 | 2.09 | 518.2 | A |
| I-27 | 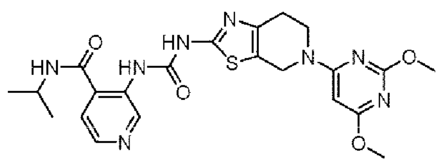 | 1.36 | 499.2 | A |
| I-28 | 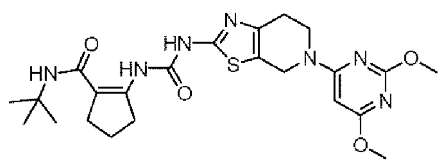 | 2.14 | 502.2 | A |
| I-29 | 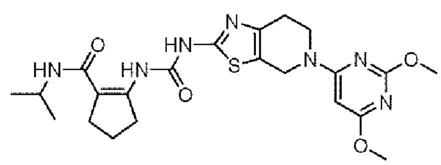 | 1.9 | 488.2 | A |

(continued)

| Compound No. | Structural Formula | LC/MS Retention Time (min) | [M+H]+ | LC Method |
|---|---|---|---|---|
| I-30 | 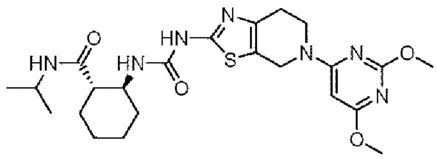 | 1.48 | 504.2 | A |

[Table 1-6]

| Compound No. | Structural Formula | LC/MS Retention Time (min) | [M+H]+ | LC Method |
|---|---|---|---|---|
| I-31 | 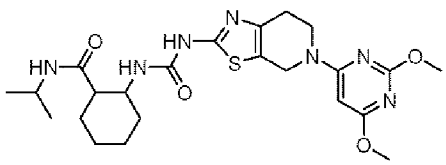 | 1.58 | 504.2 | A |
| I-32 | 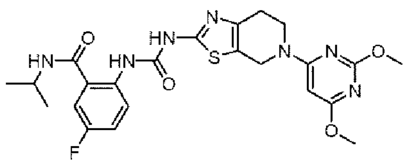 | 1.88 | 516.1 | A |

[Table 1-7]

| Compound No. | Structural Formula | LC/MS Retention Time (min) | [M+H]+ | LC Method |
|---|---|---|---|---|
| I-33 | 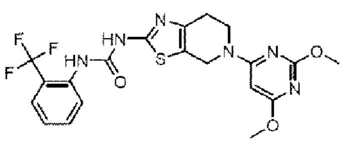 | 2.35 | 481 | C |
| I-34 | 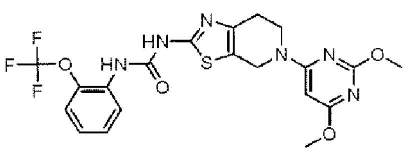 | 2.51 | 497 | C |
| I-35 | 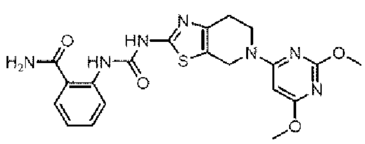 | 1.86 | 456 | C |

(continued)

| Compound No. | Structural Formula | LC/MS Retention Time (min) | [M+H]+ | LC Method |
|---|---|---|---|---|
| I-36 | 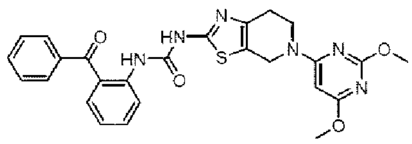 | 2.49 | 517 | C |
| I-37 | 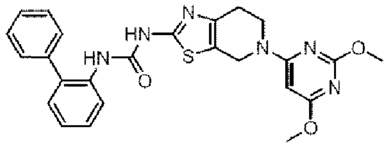 | 2.51 | 489 | C |
| I-38 | 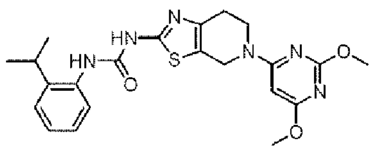 | 2.43 | 455 | C |

[Table 1-8]

| Compound No. | Structural Formula | LC/MS Retention Time (min) | [M+H]+ | LC Method |
|---|---|---|---|---|
| I-39 | 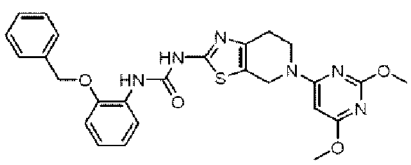 | 2.60 | 519 | C |
| I-40 | 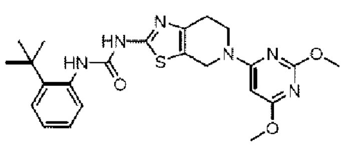 | 2.46 | 469 | C |
| I-41 | 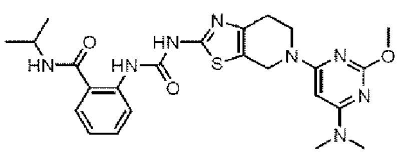 | 1.33 | 511 | D |
| I-42 | 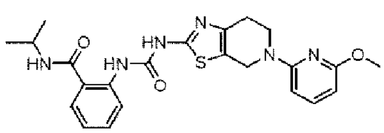 | 2.11 | 467 | D |

(continued)

| Compound No. | Structural Formula | LC/MS Retention Time (min) | [M+H]+ | LC Method |
|---|---|---|---|---|
| I-43 | 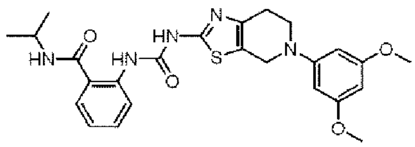 | 2.01 | 496 | D |
| I-44 | 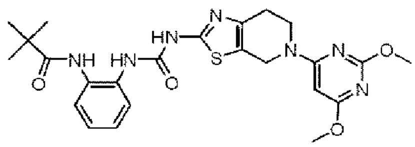 | 2.12 | 512 | C |
| I-45 | 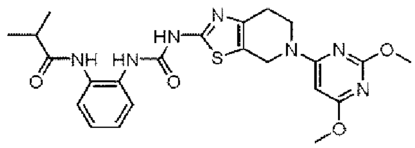 | 1.97 | 512 | C |

[Table 1-9]

| Compound No. | Structural Formula | LC/MS Retention Time (min) | [M+H]+ | LC Method |
|---|---|---|---|---|
| I-46 | 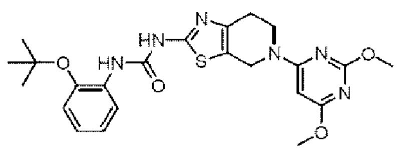 | 2.51 | 485 | C |
| I-47 | 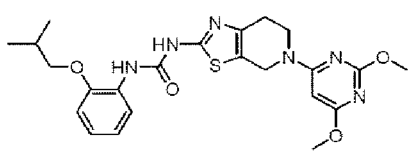 | 2.63 | 485 | C |
| I-48 | 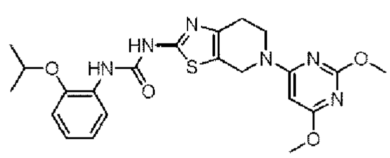 | 2.46 | 471 | C |
| I-49 | 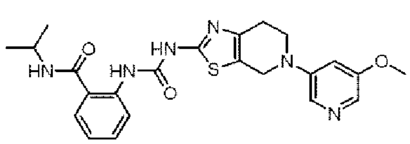 | 1.22 | 467 | D |

(continued)

| Compound No. | Structural Formula | LC/MS Retention Time (min) | [M+H]+ | LC Method |
|---|---|---|---|---|
| I-50 | 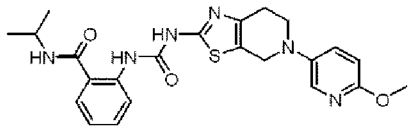 | 1.71 | 467 | D |
| I-51 | 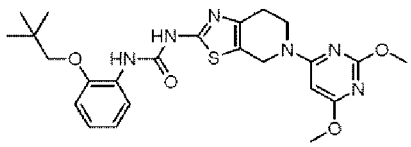 | 2.71 | 499 | C |

[Table 1-10]

| Compound No. | Structure Formula | LC/MS Retention Time (min) | [M+H]+ | LC Methods |
|---|---|---|---|---|
| I-52 | 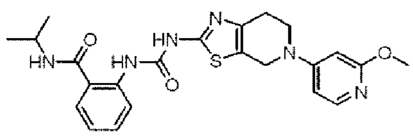 | 1.19 | 467 | D |
| I-53 | 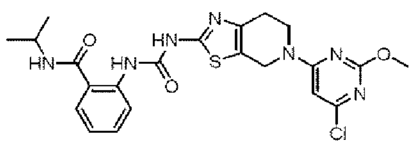 | 1.93 | 502 | D |
| I-54 | 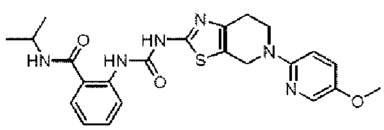 | 1.40 | 467 | D |
| I-55 | 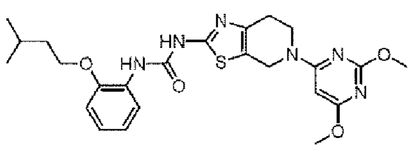 | 2.76 | 499 | C |
| I-56 | 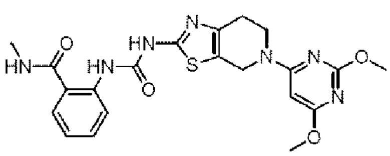 | 1.93 | 470 | C |
| I-57 | 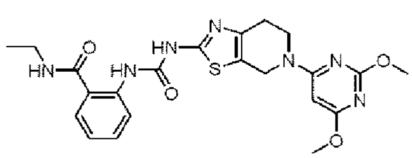 | 2.05 | 484 | C |

(continued)

| Compound No. | Structure Formula | LC/MS Retention Time (min) | [M+H]+ | LC Methods |
|---|---|---|---|---|
| I-58 | 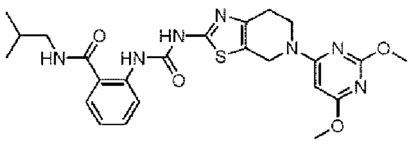 | 2.30 | 512 | C |

[Table 1-11]

| Compound No. | Structural Formula | LC/MS Retention Time (min) | [M+H]+ | LC Method |
|---|---|---|---|---|
| I-59 | 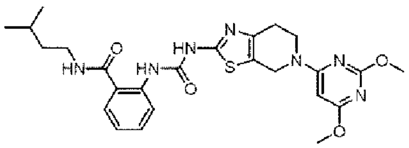 | 2.42 | 526 | C |
| I-60 | 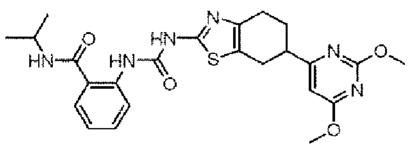 | 2.00 | 497 | D |
| I-61 | 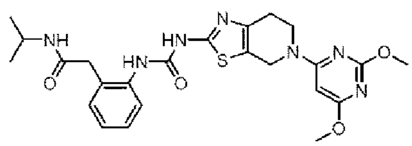 | 2.04 | 512 | C |
| I-62 | 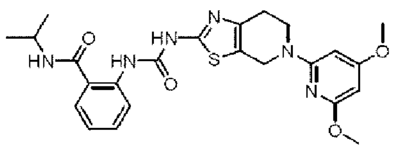 | 2.04 | 497 | D |
| I-63 | 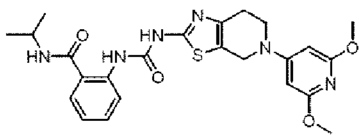 | 1.72 | 497 | D |
| I-64 | 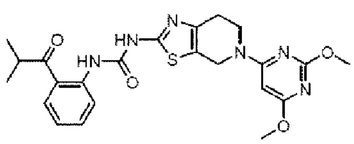 | 2.50 | 482 | C |

[Table 1-12]

| Compound No. | Structural Formula | LC/MS RetentionTime | [M+H]+ | LC Method |
|---|---|---|---|---|
| I-65 | 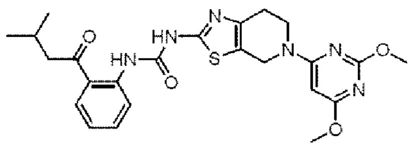 | 2.65 | 497 | C |
| I-66 | 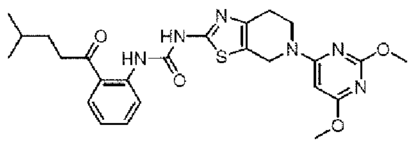 | 2.75 | 511 | C |
| I-67 | 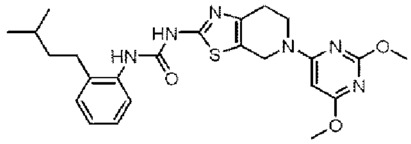 | 2.70 | 482 | C |
| I-68 | 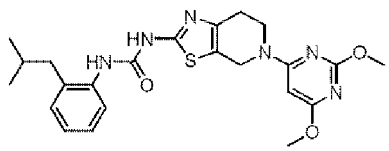 | 2.57 | 469 | C |
| I-69 | 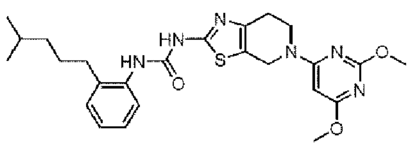 | 2.84 | 497 | C |
| I-70 | 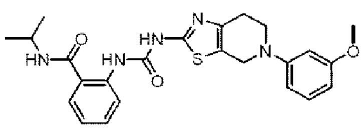 | 1.98 | 466 | D |
| I-71 | 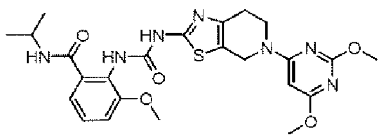 | 1.84 | 528 | C |

[Table 1-13]

| Compound No. | Structural Formula | LC/MS Retention Time (min) | [M+H]+ | LC Method |
|---|---|---|---|---|
| I-72 | 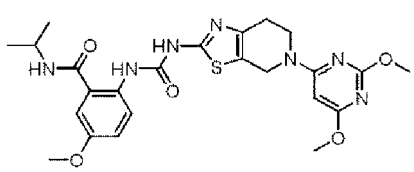 | 2.12 | 528 | C |

(continued)

| Compound No. | Structural Formula | LC/MS Retention Time (min) | [M+H]+ | LC Method |
|---|---|---|---|---|
| I-73 | 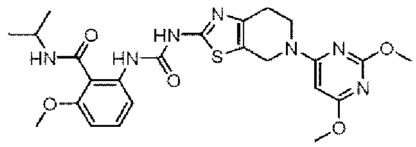 | 2.16 | 528 | C |
| I-74 | 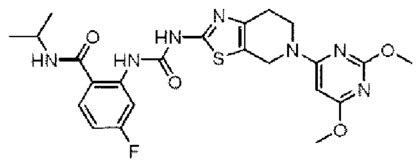 | 2.32 | 516 | C |
| I-75 | 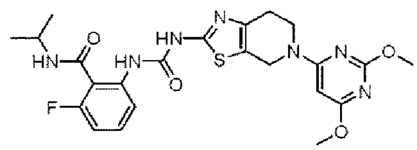 | 2.17 | 516 | C |
| I-76 | 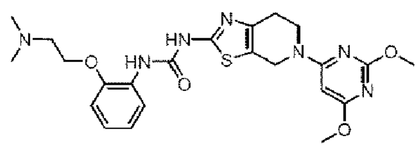 | 1.45 | 500 | C |
| I-77 | 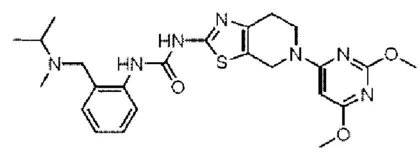 | 1.38 | 498 | C |
| I-78 | 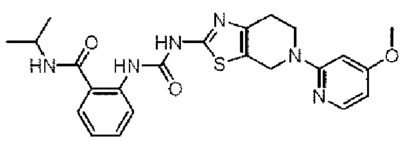 | 1.21 | 467 | D |

[Table 1-14]

| Compound | Structural Formula | LC/MS Retention Time (min) | [M+H]+ | LC Method |
|---|---|---|---|---|
| I-79 | 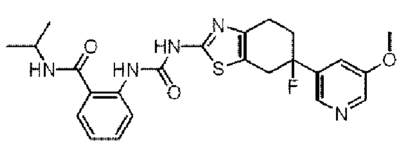 | 1.59 | 484 | D |

(continued)

| Compound | Structural Formula | LC/MS Retention Time (min) | [M+H]+ | LC Method |
|---|---|---|---|---|
| I-80 | 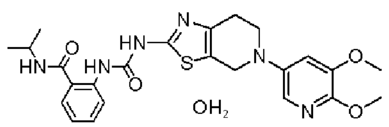 | 1.72 | 497 | D |
| I-81 | 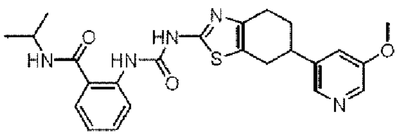 | 1.30 | 466 | D |
| I-82 | 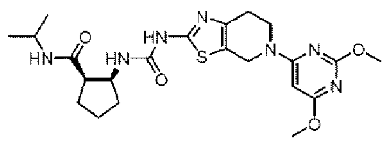 | 1.84 | 490 | C |
| I-83 | 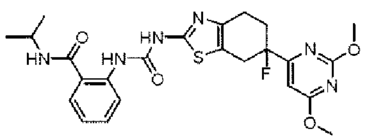 | 2.08 | 515 | D |
| I-84 | 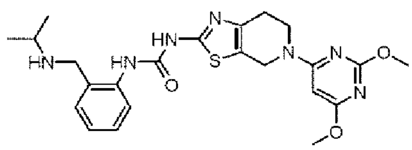 | 1.32 | 484 | C |
| I-85 | 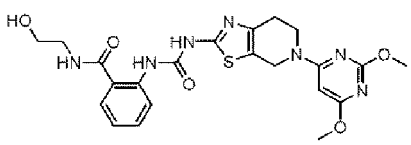 | 1.78 | 500 | C |

It is noted that Compound No. I-80 in Table 1-14 is a monohydrate.

[Table 1-15]

| Compound No. | Structural Formula | LC/M Retention Time (min) | [M+H]+ | LC Method |
|---|---|---|---|---|
| I-86 | 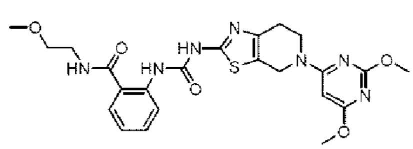 | 1.99 | 514 | C |
| I-87 | 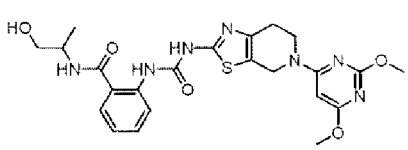 | 1.85 | 514 | C |

(continued)

| Compound No. | Structural Formula | LC/M Retention Time (min) | [M+H]+ | LC Method |
|---|---|---|---|---|
| I-88 | 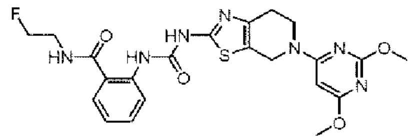 | 2.01 | 502 | C |
| I-89 | 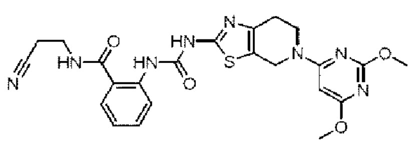 | 1.94 | 509 | C |
| I-90 | 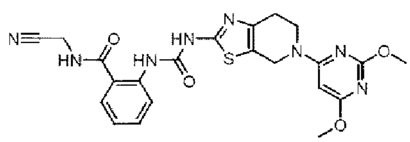 | 1.95 | 495 | C |
| I-91 | 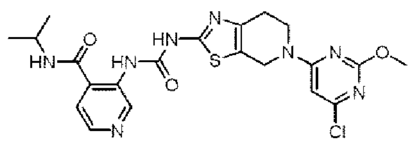 | 1.83 | 503, 505 | C |
| I-92 | 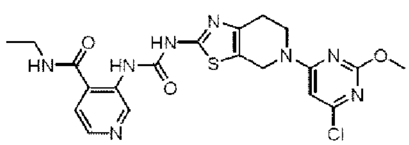 | 1.73 | 489, 491 | C |

[Table 1-16]

| Compound No. | Structural Formula | LC/MS Retention Time (min) | [M+H]+ | LC Method |
|---|---|---|---|---|
| I-93 | 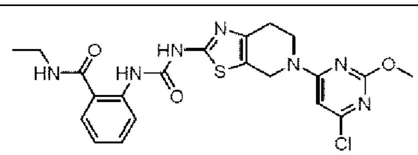 | 2.17 | 488, 490 | C |
| I-94 | 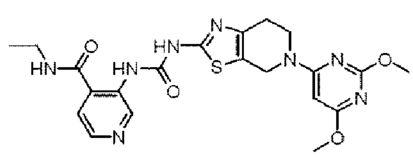 | 1.61 | 485 | C |
| I-95 | 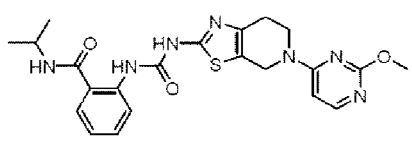 | 1.36 | 468 | C |

(continued)

| Compound No. | Structural Formula | LC/MS Retention Time (min) | [M+H]+ | LC Method |
|---|---|---|---|---|
| I-96 | 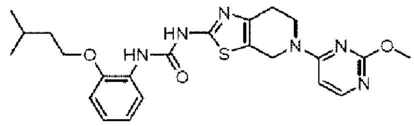 | 1.84 | 469 | C |
| I-97 | 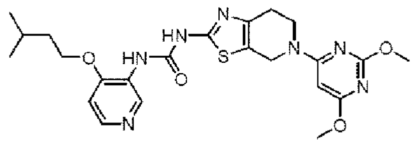 | 1.60 | 500 | C |
| I-98 | 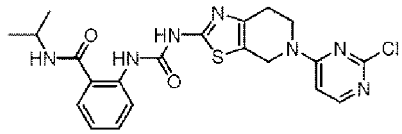 | 2.06 | 472 | C |
| I-99 | 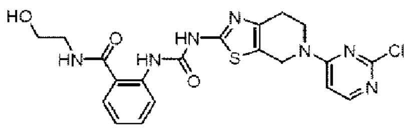 | 1.69 | 474 | C |

[Table 1-17]

| Compound No. | Structural Formula | LC/MS Retention Time (min) | [M+H]+ | LC Method |
|---|---|---|---|---|
| I-100 | 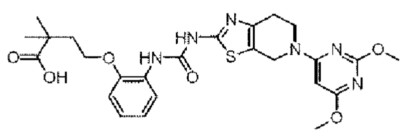 | 2.51 | 543 | C |
| I-101 | 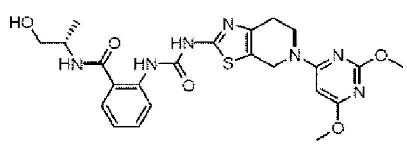 | 1.86 | 514 | C |
| I-102 | 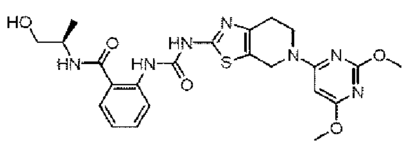 | 1.86 | 514 | C |
| I-103 | 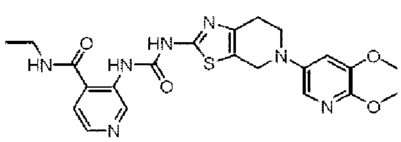 | 1.48 | 484 | C |

(continued)

| Compound No. | Structural Formula | LC/MS Retention Time (min) | [M+H]+ | LC Method |
|---|---|---|---|---|
| I-104 | 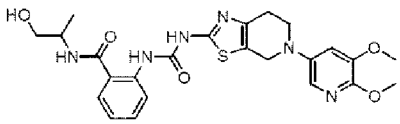 | 1.75 | 513 | C |
| I-105 | 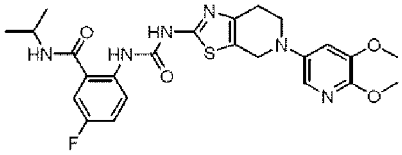 | 2.10 | 515 | C |
| I-106 | 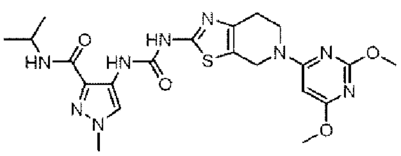 | 2.07 | 502 | C |

[Table 1-18]

| Compound No. | Structural Formula | LC/MS Retention Time (min) | [M+H]+ | LC Method |
|---|---|---|---|---|
| I-107 | 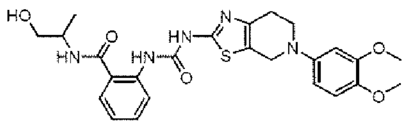 | 1.70 | 512 | C |
| I-108 | 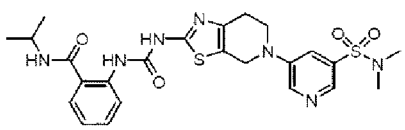 | 1.99 | 544 | C |
| I-109 | 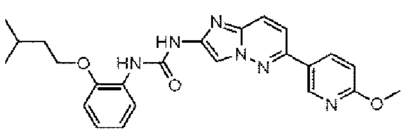 | 2.41 | 447 | D |
| I-110 | 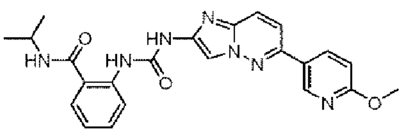 | 1.78 | 446 | D |
| I-111 | 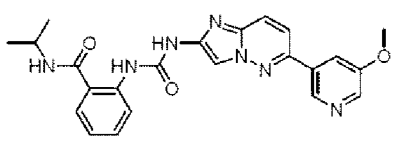 | 1.51 | 446 | D |

(continued)

| Compound No. | Structural Formula | LC/MS Retention Time (min) | [M+H]+ | LC Method |
|---|---|---|---|---|
| I-112 | 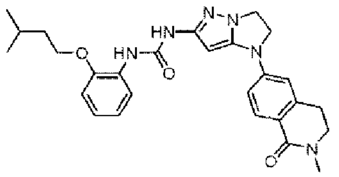 | 2.37 | 489 | C |
| I-113 | 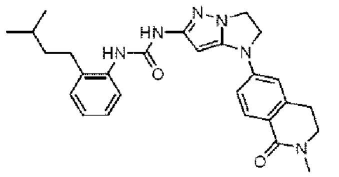 | 2.38 | 473 | C |

[Table 1-19]

| Compound No. | Structural Formula Retention | LC/MS Time (min) | [M+H]+ | LC Method |
|---|---|---|---|---|
| I-114 | 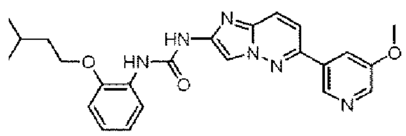 | 2.14 | 447 | D |
| I-115 | 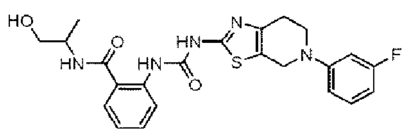 | 2.03 | 470 | C |
| I-116 | 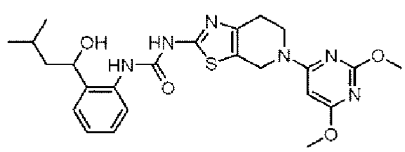 | 2.20 | 499 | C |
| I-117 | 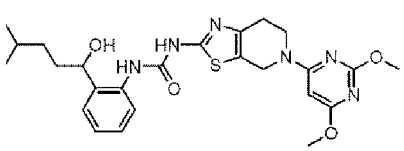 | 2.33 | 513 | C |
| I-118 | 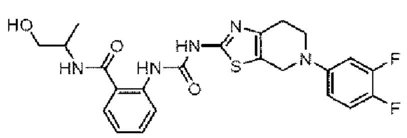 | 2.06 | 488 | C |
| I-119 | 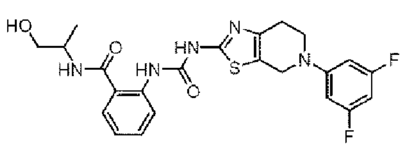 | 2.14 | 488 | C |

(continued)

| Compound No. | Structural Formula Retention | LC/MS Time (min) | [M+H]+ | LC Method |
|---|---|---|---|---|
| I-120 | 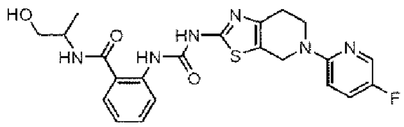 | 1.78 | 471 | C |

[Table 1-20]

| Compound No. | Structural Formula | LC/MS Retention Time (min) | [M+H]+ | LC Method |
|---|---|---|---|---|
| I-121 | 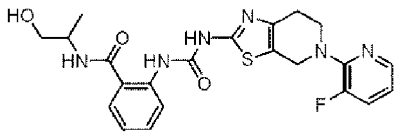 | 1.80 | 471 | C |
| I-122 | 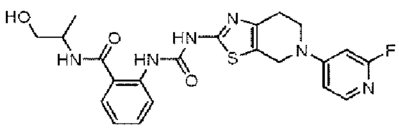 | 1.57 | 471 | C |
| I-123 | 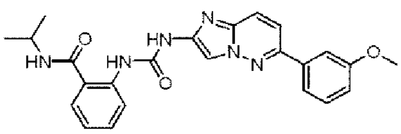 | 1.96 | 445 | D |
| I-124 | 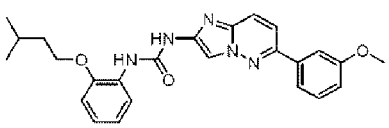 | 2.55 | 446 | D |
| I-125 | 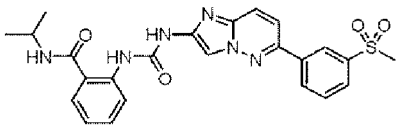 | 1.62 | 493 | D |
| I-126 | 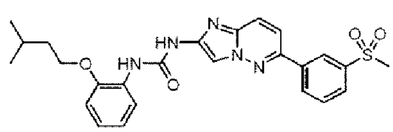 | 2.20 | 494 | D |

[Table 1-21]

| Compound No. | Structural Formula | LC/MS Retention Time (min) | [M+H]+ | LC Method |
|---|---|---|---|---|
| I-127 | 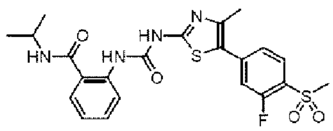 | 2.38 | 492.28 | D |
| I-128 | 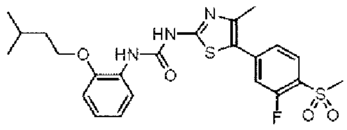 | 1.81 | 491.23 | D |
| I-129 | 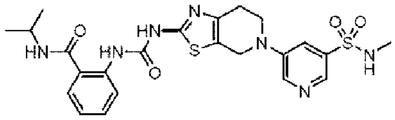 | 1.70 | 530 | C |
| I-130 | 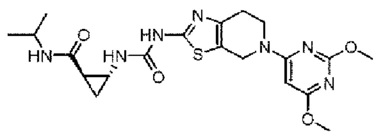 | 1.51 | 462.2 | C |
| I-131 | 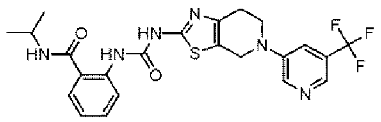 | 1.92 | 505.22 | D |
| I-132 | 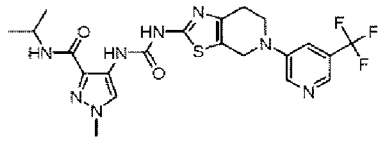 | 1.80 | 509.29 | D |
| I-133 | 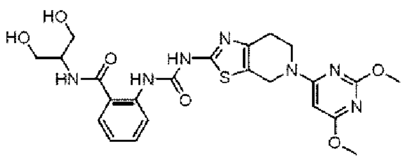 | 1.60 | 553 | C |
| I-134 | 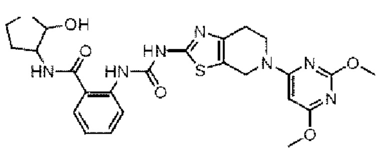 | 1.87 | 540 | C |

[Table 1-22]

| Compound No. | Structural Formula Retention | LC/MS Time (min) | [M+H]+ | LC Method |
|---|---|---|---|---|
| I-135 | 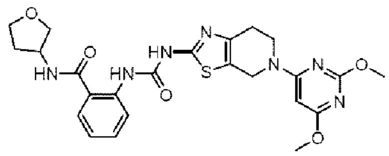 | 1.85 | 526 | C |
| I-136 | 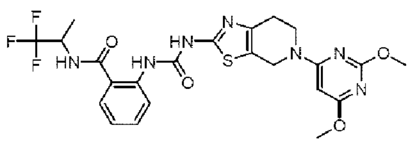 | 2.17 | 552 | C |
| I-137 | 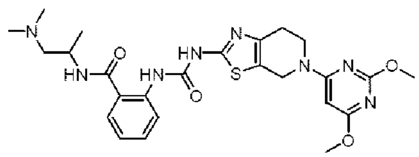 | 1.39 | 541 | C |
| I-138 | 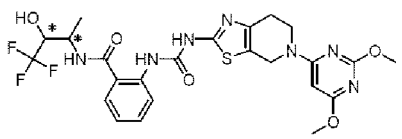 | 3.09 | 582 | F |
| I-139 | 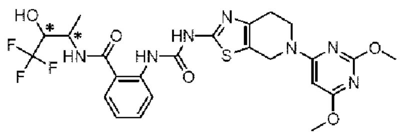 | 3.02 | 582 | F |
| I-140 | 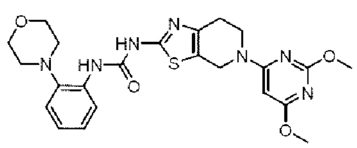 | 3.12 | 498 | F |
| I-141 | 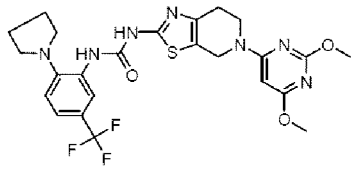 | 3.40 | 550 | F |

[Table 1-23]

| Compound No. | Structure Formula | LC/MS Retention Time (min) | [M+H]+ | LC Method |
|---|---|---|---|---|
| I-142 | 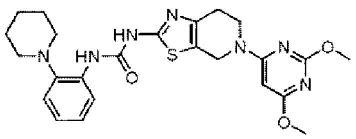 | 3.40 | 496 | F |

(continued)

| Compound No. | Structure Formula | LC/MS Retention Time (min) | [M+H] + | LC Method |
|---|---|---|---|---|
| I-143 | 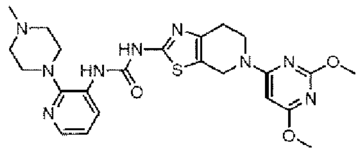 | 2.02 | 512 | F |
| I-144 | 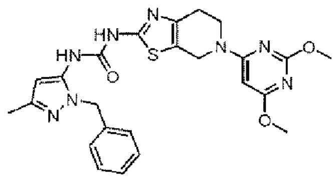 | 3.03 | 507 | F |
| I-145 | 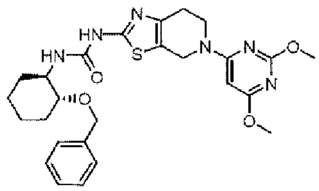 | 3.26 | 525 | F |
| I-146 | 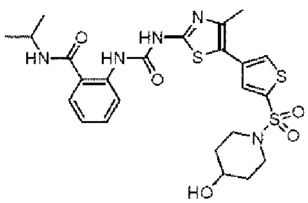 | 1.73 | 564.19 | D |
| I-147 | 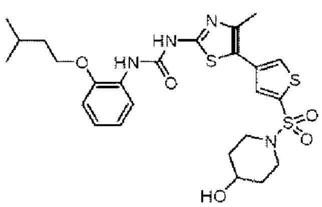 | 2.26 | 565.17 | D |

[Table 1-24]

| Compound No. | Structural Formula | LC/MS Retention Time (min) | [M+H]+ | LC Method |
|---|---|---|---|---|
| I-148 | 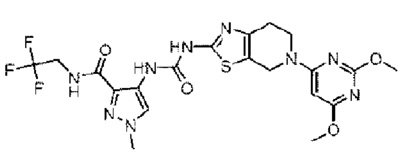 | 1.98 | 541.9 | C |
| I-149 | 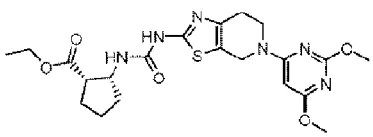 | 2.93 | 477 | F |

(continued)

| Compound No. | Structural Formula | LC/MS Retention Time (min) | [M+H]+ | LC Method |
|---|---|---|---|---|
| I-150 | 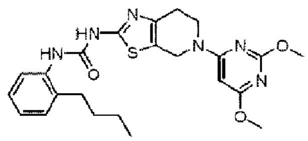 | 3.37 | 469 | F |
| I-152 | 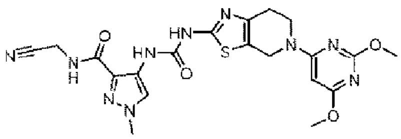 | 1.71 | 498.9 | C |
| I-153 | 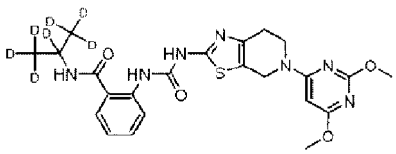 | 2.05 | 504.9 | C |
| I-154 | 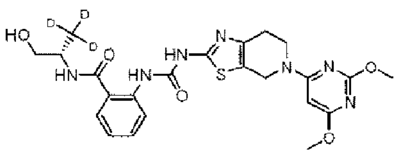 | 1.34 | 516.9 | C |

[Table 1-25]

| Compound No. | Structural Formula | LC/MS Retention Time (min) | [M+H]+ | LC Method |
|---|---|---|---|---|
| I-155 | 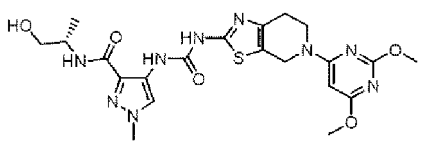 | 1,63 | 517.9 | C |
| I-156 | 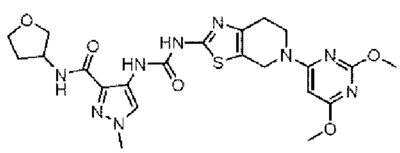 | 1.72 | 529.9 | C |
| I-157 | 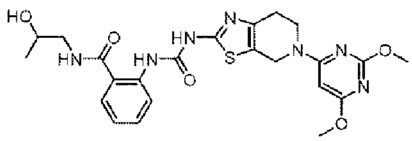 | 1.74 | 514.15 | C |
| I-158 | 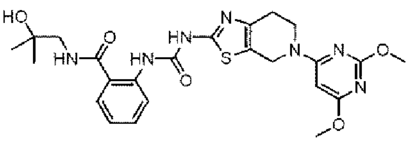 | 1.81 | 527.95 | C |

(continued)

| Compound No. | Structural Formula | LC/MS Retention Time (min) | [M+H]+ | LC Method |
|---|---|---|---|---|
| I-159 | 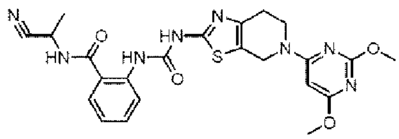 | 1.93 | 508.9 | C |
| I-160 | 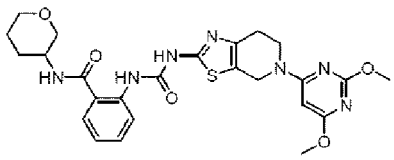 | 1.94 | 539.9 | C |
| I-161 | 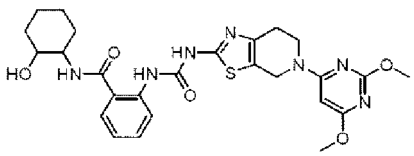 | 1.98 | 553.95 | C |
| I-162 | 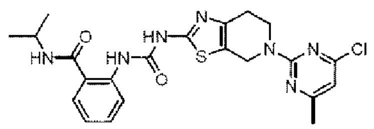 | 2.53 | 485.9 | C |

[Table 1-26]

| Compound No. | Structural Formula | LC/MS Retention Time (min) | [M+H]+ | LC Method |
|---|---|---|---|---|
| I-163 | 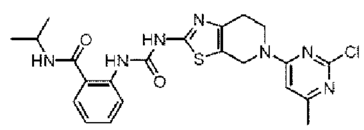 | 2.03 | 485.85 | C |
| I-164 | 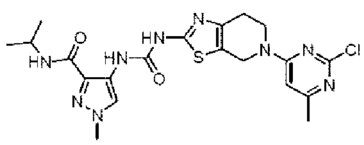 | 1.93 | 489.85 | D |
| I-165 | 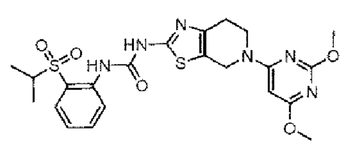 | 2.11 | 18.85 | |
| I-166 | 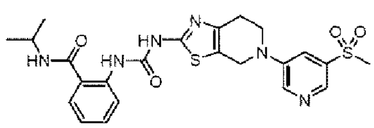 | 1.70 | 514.8 | C |
| I-167 | 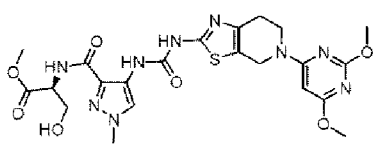 | 1.66 | 561.9 | C |

(continued)

| Compound No. | Structural Formula | LC/MS Retention Time (min) | [M+H]+ | LC Method |
|---|---|---|---|---|
| I-168 | 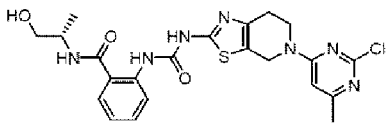 | 1.72 | 501.85 | C |
| I-169 | 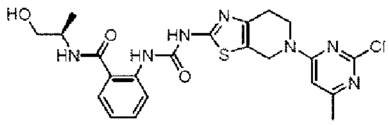 | 1.72 | 501.85 | C |
| I-170 | 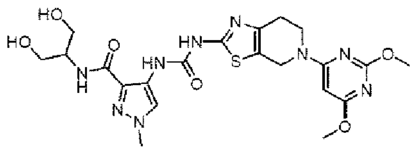 | 1.47 | 533.9 | C |

[Table 1-27]

| Compound No. | Structural Formula | LC/MS Retention Time (min) | [M+H]+ | LC Method |
|---|---|---|---|---|
| I-171 | 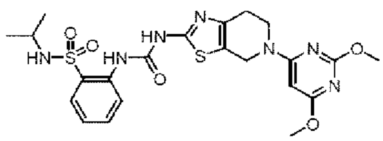 | 2.18 | 533.9 | C |
| I-172 | 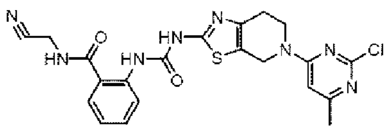 | 1.83 | 483.1 | C |
| I-173 | 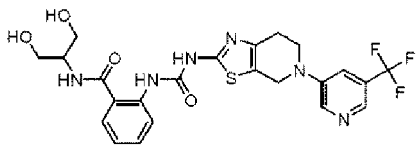 | 1.68 | 537.2 | C |
| I-174 | 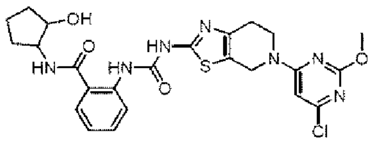 | 1.97 | 543.80, 546.20 | C |
| I-175 | 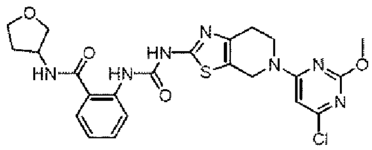 | 1.96 | 529.85, 531.90 | C |

(continued)

| Compound No. | Structural Formula | LC/MS Retention Time (min) | [M+H]+ | LC Method |
|---|---|---|---|---|
| I-176 | 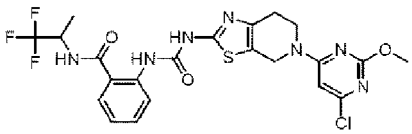 | 2.27 | 555.85, 557.80 | C |
| I-177 | 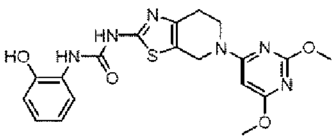 | 1.88 | 428.9 | C |
| I-178 | 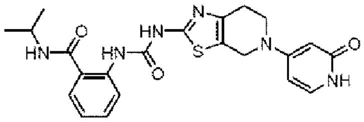 | 1.37 | 453.05 | C |

[Table 1-28]

| Compound No. | Structural Formula | LC/MS Retention Time (min) | [M+H] + | LC Method |
|---|---|---|---|---|
| I-179 | 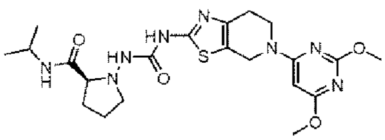 | 1.69 | 490.95 | C |
| I-180 | 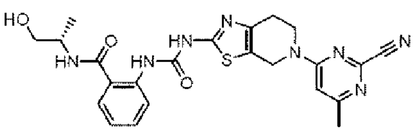 | 1.78 | 493.15 | C |
| I-181 | 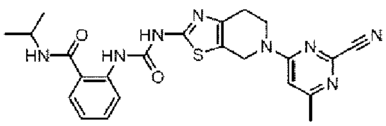 | 2.09 | 477.2 | C |
| I-182 | 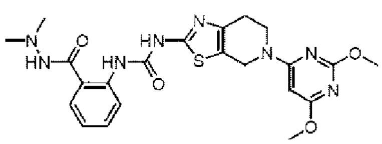 | 1.72 | 498.95 | C |
| I-183 | 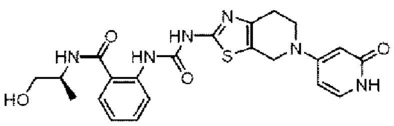 | 1.14 | 469.2 | C |

(continued)

| Compound No. | Structural Formula | LC/MS Retention Time (min) | [M+H]+ | LC Method |
|---|---|---|---|---|
| I-184 | 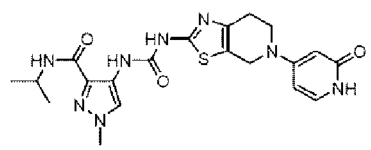 | 1.31 | 457.1 | C |
| I-185 | 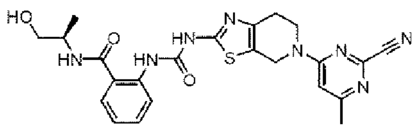 | 1.78 | 493 | C |
| I-186 | 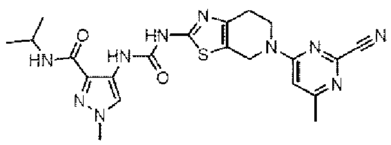 | 1.98 | 480.95 | C |

[Table 1-29]

| Compound No. | Structural Formula | LC/MS Retention Time (min) | [M+H]+ | LC Method |
|---|---|---|---|---|
| I-187 | 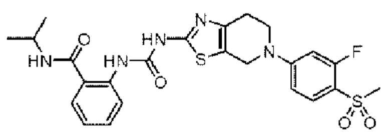 | 1.96 | 532 | C |
| I-188 | 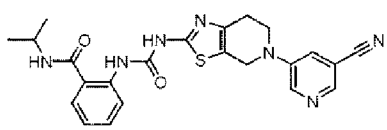 | 1.89 | 462 | C |
| I-189 | 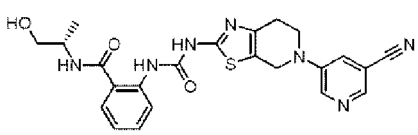 | 1.6 | 477.9 | C |
| I-190 | 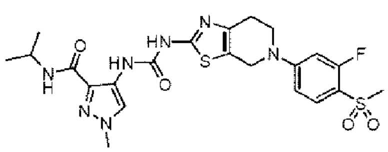 | 1.87 | 536 | C |
| I-191 | 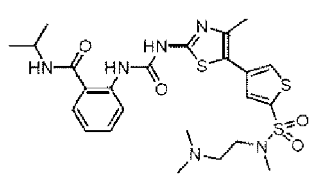 | 1.35 | 565.2 | D |

(continued)

| Compound No. | Structural Formula | LC/MS Retention Time (min) | [M+H]+ | LC Method |
|---|---|---|---|---|
| I-192 | 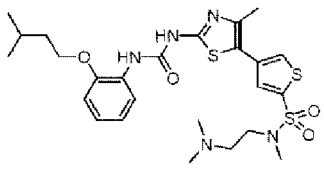 | 1.84 | 566.23 | D |
| I-193 | 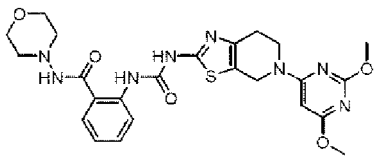 | 1.74 | 541.05 | C |
| I-194 | 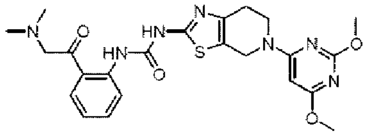 | 1.28 | 497.95 | C |

[Table 1-30]

| Compound No. | Structural Formula | LC/MS Retention Time (min) | [M+H]+ | LC Method |
|---|---|---|---|---|
| I-195 | 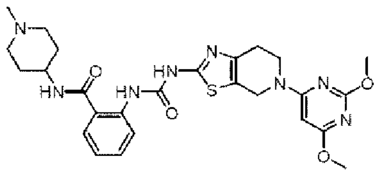 | 1.3 | 553.05 | C |
| I-196 | 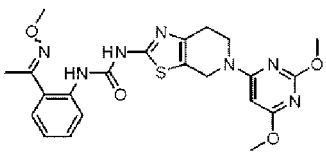 | 2.28 | 483.95 | C |
| I-197 | 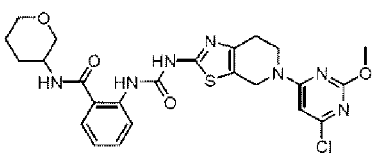 | 2.06 | 543.95, 545.90 | C |
| I-198 | 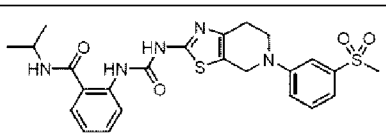 | 1.93 | 514.15 | C |
| I-199 | 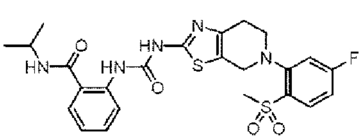 | 2.1 | 531.95 | C |

(continued)

| Compound No. | Structural Formula | LC/MS Retention Time (min) | [M+H]+ | LC Method |
|---|---|---|---|---|
| I-200 | 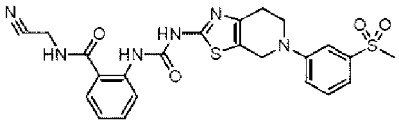 | 1.74 | 510.95 | C |
| I-201 | 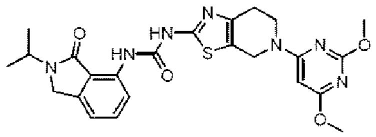 | 2.29 | 510 | C |
| I-202 | 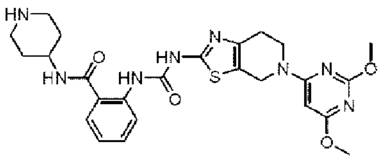 | 1.28 | 270.1 | C |

It is noted that the [M+H]+ value of Compound No. I-202 in Table 1-30 exhibits a value as [M+2H]/2.

[Table 1-31]

| Compound No. | Structural Formula | LC/MS Retention Time (min) | [M+H]+ | LC Method |
|---|---|---|---|---|
| I-203 | 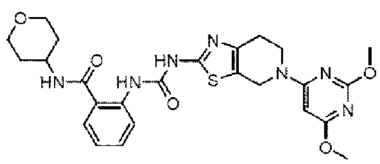 | 1.89 | 540 | C |
| I-204 | 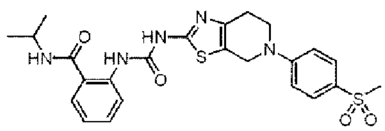 | 1.87 | 513.9 | C |
| I-205 | 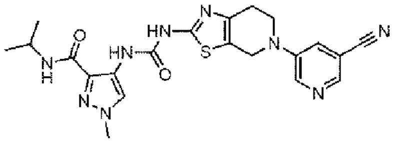 | 1.78 | 466 | C |
| I-206 | 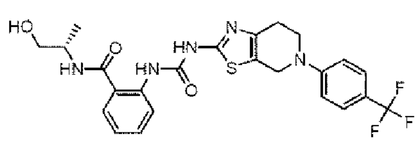 | 2.26 | 519.90, 520.95, 521.95 | C |
| I-207 | 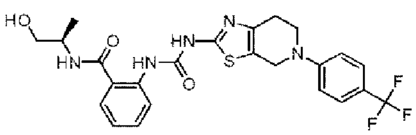 | 2.27 | 520.20, 520.95 | C |

(continued)

| Compound No. | Structural Formula | LC/MS Retention Time (min) | [M+H]+ | LC Method |
|---|---|---|---|---|
| I-208 | 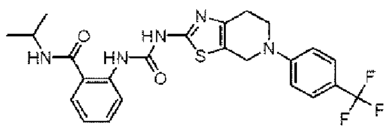 | 2.54 | 503.95, 504.95 | C |
| I-209 | 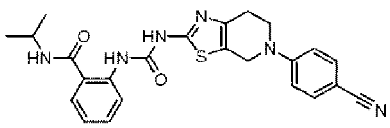 | 2.14 | 460.95 | C |
| I-210 | 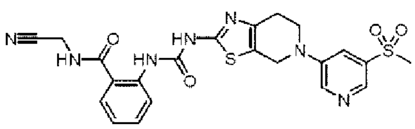 | 1.5 | 511.9 | C |

[Table 1-32]

| Compound No. | Structural Formula | LC/MS Retention Time (min) | [M+H]+ | LC Method |
|---|---|---|---|---|
| I-211 | 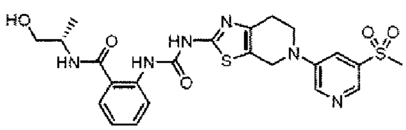 | 1.42 | 531.05 | C |
| I-212 | 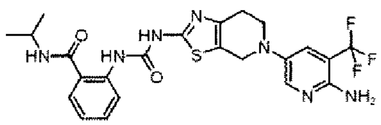 | 1.83 | 519.95 | C |

[Table 1-33]

| Compound No. | Structural Formula | Retention Time (min) | [M+H]+ | LC Method |
|---|---|---|---|---|
| I-213 | 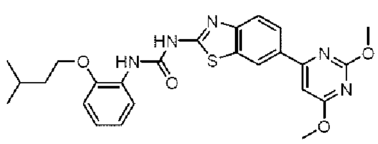 | 2.69 | 494.14 | D |
| I-214 | 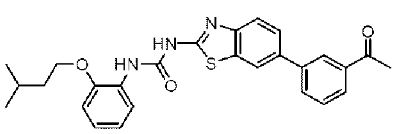 | 2.64 | 474.15 | D |
| I-215 | 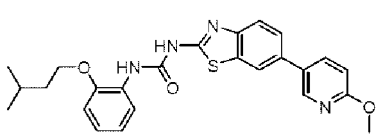 | 2.63 | 463.14 | D |

(continued)

| Compound No. | Structural Formula | Retention Time (min) | [M+H]+ | LC Method |
|---|---|---|---|---|
| I-224 | 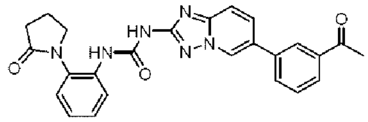 | 1.48 | 455.27 | D |
| I-216 | 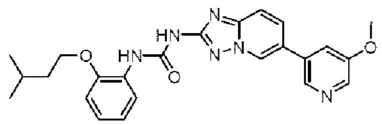 | 2.05 | 447.17 | D |
| I-217 | 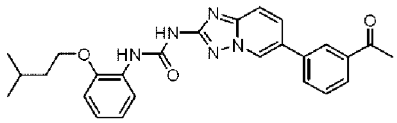 | 2.36 | 458.17 | D |
| I-218 | 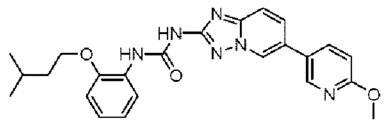 | 2.37 | 447.16 | D |
| I-225 | 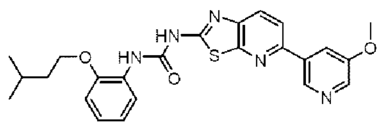 | 2.21 | 464.12 | D |
| I-219 | 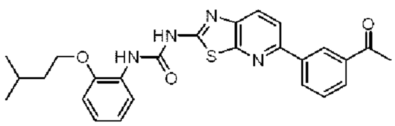 | 2.58 | 465.14 | D |

[Table 1-34]

| Compound No. | Structural Formula | Retention Time (min) | [M+H]+ | LC Method |
|---|---|---|---|---|
| I-220 | 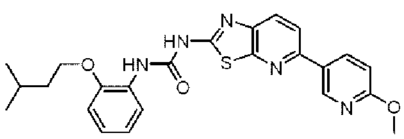 | 2.6 | 464.12 | D |
| I-226 | 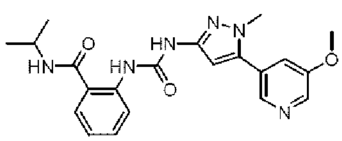 | 1.33 | 409.16 | D |
| I-227 | 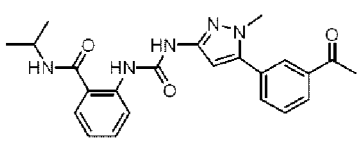 | 1.64 | 420.17 | D |
| I-228 | 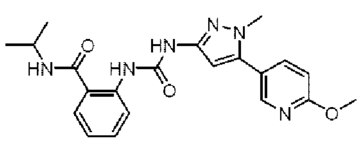 | 1.6 | 409.16 | D |

(continued)

| Compound No. | Structural Formula | Retention Time (min) | [M+H]+ | LC Method |
|---|---|---|---|---|
| I-221 | 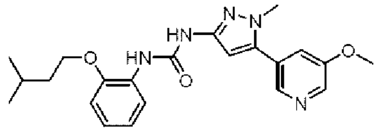 | 2.05 | 410.21 | D |
| I-222 | 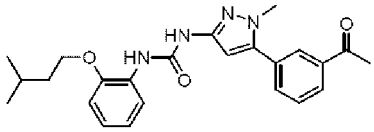 | 2.3 | 421.22 | D |
| I-223 | 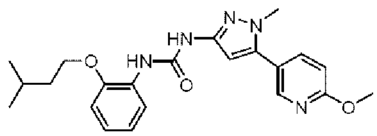 | 2.32 | 410.21 | D |
| I-229 | 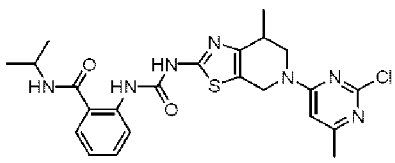 | 1.06 | 501 | C |

By carrying out the same reactions as in the above-described methods, the following compounds can be synthesized.

In formula (I-Y3a):

[Formula 117]

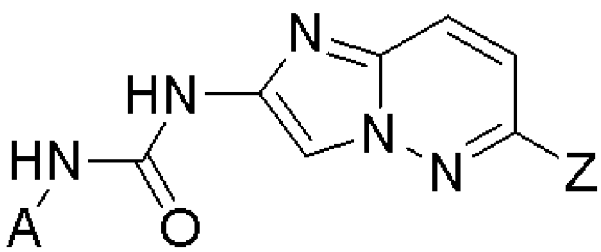

(I-Y3a)

wherein

A is a group represented by a formula selected from the following:

[Formula 118]

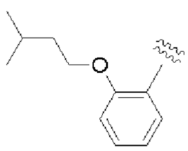
(A1) ,
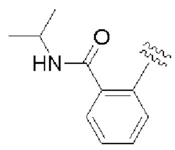
(A2) ,
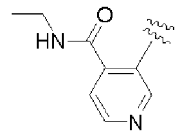
(A3) ,
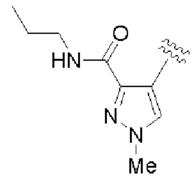
(A4) ,
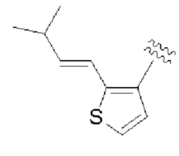
(A5)
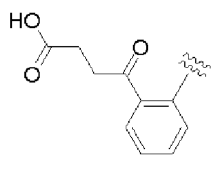
(A6) ,
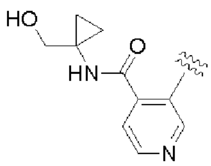
(A7) ,
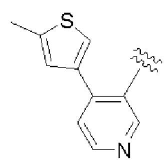
(A8) ,
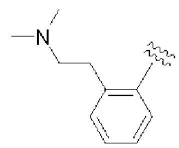
(A9) ,
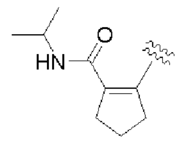
(A10)
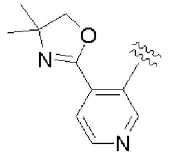
(A11) ,
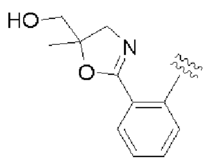
(A12) ,
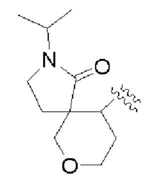
(A13) ,
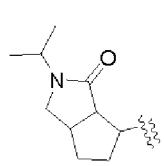
(A14) ,
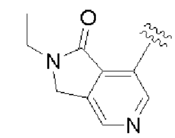
(A15)
,
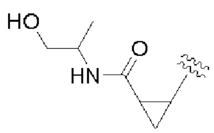
(A16) , or
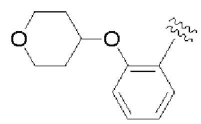
(A17)

and Z is a group represented by a formula selected from the following:

[Formula 119]

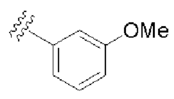
(Z1) ,
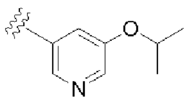
(Z2) ,
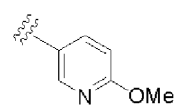
(Z3) ,
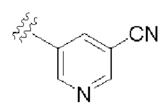
(Z4) ,
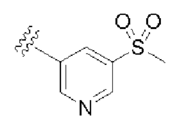
(Z5)
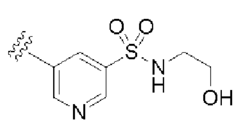
(Z6) ,
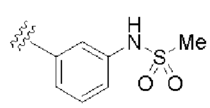
(Z7) ,
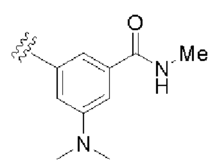
(Z8) ,
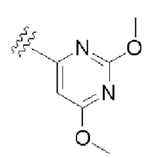
(Z9) ,
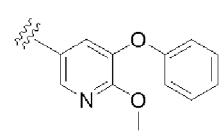
(Z10)
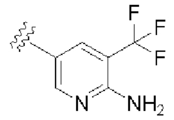
(Z11) ,
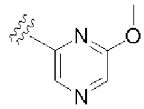
(Z12) ,
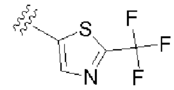
(Z13) ,
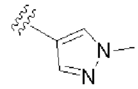
(Z14) or
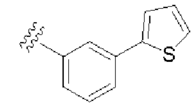
(Z15)

compounds represented by the following combinations: (Compound No., A, Z) = (P-1, A1, Z1), (P-2, A1, Z2), (P-3, A1, Z3), (P-4, A1, Z4), (P-5, A1, Z5), (P-6, A1, Z6), (P-7, A1, Z7), (P-8, A1, Z8), (P-9, A1, Z9), (P-10, A1, Z10), (P-11, A1, Z11), (P-12, A1, Z12), (P-13, A1, Z13), (P-14, A1, Z14), (P-15, A1, Z15), (P-16, A2, Z1), (P-17, A2, Z2), (P-18, A2, Z3), (P-19, A2, Z4), (P-20, A2, Z5), (P-21, A2, Z6), (P-22, A2, Z7), (P-23, A2, Z8), (P-24, A2, Z9), (P-25, A2, Z10), (P-26, A2, Z11), (P-27, A2, Z12), (P-28, A2, Z13), (P-29, A2, Z14), (P-30, A2, Z15), (P-31, A3, Z1), (P-32, A3, Z2), (P-33, A3, Z3), (P-34, A3, Z4), (P-35, A3, Z5), (P-36, A3, Z6), (P-37, A3, Z7), (P-38, A3, Z8), (P-39, A3, Z9), (P-40, A3, Z10), (P-41, A3, Z11), (P-42, A3, Z12), (P-43, A3, Z13), (P-44, A3, Z14), (P-45, A3, Z15), (P-46, A4, Z1), (P-47, A4, Z2), (P-48, A4, Z3), (P-49, A4, Z4), (P-50, A4, Z5), (P-51, A4, Z6), (P-52, A4, Z7), (P-53, A4, Z8), (P-54, A4, Z9), (P-55, A4, Z10), (P-56, A4, Z11), (P-57, A4, Z12), (P-58, A4, Z13), (P-59, A4, Z14), (P-60, A4, Z15), (P-61, A5, Z1), (P-62, A5, Z2), (P-63, A5, Z3), (P-64, A5, Z4), (P-65, A5, Z5), (P-66, A5, Z6), (P-67, A5, Z7), (P-68, A5, Z8), (P-69, A5, Z9), (P-70, A5, Z10), (P-71, A5, Z11), (P-72, A5, Z12), (P-73, A5, Z13), (P-74, A5, Z14), (P-75, A5, Z15), (P-76, A6, Z1), (P-77, A6, Z2), (P-78, A6, Z3), (P-79, A6, Z4), (P-80, A6, Z5), (P-81, A6, Z6), (P-82, A6, Z7), (P-83, A6, Z8), (P-84, A6, Z9), (P-85, A6, Z10), (P-86, A6, Z11), (P-87, A6, Z12), (P-88, A6, Z13), (P-89, A6, Z14), (P-90, A6, Z15), (P-91, A7, Z1), (P-92, A7, Z2), (P-93, A7, Z3), (P-94, A7, Z4), (P-95, A7, Z5), (P-96, A7, Z6), (P-97, A7, Z7), (P-98, A7, Z8), (P-99, A7, Z9), (P-100, A7, Z10), (P-101, A7, Z11), (P-102, A7, Z12), (P-103, A7, Z13), (P-104, A7, Z14), (P-105, A7, Z15), (P-106, A8, Z1), (P-107, A8, Z2), (P-108, A8, Z3), (P-109, A8, Z4), (P-110, A8, Z5), (P-111, A8, Z6), (P-112, A8, Z7), (P-113, A8, Z8), (P-114, A8, Z9), (P-115, A8, Z10), (P-116, A8, Z11), (P-117, A8, Z12), (P-118, A8, Z13), (P-119, A8, Z14), (P-120, A8, Z15), (P-121, A9, Z1), (P-122, A9, Z2), (P-123, A9, Z3), (P-124, A9, Z4), (P-125, A9, Z5), (P-126, A9, Z6), (P-127, A9, Z7), (P-128, A9, Z8), (P-129, A9, Z9), (P-130, A9, Z10), (P-131, A9, Z11), (P-132, A9, Z12), (P-133, A9, Z13), (P-134, A9, Z14), (P-135, A9, Z15), (P-136, A10, Z1), (P-137, A10, Z2), (P-138, A10, Z3), (P-139, A10, Z4), (P-140, A10, Z5), (P-141, A10, Z6), (P-142, A10, 27), (P-143, A10, Z8), (P-144, A10, Z9), (P-145, A10, Z10), (P-146, A10, Z11), (P-147, A10, Z12), (P-148, A10, Z13), (P-149, A10, Z14), (P-150, A10, Z15), (P-151, A11, Z1), (P-152, A11, Z2), (P-153, A11, Z3), (P-154, A11, Z4), (P-155, A11, Z5), (P-156, A11, Z6), (P-157, A11, Z7), (P-158, A11, Z8), (P-159, A11, Z9), (P-160, A11, Z10), (P-161, A11, Z11), (P-162, A11, Z12), (P-163, A11, Z13), (P-164, A11, Z14), (P-165, A11, Z15), (P-166, A12, Z1), (P-167, A12, Z2), (P-168, A12, Z3), (P-169, A12, 24), (P-170, A12, Z5), (P-171, A12, Z6), (P-172, A12, Z7), (P-173, A12, Z8), (P-174, A12, Z9), (P-175, A12, Z10), (P-176, A12, Z11), (P-177, A12, Z12), (P-178, A12, Z13), (P-179, A12, Z14), (P-180, A12, Z15), (P-181, A13, Z1), (P-182, A13, Z2), (P-183, A13, Z3), (P-184, A13, Z4), (P-185, A13, Z5), (P-186, A13, Z6), (P-187, A13, Z7), (P-188, A13, Z8), (P-189, A13, Z9), (P-190, A13, Z10), (P-191, A13, Z11), (P-192, A13, Z12), (P-193, A13, Z13), (P-194, A13, Z14), (P-195, A13, Z15), (P-196, A14, Z1), (P-197, A14, Z2), (P-198, A14, Z3), (P-199, A14, Z4), (P-200, A14, Z5), (P-201, A14, Z6), (P-202, A14, Z7), (P-203, A14, Z8), (P-204, A14, Z9), (P-205, A14, Z10), (P-206, A14, Z11), (P-207, A14, Z12), (P-208, A14, Z13), (P-209, A14, Z14), (P-210, A14, Z15), (P-211, A15, Z1), (P-212, A15, Z2), (P-213, A15, Z3), (P-214, A15, Z4), (P-215, A15, Z5), (P-216, A15, Z6), (P-217, A15, Z7), (P-218, A15, Z8), (P-219, A15, Z9), (P-220, A15, Z10), (P-221, A15, Z11), (P-222, A15, Z12), (P-223, A15, Z13), (P-224, A15, Z14), (P-225, A15, Z15), (P-226, A16, Z1), (P-227, A16, Z2), (P-228, A16, Z3), (P-229, A16, Z4), (P-230, A16, Z5), (P-231, A16, Z6), (P-232, A16, Z7), (P-233, A16, Z8), (P-234, A16, Z9), (P-235, A16, Z10), (P-236, A16, Z11), (P-237, A16, Z12), (P-238, A16, Z13), (P-239, A16, Z14), (P-240, A16, Z15), (P-241, A17, Z1), (P-242, A17, Z2), (P-243, A17, Z3), (P-244, A17, Z4), (P-245, A17, Z5), (P-246, A17, Z6), (P-247, A17, Z7), (P-248, A17, Z8), (P-249, A17, Z9), (P-250, A17, Z10), (P-251, A17, Z11), (P-252, A17, Z12), (P-253, A17, Z13), (P-254, A17, Z14), (P-255, A17, Z15).

Further, by carrying out the same reactions as in the above-described methods, compounds can be synthesized in which a combination of AA and ZZ, *i.e.,* (AA, ZZ), in formulas (I-Y3b), (I-Y3e), (I-Y3f), (I-Y3g), (I-Y3h), (I-Y3j), (I-Y3k), (I-Y4a), and (I-Y4c) described below is a combination selected from the following.
In the following formulas:
[Formula 120]

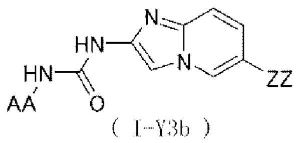
( I-Y3b ) ,
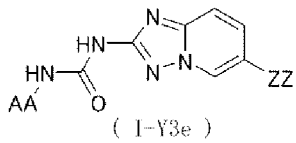
( I-Y3e ) ,
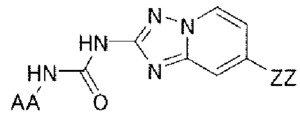
( I-Y3f ) ,
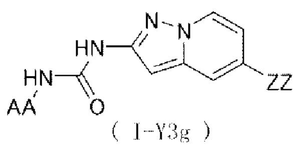
( I-Y3g ) ,
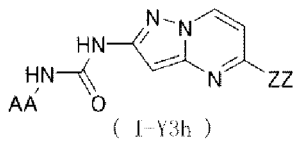
( I-Y3h ) ,
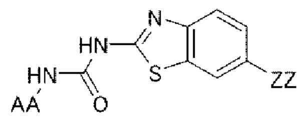
( I-Y3j ) ,
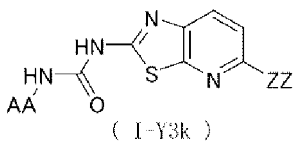
( I-Y3k ) ,
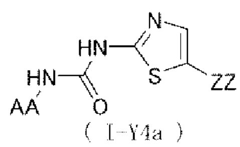
( I-Y4a ) and
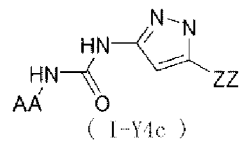
( I-Y4c )

wherein
AA is a group represented by a formula selected from the following:
[Formula 121]

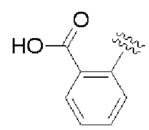
(AA1) ,
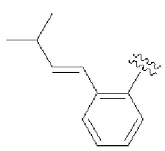
(AA2) ,
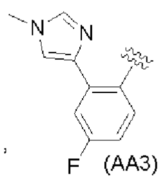
(AA3) ,
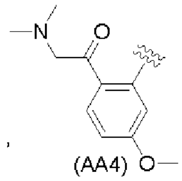
(AA4) ,
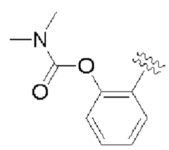
(AA5) ,
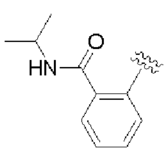
(AA6) ,
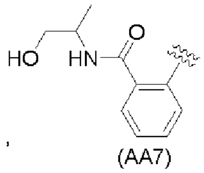
(AA7) ,
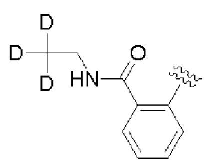
(AA8) ,
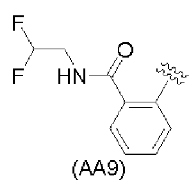
(AA9) ,
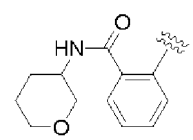
(AA10) ,
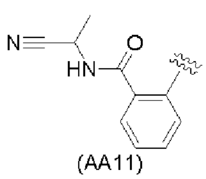
(AA11) ,
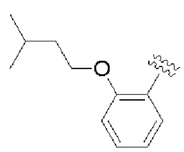
(AA12) ,
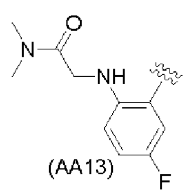
(AA13) ,
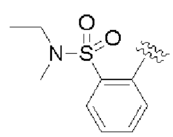
(AA14) ,
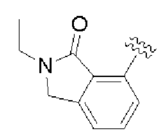
(AA15) ,
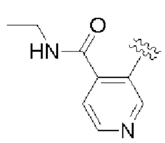
(AA16) ,
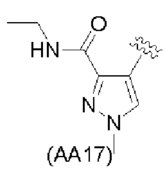
(AA17) ,
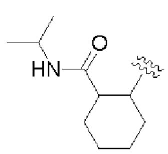
(AA18) ,
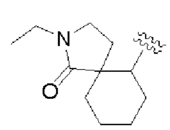
(AA19) ,
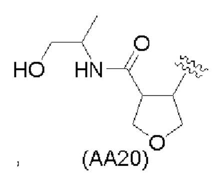
(AA20)

or
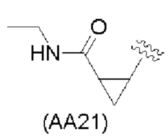
(AA21)

and ZZ is a group represented by a formula selected from the following:

[Formula 122]

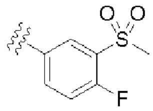
(ZZ1),
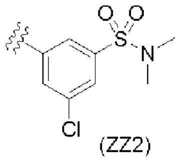
(ZZ2),
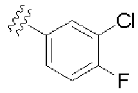
(ZZ3),
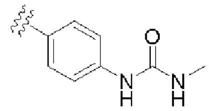
(ZZ4),
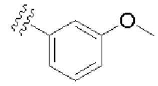
(ZZ5),
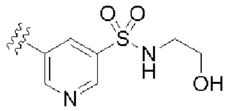
(ZZ6),
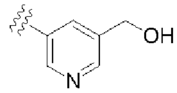
(ZZ7),
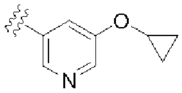
(ZZ8),
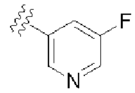
(ZZ9),
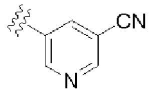
(ZZ10),
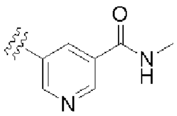
(ZZ11),
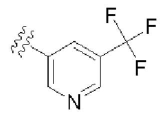
(ZZ12),
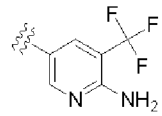
(ZZ13),
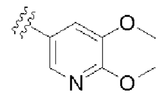
(ZZ14),
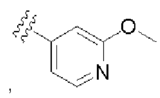
(ZZ15),
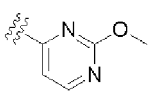
(ZZ16),
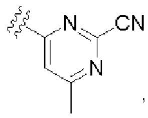
(ZZ17),
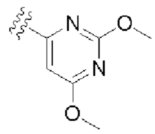
(ZZ18),
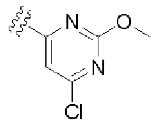
(ZZ19),
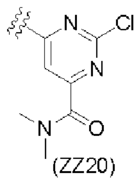
(ZZ20),
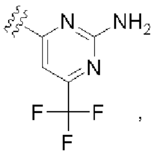
(ZZ21),
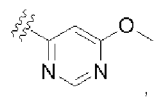
(ZZ22),
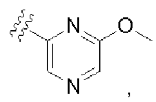
(ZZ23),
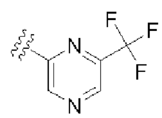
(ZZ24),
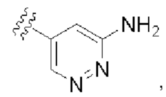
(ZZ25),
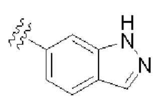
(ZZ26),
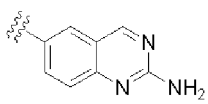
(ZZ27),
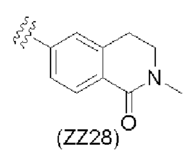
(ZZ28),
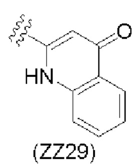
(ZZ29),
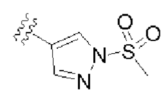
(ZZ30),
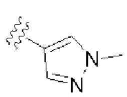
(ZZ31),
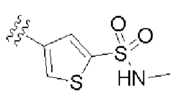
(ZZ32),
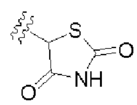
(ZZ33) or
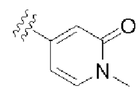
(ZZ34)

combinations of AA and ZZ represented by the following: (AA, ZZ) = (AA1, ZZ1), (AA1, ZZ2), (AA1, ZZ3), (AA1, ZZ4), (AA1, ZZ5), (AA1, ZZ6), (AA1, ZZ7), (AA1, ZZ8), (AA1, ZZ9), (AA1, ZZ10), (AA1, ZZ11), (AA1, ZZ12), (AA1, ZZ13), (AA1, ZZ14), (AA1, ZZ15), (AA1, ZZ16), (AA1, ZZ17), (AA1, ZZ18), (AA1, ZZ19), (AA1, ZZ20), (AA1, ZZ21), (AA1, ZZ22), (AA1, ZZ23), (AA1, ZZ24), (AA1, ZZ25), (AA1, ZZ26), (AA1, ZZ27), (AA1, ZZ28), (AA1, ZZ29), (AA1, ZZ30), (AA1, ZZ31), (AA1, ZZ32), (AA1, ZZ33), (AA1, ZZ34), (AA2, ZZ1), (AA2, ZZ2), (AA2, ZZ3), (AA2, ZZ4), (AA2, ZZ5), (AA2, ZZ6), (AA2, ZZ7), (AA2, ZZ8), (AA2, ZZ9), (AA2, ZZ10), (AA2, ZZ11), (AA2, ZZ12), (AA2, ZZ13), (AA2, ZZ14), (AA2, ZZ15), (AA2, ZZ16), (AA2, ZZ17), (AA2, ZZ18), (AA2, ZZ19), (AA2, ZZ20), (AA2, ZZ21), (AA2, ZZ22), (AA2, ZZ23), (AA2, ZZ24), (AA2, ZZ25), (AA2, ZZ26), (AA2, ZZ27), (AA2, ZZ28), (AA2, ZZ29), (AA2, ZZ30), (AA2, ZZ31), (AA2, ZZ32), (AA2, ZZ33), (AA2, ZZ34), (AA3, ZZ1), (AA3, ZZ2), (AA3, ZZ3), (AA3, ZZ4), (AA3, ZZ5), (AA3,

ZZ6), (AA3, ZZ7), (AA3, ZZ8), (AA3, ZZ9), (AA3, ZZ10), (AA3, ZZ11), (AA3, ZZ12), (AA3, ZZ13), (AA3, ZZ14), (AA3, ZZ15), (AA3, ZZ16), (AA3, ZZ17), (AA3, ZZ18), (AA3, ZZ19), (AA3, ZZ20), (AA3, ZZ21), (AA3, ZZ22), (AA3, ZZ23), (AA3, ZZ24), (AA3, ZZ25), (AA3, ZZ26), (AA3, ZZ27), (AA3, ZZ28), (AA3, ZZ29), (AA3, ZZ30), (AA3, ZZ31), (AA3, ZZ32), (AA3, ZZ33), (AA3, ZZ34), (AA4, ZZ1), (AA4, ZZ2), (AA4, ZZ3), (AA4, ZZ4), (AA4, ZZ5), (AA4, ZZ6), (AA4, ZZ7), (AA4, ZZ8), (AA4, ZZ9), (AA4, ZZ10), (AA4, ZZ11), (AA4, ZZ12), (AA4, ZZ13), (AA4, ZZ14), (AA4, ZZ15), (AA4, ZZ16), (AA4, ZZ17), (AA4, ZZ18), (AA4, ZZ19), (AA4, ZZ20), (AA4, ZZ21), (AA4, ZZ22), (AA4, ZZ23), (AA4, ZZ24), (AA4, ZZ25), (AA4, ZZ26), (AA4, ZZ27), (AA4, ZZ28), (AA4, ZZ29), (AA4, ZZ30), (AA4, ZZ31), (AA4, ZZ32), (AA4, ZZ33), (AA4, ZZ34), (AA5, ZZ1), (AA5, ZZ2), (AA5, ZZ3), (AA5, ZZ4), (AA5, ZZ5), (AA5, ZZ6), (AA5, ZZ7), (AA5, ZZ8), (AA5, ZZ9), (AA5, ZZ10), (AA5, ZZ11), (AA5, ZZ12), (AA5, ZZ13), (AA5, ZZ14), (AA5, ZZ15), (AA5, ZZ16), (AA5, ZZ17), (AA5, ZZ18), (AA5, ZZ19), (AA5, ZZ20), (AA5, ZZ21), (AA5, ZZ22), (AA5, ZZ23), (AA5, ZZ24), (AA5, ZZ25), (AA5, ZZ26), (AA5, ZZ27), (AA5, ZZ28), (AA5, ZZ29), (AA5, ZZ30), (AA5, ZZ31), (AA5, ZZ32), (AA5, ZZ33), (AA5, ZZ34), (AA6, ZZ1), (AA6, ZZ2), (AA6, ZZ3), (AA6, ZZ4), (AA6, ZZ5), (AA6, ZZ6), (AA6, ZZ7), (AA6, ZZ8), (AA6, ZZ9), (AA6, ZZ10), (AA6, ZZ11), (AA6, ZZ12), (AA6, ZZ13), (AA6, ZZ14), (AA6, ZZ15), (AA6, ZZ16), (AA6, ZZ17), (AA6, ZZ18), (AA6, ZZ19), (AA6, ZZ20), (AA6, ZZ21), (AA6, ZZ22), (AA6, ZZ23), (AA6, ZZ24), (AA6, ZZ25), (AA6, ZZ26), (AA6, ZZ27), (AA6, ZZ28), (AA6, ZZ29), (AA6, ZZ30), (AA6, ZZ31), (AA6, ZZ32), (AA6, ZZ33), (AA6, ZZ34), (AA7, ZZ1), (AA7, ZZ2), (AA7, ZZ3), (AA7, ZZ4), (AA7, ZZ5), (AA7, ZZ6), (AA7, ZZ7), (AA7, ZZ8), (AA7, ZZ9), (AA7, ZZ10), (AA7, ZZ11), (AA7, ZZ12), (AA7, ZZ13), (AA7, ZZ14), (AA7, ZZ15), (AA7, ZZ16), (AA7, ZZ17), (AA7, ZZ18), (AA7, ZZ19), (AA7, ZZ20), (AA7, ZZ21), (AA7, ZZ22), (AA7, ZZ23), (AA7, ZZ24), (AA7, ZZ25), (AA7, ZZ26), (AA7, ZZ27), (AA7, ZZ28), (AA7, ZZ29), (AA7, ZZ30), (AA7, ZZ31), (AA7, ZZ32), (AA7, ZZ33), (AA7, ZZ34), (AA8, ZZ1), (AA8, ZZ2), (AA8, ZZ3), (AA8, ZZ4), (AA8, ZZ5), (AA8, ZZ6), (AA8, ZZ7), (AA8, ZZ8), (AA8, ZZ9), (AA8, ZZ10), (AA8, ZZ11), (AA8, ZZ12), (AA8, ZZ13), (AA8, ZZ14), (AA8, ZZ15), (AA8, ZZ16), (AA8, ZZ17), (AA8, ZZ18), (AA8, ZZ19), (AA8, ZZ20), (AA8, ZZ21), (AA8, ZZ22), (AA8, ZZ23), (AA8, ZZ24), (AA8, ZZ25), (AA8, ZZ26), (AA8, ZZ27), (AA8, ZZ28), (AA8, ZZ29), (AA8, ZZ30), (AA8, ZZ31), (AA8, ZZ32), (AA8, ZZ33), (AA8, ZZ34), (AA9, ZZ1), (AA9, ZZ2), (AA9, ZZ3), (AA9, ZZ4), (AA9, ZZ5), (AA9, ZZ6), (AA9, ZZ7), (AA9, ZZ8), (AA9, ZZ9), (AA9, ZZ10), (AA9, ZZ11), (AA9, ZZ12), (AA9, ZZ13), (AA9, ZZ14), (AA9, ZZ15), (AA9, ZZ16), (AA9, ZZ17), (AA9, ZZ18), (AA9, ZZ19), (AA9, ZZ20), (AA9, ZZ21), (AA9, ZZ22), (AA9, ZZ23), (AA9, ZZ24), (AA9, ZZ25), (AA9, ZZ26), (AA9, ZZ27), (AA9, ZZ28), (AA9, ZZ29), (AA9, ZZ30), (AA9, ZZ31), (AA9, ZZ32), (AA9, ZZ33), (AA9, ZZ34), (AA10, ZZ1), (AA10, ZZ2), (AA10, ZZ3), (AA10, ZZ4), (AA10, ZZ5), (AA10, ZZ6), (AA10, ZZ7), (AA10, ZZ8), (AA10, ZZ9), (AA10, ZZ10), (AA10, ZZ11), (AA10, ZZ12), (AA10, ZZ13), (AA10, ZZ14), (AA10, ZZ15), (AA10, ZZ16), (AA10, ZZ17), (AA10, ZZ18), (AA10, ZZ19), (AA10, ZZ20), (AA10, ZZ21), (AA10, ZZ22), (AA10, ZZ23), (AA10, ZZ24), (AA10, ZZ25), (AA10, ZZ26), (AA10, ZZ27), (AA10, ZZ28), (AA10, ZZ29), (AA10, ZZ30), (AA10, ZZ31), (AA10, ZZ32), (AA10, ZZ33), (AA10, ZZ34), (AA11, ZZ1), (AA11, ZZ2), (AA11, ZZ3), (AA11, ZZ4), (AA11, ZZ5), (AA11, ZZ6), (AA11, ZZ7), (AA11, ZZ8), (AA11, ZZ9), (AA11, ZZ10), (AA11, ZZ11), (AA11, ZZ12), (AA11, ZZ13), (AA11, ZZ14), (AA11, ZZ15), (AA11, ZZ16), (AA11, ZZ17), (AA11, ZZ18), (AA11, ZZ19), (AA11, ZZ20), (AA11, ZZ21), (AA11, ZZ22), (AA11, ZZ23), (AA11, ZZ24), (AA11, ZZ25), (AA11, ZZ26), (AA11, ZZ27), (AA11, ZZ28), (AA11, ZZ29), (AA11, ZZ30), (AA11, ZZ31), (AA11, ZZ32), (AA11, ZZ33), (AA11, ZZ34), (AA12, ZZ1), (AA12, ZZ2), (AA12, ZZ3), (AA12, ZZ4), (AA12, ZZ5), (AA12, ZZ6), (AA12, ZZ7), (AA12, ZZ8), (AA12, ZZ9), (AA12, ZZ10), (AA12, ZZ11), (AA12, ZZ12), (AA12, ZZ13), (AA12, ZZ14), (AA12, ZZ15), (AA12, ZZ16), (AA12, ZZ17), (AA12, ZZ18), (AA12, ZZ19), (AA12, ZZ20), (AA12, ZZ21), (AA12, ZZ22), (AA12, ZZ23), (AA12, ZZ24), (AA12, ZZ25), (AA12, ZZ26), (AA12, ZZ27), (AA12, ZZ28), (AA12, ZZ29), (AA12, ZZ30), (AA12, ZZ31), (AA12, ZZ32), (AA12, ZZ33), (AA12, ZZ34), (AA13, ZZ1), (AA13, ZZ2), (AA13, ZZ3), (AA13, ZZ4), (AA13, ZZ5), (AA13, ZZ6), (AA13, ZZ7), (AA13, ZZ8), (AA13, ZZ9), (AA13, ZZ10), (AA13, ZZ11), (AA13, ZZ12), (AA13, ZZ13), (AA13, ZZ14), (AA13, ZZ15), (AA13, ZZ16), (AA13, ZZ17), (AA13, ZZ18), (AA13, ZZ19), (AA13, ZZ20), (AA13, ZZ21), (AA13, ZZ22), (AA13, ZZ23), (AA13, ZZ24), (AA13, ZZ25), (AA13, ZZ26), (AA13, ZZ27), (AA13, ZZ28), (AA13, ZZ29), (AA13, ZZ30), (AA13, ZZ31), (AA13, ZZ32), (AA13, ZZ33), (AA13, ZZ34), (AA14, ZZ1), (AA14, ZZ2), (AA14, ZZ3), (AA14, ZZ4), (AA14, ZZ5), (AA14, ZZ6), (AA14, ZZ7), (AA14, ZZ8), (AA14, ZZ9), (AA14, ZZ10), (AA14, ZZ11), (AA14, ZZ12), (AA14, ZZ13), (AA14, ZZ14), (AA14, ZZ15), (AA14, ZZ16), (AA14, ZZ17), (AA14, ZZ18), (AA14, ZZ19), (AA14, ZZ20), (AA14, ZZ21), (AA14, ZZ22), (AA14, ZZ23), (AA14, ZZ24), (AA14, ZZ25), (AA14, ZZ26), (AA14, ZZ27), (AA14, ZZ28), (AA14, ZZ29), (AA14, ZZ30), (AA14, ZZ31), (AA14, ZZ32), (AA14, ZZ33), (AA14, ZZ34), (AA15, ZZ1), (AA15, ZZ2), (AA15, ZZ3), (AA15, ZZ4), (AA15, ZZ5), (AA15, ZZ6), (AA15, ZZ7), (AA15, ZZ8), (AA15, ZZ9), (AA15, ZZ10), (AA15, ZZ11), (AA15, ZZ12), (AA15, ZZ13), (AA15, ZZ14), (AA15, ZZ15), (AA15, ZZ16), (AA15, ZZ17), (AA15, ZZ18), (AA15, ZZ19), (AA15, ZZ20), (AA15, ZZ21), (AA15, ZZ22), (AA15, ZZ23), (AA15, ZZ24), (AA15, ZZ25), (AA15, ZZ26), (AA15, ZZ27), (AA15, ZZ28), (AA15, ZZ29), (AA15, ZZ30), (AA15, ZZ31), (AA15, ZZ32), (AA15, ZZ33), (AA15, ZZ34), (AA16, ZZ1), (AA16, ZZ2), (AA16, ZZ3), (AA16, ZZ4), (AA16, ZZ5), (AA16, ZZ6), (AA16, ZZ7), (AA16, ZZ8), (AA16, ZZ9), (AA16, ZZ10), (AA16, ZZ11), (AA16, ZZ12), (AA16, ZZ13), (AA16, ZZ14), (AA16, ZZ15), (AA16, ZZ16), (AA16, ZZ17), (AA16, ZZ18), (AA16, ZZ19), (AA16, ZZ20), (AA16, ZZ21), (AA16, ZZ22), (AA16, ZZ23), (AA16, ZZ24), (AA16, ZZ25), (AA16, ZZ26), (AA16, ZZ27), (AA16, ZZ28), (AA16, ZZ29), (AA16, ZZ30), (AA16, ZZ31), (AA16, ZZ32), (AA16, ZZ33), (AA16, ZZ34), (AA17, ZZ1), (AA17, ZZ2), (AA17, ZZ3), (AA17, ZZ4), (AA17, ZZ5), (AA17, ZZ6), (AA17, ZZ7), (AA17, ZZ8), (AA17, ZZ9), (AA17, ZZ10), (AA17, ZZ11), (AA17, ZZ12), (AA17, ZZ13), (AA17, ZZ14), (AA17, ZZ15), (AA17, ZZ16), (AA17, ZZ17), (AA17, ZZ18), (AA17, ZZ19), (AA17, ZZ20), (AA17, ZZ21),

(AA17, ZZ22), (AA17, ZZ23), (AA17, ZZ24), (AA17, ZZ25), (AA17, ZZ26), (AA17, ZZ27), (AA17, ZZ28), (AA17, ZZ29), (AA17, ZZ30), (AA17, ZZ31), (AA17, ZZ32), (AA17, ZZ33), (AA17, ZZ34), (AA18, ZZ1), (AA18, ZZ2), (AA18, ZZ3), (AA18, ZZ4), (AA18, ZZ5), (AA18, ZZ6), (AA18, ZZ7), (AA18, ZZ8), (AA18, ZZ9), (AA18, ZZ10), (AA18, ZZ11), (AA18, ZZ12), (AA18, ZZ13), (AA18, ZZ14), (AA18, ZZ15), (AA18, ZZ16), (AA18, ZZ17), (AA18, ZZ18), (AA18, ZZ19), (AA18, ZZ20), (AA18, ZZ21), (AA18, ZZ22), (AA18, ZZ23), (AA18, ZZ24), (AA18, ZZ25), (AA18, ZZ26), (AA18, ZZ27), (AA18, ZZ28), (AA18, ZZ29), (AA18, ZZ30), (AA18, ZZ31), (AA18, ZZ32), (AA18, ZZ33), (AA18, ZZ34), (AA19, ZZ1), (AA19, ZZ2), (AA19, ZZ3), (AA19, ZZ4), (AA19, ZZ5), (AA19, ZZ6), (AA19, ZZ7), (AA19, ZZ8), (AA19, ZZ9), (AA19, ZZ10), (AA19, ZZ11), (AA19, ZZ12), (AA19, ZZ13), (AA19, ZZ14), (AA19, ZZ15), (AA19, ZZ16), (AA19, ZZ17), (AA19, ZZ18), (AA19, ZZ19), (AA19, ZZ20), (AA19, ZZ21), (AA19, ZZ22), (AA19, ZZ23), (AA19, ZZ24), (AA19, ZZ25), (AA19, ZZ26), (AA19, ZZ27), (AA19, ZZ28), (AA19, ZZ29), (AA19, ZZ30), (AA19, ZZ31), (AA19, ZZ32), (AA19, ZZ33), (AA19, ZZ34), (AA20, ZZ1), (AA20, ZZ2), (AA20, ZZ3), (AA20, ZZ4), (AA20, ZZ5), (AA20, ZZ6), (AA20, ZZ7), (AA20, ZZ8), (AA20, ZZ9), (AA20, ZZ10), (AA20, ZZ11), (AA20, ZZ12), (AA20, ZZ13), (AA20, ZZ14), (AA20, ZZ15), (AA20, ZZ16), (AA20, ZZ17), (AA20, ZZ18), (AA20, ZZ19), (AA20, ZZ20), (AA20, ZZ21), (AA20, ZZ22), (AA20, ZZ23), (AA20, ZZ24), (AA20, ZZ25), (AA20, ZZ26), (AA20, ZZ27), (AA20, ZZ28), (AA20, ZZ29), (AA20, ZZ30), (AA20, ZZ31), (AA20, ZZ32), (AA20, ZZ33), (AA20, ZZ34), (AA21, ZZ1), (AA21, ZZ2), (AA21, Z3), (AA21, ZZ4), (AA21, ZZ5), (AA21, ZZ6), (AA21, ZZ7), (AA21, ZZ8), (AA21, ZZ9), (AA21, ZZ10), (AA21, ZZ11), (AA21, ZZ12), (AA21, ZZ13), (AA21, ZZ14), (AA21, ZZ15), (AA21, ZZ16), (AA21, ZZ17), (AA21, ZZ18), (AA21, ZZ19), (AA21, ZZ20), (AA21, ZZ21), (AA21, ZZ22), (AA21, ZZ23), (AA21, ZZ24), (AA21, ZZ25), (AA21, ZZ26), (AA21, ZZ27), (AA21, ZZ28), (AA21, ZZ29), (AA21, ZZ30), (AA21, ZZ31), (AA21, ZZ32), (AA21, ZZ33), (AA21, ZZ34).

Moreover, by carrying out the same reactions as in the above-described methods, compounds shown below can be synthesized.
The formulas:

[Formula 123]

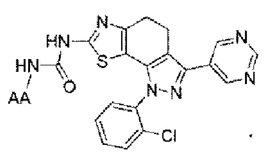
(I-Y5a-α)
,
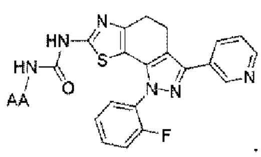
(I-Y5a-β)
,
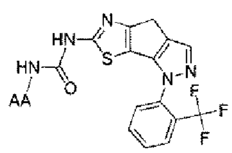
(I-Y5c-α)
and
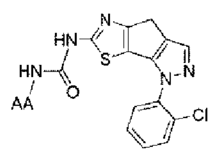
(I-Y5c-β)

wherein
AA is a group represented by a formula selected from formula (AA1) to (AA21) described above.

(Example 2: Measurement of PI3Kγ inhibitory activity)

For each compound synthesized in the above Examples, PI3Kγ inhibitory activity was measured.

(Method)

The PI3Kγ inhibitory activity of a compound was evaluated using PI3-kinase HTRF™ assay (Millipore) according to the following procedure:

To each well of a testing plate, 5 μL of a compound solution containing 10% DMSO (40 μmol/L as the concentration of the compound), 5 μL of a substrate solution (40 μmol/L phosphatidylinositol (4,5)-bisphosphate, 20 mmol/L $MgCl_2$, 10 mmol/L DTT), and 5 μL of an enzyme solution (80 μg/mL PI-3 kinase γ, 10 mmol/L $MgCl_2$, 5 mmol/L DTT) were added, and then allowed to stand for 10 minutes.

Then, 5 μL of a reaction solution (40 μmol/L ATP, 10 mmol/L $MgCl_2$, 5 mmol/L DTT) was added thereto. After reacting for 30 minutes at room temperature, 5 μL of a solution containing EDTA and biotinylated phosphatidylinositol (3,4,5)-triphosphate was added to quench the reaction.

5 μL of a detection reagent containing a europium-labeled anti-GST antibody, the GST-tagged PH domain, and allophycocyanin-labeled streptavidin was added thereto. After 18 hours, HTRF (excitation wavelength: 330 nm, measured wavelengths: 620 nm and 665 nm) was measured.

HTRF ratio was defined as the value obtained by dividing the amount of fluorescence obtained at the measured wavelength 665 nm by the amount of fluorescence obtained at 620 nm. The HTRF ratio in the absence of a compound

was defined as 100% activity and the HTRF ratio in the absence of PI-3 kinase γ was defined as 0% activity to calculate an inhibition ratio, which was defined as the PI3Kγ inhibitory activity of a compound at 10 μmol/L.

(Results)

Results are shown in Tables 2-1 to 2-5 below. In the tables, "μM" denotes "μmol/L," and "PI3Kg" denotes "PI3Kγ."

[Table 2-1]

| Compound No. | PI3Kg Inhibition Ratio% (10 μM) | Compound No. | PI3Kg Inhibition Ratio % (10 μM) |
|---|---|---|---|
| I-1 | 79 | I-17 | ≧95 |
| I-2 | 69 | I-18 | 94 |
| I-3 | ≧95 | I-19 | ≧95 |
| I-4 | 72 | I-20 | ≧95 |
| I-5 | 85 | I-21 | 74 |
| I-6 | ≧95 | I-22 | ≧95 |
| I-7 | 93 | I-3 | ≧95 |
| I-8 | 93 | I-24 | ≧95 |
| I-9 | 93 | I-25 | ≧95 |
| I-10 | 68 | I-26 | ≧95 |
| I-11 | 94 | I-27 | ≧95 |
| I-12 | 94 | I-28 | ≧95 |
| I-13 | ≧95 | I-29 | 95 |
| I-14 | 94 | I-31 | 90 |
| I-15 | 94 | I-32 | ≧95 |
| I-16 | 92 | | |

[Table 2-2]

| Compound No. | PI3Kg Inhibition Ratio % (10 μM) | Compound No. | PI3Kg inhibition Ratio % (10 μM) |
|---|---|---|---|
| I-35 | ≧95 | I-94 | ≧95 |
| I-44 | ≧95 | I-95 | ≧95 |
| I-47 | ≧95 | I-96 | ≧95 |
| I-60 | ≧95 | I-97 | ≧95 |
| I-63 | ≧95 | I-98 | ≧95 |
| I-65 | ≧95 | I-99 | ≧95 |
| I-66 | ≧95 | I-100 | ≧95 |
| I-67 | ≧95 | I-101 | ≧95 |
| I-68 | ≧95 | I-102 | ≧95 |
| I-83 | ≧95 | I-103 | ≧95 |
| I-85 | ≧95 | I-115 | ≧95 |
| I-86 | ≧95 | I-116 | ≧95 |

(continued)

| Compound No. | PI3Kg Inhibition Ratio % (10 μM) | Compound No. | PI3Kg inhibition Ratio % (10 μM) |
|---|---|---|---|
| I-87 | ≧95 | I-118 | ≧95 |
| I-88 | ≧95 | I-119 | ≧95 |
| I-89 | ≧95 | I-120 | ≧95 |
| I-91 | ≧95 | I-122 | ≧95 |
| I-93 | ≧95 | | |

[Table 2-3]

| Compound No. | PI3Kg Inhibition Ratio % (10 μM) | Compound No. | PI3Kg Inhibition Ratio % (10 μM) |
|---|---|---|---|
| I-127 | ≧95 | I-168 | 89 |
| I-128 | ≧95 | I-169 | ≧95 |
| I-129 | ≧95 | I-170 | ≧95 |
| I-131 | 78 | I-171 | 85 |
| I-132 | 85 | I-172 | ≧95 |
| I-133 | ≧95 | I-173 | 71 |
| I-134 | ≧95 | II-174 | ≧95 |
| I-135 | ≧95 | I-175 | ≧95 |
| I-136 | ≧95 | I-177 | 88 |
| I-137 | ≧95 | I-178 | ≧95 |
| I-138 | ≧95 | I-180 | 89 |
| I-139 | ≧95 | I-181 | 79 |
| I-146 | 77 | I-182 | ≧95 |
| I-148 | 90 | I-183 | 91 |
| I-152 | ≧95 | I-184 | 75 |
| I-153 | ≧95 | I-185 | ≧95 |
| I-154 | ≧95 | I-186 | 91 |
| I-155 | 90 | I-188 | 80 |
| I-156 | 80 | I-189 | 92 |
| I-157 | 87 | I-191 | 87 |
| I-158 | 86 | I-192 | 94 |
| I-159 | 77 | I-193 | 92 |
| I-160 | ≧95 | I-194 | 92 |
| I-161 | ≧95 | I-195 | ≧95 |
| I-163 | 83 | I-196 | 83 |
| I-164 | 93 | I-197 | ≧95 |
| I-166 | ≧95 | I-198 | ≧95 |
| 1-167 | ≧95 | I-200 | ≧95 |

[Table 2-4]

| Compound No. | PI3Kg Inhibition Ratio (%) (10 μM) |
|---|---|
| I-201 | 78 |
| I-202 | ≧ 95 |
| I-203 | 91 |
| I-204 | ≧ 95 |
| I-205 | ≧ 95 |
| I-206 | 87 |
| I-207 | 93 |
| I-209 | ≧ 95 |
| I-210 | 91 |
| I-211 | 87 |
| I-212 | ≧ 95 |

[Table 2-5]

| **Compound No.** | PI3Kg **Inhibition Ratio(%)** (10 μM) |
|---|---|
| I-214 | ≧ 95 |
| I-215 | ≧ 95 |
| I-216 | ≧ 95 |
| I-217 | 88 |
| I-218 | 90 |
| I-225 | ≧ 95 |
| I-219 | ≧ 95 |
| I-220 | ≧ 95 |
| I-226 | ≧ 93 |
| I-227 | ≧ 87 |
| I-228 | 88 |
| I-221 | ≧ 95 |
| I-222 | ≧ 95 |
| I-229 | ≧ 95 |

(Example 3: Measurement of AKT phosphorylation inhibitory activity)

Cells were used to measure whether or not they exhibit the inhibitory activity.

(Method)

(1) The AKT phosphorylation inhibitory activity of a compound was evaluated according to the following procedure.
(2) Human monocyte-like cell line THP-1 was washed with RPMI-1640 media, incubated in the presence of 5% $CO_2$

at 37°C for 3 hours, washed with Hank's balanced salt solution (HBSS), adjusted to a cell concentration of 6.7 x $10^6$ /mL, and then used in an experiment.
(3) 30 μL of the cell suspension and 60 μL of each compound solution containing 0.2% DMSO/HBSS were mixed and then preincubated at 37°C for 5 minutes. 30 μL of HBSS containing 1.2 μg/mL of MCP-1 was added thereto and then incubated for 60 seconds at 37°C.
(4) 30 μL of a cell lysate (the final concentrations: 20 mmol/L Tris-HCl (pH 7.5), 150 mmol/L NaCl, 1 mmol/L EDTA, 1 mmol/L EDTA, 1% Triton X-100, 2.5 mmol/L sodium pyrophosphate, 1 mmol/L β-glycerophosphate, 1 mmol/L $Na_3VO_4$, 1 μg/ml leupeptin, 50 nmol/L APMSF) was added thereto to lyse cells.
(5) The amount of AKT phosphorylation in the cell solution was measured by sandwich ELISA method using anti-Phospho-Akt (Ser473) antibody and anti-AKT1 antibody.
(6) Anti-Phospho-Akt (Ser473) antibodies or anti-AKT1 antibodies were immobilized onto the solid phase of a micro well plate. The prepared cell lysate was added to the micro well plate, incubated, and then washed with ELISA wash solution.
(7) An anti-AKT1 antibody or anti-Phospho-Akt (Ser473) antibody, which forms a counterpart to an antibody immobilized on the solid phase, was added thereto, incubated, washed similarly, and then reacted with an HRP-labeled anti-mouse IgG antibody.
(8) After incubating for 30 minutes and then washing similarly, 100 μL of TMB (3,3',5,5''-tetramethylbenzidine) was added thereto, followed by reacting for 30 minutes.
(9) 100 μL of stop solution was added to quench the color reaction, and then the absorbance at 450 nm was measured.
(10) The amount of AKT phosphorylation in a sample in the absence of a compound was defined as 100% activity, and the amount of AKT phosphorylation in a sample in the absence of MCP-1 was defined as 0% activity to calculate an inhibition ratio of each compound. The measurement was performed for a 1 μmol/L solution of each compound.

(Results) Inhibition ratios for respective compound at 1 μmol/L are shown.

Compound No. I-7: 77.6%
Compound No. I-12: ≥95%
Compound No. I-27: 94.1%
Compound No. I-88: 87.3%
Compound No. I-91: 94.7%
Compound No. I-97: 80.4%
Compound No. I-114: 64.9%
Compound No. I-127: 77.7%
Compound No. I-129: ≥95%
Compound No. I-131: 84%
Compound No. I-134: 93%
Compound No. I-135: 89%
Compound No. I-136: 84%
Compound No. I-137: 85%
Compound No. I-138: ≥95%
Compound No. I-139: ≥95%
Compound No. I-146: ≥95%
Compound No. I-148: ≥95%
Compound No. I-152: ≥95%
Compound No. I-153: ≥95%
Compound No. I-154: ≥95%
Compound No. I-155: ≥95%
Compound No. I-156: ≥95%
Compound No. I-157: ≥95%
Compound No. I-158: ≥95%
Compound No. I-159: ≥95%
Compound No. I-160: ≥95%
Compound No. I-161: 93%
Compound No. I-166: 76%
Compound No. I-167: ≥95%
Compound No. I-169: 75%
Compound No. I-170: 80%
Compound No. I-174: 81%

Compound No. I-175: 88%
Compound No. I-176: 83%
Compound No. I-182: ≥95%
Compound No. I-188: 84%
Compound No. I-191: 93%
Compound No. I-193: 81%
Compound No. I-195: 82%
Compound No. I-197: 90%
Compound No. I-198: 94%
Compound No. I-203: ≥95%
Compound No. I-205: 90%
Compound No. I-210: 93%
Compound No. I-211: 85%
Compound No. I-212: ≥95%.

(Example 4: Measurement of PI3Kγ inhibitory activity (Ki value))

The PI3Kγ inhibitory activity (Ki value) of a compound was evaluated according to the following procedure:
5 μL of a compound solution containing 10% DMSO and 200 μmol/L of a compound was changed to 5 μL of a compound solution containing 10% DMSO and either 200, 64, 20, 6.4, 2, 0.64, or 0.20 μmol/L of the compound (optionally, this is diluted to a lower concentration). Using a method similar to the method for measuring PI3Kγ inhibitory activity, inhibition ratios of PI3Kγ were measured in the presence of the compound at 50, 16, 5, 1.6, 0.5, 0.16, and 0.05 μmol/L (optionally, a lower concentration). Then an $IC_{50}$ value was calculated by a logistic approximation method or the linear regression method using two concentrations that cross 50% inhibition. Separately, the ATP concentration in the reaction solution (40 μmol/L ATP, 10 mmol/L $MgCl_2$, 5 mmol/L DTT) at the time of the start of a reaction in the absence of the compound was changed to 80, 40, 20, 10, 5, 2.5, 1.25, or 0.625 μmol/L. Then, HTRF ratios were measured by a similar method. The value obtained by subtracting the HTRF ratio at each ATP concentration from the HTRF ratio in the absence of PI-3 kinase γ was defined as the value obtained by multiplying reaction rate (v) at each ATP concentration with a constant. The Michaelis-Menten constant Km was then calculated by the Lineweaver-Burk plot method. The Ki value of a compound was calculated by the following formula wherein "μM" denotes "μmol/L."
$$\text{Ki value} = IC_{50}\ \text{value}/(1 + \text{test ATP concentration}\ (\mu M)/\text{Km}\ (\mu M))$$

(Results)

Compound No. I-12: 0.0096 μmol/L
Compound No. I-14: 0.054 μmol/L
Compound No. I-28: 0.047 μmol/L
Compound No. I-91: 0.0014 μmol/L
Compound No. I-131: 0.0008 μmol/L
Compound No. I-148: 0.0055 μmol/L
Compound No. I-174: 0.0036 μmol/L
Compound No. I-183: 0.09 μmol/L
Compound No. I-197: 0.0023 μmol/L
Compound No. I-83: 0.11 μmol/L
Compound No. I-112: 1.1 μmol/L
Compound No. I-113: 2.3 μmol/L
Compound No. I-114: 0.033 μmol/L
Compound No. I-123: 0.025 μmol/L
Compound No. I-125: 0.013 μmol/L
Compound No. I-126: 0.061 μmol/L
Compound No. I-127: 0.016 μmol/L

Compound No. I-128: 0.12 μmol/L
Compound No. I-146: 0.047 μmol/L
Compound No. I-191: 0.3 μmol/L
Compound No. I-192: 0.48 μmol/L
Compound No. I-213: 0.058 μmol/L
Compound No. I-216: 0.23 μmol/L
Compound No. I-225: 0.015 μmol/L
Compound No. I-226: 0.83 μmol/L
Compound No. I-221: 0.68 μmol/L
Compound No. I-229: 0.0094 μmol/L

(Example 5: Measurement of PI3Kα inhibitory activity)

The PI3Kα inhibitory activity of a compound was evaluated according to the following procedure.
According to Example 2 above, after 80 μg/mL PI-3 kinase γ of the enzyme solution (80 μg/mL PI-3 kinase γ, 10 mmol/L $MgCl_2$, 5 mmol/L DTT) was changed to 0.8 μg/mL PI-3 kinase α, an inhibition ratio was calculated by a method similar to the method for measuring PI3Kγ inhibitory activity, and then defined as the PI3Kα inhibitory activity.

(Example 6: Measurement of PI3Kα inhibitory activity (Ki value))

The α inhibitory activity (Ki value) of a compound was evaluated according to the following procedure.
According to Example 4 above, a Km value for PI3Kα was determined to calculate a Ki value.
For example, the Ki value of Compound No. I-12 is >12 μmol/L, the Ki value of Compound No. I-83 is >13 μmol/L, the Ki value of Compound No. I-112 is 7.5 μmol/L, the Ki value of Compound No. I-114 is 1.1 μmol/L, and the Ki value of Compound No. I-127 is >7.0 μmol/L.

(Example 7: Measurement of PI3Kβ inhibitory activity)

The PI3Kβ inhibitory activity of a compound was evaluated according to the following procedure.
According to Example 2 above, after 80 μg/mL PI-3 kinase γ of the enzyme solution (80 μg/mL PI-3 kinase γ, 10 mmol/L $MgCl_2$, 5 mmol/L DTT) was changed to 60 μg/mL PI-3 kinase β, a method similar to the method for measuring PI3Kγ inhibitory activity was used to calculate an inhibition ratio, which was defined as the PI3Kβ inhibitory activity.

(Example 8: Measurement of PI3Kβ inhibitory activity (Ki value))

According to Example 4 above, a Km value for PI3Kβ was determined to calculate a Ki value.
For example, the Ki value of Compound No. I-12 is 0.34 μmol/L, the Ki value of Compound No. I-83 is >12 μmol/L, the Ki value of Compound No. I-112 is >11 μmol/L, the Ki value of Compound No. I-114 is 0.66 μmol/L, and the Ki value of Compound No. I-127 is 0.34 μmol/L.

(Example 9: Measurement of P13Kδ inhibitory activity)

The PI3Kδ inhibitory activity of a compound was evaluated according to the following procedure.
According to Example 2 above, after 80 μg/mL PI-3 kinase γ of the enzyme solution (80 μg/mL PI-3 kinase γ, 10 mmol/L $MgCl_2$, 5 mmol/L DTT) was changed to 158 μg/mL PI-3 kinase δ, a method similar to the method for measuring PI3Kγ inhibitory activity was used to calculate an inhibition ratio, which was defined as the P13Kδ inhibitory activity.

(Example 10: Measurement of PI3Kδ inhibitory activity (Ki value))

According to Example 4 above, a Km value for PI3Kδ was determined to calculate a Ki value.
For example, the Ki value of Compound No. I-12 is 0.59 μmol/L, the Ki value of Compound No. I-83 is >12 μmol/L, the Ki value of Compound No. I-112 is 2.1 μmol/L, the Ki value of Compound No. I-114 is 2.0 μmol/L, and the Ki value of Compound No. I-127 is 0.081 μmol/L.
In addition, Ki values of PI3Kγ, α, β, and δ are shown in Table 3-1 and Table 3-2. In the tables, "+," "++," "+++," "++++," and "+++++" refer to the following, respectively.
"+": Ki value >3 μmol/L
"++": Ki value ≤3 μmol/L
"+++": Ki value ≤0.3 μmol/L

"++++": Ki value ≤0.03 μmol/L
"+++++": Ki value ≤0.003 μmol/L

[Table 3-1]

| Compound No. | PI3K γ Ki | PI3K α Ki | PI3K β Ki | PI3K δ Ki |
|---|---|---|---|---|
| I-12 | ++++ | + | ++ | ++ |
| I-14 | +++ | + | + | + |
| I-28 | +++ | + | ++ | ++ |
| I-91 | +++++ | + | ++ | ++ |
| I-131 | +++++ | + | + | + |
| I-148 | ++++ | ++ | +++ | +++ |
| I-174 | ++++ | + | ++ | ++ |
| I-483 | +++ | + | + | + |
| I-197 | +++++ | + | + | + |
| I-83 | +++ | + | + | + |
| I-112 | ++ | + | + | ++ |
| I-113 | ++ | + | + | ++ |
| I-114 | +++ | ++ | ++ | ++ |
| I-123 | ++++ | + | ++ | +++ |
| I-425 | ++++ | ++ | +++ | +++ |
| I-126 | +++ | + | ++ | ++ |
| I-127 | ++++ | + | ++ | +++ |
| I-128 | +++ | + | ++ | + |

[Table 3-2]

| Compound No. | PI3K γ Ki | PI3K α Ki | PI3K β Ki | PI3K δ Ki |
|---|---|---|---|---|
| I-213 | +++ | + | + | + |
| I-216 | +++ | + | ++ | + |
| I-225 | ++++ | + | +++ | + |
| I-226 | ++ | + | + | ++ |
| I-229 | ++++ | + | + | + |

(Example 11: Method for calculating the selectivity of PI3Kγ and PI3Kα)

The PI3Kγ/α selectivity of a compound was expressed by the value obtained by dividing the Ki value for PI3Kα by the Ki value for PI3Kγ.

(Example 12: Method for calculating the selectivity of PI3Kγ and PI3Kβ)

The P13Kγ/β selectivity of a compound was expressed by the value obtained by dividing the Ki value for PI3Kβ by the Ki value for PI3Kγ.

(Example 13: Solubility test)

(Method)

The solubility of a compound was determined under a condition in which 1% DMSO was added. 10 mmol/L compound solution was prepared using DMSO, and then 6 μL of the compound solution was added to 594 μL of artificial intestinal juice in pH 6.8 (to 250 mL of 0.2 mol/L potassium dihydrogen phosphate reagent solution was added 118 mL of 0.2 mol/L NaOH reagent solution and water to provide a final volume of 1000 mL). After standing at 25˚C for 16 hours, the mixed solution was filtered with suction. The filtrate was diluted twice with methanol/water (1/1), and then a concentration in the filtration was measured with HPLC or LC/MS/MS by the absolute calibration method.

(Results)

Results are shown in Table 4 below. In the table, "μM" denotes "μmol/L."

[Table 4]

| Compound No. | Solubility (μM) |
|---|---|
| I-4 | 12.3 |
| I-10 | >50.0 |
| I-13 | 15.4 |
| I-19 | >50.0 |
| I-31 | 32.3 |
| I-76 | 26.3 |
| I-84 | >50.0 |
| I-100 | 34.4 |
| I-103 | 30.1 |
| I-104 | 39.6 |
| I-107 | 42.6 |
| I-120 | 17.5 |
| I-137 | >50.0 |
| I-149 | 36.1 |
| I-158 | 24.9 |
| I-178 | 10 |
| I-179 | >50.0 |
| I-182 | 19.6 |
| I-183 | 28.1 |
| I-189 | 10.3 |
| I-191 | >50.0 |
| I-195 | >50.0 |
| I-198 | 10.1 |
| I-202 | >50.0 |
| I-212 | 14 |
| I-227 | 36.8 |

(Example 14: Metabolic stability test)

(Method)

After a subject compound was reacted for a certain time using a pooled human liver microsome commercially available, a reacted sample and an unreacted sample are compared to calculate a survival ratio, and then the degree of metabolism in the liver was evaluated.

0.2 mL of a buffer solution (50 mmol/L of Tris-HCl in pH 7.4, 150 mmol/L of potassium chloride, and 10 mmol/L of magnesium chloride) containing a human liver microsome of 0.5 mg protein/mL was reacted in the presence of 1 mmol/L NADPH at 37˚C for 0 or 30 minutes (oxidative reaction). After reacting, 50 μL of the reaction solution was added to 100 μL of a solution of methanol/acetonitrile (1/1 (v/v)), mixed, and then centrifuged at 3000 rpm for 15 minutes. The test compound in the supernatant was quantitated with LC/MS/MS. The amount of the compound at a reaction time of 0 minute was defined as 100% and, based on that, the percentage of the test compound remained after reacting for 30 minutes was calculated.

(Results)

Results when the concentration of a compound is 0.5 μmol/L or 0.2 μmol/L are shown in Table 5 below. In the table, "μM" denotes "μmol/L."

[Table 5]

| Compound No. | HLMS_ox(at 0.5 μM) % | Compound No. | hLMS_ox( at 0.5 μM) % |
|---|---|---|---|
| I-19 | 87.2 | I-134 | 88.4 |
| I-35 | 101 | I-135 | 85.8 |
| I-56 | 95.5 | I-137 | 94.5 |
| I-57 | 83.5 | I-138 | 82.6 |
| I-60 | 81.9 | I-139 | 84.8 |
| I-63 | 80.1 | I-148 | 87.9 |
| I-74 | 83.3 | I-152 | 96.8 |
| I-80 | 82. | I-153 | 83 |
| I-83 | 89.1 | I-154 | 98.7 |
| I-85 | 90.2 | I-155 | 95.9 |
| I-87 | 91.9 | I-157 | 96.2 |
| I-88 | 88.2 | I-158 | 99 |
| I-89 | 93.6 | I-159 | 85.6 |
| I-90 | 83.4 | I-163 | 90.5 |
| I-99 | 90.6 | I-168 | 104 |
| I-100 | 89.3 | I-169 | 94.4 |
| I-101 | 91.9 | I-170 | 91.6 |
| I-102 | 84.2 | I-172 | 98 |
| I-103 | 85.8 | I-178 | 97.9 |
| I-104 | 103 | I-180 | 92.6 |
| I-106 | 83.9 | I-181 | 85.9 |
| I-107 | 98 | I-182 | 88.1 |
| I-115 | 84.7 | I-183 | 109 |
| I-118 | 89.8 | I-184 | 98.1 |

(continued)

| Compound No. | HLMS_ox(at 0.5 μM) % | Compound No. | hLMS_ox( at 0.5 μM) % |
|---|---|---|---|
| I-119 | 90.8 | I-185 | 87.6 |
| I-120 | 92.3 | I-193 | 91.2 |
| I-122 | 90.6 | I-195 | 110 |
| I-125 | 103 | I-202 | 104 |
| I-127 | 92.6 | I-203 | 88.5 |
| I-130 | 104 | I-207 | 93.5 |
| I-133 | 106 | I-226 | 82.5 |
| | | | |
| Compound No. | hLMS_ox(at 2 μM) % | | |
| I-3 | 82 | | |
| I-4 | 103 | | |

(Example 15: CYP inhibition test) (Method)

A commercially available pooled human liver microsome was used in evaluating the degree of produced-metabolite inhibition exhibited by a test compound. O-de-ethylation of 7-ethoxyresorufin (CYP1A2), methyl-hydroxylation of tolbutamide (CYP2C9), 4'-hydroxylation of mephenytoin (CYP2C19), O-demethylation of dextromethorphan (CYP2D6), and hydroxylation of terfenadine (CYP3A4), which are typical substrate for metabolic reactions of human main CYP5 molecular species (CYP1A2, 2C9, 2C19, 2D6, and 3A4), were adopted as indexes.

The reaction conditions are as follows: substrate: 0.5 μmol/L of ethoxyresorufin (CYP1A2), 100 μmol/L of tolbutamide (CYP2C9), 50 μmol/L of S-mephenytoin (CYP2C19), 5 μmol/L of dextromethorphan (CYP2D6), and 1 μmol/L of terfenadine (CYP3A4); reaction time; 15 minutes; reaction temperature: 37˚C; enzyme: pooled human liver microsome 0.2 mg protein/mL; test drug concentration: 1, 5, 10, and 20 μmol/L (4 points).

In a 96-well plate, as a reaction solution, five kinds of substrates, a human liver microsome, and a test drug were added to 50 mmol/L Hepes buffer solution in the aforementioned ratio. A coenzyme NADPH was added to start a metabolic reaction, which is an index. After reacting at 37˚C for 15 minutes, a solution of methanol/acetonitrile (1/1 (v/v)) was added to quench the reaction. After centrifugation at 3000 rpm for 15 minutes, resorufin (CYP1A2 metabolite) in the supernatant was quantitated with a fluorescence multilabel counter, and hydroxylated tolbutamide (CYP2C9 metabolite), 4'-hydroxylated mephenytoin (CYP2C19 metabolite), dextromethorphan (CYP2D6 metabolite), terfenadine in alcohol form (CYP3A4 metabolite) were quantitated with LC/MS/MS.

The case wherein only DMSO (a solvent which dissolved a drug) was added to a reaction system was defined as a control (100%). When a test drug solution was added, the remaining activity (%) at each concentration was calculated, and then $IC_{50}$ was calculated with a concentration and an inhibition ratio by an inverse estimation using a logistic model.

(Results)

$IC_{50}$ for five kinds of substances are shown in Table 6 below. The unit of concentration in the table is "μmol/L."

[Table 6]

| Compound No. | 5CYPs_IC50 | Compound No. | 5CYPs_IC50 |
|---|---|---|---|
| I-5 | ≧ 10 | I-117 | >20 |
| I-9 | ≧ 10 | I-131 | ≧ 10 |
| I-10 | >20 | I-149 | >20 |
| I-19 | >20 | I-170 | >20 |
| I-31 | ≧ 10 | I-172 | ≧ 10 |
| I-50 | ≧ 10 | I-173 | ≧ 10 |

(continued)

| Compound No. | 5CYPs_IC50 | Compound No. | 5CYPs_IC50 |
|---|---|---|---|
| I-60 | >20 | I-175 | ≧ 10 |
| I-63 | >20 | I-176 | ≧ 10 |
| I-64 | ≧ 10 | I-188 | >20 |
| I-81 | >20 | I-191 | ≧ 10 |
| I-84 | >20 | I-198 | >20 |
| I-86 | ≧ 10 | I-202 | ≧ 10 |
| I-88 | ≧ 10 | I-203 | >20 |
| I-89 | ≧ 10 | I-204 | >20 |
| I-90 | ≧ 10 | I-208 | >20 |
| I-91 | ≧ 10 | I-209 | ≧ 10 |
| I-105 | ≧ 10 | I-211 | ≧ 10 |
| I-106 | ≧ 10 | I-212 | ≧ 10 |
| I-107 | ≧ 10 | I-224 | ≧ 10 |
| I-108 | >20 | I-219 | ≧ 10 |
| I-114 | >20 | I-226 | ≧ 10 |
| I-115 | ≧ 10 | | |

(Example 16: CYP3A4 fluorescence MBI test)

(Method)

The CYP3A4 fluorescence MBI test is a test to examine the enhancement of CYP3A4 inhibition of a compound by a metabolic reaction. The test was performed using as an index, a reaction to produce a metabolite 7-hydroxytrifluoromethylcoumarin (HFC) which emits fluorescence, in which CYP3A4 expressed in *E. coli* was used as an enzyme and 7-benzyloxytrifluoromethylcoumarin (7-BFC) is debenzylated by CYP3A4 enzyme.

The reaction conditions are as follows: substrate: 5.6 μmol/L 7-BFC; pre-reaction time; 0 or 30 minutes; reaction time: 15 minutes; reaction temperature: 25°C (room temperature); content of CYP3A4 (an enzyme expressed in *E. coli*): 62.5 pmol/mL at the time of a pre-reaction and 6.25 pmol/mL (when diluted 10 times) at the time of a reaction; the concentrations of a test drug: 0.625, 1.25, 2.5, 5, 10, and 20 μmol/L (6 points).

In a 96-well plate, an enzyme solution and a test drug solution were added as a pre-reaction solution into K-Pi buffer solution (pH 7.4) in the aforementioned ratio. A portion thereof was transferred to another 96-well plate to be diluted ten times with a substrate and a K-Pi buffer solution. A coenzyme, NADPH, was then added to start a reaction, which is the index (this is without a pre-reaction). After reacting for a predetermined time, 4:1 of acetonitrile:0.5 mol/L Tris (trishydroxyaminomethane) was added to quench the reaction. To the remaining pre-reaction solution, NADPH was added to start a pre-reaction (this is with a pre-reaction). After prereacting for a predetermined time, a portion thereof was transferred to another plate to be diluted ten times with a substrate and K-Pi buffer solution, and thereby the index reaction is started. After reacting for a predetermined time, 4:1 of acetonitrile:0.5 mol/L Tris (trishydroxyaminomethane) was added to quench the reaction. For each plate in which the index reaction was carried out, the fluorescence value of a metabolite 7-HFC was measured with a fluorescent plate reader (Ex = 420 nm, Em = 535 nm).

The case wherein only DMSO (a solvent which dissolved a drug) was added to a reaction system was defined as a control (100%). When a test drug solution was added, the remaining activity (%) at each concentration was calculated, and then $IC_{50}$ was calculated with a concentration and an inhibition ratio by an inverse estimation using a logistic model. When the difference in $IC_{50}$ value is 5 μmol/L or more, the compound is shown as (+). When 3 μmol/L or less, the compound is shown as (-).

(Results)

Results are shown in Table 7 below.

[Table 7]

| Compound No. | MBI | Compound No. | MBI |
|---|---|---|---|
| I-5 | (-) | I-89 | (-) |
| I-9 | (-) | I-90 | (-) |
| I-10 | (-) | I-91 | (-) |
| I-12 | (-) | I-93 | (-) |
| I-14 | (-) | I-94 | (-) |
| I-20 | (-) | I-97 | (-) |
| I-22 | (-) | I-98 | (-) |
| I-23 | (-) | I-108 | (-) |
| I-24 | (-) | I-111 | (-) |
| I-27 | (-) | I-114 | (-) |
| I-29 | (-) | I-129 | (-) |
| I-33 | (-) | I-131 | (-) |
| I-34 | (-) | I-139 | (-) |
| I-35 | (-) | I-153 | (-) |
| I-39 | (-) | I-163 | (-) |
| I-40 | (-) | I-168 | (-) |
| I-49 | (-) | I-172 | (-) |
| I-52 | (-) | I-173 | (-) |
| I-53 | (-) | I-176 | (-) |
| I-56 | (-) | I-180 | (-) |
| I-57 | (-) | I-185 | (-) |
| I-58 | (-) | I-186 | (-) |
| I-559 | (-) | I-189 | (-) |
| I-62 | (-) | I-195 | (-) |
| I-65 | (-) | I-200 | (-) |
| I-74 | (-) | I-204 | (-) |
| I-75 | (-) | I-209 | (-) |
| I-79 | (-) | I-210 | (-) |
| I-81 | (-) | I-211 | (-) |
| I-86 | (-) | I-225 | (-) |
| I-88 | (-) | I-226 | (-) |

(Example 17: FAT test)

(Method)

20 µL of *Salmonella enterica* subsp. *typhimurium* (*Salmonella typhimurium* TA98 line, TA100 line) cryopreserved was inoculated to 10 mL of liquid nutrient medium (2.5% Oxoid nutrient broth No. 2) and then precultured at 37˚C for 10 hours with shaking. Regarding TA98 line, after 9 mL of a bacterial suspension was centrifuged (2000 x g, 10 minutes) to remove the culture solution, the bacteria was suspended in 9 mL of Micro F buffer solution ($K_2HPO_4$: 3.5

g/L, $KH_2PO_4$: 1 g/L, $(NH_4)_2SO_4$: 1 g/L, tri-sodium citric acid dihydrate: 0.25 g/L, $MgSO_4 \cdot 7H_2O$: 0.1 g/L), and then added to 110 mL of Exposure media (Micro F buffer solution containing biotin: 8 μg/mL, histidine: 0.2 μg/mL, glucose: 8 mg/mL). Regarding TA 100 line, 120 mL of Exposure media was added to 3.16 mL of the bacterial suspension to prepare a test bacterial suspension. 12 μL of a solution of a test substance in DMSO (diluted eight times in a common ratio of 2 from the maximum dose of 50 mg/mL); 12 μL of DMSO as a negative control; 50 μg/mL 4-nitroquinoline-1-oxide solution in DMSO for TA98 line or 0.25 μg/mL 2-(2-furyl)-3-(5-nitro-2-furyl)acrylamide solution in DMSO as a positive control for TA 100 line in the case of a non-metabolism-activation condition; 12 μL of 40 μg/mL 2-aminoanthracene solution in DMSO for TA98 line or 20 μg/mL of 2-aminoanthracene solution in DMSO for TA100 line in the case of a metabolism-activation condition; were each mixed with 588 μL of the test bacterial suspension (in the case of a metabolism activation condition, a mixed solution of 498 μL of the test bacterial suspension and 90 μL S9 mix) and then cultured at 37°C at 90 minutes with shaking. 460 μL of the bacterial suspension to which a test substance was exposed was mixed with 2300 μL of Indicator media (a Micro F buffer solution containing 8 μg/mL biotin, 0.2 μg/mL histidine, 8 mg/mL glucose, and 37.5 μg/mL bromocresol purple). 50 μL thereof was dispensed to microplate 48 wells/dose and then statically cultured at 37°C for 3 days. In a well containing bacteria which obtained proliferation potency by mutation of the amino acid (histidine) synthetase gene, the change in pH caused the color change from purple to yellow. Thus, in 48 wells per dose, the number of the bacteria-proliferation wells in which the color changed to yellow was counted, compared with a group of negative controls, and then evaluated. When the mutagenicity is negative, the compound is shown as (-). When positive, the compound is shown as (+).

(Results)

Results are shown in Table 8 below.

[Table 8]

| Compound No. | xFAT | Compound No. | FAT |
|---|---|---|---|
| I-8 | (-) | I-128 | (-) |
| I-10 | (-) | I-129 | (-) |
| I-12 | (-) | I-131 | (-) |
| I-27 | (-) | I-134 | (-) |
| I-29 | (-) | I-135 | (-) |
| I-32 | (-) | I-131 | (-) |
| I-41 | (-) | I-138 | (-) |
| I-45 | (-) | I-139 | (-) |
| I-48 | (-) | I-148 | (-) |
| I-52 | (-) | I-152 | (-) |
| I-53 | (-) | I-155 | (-) |
| I-55 | (-) | I-156 | (-) |
| I-57 | (-) | I-157 | (-) |
| I-80 | (-) | I-159 | (-) |
| I-83 | (-) | 1-160 | (-) |
| I-88 | (-) | I-161 | (-) |
| I-92 | (-) | I-169 | (-) |
| I-93 | (-) | I-175 | (-) |
| I-94 | (-) | I-176 | (-) |
| I-95 | (-) | I-182 | (-) |
| I-101 | (-) | I-191 | (-) |
| I-102 | (-) | I-193 | (-) |

(continued)

| Compound No. | xFAT | Compound No. | FAT |
|---|---|---|---|
| I-106 | (-) | I-195 | (-) |
| I-108 | (-) | I-197 | (-) |
| I-122 | (-) | I-198 | (-) |
| I-127 | (-) | I-203 | (-) |

(Example 18: hERG test)

(Method)

For the purpose of risk evaluation of QT interval extension of electrocardiogram, the activity on delayed rectification $K^+$ current ($I_{Kr}$), which plays an important role in a cardiac ventricle repolarization process, was examined using the HEK293 cell that was made to express human ether-a-go-go related gene (hERG) channel.

Using an automatic patch-clamp system (PatchXpress 7000A, Axon Instruments Inc.), by a whole-cell patch-clamp method, after the cell was maintained at a membrane potential of -80 mV, $I_{Kr}$ induced upon applying depolarizing stimulation of +50 mV for 2 seconds and further applying repolarizing stimulation of -50 mV for 2 seconds was recorded. After generated electric current became stable, a test substance dissolved at an intended concentration in extracellular fluid (137 mmol/L NaCl, 4 mmol/L KCl, 1.8 mmol/L $CaCl_2 \cdot 2H_2O$, 1 mmol/L $MgCl_2 \cdot 6H_2O$, 10 mmol/L glucose, 10 mmol/L HEPES (4-(2-hydroxyethyl)-1-piperazineethanesulfonic acid), pH = 7.4) was applied to the cell for 10 minutes under room temperature condition. From the obtained $I_{Kr}$, the absolute value of the maximum tail current was measured using analysis software (DataXpress ver. 1, Molecular Devices Corporation) and the value of the electric current in a maintained membrane potential as baseline. Moreover, an inhibition ratio for the maximum tail current prior to the application of the test substance was calculated and then compared with a group of media-application (0.1% dimethylsulfoxide solution) to evaluate influence of the test substance on $I_{Kr}$.

(Results)

Results when the compound concentration is 1 μmol/L or 10 μmol/L are shown in Table 9 below. In the table, "μM" denotes "μmol/L."

[Table 9]

| Compound No. | hERG(at 1 μM) % Inhibition | Compound No. | hERG(at 1 μM)% Inhibition |
|---|---|---|---|
| I-16 | 9.3 | I-128 | 1 |
| I-27 | 2 | I-129 | 5.5 |
| I-29 | 8.7 | I-131 | 3.9 |
| I-41 | 5.6 | I-134 | 1.7 |
| I-45 | 7.3 | I-135 | 1.8 |
| I-48 | 3.2 | I-138 | 2.8 |
| I-53 | 5.1 | I-139 | 8.5 |
| I-55 | 7.7 | I-148 | 3.3 |
| I-57 | 5.3 | I-152 | 4.7 |
| I-60 | 3.6 | I-155 | 2.9 |
| I-63 | 2.7 | I-156 | 2.7 |
| I-80 | 5.5 | I-157 | 0.4 |
| I-83 | 3.3 | I-158 | 9.2 |
| I-88 | 5.7 | I-159 | 3.8 |
| I-91 | 0.9 | I-160 | 3 |

(continued)

| Compound No. | hERG(at 1 μM) % Inhibition | Compound No. | hERG(at 1 μM)% Inhibition |
|---|---|---|---|
| I-92 | 2.4 | I-161 | 6.1 |
| I-93 | 6.8 | I-166 | 1.8 |
| I-94 | 6.8 | I-169 | 2.7 |
| I-95 | 3.7 | I-175 | 1.6 |
| I-101 | 9.6 | I-176 | 0.4 |
| I-102 | 8.8 | I-182 | 4.9 |
| I-103 | 3.6 | I-188 | 3.9 |
| I-107 | 5.4 | I-197 | 4.8 |
| I-111 | 2.4 | I-198 | 5.9 |
| I-125 | 1.3 | I-203 | 6.7 |
| I-127 | 0.6 | I-205 | 0.2 |
| | | | |
| Compound No. | hERG(at 10 μM) % Inhibition | | |
| I-19 | 9.5 | | |

(Example 19 BA Test)

An experimental material and a method for examining oral absorbability

(1) Animals used: rats or mice were used.
(2) Breeding condition: chow and sterilized tap water were allowed to be taken in freely.
(3) Setting of a dosage and grouping: a predetermined dosage was administered orally or intravenously. Groups were formed as shown below. (A dosage varied depending on each compound)

Oral administration 1-30 mg/kg (n = 2 to 3)

Intravenous administration 0.5-10 mg/kg (n = 2 to 3)
(4) Preparation of administered liquid: In oral administration, a solution or suspension was administered. In intravenous administration, after solubilization, the administration was performed.
(5) Method of Administration: In oral administration, compulsory administration to the stomach was conducted using an oral probe.
In intravenous administration, administration from the caudal vein was conducted using a syringe with an injection needle.
(6) Evaluation item: Blood was chronologically collected, and then the drug concentration in blood plasma was measured using a LC/MS/MS.
(7) Statistical analysis: With regard to a shift in plasma concentration, the plasma concentration-time area under the curve (AUC) was calculated using a nonlinear least-squares program WinNonlin®. Bioavailability (BA) was calculated from the AUCs of the oral administration group and the intravenous administration group, respectively.

Results are shown in Table 10 below.

[Table 10]

| Compound No. | BA (Rat, 1 mg/kg, Methylcellulose suspension Oral Administration) |
|---|---|
| I-12 | 46.8% |
| I-52 | 40.9% |
| I-148 | 45.1% |

(Discussion)

As described above, the compound of the present invention exhibits excellent PI3-kinaseγ inhibitory activity *in vitro* and *in vino.* Accordingly, the pharmaceutical composition of the present invention may be used for the prophylaxis of and/or as a therapeutic agent for diseases such as encephalitis, myelitis and encephalomyelitis, meningitis, inflammatory polyneuropathy, neuritis, dacryoadenitis, orbital inflammation, conjunctivitis (allergic conjunctivitis, vernal keratoconjunctivitis, and the like), keratitis, chorioretinitis scar, endophthalmitis, retrobulbar neuritis, retinopathy, glaucoma, phlegmon, external otitis, perichondritis, tympanitis, eustachitis, mastoiditis, myringitis, labyrinthitis, pulpitis, periodontitis, sialadenitis, stomatitis, glossitis, thyroiditis, pericarditis, endocarditis, myocarditis, hypertension, heart failure, arteriosclerosis (atherosclerosis and the like), restenosis, ischemia-reperfusion injury, thrombosis (myocardial infarction, cerebral infarction, and the like), obesity, angiitis, vasculitis, polyarteritis, lymphadenitis, lymphoma, Hodgkin disease, eosinophilic diseases (eosinophilia, pulmonary eosinophilia, pulmonary aspergillosis, and the like), inflammatory or obstructive airway diseases (allergic rhinitis, chronic sinusitis, pneumonia, laryngitis, laryngotracheitis, bronchitis, asthma, acute lung disorder, acute respiratory distress syndrome, pulmonary emphysema, chronic obstructive pulmonary diseases, and the like), pleurisy, pneumoconiosis, mesothelioma, esophagitis, gastro-jejunal ulcer, gastritis, duodenitis, food allergy, sepsis, hepatitis, hepatic fibrosis, cirrhosis, cholecystitis, pancreatitis, peritonitis, diabetes (type I diabetes, type II diabetes), inflammatory or allergic skin diseases (atopic dermatitis, contact dermatitis (allergic contact dermatitis, irritant contact dermatitis, and the like), psoriasis, urticaria, photoallergic reaction, alopecia areata, and the like), skin-thickening disorder (cutaneous eosinophilic granuloma and the like), cutaneous polymyositis, panniculitis, hyperthyroidism, sarcoidosis, autoimmune blood diseases (hemolytic anemia, idiopathic thrombocytopenic purpura, and the like), (systemic) lupus erythematosus, relapsing polychondritis, polychondritis, sclerodoma, Wegener granulomatosis, dermatomyositis, chronic active hepatitis, myasthenia gravis, Stevens-Johnson syndrome, idiopathic sprue, autoimmune inflammatory bowel diseases (ulcerative colitis, Crohn disease, and the like), endocrine eye diseases, alveolitis, chronic hypersensitivity pneumonitis, multiple sclerosis, primary biliary cirrhosis, uveitis, keratoconjunctivitis sicca, interstitial pulmonary fibrosis, iridocyclitis, psoriatic arthritis, glomerulonephritis, systemic sclerosis, systemic connective tissue diseases (Sjoegren syndrome, Behcet disease, diffuse fasciitis, and the like), interstitial myositis, inflammatory polyarthropathy, inflammatory arthritis, articular rheumatism, osteoarthritis, synovitis, bursitis, tendovaginitis, chronic multifocal osteomyelitis, nephritic syndrome, tubulointerstitial nephritis, cystitis, prostatitis, orchitis, epididymitis, salpingitis, oophoritis, trachelitis, female pelvic inflammation, vulvovaginitis, organ transplantation rejection, bone marrow transplantation rejection, graft-versus-host diseases, and the like; or used as a therapeutic agent for burn or traumatic inflammation.

(Example 17: Formulation example 1 Tablet)

A tablet consisting of the following composition is produced by a conventional method.

| | |
|---|---|
| The compound of the present invention | 100 mg |
| Lactose | 60 mg |
| Potato starch | 30 mg |
| Polyvinyl alcohol | 2 mg |
| Magnesium stearate | 1 mg |
| Tar dye | minute amount |

(Example 18: Formulation example 2 Powder)

A powder consisting of the following composition is produced by a conventional method.

| | |
|---|---|
| The compound of the present invention | 150 mg |
| Lactose | 280 mg |

(Example 19: Formulation example 3 Syrup)

Syrup consisting of the following composition is produced by a conventional method.

| | |
|---|---|
| The compound of the present invention | 100 mg |
| Refined white sugar | 40 g |
| Ethyl *p*-hydroxybenzoate | 40 mg |

(continued)

| | |
|---|---|
| Propyl *p*-hydroxybenzoate | 10 mg |
| Chocolate flavor | 0.1 cc |
| Water was added to this to provide a total amount of 100 cc. | |

The present invention has been exemplified so far by referring to preferable embodiments of the present invention, but it should not be construed that the present invention is restricted by the embodiments of the present invention. It should be understood that the scope of the present invention should be construed only by the claims. It would be understood that those skilled in the art can perform an invention practically equivalent to the present invention, based on the description of the present invention and technical common sense from the specific description of preferable embodiments of the present invention. It would be understood that the patents, patent applications and literature cited herein should be incorporated herein by reference to the present specification in their entire contents, similarly to the case where the contents themselves are described specifically herein.

The present application claims priority to Japanese Patent Application Nos. 2009-108526, 2009-235769, and 2010-043072, which were filed in Japan, and which are herein incorporated by reference in their entirety.

INDUSTRIAL APPLICABILITY

The present invention provides medicaments for the treatment of phosphatidylinositol-3-kinase dependent diseases, a compound used therefor, a pharmaceutically acceptable salt thereof, or a solvate (in particular, a hydrate) or prodrug thereof. Compounds of the present invention exhibit excellent PI3-kinaseγ inhibitory activity, as described in the Examples above.

**Claims**

**1.** A compound represented by formula (I):

[Formula 1]

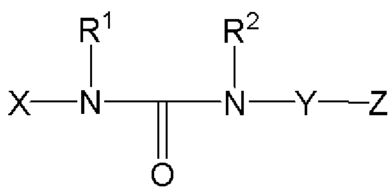

(I)

wherein

$R^1$ and $R^2$ are each independently hydrogen or substituted or unsubstituted alkyl;
X is a group represented by a formula selected from the following:

[Formula 2]

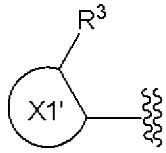

(X1)

or

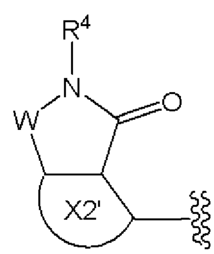

(X2)

;

X1' is a substituted or unsubstituted 6-membered aromatic hydrocarbon ring, a substituted or unsubstituted 5-

or 6-membered aromatic heterocycle, a substituted or unsubstituted 3- to 7-membered non-aromatic hydrocarbon ring, or a substituted or unsubstituted 3- to 7-membered non-aromatic heterocycle;
X2' is a substituted or unsubstituted monocyclic aromatic hydrocarbon ring, a substituted or unsubstituted monocyclic aromatic heterocycle, a substituted or unsubstituted monocyclic non-aromatic hydrocarbon ring, or a substituted or unsubstituted monocyclic non-aromatic heterocycle;
$R^3$ is carboxy, substituted or unsubstituted alkyl, substituted or unsubstituted alkenyl, substituted or unsubstituted alkynyl, substituted or unsubstituted aryl, substituted or unsubstituted cycloalkyl, substituted or unsubstituted cycloalkenyl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, a group represented by the formula: $-C(-R^a)=N-OH$, a group represented by the formula: $-SO-R^a$, a group represented by the formula: $-SO_2-R^a$, or a group represented by the formula :$-SR^a$;
$R^a$ is hydrogen, substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;
$R^4$ is hydrogen, cyano, substituted or unsubstituted alkyl, substituted or unsubstituted aryl, substituted or unsubstituted cycloalkyl, substituted or unsubstituted cycloalkenyl, substituted or unsubstituted heteroaryl, or substituted or unsubstituted heterocyclyl;
W is a group represented by the formula: $-(CR^5R^6)_a-$ or a group represented by the formula: $-(CR^7=CR^8)-$;
$R^5$ to $R^8$ are each independently hydrogen, halogen, cyano, hydroxy, carboxy, substituted or unsubstituted alkyl, substituted or unsubstituted alkoxy, substituted or unsubstituted acyl, substituted or unsubstituted carbamoyl, or substituted or unsubstituted amino;
a is 1 or 2;
Y is a group represented by a formula selected from the following:

[Formula 3]

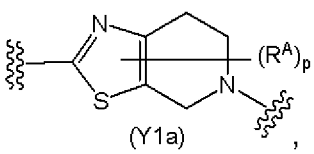
(Y1a) ,
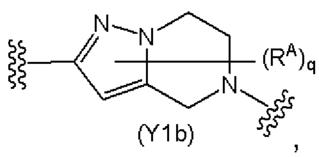
(Y1b) ,
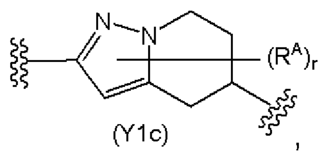
(Y1c) ,

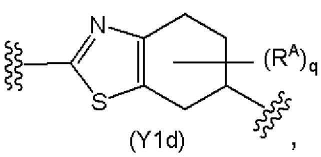
(Y1d) ,

[Formula 4]

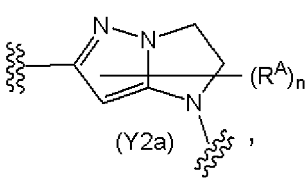
(Y2a) ,
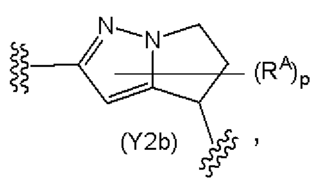
(Y2b) ,
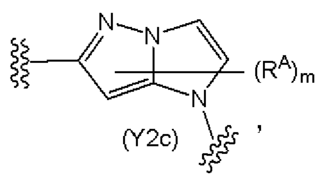
(Y2c) ,

[Formula 5]

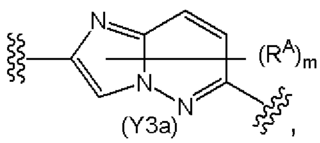
(Y3a),
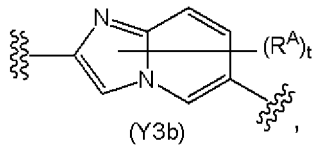
(Y3b),
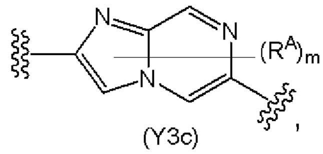
(Y3c),
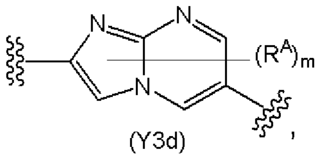
(Y3d),
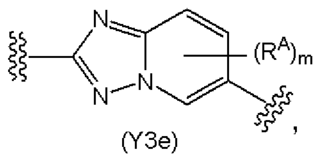
(Y3e),
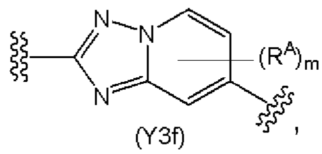
(Y3f),
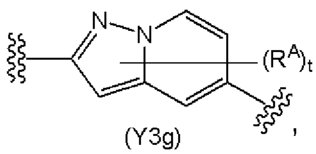
(Y3g),
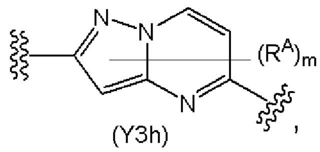
(Y3h),
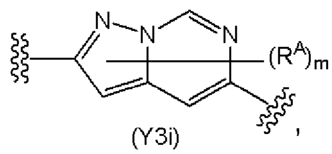
(Y3i),
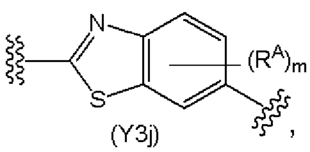
(Y3j),
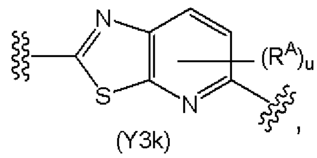
(Y3k),
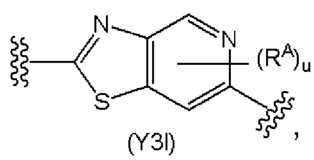
(Y3l),

[Formula 6]

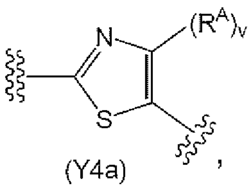
(Y4a),
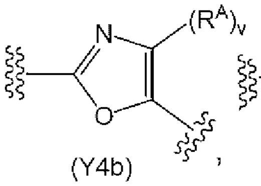
(Y4b),
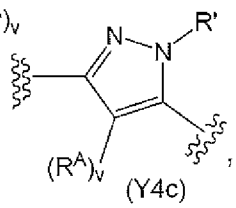
(Y4c),
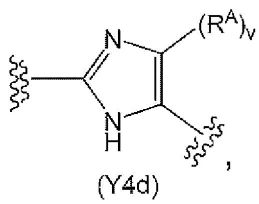
(Y4d),

[Formula 7]

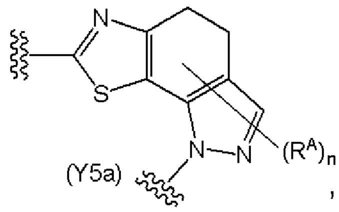
(Y5a) ,

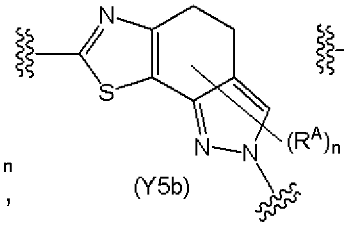
(Y5b) ,

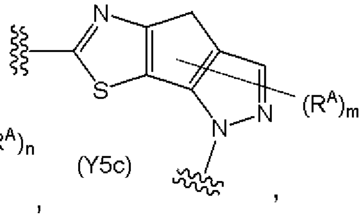
(Y5c) ,

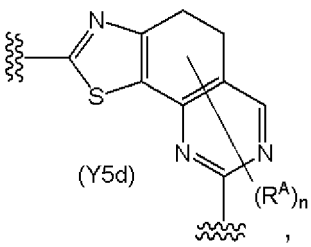
(Y5d) ,

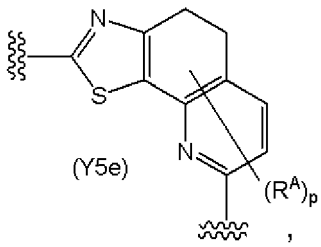
(Y5e) ,

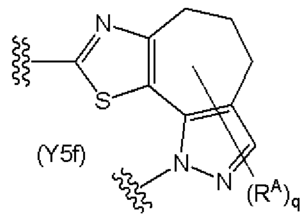
(Y5f) ,

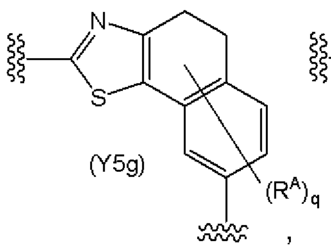
(Y5g) ,

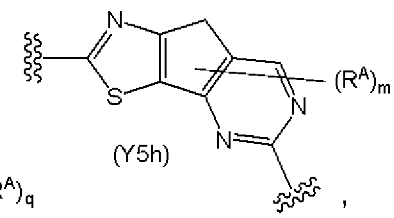
(Y5h) ,

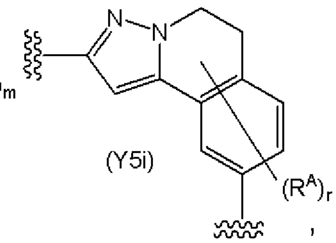
(Y5i) ,

[Formula 8]

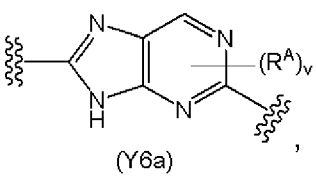
(Y6a) ,

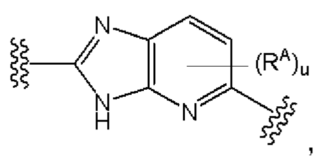
(Y6b) ,

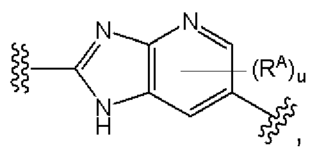
(Y6c) ,

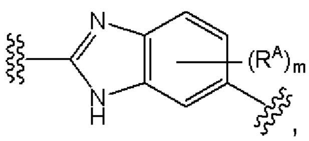
(Y6d) ,

[Formula 9]

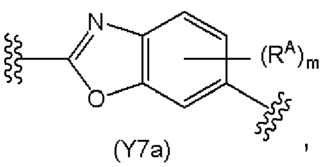
(Y7a) ,

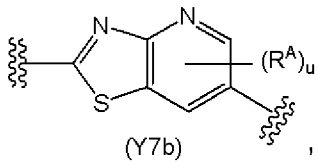
(Y7b) ,

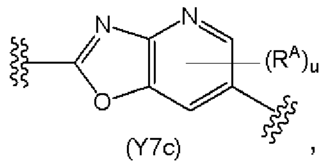
(Y7c) ,

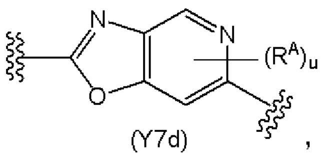
(Y7d) ,

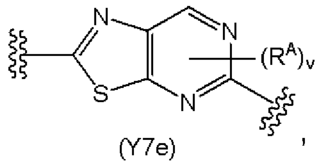
(Y7e) ,

[Formula 10]

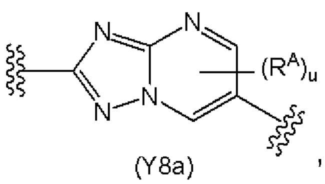
(Y8a) ,

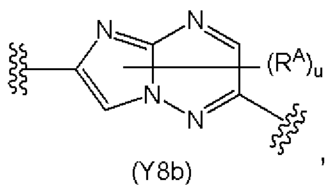
(Y8b) ,

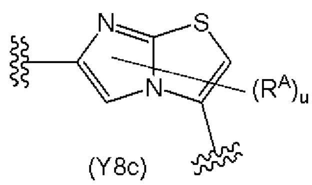
(Y8c)

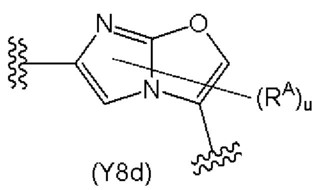
(Y8d) ,

[Formula 11]

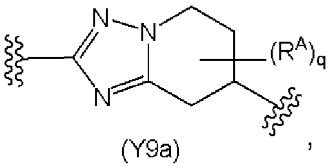
(Y9a) ,
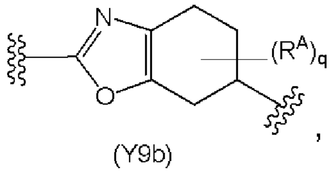
(Y9b) ,
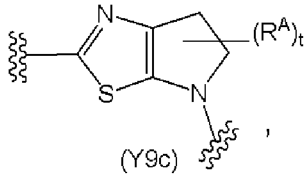
(Y9c) ,
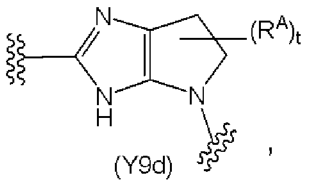
(Y9d) ,
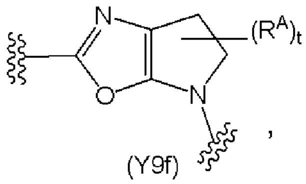
(Y9f) ,
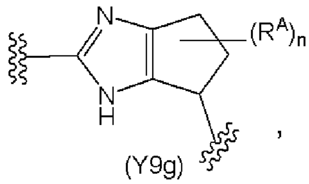
(Y9g) ,
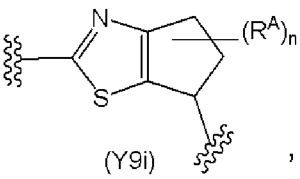
(Y9i) ,
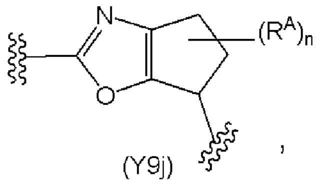
(Y9j) ,
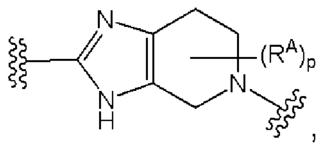
(Y9k) ,
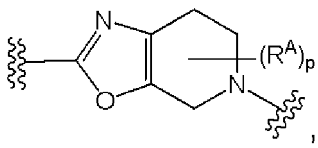
(Y9l) ,
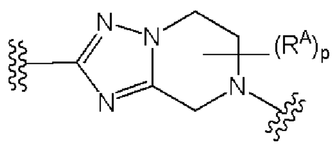
(Y9m) or
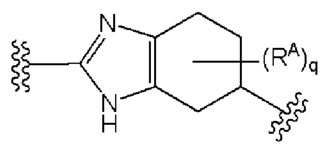
(Y9n) ;

$R^A$ is each independently halogen, cyano, carboxy, hydroxy, nitro, substituted or unsubstituted alkoxy, substituted or unsubstituted alkyl, substituted or unsubstituted alkenyl, substituted or unsubstituted alkynyl, substituted or unsubstituted amino, substituted or unsubstituted carbamoyl, substituted or unsubstituted acyl, substituted or unsubstituted aryl, substituted or unsubstituted cycloalkyl, substituted or unsubstituted cycloalkenyl, substituted or unsubstituted heteroaryl, substituted or unsubstituted heterocyclyl, a group represented by the formula: $-SO-R^{a'}$, a group represented by the forumula: $-SO_2-R^{a'}$, or a group represented by the formula: $-SR^{a'}$;

$R^{a'}$ is hydrogen, substituted or unsubstituted alkyl, substituted or unsubstituted amino, substituted or unsubstituted aryl, substituted or unsubstituted cycloalkyl, substituted or unsubstituted heteroaryl, or substituted or unsubstituted heterocyclyl;

R' is hydrogen or substituted or unsubstituted alkyl;

m is an integer from 0 to 3;

n is an integer from 0 to 5;

p is an integer from 0 to 6;

q is an integer from 0 to 7;

r is an integer from 0 to 8;

t is an integer from 0 to 4;

u is an integer from 0 to 2;

v is 0 or 1; and

Z is substituted or unsubstituted cycloalkyl, substituted or unsubstituted cycloalkenyl, substituted or unsubstituted aryl, substituted or unsubstituted heteroaryl, or substituted or unsubstituted heterocyclyl,

with proviso that:

when Y is a group represented by formula (Y4a) or a group represented by formula (Y4b), $R^A$ is not substituted or unsubstituted aryl;

when Y is a group represented by formula (Y4a), $R^3$ is not methyl or methoxy;
when Y is a group represented by formula (Y5a), $R^3$ is not carboxy, ethoxycarbonyl, phenyl, or alkyl substituted with amino;
when Y is a group represented by formula (Y5c), $R^3$ is not phenyl, or alkyl substituted with substituted amino or imidazolidinone; and
when Y is a group represented by formula (Y5d), $R^3$ is not phenyl, or alkyl substituted with substituted amino, and

wherein the following compound is excluded:

[Formula 12]

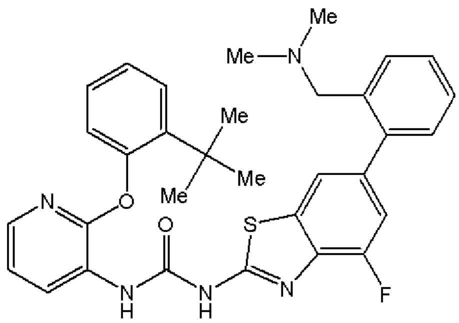

,

or a pharmaceutically acceptable salt thereof, or a solvate thereof.

**2.** The compound according to claim 1, wherein X1' is a substituted or unsubstituted 6-membered aromatic hydrocarbon ring, a substituted or unsubstituted 5- or 6-membered aromatic heterocycle, a substituted or unsubstituted 5- or 6-membered non-aromatic hydrocarbon ring, or a substituted or unsubstituted 5- or 6-membered non-aromatic heterocycle,
or a pharmaceutically acceptable salt thereof, or a solvate thereof.

**3.** The compound according to claim 1 or 2, wherein X is a group represented by formula (X1),
or a pharmaceutically acceptable salt thereof, or a solvate thereof.

**4.** The compound according to any of claims 1 to 3, wherein Y is a group represented by formula (Y1a), a group represented by formula (Y1b), a group represented by formula (Y1c), or a group represented by formula (Y1d),
or a pharmaceutically acceptable salt thereof, or a solvate thereof.

**5.** The compound according to any of claims 1 to 3, wherein Y is a group represented by formula (Y1a) and p is 0 or 1,
or a pharmaceutically acceptable salt thereof, or a solvate thereof.

**6.** The compound according to any of claims 1 to 3, wherein Y is a group represented by formula (Y1d) and q is 0 or 1,
or a pharmaceutically acceptable salt thereof, or a solvate thereof.

**7.** The compound according to any of claims 1 to 3, wherein Y is a group represented by formula (Y2a), a group represented by formula (Y2b), or a group represented by formula (Y2c),
or a pharmaceutically acceptable salt thereof, or a solvate thereof.

**8.** The compound according to any of claims 1 to 3, wherein Y is a group represented by formula (Y2a) and n is 0,
or a pharmaceutically acceptable salt thereof, or a solvate thereof.

**9.** The compound according to any of claims 1 to 3, wherein Y is a group represented by formula (Y3a), a group represented by formula (Y3b), a group represented by formula (Y3c), a group represented by formula (Y3d), a group represented by formula (Y3e), a group represented by formula (Y3f), a group represented by formula (Y3g), a group represented by formula (Y3h), a group represented by formula (Y3i), a group represented by formula (Y3j), a group represented by formula (Y3k), or a group represented by formula (Y31),
or a pharmaceutically acceptable salt thereof, or a solvate thereof.

10. The compound according to any of claims 1 to 3, wherein Y is a group represented by formula (Y3a), a group represented by formula (Y3e), or a group represented by formula (Y3j), and m is 0,
or a pharmaceutically acceptable salt thereof, or a solvate thereof.

11. The compound according to any of claims 1 to 3, wherein Y is a group represented by formula (Y3k) and u is 0, or a pharmaceutically acceptable salt thereof, or a solvate thereof.

12. The compound according to any of claims 1 to 3, wherein Y is a group represented by formula (Y4a), a group represented by formula (Y4b), a group represented by formula (Y4c), or a group represented by formula (Y4d),
or a pharmaceutically acceptable salt thereof, or a solvate thereof.

13. The compound according to any of claims 1 to 3, wherein Y is a group represented by formula (Y4a) or a group represented by formula (Y4c), v is 0 or 1, and R' is hydrogen or substituted or unsubstituted alkyl,
or a pharmaceutically acceptable salt thereof, or a solvate thereof.

14. The compound according to any of claims 1 to 3, wherein Y is a group represented by formula (Y5a), a group represented by formula (Y5b), a group represented by formula (Y5c), a group represented by formula (Y5d), a group represented by formula (Y5e), a group represented by formula (Y5f), a group represented by formula (Y5g), a group represented by formula (Y5h), or a group represented by formula (Y5i),
or a pharmaceutically acceptable salt thereof, or a solvate thereof.

15. The compound according to any of claims 1 to 3, wherein Y is a group represented by formula (Y5a) and n is 0 or 1, or a pharmaceutically acceptable salt thereof, or a solvate thereof.

16. The compound according to any of claims 1 to 3, wherein Y is a group represented by formula (Y6a), a group represented by formula (Y6b), a group represented by formula (Y6c), or a group represented by formula (Y6d),
or a pharmaceutically acceptable salt thereof, or a solvate thereof.

17. The compound according to any of claims 1 to 3, wherein Y is a group represented by formula (Y7a), a group represented by formula (Y7b), a group represented by formula (Y7c), a group represented by formula (Y7d), or a group represented by formula (Y7e),
or a pharmaceutically acceptable salt thereof, or a solvate thereof.

18. The compound according to any of claims 1 to 3, wherein Y is a group represented by formula (Y8a), a group represented by formula (Y8b), a group represented by formula (Y8c), or a group represented by formula (Y8d),
or a pharmaceutically acceptable salt thereof, or a solvate thereof.

19. The compound according to any of claims 1 to 3, wherein Y is a group represented by formula (Y9a), a group represented by formula (Y9b), a group represented by formula (Y9c), a group represented by formula (Y9d), a group represented by formula (Y9f), a group represented by formula (Y9g), a group represented by formula (Y9i), a group represented by formula (Y9j), a group represented by formula (Y9k), a group represented by formula (Y91), a group represented by formula (Y9m), or a group represented by formula (Y9n),
or a pharmaceutically acceptable salt thereof, or a solvate thereof.

20. The compound according to any of claims 1 to 19, wherein Z is substituted or unsubstituted aryl, substituted or unsubstituted heteroaryl, or substituted or unsubstituted heterocyclyl,
or a pharmaceutically acceptable salt thereof, or a solvate thereof.

21. The compound according to any of claims 1 to 20, wherein $R^3$ is carboxy, substituted or unsubstituted alkyl, substituted or unsubstituted aryl, substituted or unsubstituted heteroaryl, substituted or unsubstituted heterocyclyl, substituted or unsubstituted acyl, substituted or unsubstituted alkoxycarbonyl, substituted or unsubstituted carbamoyl, substituted or unsubstituted alkoxy, substituted or unsubstituted amino, substituted or unsubstituted alkylsulfonyl, substituted or unsubstituted sulfamoyl, or a group represented by the formula: $-C(-R^a)=N-OH$, and $R^a$ is hydrogen or substituted or unsubstituted alkyl,
or a pharmaceutically acceptable salt thereof, or a solvate thereof.

22. The compound according to claim 21, wherein $R^3$ is substituted or unsubstituted carbamoyl, substituted or unsubstituted amino, or substituted or unsubstituted alkoxy,

or a pharmaceutically acceptable salt thereof, or a solvate thereof.

**23.** The compound according to any of claims 1 to 22, wherein $R^1$ and $R^2$ are both hydrogen,
or a pharmaceutically acceptable salt thereof, or a solvate thereof.

**24.** A pharmaceutical composition containing the compound according to any of claims 1 to 23, or a pharmaceutically acceptable salt thereof, or a solvate thereof.

**25.** The pharmaceutical composition according to claim 24, which is a phosphatidylinositol-3-kinase inhibitor.

**26.** A method for the therapy and/or prophylaxis of inflammation, **characterized by** administering the compound according to any of claims 1 to 23, or a pharmaceutically acceptable salt thereof, or a solvate thereof.

**27.** The compound according to any of claims 1 to 23, or a pharmaceutically acceptable salt thereof, or a solvate thereof for the therapy and/or prophylaxis of inflammation.

## INTERNATIONAL SEARCH REPORT

International application No.
PCT/JP2010/002998

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>A | JP 2005-522458 A (Novartis AG.),<br>28 July 2005 (28.07.2005),<br>entire text; particularly, claim 1; paragraph [0075]; examples 101, 120, 125 to 131, 133, 135, 136, 144, 145, 163<br>& WO 2003/072557 A1 & TW 314928 B<br>& CA 2477601 A1 & AU 2003214080 A1<br>& EP 1480962 A1 & BR 2003008030 A<br>& CN 1639139 A & CN 100548996 C<br>& JP 4408701 B2 & NZ 534657 A<br>& RU 2378263 C2 & IN 227180 A1<br>& MX 2004008362 A & NO 2004004095 A<br>& US 2005/0119320 A1 | 1-3,12,13,<br>20-25,27<br>4-11,17-19 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 July, 2010 (22.07.10) | 03 August, 2010 (03.08.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No.
PCT/JP2010/002998

C (Continuation). DOCUMENTS CONSIDERED TO BE RELEVANT

| Category* | Citation of document, with indication, where appropriate, of the relevant passages | Relevant to claim No. |
|---|---|---|
| X<br><br>A | JP 2008-519021 A (ASTRAZENECA AB.),<br>05 June 2008 (05.06.2008),<br>entire text; particularly, claim 1; table 2, no.6; table 5<br>& WO 2006/051270 A1 & AU 2005303566 A1<br>& CA 2585310 A1 & EP 1841763 A1<br>& EP 1841763 B1 & CN 101098869 A<br>& BR 2005017634 A & NO 2007002259 A<br>& US 2008/0132502 A1 & MX 2007005546 A<br>& ZA 2007003714 A & KR 2007085837 A | 1-3,12,13,<br>20-25,27<br>4-11,17-19 |
| X<br><br>A | JP 2006-525266 A (Novartis AG.),<br>09 November 2006 (09.11.2006),<br>entire text; particularly, claim 1; paragraphs [0009], [0023], [0086]; examples 116, 137<br>& WO 2004/096797 A1 & AU 2004234068 A1<br>& AU 2004234068 B2 & CA 2524401 A1<br>& EP 1622897 A1 & EP 1622897 B1<br>& BR 2004010037 A & CN 1816549 A<br>& AT 445614 T & PT 1622897 E<br>& ES 2331883 T3 & CN 101648949 A<br>& EP 2157091 A1 & RU 2384580 C2<br>& MX 2005011740 A | 1-3,12,13,<br>20-25,27<br>4-11,17-19 |
| X<br><br>A | WO 2008/042867 A2 (EMILIEM INC., USA),<br>10 April 2008 (10.04.2008),<br>entire text; particularly, claims 4, 13; table 6b; page 82<br>& WO 2008/042867 A3 | 1-3,12,13,<br>20-25,27<br>4-11,17-19 |
| X<br><br>A | WO 2008/023159 A1 (ASTRAZENECA AB., SWED.),<br>28 February 2008 (28.02.2008),<br>entire text; particularly, claim 1; page 105; examples 46c to 46f, 46h, 46i, 47a, 47b<br>& CA 2660758 A1 & AU 2007287428 A1<br>& EP 2057140 A1 & JP 2010-501534 A<br>& US 2008/0171743 A1 & US 7750003 B2<br>& NO 2009000631 A & MX 2009002046 A<br>& KR 2009053928 A & CN 101541781 A | 1-3,12,13,<br>20-25,27<br>4-11,17-19 |
| X<br><br>A | JP 2005-538152 A (Neurosearch A/S),<br>15 December 2005 (15.12.2005),<br>entire text; particularly, claim 1; paragraph [0138]; page 50, example 18, compound '4-chloro-2-[3-(5-phenyl-2H-pyrazole-3-yl)-ureido]-benzoic acid'<br>& WO 2004/022529 A2 & WO 2004/022529 A3<br>& CA 2495284 A1 & AU 2003258490 A1<br>& EP 1537075 A2 & EP 1537075 B1<br>& CN 1678573 A & CN 100497302 C<br>& NZ 538513 A & AT 435199 T<br>& MX 2005002493 A & US 2006/0160856 A1 | 1-3,12,13,<br>20-25,27<br>4-11,17-19 |

Form PCT/ISA/210 (continuation of second sheet) (July 2009)

**INTERNATIONAL SEARCH REPORT**

International application No.
PCT/JP2010/002998

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 2010/024258 A1 (Shionogi & Co., Ltd.), 04 March 2010 (04.03.2010), entire text; particularly, claim 1; paragraph [0153]; compounds no. I-134, I-203, I-213, I-289, I-290, (I-294)-(I-297), I-302, I-303, I-321, (I-366)-(I-369), I-412, I-416, I-417, (I-420)-(I-422), I-425, I-426, I-430, I-441, I-442, I-460, (I-483)-(I-488), (I-507)-(I-515), (I-540)-(I-545), I-550; example 8 (Family: none) | 1-5,7,8, 20-25,27 |
| P,X | WO 2009/128520 A1 (Shionogi & Co., Ltd.), 22 October 2009 (22.10.2009), entire text; particularly, claim 1; paragraphs [0124] to [0125]; compounds no. I-108, I-111, I-115, I-118, I-232, I-233, I-242, I-243, I-251, I-252, I-271, I-277; example 21 (Family: none) | 1-3,9,10, 20-25,27 |
| P,X | WO 2010/024903 A1 (FENG, Yangbo), 04 March 2010 (04.03.2010), entire text; particularly, claims 1, 46 (Family: none) | 1-3,9,20-25, 27 |

Form PCT/ISA/210 (continuation of second sheet) (July 2009)

INTERNATIONAL SEARCH REPORT

International application No.
PCT/JP2010/002998

Continuation of A. CLASSIFICATION OF SUBJECT MATTER
(International Patent Classification (IPC))

*C07D513/04*(2006.01)i, *A61K31/415*(2006.01)i, *A61K31/421*(2006.01)i, *A61K31/437*(2006.01)i, *A61K31/444*(2006.01)i, *A61K31/4725*(2006.01)i, *A61K31/5025*(2006.01)i, *A61K31/506*(2006.01)i, *A61K31/5377*(2006.01)i, *A61P1/02*(2006.01)i, *A61P1/04*(2006.01)i, *A61P1/16*(2006.01)i, *A61P1/18*(2006.01)i, *A61P3/04*(2006.01)i, *A61P3/10*(2006.01)i, *A61P5/14*(2006.01)i, *A61P7/06*(2006.01)i, *A61P9/00*(2006.01)i, *A61P9/04*(2006.01)i, *A61P9/10*(2006.01)i, *A61P9/12*(2006.01)i, *A61P11/00*(2006.01)i, *A61P11/06*(2006.01)i, *A61P13/00*(2006.01)i, *A61P13/10*(2006.01)i, *A61P13/12*(2006.01)i, *A61P15/02*(2006.01)i, *A61P17/02*(2006.01)i, *A61P17/06*(2006.01)i, *A61P17/14*(2006.01)i, *A61P19/02*(2006.01)i, *A61P19/08*(2006.01)i, *A61P21/00*(2006.01)i, *A61P21/04*(2006.01)i, *A61P25/00*(2006.01)i, *A61P25/02*(2006.01)i, *A61P27/02*(2006.01)i, *A61P27/06*(2006.01)i, *A61P27/14*(2006.01)i, *A61P27/16*(2006.01)i, *A61P29/00*(2006.01)i, *A61P31/00*(2006.01)i, *A61P31/04*(2006.01)i, *A61P35/00*(2006.01)i, *A61P37/02*(2006.01)i, *A61P37/06*(2006.01)i, *A61P37/08*(2006.01)i, *A61P43/00*(2006.01)i, *C07D231/40*(2006.01)i, *C07D277/18*(2006.01)i, *C07D277/80*(2006.01)i, *C07D401/04*(2006.01)i, *C07D417/04*(2006.01)i, *C07D417/14*(2006.01)i, *C07D471/04*(2006.01)i, *C07D487/04*(2006.01)i

(According to International Patent Classification (IPC) or to both national classification and IPC)

Continuation of B. FIELDS SEARCHED
Minimum documentation searched (International Patent Classification (IPC))

C07D513/04, A61K31/415, A61K31/421, A61K31/437, A61K31/444, A61K31/4725, A61K31/5025, A61K31/506, A61K31/5377, A61P1/02, A61P1/04, A61P1/16, A61P1/18, A61P3/04, A61P3/10, A61P5/14, A61P7/06, A61P9/00, A61P9/04, A61P9/10, A61P9/12, A61P11/00, A61P11/06, A61P13/00, A61P13/10, A61P13/12, A61P15/02, A61P17/02, A61P17/06, A61P17/14, A61P19/02, A61P19/08, A61P21/00, A61P21/04, A61P25/00, A61P25/02, A61P27/02, A61P27/06, A61P27/14, A61P27/16, A61P29/00, A61P31/00, A61P31/04, A61P35/00, A61P37/02, A61P37/06, A61P37/08, A61P43/00, C07D231/40, C07D277/18, C07D277/80, C07D401/04, C07D417/04, C07D417/14, C07D471/04, C07D487/04

Minimum documentation searched (classification system followed by classification symbols)

Form PCT/ISA/210 (extra sheet) (July 2009)

**INTERNATIONAL SEARCH REPORT**

International application No.
PCT/JP2010/002998

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 26
because they relate to subject matter not required to be searched by this Authority, namely:
Claim 26 pertains to a method for treatment of the human body by therapy and thus relates to a subject matter on which the International Searching Authority is not required to carry out a search under the provision of PCT Rule 39.1(iv).

2. ☒ Claims Nos.: 14 - 16
because they relate to parts of the international application that do not comply with the prescribed requirements to such an extent that no meaningful international search can be carried out, specifically:
The inventions in claims 14 - 16 are not concretely set forth in the description, are not disclosed in the meaning of PCT Article 5 and lack the support in the meaning of PCT Article 6, and therefore are decided as a subject on which this International Searching Authority is not required to carry out a search.

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.
PCT/JP2010/002998

Subject to be searched:

Although the invention in claim 1 of the present application involves a plurality of compounds, only the compound having a specific Y-group represented by formula (I) that is concretely set forth in the description is disclosed in the meaning of PCT Article 5, and therefore, the invention in claim 1 lacks the support in the meaning of PCT Article 6.

Therefore, in this international search report, the search has been carried out with respect to the compound having the above-said specific Y-group among the compounds set forth in claim 1 of the present application.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7145051 A
- JP 7029673 A
- JP 2001098269 A
- JP 2001098270 A
- JP 2000035451 A
- JP 2000035452 A
- JP 2000035453 A
- JP 2000035454 A
- JP 2000035449 A
- JP 2002100433 A
- JP 2002051442 A
- WO 2001010865 A
- WO 2003072557 A
- WO 2005113511 A
- WO 2007115930 A
- WO 2007115932 A
- WO 2008039520 A
- JP 2001281820 A
- JP 6003784 A
- JP 5333503 A
- EP 578248 A
- EP 569979 A
- EP 572029 A
- WO 2007115931 A
- US 20070238730 A
- WO 2009128520 A
- WO 2010024258 A
- WO 2007095588 A
- WO 2009010530 A
- WO 2007129052 A
- WO 2008116129 A
- WO 2008118468 A
- WO 2007135398 A
- WO 2009017822 A
- WO 2008152390 A
- WO 2009021990 A
- WO 2009021992 A
- US 20060063799 A
- JP 2009108526 A
- JP 2009235769 A
- JP 2010043072 A


### Non-patent literature cited in the description


- **M. P. Wymann et al.** *Biochemical Society Transactions,* 2003, vol. 31, 275-280
- **Rueckle T. et al.** *NATURE REVIEWS DRUG DISCOVERY,* 2006, vol. 5, 903-918
- **Laffargue M. et al.** *Immunity,* 2002, vol. 16, 441-451
- **Hirsch E. et al.** *Science,* 2000, vol. 287, 1049-1053
- **Li Z. et al.** *Science,* 2000, vol. 287, 982-983
- **Sasaki T. et al.** *Science,* 2000, vol. 287, 1040-1046
- **Lupia E. et al.** *Am J Pathol.,* 2004, vol. 165, 2003-2011
- **Barber DF et al.** *Nat Med,* 2005, vol. 11, 933-935
- **Camps.** *Nat Med,* 2005, vol. 11, 936-943
- **Campbell et al.** *Circ Res.,* 2005, vol. 96, 197-206
- **Yadav M. et al.** *J Immunol.,* 2006, vol. 176, 5494-503
- *Japanese Journal of Clinical Immunology,* 2007, vol. 30, 369-374
- **Ui MT et al.** *Trends Biochem. Sci.,* 1995, vol. 20, 303-307
- **Shao L. et al.** *Heteroatom Chemistry,* 2008, vol. 19 (1), 2-6
- **Vovk, M. V. et al.** *Zhurnal Organichnoi ta Farmatsevtichnoi Khimii,* 2006, vol. 4 (4), 63-66
- **Salvatore G. et al.** *Scientia Pharmaceutica,* 2000, vol. 68 (1), 65-73
- **Romeo G. et al.** *Pharmazie,* 1999, vol. 54 (1), 19-23
- Isotopes in the Physical and Biomedical Sciences. 1987, vol. 1
- **Theodora W. Greene.** Protective Groups in Organic Synthesis. John Wiley & Sons, Inc, 1991
- **Theodora W. Greene.** Protective Groups in Organic Synthesis. John Wiley & Sons, 1991
- *Journal of the Chemical Society, Perkin Transactions 1: Organic and Bio-Organic Chemistry,* 1972, 9-12
- *Bioorganic & Medicinal Chemistry Letters,* 2005, vol. 15 (22), 4910-4914
- *Journal of Medicinal Chemistry,* 1987, vol. 30 (3), 494-8
- *Journal of Medicinal Chemistry,* 2008, vol. 51 (10), 3005-3019

- *Journal of Combinatorial Chemistry,* 2008, vol. 10 (1), 118-122
- *Journal of the Chemical Society, Perkin Transactions,* 1986, vol. 1 (1), 9-12
- **T. W. Green.** Protective Groups in Organic Synthesis. John Wiley & Sons Inc, 1991
- **T. W. Greene.** Protective Groups in Organic Synthesis. John Wiley & Sons Inc, 1991
- **R. C. Larock.** *Comprehensive Organic Transformations,* 1989
- *Journal of the Chemical Society, Perkin Transaction,* 2000, vol. 1, 999-1001
- *Heterocycles,* 2004, vol. 63 (7), 1555-1561